# EUROPEAN PATENT APPLICATION

(11) **EP 3 910 059 A1**
(43) Date of publication of application: **17.11.2021**
(21) Application number: 21170389.7
(22) Date of filing: 26.05.2017
(51) Int. Cl.: C12N 15/11, C07K 7/08, C12N 15/63, C12N 9/22, B82Y 5/00, A61K 39/00

(54) **PEPTIDES AND NANOPARTICLES FOR INTRACELLULAR DELIVERY OF GENOME-EDITING MOLECULES**

(30) Priority: 27.05.2016 US 201662342823 P; 13.09.2016 US 201662394140 P; 27.03.2017 US 201762477357 P
(62) Divisional of application: 17733918.1
(71) Applicant: AADIGEN, LLC, Pacific Palisades, CA 90272-9992 (US)
(72) Inventor: DIVITA, Gilles, 34725 Saint-Andre-De-Sangonis (FR); DESAI, Neil, Pacific Palisades, CA 90272 (US)
(74) Representative: J A Kemp LLP

(57) **Abstract**

The present invention pertains to peptide-containing complexes/nanoparticles that are useful for stabilizing and/or delivering one or more molecules of a genome-editing system, such as proteins and/or nucleic acids, for example CRISPR proteins and/or nucleic acids.

## Description

### CROSS-REFERENCE TO RELATED APPLICATIONS

This application claims priority to U.S. Provisional Application No. 62/342,823, filed on May 27, 2016, U.S. Provisional Application No. 62/394,140, filed on September 13, 2016, and U.S. Provisional Application No. 62/477,357, filed on March 27, 2017, all of which are hereby incorporated by reference in their entireties.

### FIELD OF THE INVENTION

The present invention pertains to peptide-containing complexes/nanoparticles that are useful for stabilizing and/or delivering into a cell one or more molecules of a genome-editing system, such as CRISPR-associated proteins and nucleic acids.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: 737372000840SeqList.txt, date recorded: May 26, 2017, size: 48 KB).

### BACKGROUND

The CRISPR interference technique has enormous potential application, including altering the germline of humans, animals, and other organisms, and modifying the genes of food crops. By delivering a CRISPR-associated nuclease, such as Cas9 or Cpfl, and appropriate guide RNAs into a cell, the organism's genome can be cut at almost any desired location (Ledford, H. (2015). Nature. 522(7554); Zetsche et al., (2015). Cell. 163(3):759-771). CRISPRs have been used in concert with specific endonuclease enzymes for genome editing and gene regulation in many different species (Mali et al., (2013). Nature Methods. 10(10):957-63). CRISPR tools have been used for various genome modifications, including double-strand break formation with non-homologous end joining (NHEJ)-mediated mutagenesis (Wang et al., (2013). Cell. 153:910-918), nick formation with NHEJ-mediated mutagenesis (Cheng et al. (2014). FEBS Lett. 588:3954-3958), knock-in with homology-dependent repair (HDR) (Inui et al. (2014). Sci. Rep. 4:5396), and genome-rearrangement (Maddalo et al. (2014) Nature. 516:423-427), and are suitable for use in methods of repressing or activating gene expression. Nuclease-deficient Cas nucleases can be used to block the binding of transcriptional activators in order to block expression of specific genes, to deliver transcriptional activators to stimulate expression of specific genes, or to deliver epigenetic modifiers to target sequences to alter histone methylation or acetylation (Ledford, H. (2016). Nature. 531:156-159).

The disclosures of all publications, patents, patent applications and published patent applications referred to herein are hereby incorporated herein by reference in their entirety.

### BRIEF SUMMARY OF THE INVENTION

The present application provides complexes and nanoparticles comprising cell-penetrating peptide that are useful for stabilizing and/or delivering into a cell one or more molecules of a genome-editing system, such as CRISPR-associated proteins and nucleic acids.

In some embodiments, there is provided a genome-editing complex for modifying a target polynucleotide comprising a cell-penetrating peptide associated with an RGEN/gRNA complex comprising an RNA-guided endonuclease (RGEN) and a guide RNA (gRNA), wherein the gRNA comprises a guide sequence complementary to a target sequence in the target polynucleotide.

In some embodiments, there is provided a genome-editing complex for modifying a target polynucleotide comprising a cell-penetrating peptide and one or more molecules of a genome-editing system targeting the target polynucleotide, wherein the cell-penetrating peptide is selected from the group consisting of VEPEP-3 peptides, VEPEP-6 peptides, VEPEP-9 peptides, and ADGN-100 peptides. In some embodiments, the one or more genome-editing system molecules are selected from the group consisting of: a) an RNA-guided endonuclease (RGEN) and a guide RNA (gRNA), wherein the gRNA comprises a guide sequence complementary to a target sequence in the target polynucleotide; b) a DNA-guided endonuclease (DGEN) and a guide DNA (gDNA), wherein the gDNA comprises a guide sequence complementary to a target sequence in the target polynucleotide; c) a zinc finger protein (ZFP), wherein the ZFP recognizes a target sequence in the target polynucleotide; d) a transcription activator-like effector nuclease (TALEN), wherein the TALEN recognizes a target sequence in the target polynucleotide; e) a homing endonuclease, wherein the homing endonuclease recognizes a target sequence in the target polynucleotide; and f) an integrase, wherein the integrase recognizes a recombination site in the target polynucleotide.

In some embodiments, according to any of the genome-editing complexes described above, the cell-penetrating peptide is a VEPEP-3 peptide. In some embodiments, the cell-penetrating peptide comprises an amino acid sequence selected from the group consisting of SEQ ID NOs: 1-14. In some embodiments, the cell-penetrating peptide comprises the amino acid sequence of SEQ ID NO: 75 or 76.

In some embodiments, the cell-penetrating peptide is a VEPEP-6 peptide. In some embodiments, the cell-penetrating peptide comprises an amino acid sequence selected from the group consisting of SEQ ID NOs: 15-40. In some embodiments, the cell-penetrating peptide comprises the amino acid sequence of SEQ ID NO: 77.

In some embodiments, according to any of the genome-editing complexes described above, the cell-penetrating peptide is a VEPEP-9 peptide. In some embodiments, the cell-penetrating peptide comprises an amino acid sequence selected from the group consisting of SEQ ID NOs: 41-52. In some embodiments, the cell-penetrating peptide comprises the amino acid sequence of SEQ ID NO: 78.

In some embodiments, according to any of the genome-editing complexes described above, the cell-penetrating peptide is an ADGN-100 peptide. In some embodiments, the cell-penetrating peptide comprises an amino acid sequence selected from the group consisting of SEQ ID NOs: 53-70. In some embodiments, the cell-penetrating peptide comprises the amino acid sequence of SEQ ID NO: 79 or 80.

In some embodiments, according to any of the genome-editing complexes described above, the cell-penetrating peptide further comprises one or more moieties covalently linked to the N-terminus of the cell-penetrating peptide, and wherein the one or more moieties are selected from the group consisting of an acetyl, a fatty acid, a cholesterol, a poly-ethylene glycol, a nuclear localization signal, nuclear export signal, an antibody, a polysaccharide and a targeting molecule. In some embodiments, the cell-penetrating peptide comprises an acetyl group covalently linked to its N-terminus. In some embodiments, the cell-penetrating peptide further comprises one or more moieties covalently linked to the C-terminus of the cell-penetrating peptide, and wherein the one or more moieties are selected from the group consisting of a cysteamide, a cysteine, a thiol, an amide, a nitrilotriacetic acid optionally substituted, a carboxyl, a linear or ramified C₁-C₆ alkyl optionally substituted, a primary or secondary amine, an osidic derivative, a lipid, a phospholipid, a fatty acid, a cholesterol, a poly-ethylene glycol, a nuclear localization signal, nuclear export signal, an antibody, a polysaccharide and a targeting molecule. In some embodiments, the cell-penetrating peptide comprises a cysteamide group covalently linked to its C-terminus.

In some embodiments, according to any of the genome-editing complexes described above, at least some of the cell-penetrating peptides in the genome-editing complex are linked to a targeting moiety by a linkage. In some embodiments, the linkage is covalent.

In some embodiments, according to any of the genome-editing complexes described above comprising a gRNA, the gRNA is a single-guide RNA (sgRNA). In some embodiments, the sgRNA comprises a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence.

In some embodiments, according to any of the genome-editing complexes described above comprising an RGEN, the RGEN is Cas9.

In some embodiments, according to any of the genome-editing complexes described above comprising an RGEN and a gRNA, the molar ratio of RGEN to gRNA is between about 1:10 and about 10:1.

In some embodiments, according to any of the genome-editing complexes described above comprising an RGEN, the molar ratio of the cell-penetrating peptide to the RGEN is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of the cell-penetrating peptide to the RGEN is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios).

In some embodiments, according to any of the genome-editing complexes described above comprising a gRNA, the genome-editing complex further comprises one or more additional gRNAs comprising different guide sequences. In some embodiments, the target sequence is present in a target gene, and the one or more additional gRNAs individually comprise a guide sequence complementary to a sequence in the target gene. In some embodiments, the target sequence is present in a first target gene, and the one or more additional gRNAs individually comprise a guide sequence complementary to a sequence present in one or more additional target genes.

In some embodiments, according to any of the genome-editing complexes described above, the one or more genome-editing system molecules comprise a ZFP. In some embodiments, according to any of the genome-editing complexes described above, the one or more genome-editing system molecules comprise a TALEN. In some embodiments, according to any of the genome-editing complexes described above, the one or more genome-editing system molecules comprise a homing endonuclease.

In some embodiments, according to any of the genome-editing complexes described above, the genome-editing complex further comprises a donor nucleic acid for introducing a modification to the target polynucleotide comprising a sequence corresponding to a portion of the target polynucleotide modified to comprise the modification. In some embodiments, the modification is addition, deletion, or substitution of one or more nucleotides in the target polynucleotide. In some embodiments, wherein the donor nucleic acid is a single-stranded DNA oligonucleotide. In some embodiments, the modification is insertion of a heterologous nucleic acid in the target polynucleotide. In some embodiments, the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified. In some embodiments, the genome-editing complex further comprises one or more additional donor nucleic acids for introducing modifications to one or more additional target polynucleotides. In some embodiments, each of the one or more additional donor nucleic acids comprises a sequence corresponding to a portion of one of the one or more additional target polynucleotides modified to comprise the modification.

In some embodiments, according to any of the genome-editing complexes described above, the one or more genome-editing system molecules comprise an integrase. In some embodiments, the genome-editing complex further comprises a donor nucleic acid for insertion into the target polynucleotide, wherein the integrase recognizes a recombination site in the donor nucleic acid and is capable of mediating recombination between the recombination site in the target polynucleotide and the recombination site in the donor nucleic acid to insert the donor nucleic acid into the target polynucleotide.

In some embodiments, according to any of the genome-editing complexes described above, the average diameter of the genome-editing complex is between about 10 nm and about 300 nm.

In some embodiments, there is provided a nanoparticle comprising a core comprising a genome-editing complex according to any of the embodiments described above. In some embodiments, the core further comprises one or more additional genome-editing complexes according to any of the embodiments described above. In some embodiments, the core further comprises a donor nucleic acid for introducing a modification to the target polynucleotide comprising a sequence corresponding to a portion of the target polynucleotide modified to comprise the modification, wherein the donor nucleic acid is complexed with a second cell-penetrating peptide. In some embodiments, the second cell-penetrating peptide is selected from the group consisting of VEPEP-3 peptides, VEPEP-6 peptides, VEPEP-9 peptides, and ADGN-100 peptides. In some embodiments, the second cell-penetrating peptide comprises an amino acid sequence selected from the group consisting of SEQ ID NOs: 1-70 and 75-80. In some embodiments, the modification is addition, deletion, or substitution of one or more nucleotides in the target polynucleotide. In some embodiments, the donor nucleic acid is a single-stranded DNA oligonucleotide. In some embodiments, the modification is insertion of a heterologous nucleic acid in the target polynucleotide. In some embodiments, the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified. In some embodiments, the core further comprises one or more additional donor nucleic acids for introducing modifications to one or more additional target polynucleotides, wherein each of the one or more additional donor nucleic acids comprises a sequence corresponding to a portion of one of the one or more additional target polynucleotides modified to comprise the modification, and wherein each of the one or more additional donor nucleic acids is complexed with a cell-penetrating peptide. In some embodiments, at least some of the cell-penetrating peptides in the nanoparticle are linked to a targeting moiety by a linkage. In some embodiments, the core is coated by a shell comprising peripheral cell-penetrating peptides. In some embodiments, at least some of the peripheral cell-penetrating peptides in the shell are linked to a targeting moiety by a linkage. In some embodiments, the linkage is covalent. In some embodiments, the peripheral cell-penetrating peptide is selected from the group consisting of VEPEP-3 peptides, VEPEP-6 peptides, VEPEP-9 peptides, and ADGN-100 peptides. In some embodiments, the peripheral cell-penetrating peptide comprises an amino acid sequence selected from the group consisting of SEQ ID NOs: 1-70 and 75-80. In some embodiments, the average diameter of the nanoparticle is between about 10 nm and about 400 nm. In some embodiments, the average diameter of the nanoparticle is between about 50 nm and about 300 nm. In some embodiments, the average diameter of the nanoparticle is between about 80 nm and about 200 nm

In some embodiments, there is provided a pharmaceutical composition comprising a genome-editing complex according to any of the embodiments described above or a nanoparticle according to any of the embodiments described above, and a pharmaceutically acceptable carrier. In some embodiments, the pharmaceutical composition is formulated for intravenous, intratumoral, intraarterial, topical, intraocular, ophthalmic, intraportal, intracranial, intracerebral, intracerebroventricular, intrathecal, intravesicular, intradermal, subcutaneous, intramuscular, intranasal, intratracheal, pulmonary, intracavity, or oral administration. In some embodiments, the pharmaceutical composition is lyophilized. In some embodiments, the pharmaceutical composition further comprises an expression complex comprising a third cell-penetrating peptide and a nucleic acid molecule encoding an exogenous protein. In some embodiments, the exogenous protein is a recombinant receptor capable of being expressed on the surface of a cell. In some embodiments, the recombinant receptor is a chimeric antigen receptor (CAR). In some embodiments, the third cell-penetrating peptide is selected from the group consisting of VEPEP-3 peptides, VEPEP-6 peptides, VEPEP-9 peptides, and ADGN-100 peptides. In some embodiments, the third cell-penetrating peptide comprises an amino acid sequence selected from the group consisting of SEQ ID NOs: 1-70 and 75-80.

In some embodiments, there is provided a method of preparing a genome-editing complex according to any of the embodiments described above, comprising combining the cell-penetrating peptide with the RGEN/gRNA complex, thereby forming the genome-editing complex. In some embodiments, the cell-penetrating peptide is combined with the RGEN/gRNA complex at a ratio from about 1:1 to about 80:1. In some embodiments, the cell-penetrating peptide is combined with the RGEN/gRNA complex at a ratio from about 5:1 to about 20:1.

In some embodiments, there is provided a method of preparing a genome-editing complex according to any of the embodiments described above, comprising combining the cell-penetrating peptide with the one or more genome-editing system molecules, thereby forming the genome-editing complex. In some embodiments, the genome-editing system molecules comprise an RGEN and a gRNA, and the cell-penetrating peptide and the RGEN are combined at a ratio from about 1:1 to about 80:1, respectively, and the RGEN and the gRNA are combined at a ratio from about 10:1 to about 1:10, respectively. In some embodiments, the cell-penetrating peptide and the RGEN are combined at a ratio from about 5:1 to about 20:1, respectively. In some embodiments, the RGEN and the gRNA are combined at a ratio of about 1:1.

In some embodiments, there is provided a method of delivering one or more molecules of a genome-editing system into a cell, comprising contacting the cell with a genome-editing complex according to any of the embodiments described above or a nanoparticle according to any of the embodiments described above, wherein the genome-editing complex or the nanoparticle comprises the one or more genome-editing system molecules. In some embodiments, the contacting of the cell with the genome-editing complex nanoparticle is carried out *in vivo.* In some embodiments, the contacting of the cell with the genome-editing complex nanoparticle is carried out *ex vivo.* In some embodiments, the contacting of the cell with the genome-editing complex nanoparticle is carried out *in vitro.* In some embodiments, the cell is a granulocyte, a mast cell, a monocyte, a dendritic cell, a B cell, a T cell, a natural killer cell, a fibroblast, or a hepatocyte. In some embodiments, the cell is a T cell. In some embodiments, the cell is a fibroblast. In some embodiments, the cell is a hepatocyte. In some embodiments, the cell is a lung progenitor cell. In some embodiments, the cell is a neuronal cell. In some embodiments, the genome-editing system targets a sequence in a gene selected from the group consisting of PD-1, PD-L1, PD-L2, TIM-3, BTLA, VISTA, LAG-3, CTLA-4, TIGIT, 4-1BB, OX40, CD27, TIM-1, CD28, HVEM, GITR, and ICOS. In some embodiments, the method further comprises contacting the cell with an expression complex comprising a fourth cell-penetrating peptide and a nucleic acid molecule encoding an exogenous protein. In some embodiments, the exogenous protein is a recombinant receptor capable of being expressed on the surface of a cell. In some embodiments, the recombinant receptor is a chimeric antigen receptor (CAR). In some embodiments, the fourth cell-penetrating peptide is selected from the group consisting of VEPEP-3 peptides, VEPEP-6 peptides, VEPEP-9 peptides, and ADGN-100 peptides. In some embodiments, the fourth cell-penetrating peptide comprises an amino acid sequence selected from the group consisting of SEQ ID NOs: 1-70 and 75-80.

In some embodiments, there is provided a method of modifying a target polynucleotide in a cell, comprising contacting the cell with a genome-editing complex according to any of the embodiments described above or a nanoparticle according to any of the embodiments described above, wherein the genome-editing complex or the nanoparticle comprises one or more molecules of a genome-editing system that targets a sequence in the target polynucleotide.

In some embodiments, there is provided a method of treating a disease in an individual comprising administering to the individual an effective amount of a pharmaceutical composition according to any of the embodiments described above. In some embodiments, the disease is selected from the group consisting of cancer, diabetes, inflammatory diseases, fibrosis, viral infectious diseases, hereditary diseases, ocular diseases, liver diseases, lung disease, kidney diseases, and aging and degenerative diseases. In some embodiments, the disease is cancer. In some embodiments, the cancer is a solid tumor, and the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, and regulators of apoptosis and metastasis. In some embodiments, the cancer is cancer of the liver, lung, or kidney. In some embodiments, the cancer is a hematological malignancy, and the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, and regulators of apoptosis and metastasis.

In some embodiments, according to any of the methods of treating a disease described above, the disease is a viral infection disease, and the pharmaceutical composition comprises a genome-editing complex or nanoparticle comprising one or more molecules of a genome-editing system that modulates the expression of one or more proteins involved in the viral infectious disease development and/or progression.

In some embodiments, according to any of the methods of treating a disease described above, the disease is a genetic disease, and the pharmaceutical composition comprises a genome-editing complex or nanoparticle comprising one or more molecules of a genome-editing system that modulates the expression of one or more proteins involved in the hereditary disease development and/or progression.

In some embodiments, according to any of the methods of treating a disease described above, the disease is an aging or degenerative disease, and the pharmaceutical composition comprises a genome-editing complex or nanoparticle comprising one or more molecules of a genome-editing system that modulates the expression of one or more proteins involved in the aging or degenerative disease development and/or progression.

In some embodiments, according to any of the methods of treating a disease described above, the disease is a fibrotic or inflammatory disease, and the pharmaceutical composition comprises a genome-editing complex or nanoparticle comprising one or more molecules of a genome-editing system that modulates the expression of two or more proteins involved in the fibrotic or inflammatory disease development and/or progression.

In some embodiments, according to any of the methods of treating a disease described above, the individual is human.

In some embodiments, there is provided a kit comprising a composition comprising a genome-editing complex according to any of the embodiments described above and/or a nanoparticle according to any of the embodiments described above.

### BRIEF DESCRIPTION OF THE FIGURES

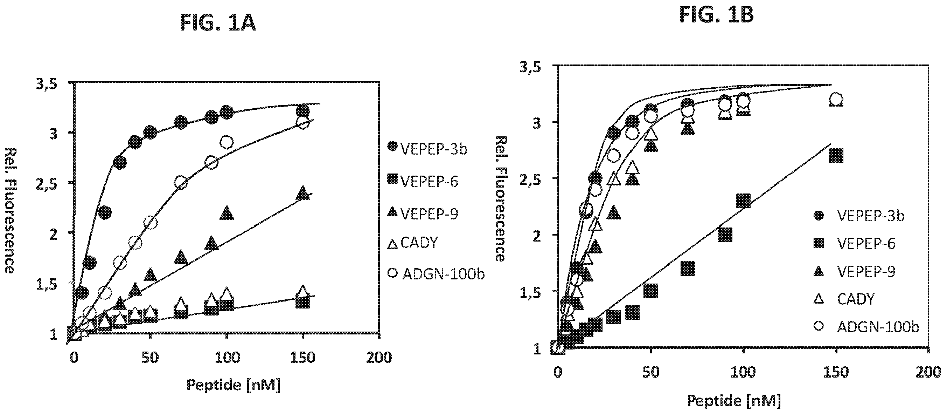

FIG. 1A shows binding titration curve of CAS9 to CPPs as monitored by CY-5 fluorescence. A fixed concentration of 5 nM of fluorescently labeled CAS9 was titrated by increasing concentration of VEPEP-3b (SEQ ID NO: 76), VEPEP-6 (SEQ ID NO: 77), VEPEP-9 (SEQ ID NO: 78), CADY (SEQ ID NO: 81) and ADGN-100b (SEQ ID NO: 80) peptides. Dissociation constants were calculated from data fitting using a quadatric equation.
FIG. 1B shows binding titration curve of CAS9:gRNA to CPPs as monitored by CY-5 fluorescence. A fixed concentration of 5 nM of fluorescently labeled CAS9:gRNA was titrated by increasing concentration of VEPEP-3b (SEQ ID NO: 76), VEPEP-6 (SEQ ID NO: 77), VEPEP-9 (SEQ ID NO: 78), CADY (SEQ ID NO: 81) and ADGN-100b (SEQ ID NO: 80) peptides. Dissociation constants were calculated from data fitting using a quadatric equation.

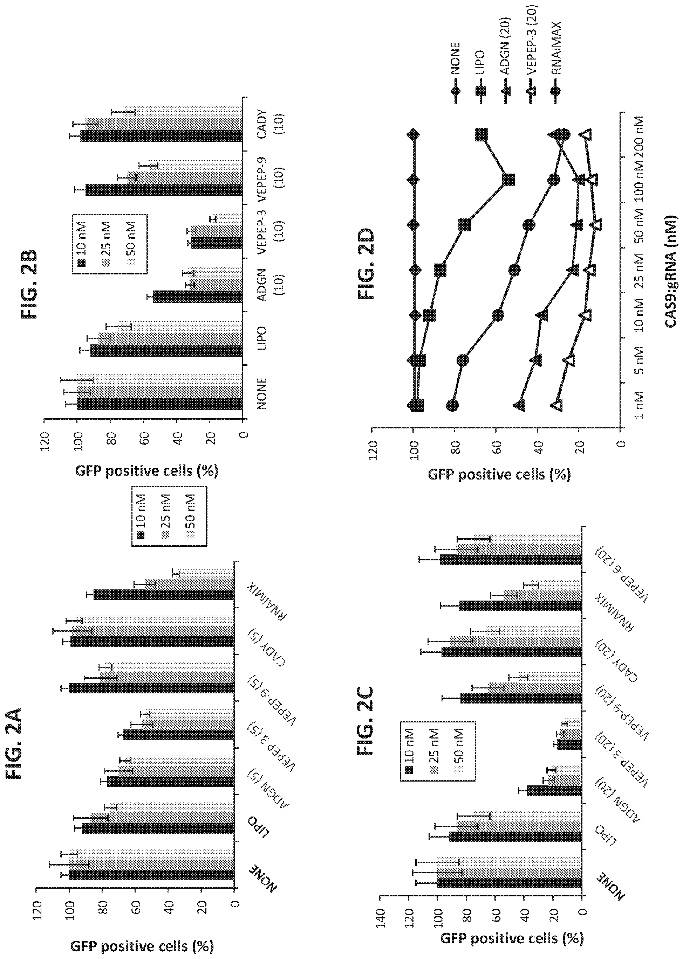

FIG. 2A shows gene disruption frequency of an EGFP reporter gene in U2OS cells from delivery of CAS9:sgRNA as quantified by flow cytometry. 10 nM, 25 nM and 50 nM of CAS9:gRNA complexes were associated with different peptides at molar ratio 1:5, respectively, and to lipofectamine 2000 and RNAiMAX.
FIG. 2B shows gene disruption frequency of an EGFP reporter gene in U2OS cells from delivery of CAS9:sgRNA as quantified by flow cytometry. 10 nM, 25 nM and 50 nM of CAS9:gRNA complexes were associated with different peptides at molar ratio 1:10, respectively, and to lipofectamine 2000 and RNAiMAX.
FIG. 2C shows gene disruption frequency of an EGFP reporter gene in U2OS cells from delivery of CAS9:sgRNA as quantified by flow cytometry. 10 nM, 25 nM and 50 nM of CAS9:gRNA complexes were associated with different peptides at molar ratio 1:20, respectively, and to lipofectamine 2000 and RNAiMAX.
FIG. 2D shows a dose response of gene disruption frequency of an EGFP reporter gene in U2OS using different peptides. Lipofectamine 2000 and RNAiMAX were used as controls. Values in parentheses indicated molar ratio of peptide to CAS9:gRNA complex.

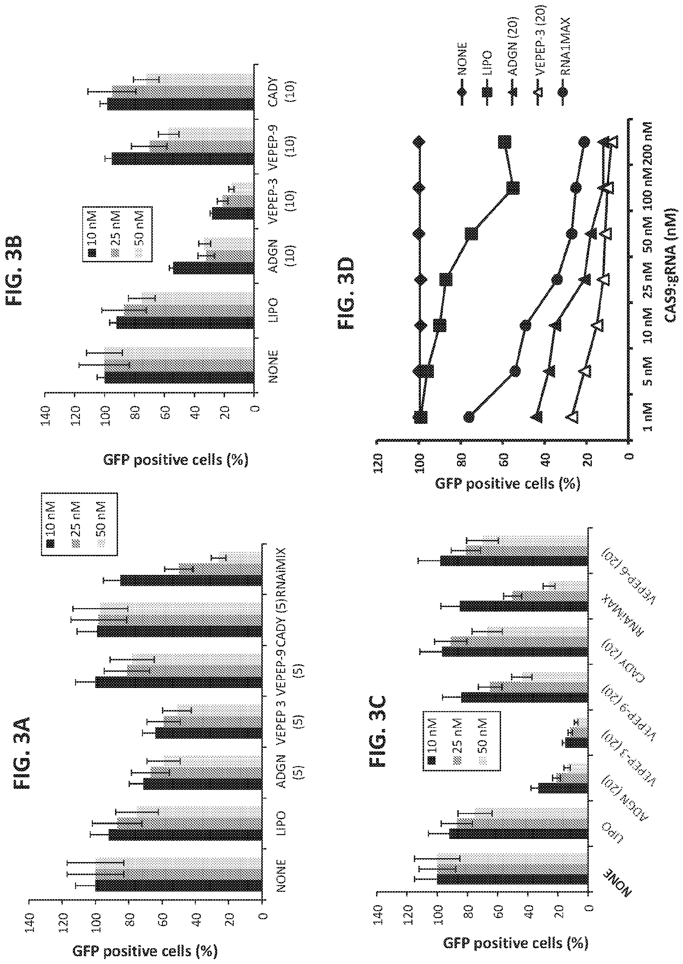

FIG. 3A shows gene disruption frequency of an EGFP reporter gene in HEK cells from delivery of CAS9:sgRNA as quantified by flow cytometry. 10 nM, 25 nM and 50 nM of CAS9:gRNA complexes were associated with different peptides at molar ratio 1:5, respectively, and to lipofectamine 2000 and RNAiMAX.
FIG. 3B shows gene disruption frequency of an EGFP reporter gene in HEK cells from delivery of CAS9:sgRNA as quantified by flow cytometry. 10 nM, 25 nM and 50 nM of CAS9:gRNA complexes were associated with different peptides at molar ratio 1:10, respectively, and to lipofectamine 2000 and RNAiMAX.
FIG. 3C shows gene disruption frequency of an EGFP reporter gene in HEK cells from delivery of CAS9:sgRNA as quantified by flow cytometry. 10 nM, 25 nM and 50 nM of CAS9:gRNA complexes were associated with different peptides at molar ratio 1:20, respectively, and to lipofectamine 2000 and RNAiMAX.
FIG. 3D shows a dose response of gene disruption frequency of an EGFP reporter gene in HEK cells using different peptides. Lipofectamine 2000 and RNAiMAX were used as controls. Values in parentheses indicated molar ratio of peptide to CAS9:gRNA complex.

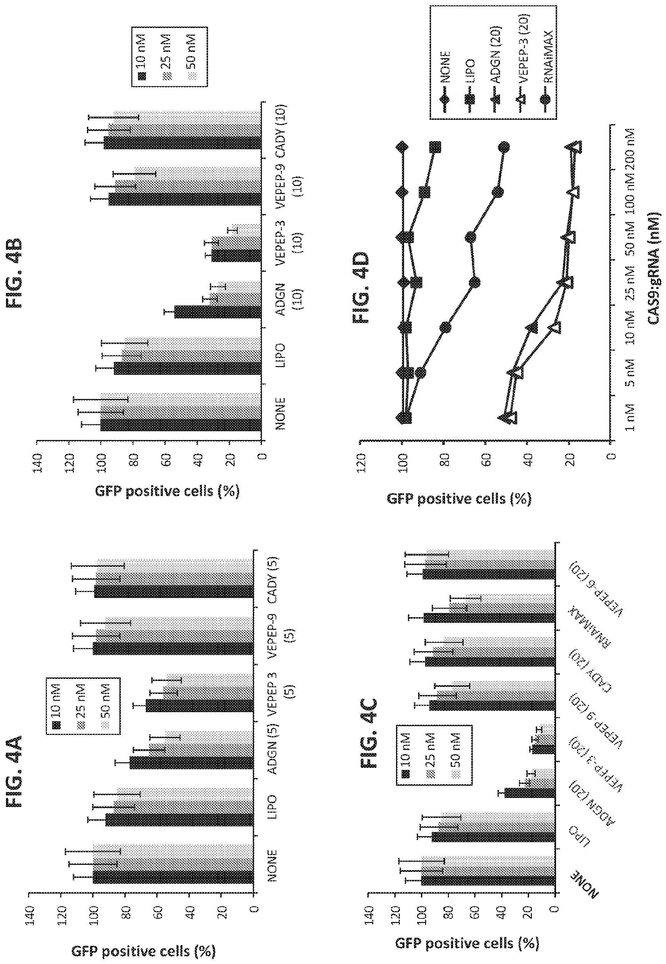

FIG. 4A shows gene disruption frequency of an EGFP reporter gene in JURKAT T cells from delivery of CAS9:sgRNA as quantified by flow cytometry. 10 nM, 25 nM and 50 nM of CAS9:gRNA complexes were associated with different peptides at molar ratio 1:50, respectively, and to lipofectamine 2000 and RNAiMAX.
FIG. 4B shows gene disruption frequency of an EGFP reporter gene in JURKAT T cells from delivery of CAS9:sgRNA as quantified by flow cytometry. 10 nM, 25 nM and 50 nM of CAS9:gRNA complexes were associated with different peptides at molar ratio 1:10, respectively, and to lipofectamine 2000 and RNAiMAX.
FIG. 4C shows gene disruption frequency of an EGFP reporter gene in JURKAT T cells from delivery of CAS9:sgRNA as quantified by flow cytometry. 10 nM, 25 nM and 50 nM of CAS9:gRNA complexes were associated with different peptides at molar ratio 1:20, respectively, and to lipofectamine 2000 and RNAiMAX.
FIG. 4D shows a dose response of gene disruption frequency of an EGFP reporter gene in JURKAT T cells using different peptides. Lipofectamine 2000 and RNAiMAX were used as controls. Values in parentheses indicated molar ratio of peptide to CAS9:gRNA complex.

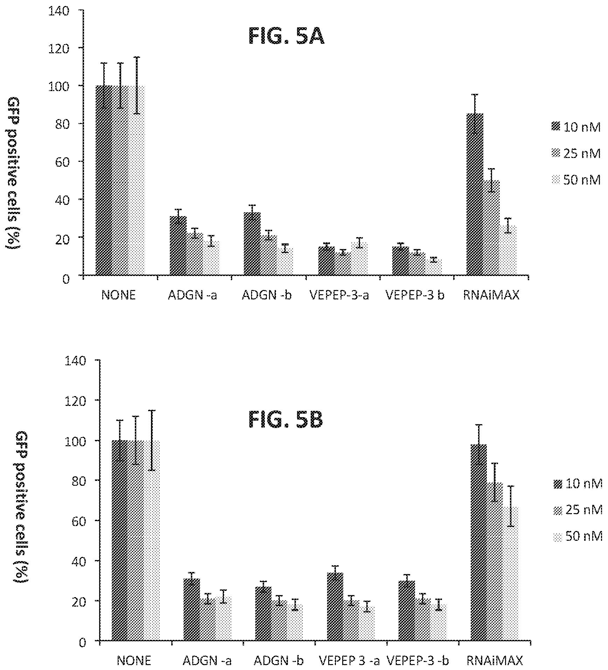

FIG. 5A shows gene disruption frequency of an EGFP reporter gene in U2OS cells from delivery of CAS9:sgRNA as quantified by flow cytometry. 10 nM, 25 nM and 50 nM of CAS9:gRNA complexes were associated with variant sequences of VEPEP-3 and ADGN-100 at molar ratio 1:20, respectively. RNAiMAX was used as control delivery system.
FIG. 5B shows gene disruption frequency of an EGFP reporter gene in JURKAT T cells from delivery of CAS9:sgRNA as quantified by flow cytometry. 10 nM, 25 nM and 50 nM of CAS9:gRNA complexes were associated with variant sequences of VEPEP-3 and ADGN-100 at molar ratio 1:20, respectively. RNAiMAX was used as control delivery system

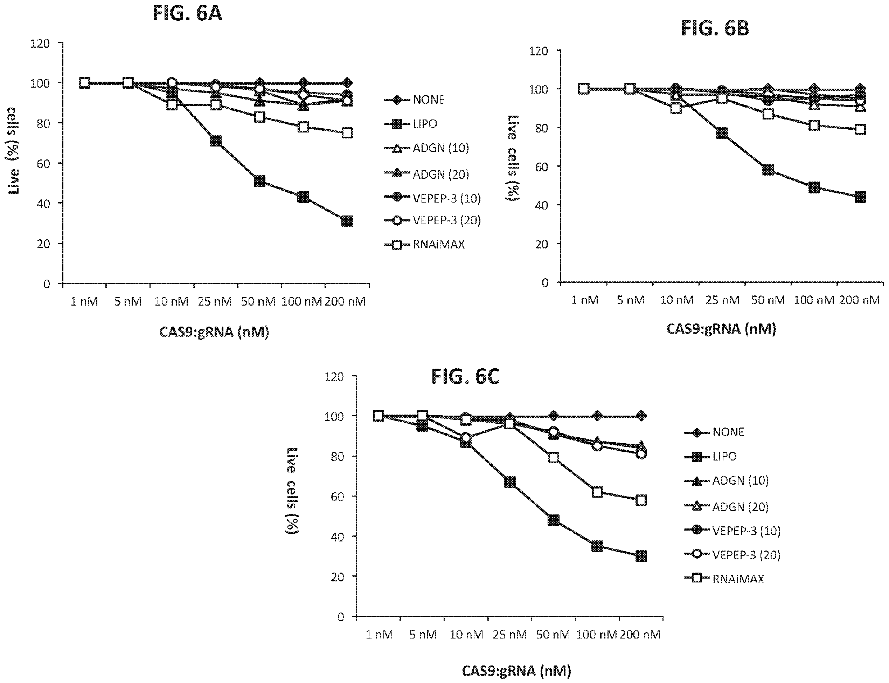

FIG. 6A shows toxicity evaluation of ADGN-100a/CAS9/gRNA and VEPEP-3b/CAS9/gRNA complexes on U2OS cells as measured by MTT assay. Peptides were compared to CAS9/gRNA complexed to Lipofectamine 2000 and RNAiMAX. Values in parentheses indicated molar ratio of peptide to CAS9:gRNA complex.
FIG. 6B shows toxicity evaluation of ADGN-100a/CAS9/gRNA and VEPEP-3b/CAS9/gRNA complexes on HEK cells as measured by MTT assay. Peptides were compared to Lipofectamine 2000 and RNAiMAX. Values in parentheses indicated molar ratio of peptide to CAS9:gRNA complex.
FIG. 6C shows toxicity evaluation of ADGN-100a/CAS9/gRNA and VEPEP-3b/CAS9/gRNA complexes on JURKAT cells as measured by MTT assay. Peptides were compared to Lipofectamine 2000 and RNAiMAX. Values in parentheses indicated molar ratio of peptide to CAS9:gRNA complex.

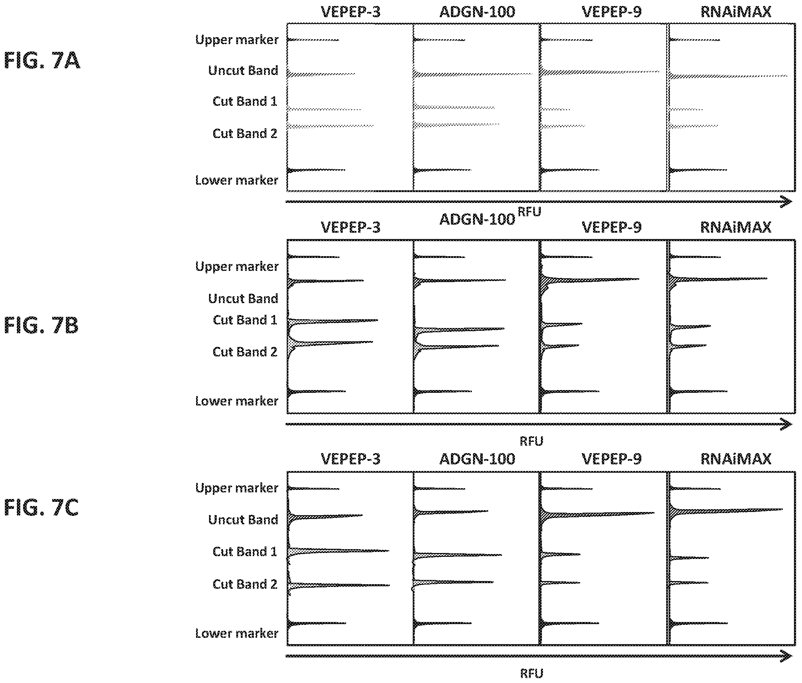

FIG. 7A shows EMX1 gene editing in U2OS cells. Cell were transfected with 25 nM CAS9/gRNA associated with either VEPEP-3b, ADGN-100a, VEPEP-9, or RNAiMAX. Cell samples were analysed by T7EI methods and indel results were determined by quantifying the amount of uncut vs cut DNA using Fragment Analyzer System and reported in Table 3.
FIG. 7B shows EMX1 gene editing in K562 cells. Cell were transfected with 25 nM CAS9/gRNA associated with either VEPEP-b3, ADGN-100a, VEPEP-9, or RNAiMAX. Cell samples were analysed by T7EI methods and indel results were determined by quantifying the amount of uncut vs cut DNA using Fragment Analyzer System and reported in Table 3.
FIG. 7C shows EMX1 gene editing in JURKAT cells. Cell were transfected with 25 nM CAS9/gRNA associated with either VEPEP-3b, ADGN-100a, VEPEP-9, or RNAiMAX. Cell samples were analysed by T7EI methods and indel results were determined by quantifying the amount of uncut vs cut DNA using Fragment Analyzer System and reported in Table 3.

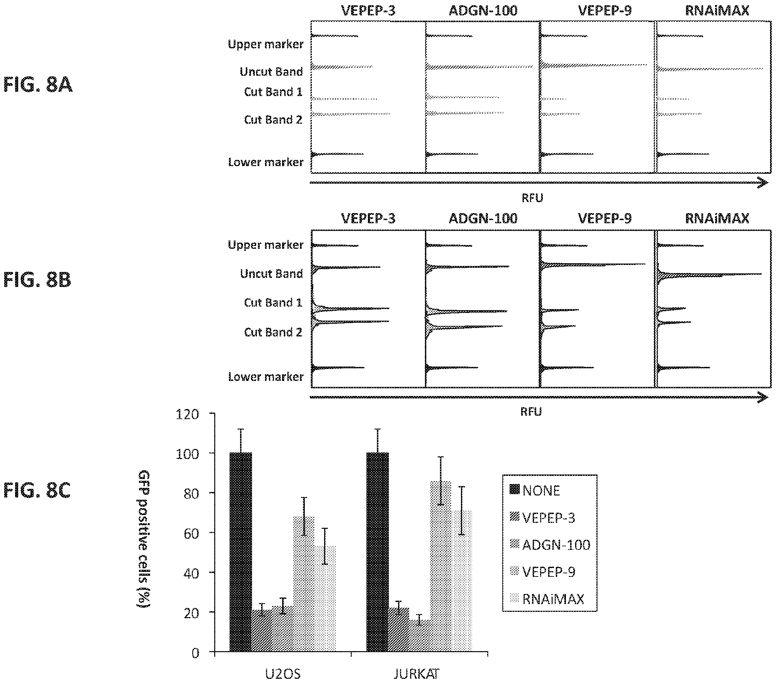

FIG. 8A shows EMX1 gene editing in EGFP-U2OS cells. Cell were transfected with 25 nM CAS9/gRNA associated with either VEPEP-3b, ADGN-100a, VEPEP-9, or RNAiMAX. Cell samples were analysed by T7EI methods and indel results were determined by quantifying the amount of uncut vs cut DNA using Fragment Analyzer System and reported in Table 4.
FIG. 8B shows EMX1 gene editing in EGFP-JURKAT cells. Cell were transfected with 25 nM CAS9/gRNA associated with either VEPEP-3b, ADGN-100a, VEPEP-9, or RNAiMAX. Cell samples were analyzed by T7EI methods and indel results were determined by quantifying the amount of uncut vs cut DNA using Fragment Analyzer System and reported in Table 4.
FIG. 8C shows gene disruption frequency of an EGFP reporter gene in both U2OS and JURKAT T cells from delivery of CAS9:sgRNA as quantified by flow cytometry. 25 nM of CAS9:gRNA complexes were associated with VEPEP-3b, VEPEP-9 and ADGN-100a at molar ratio 1:20, respectively, and to lipofectamine 2000 and RNAiMAX.

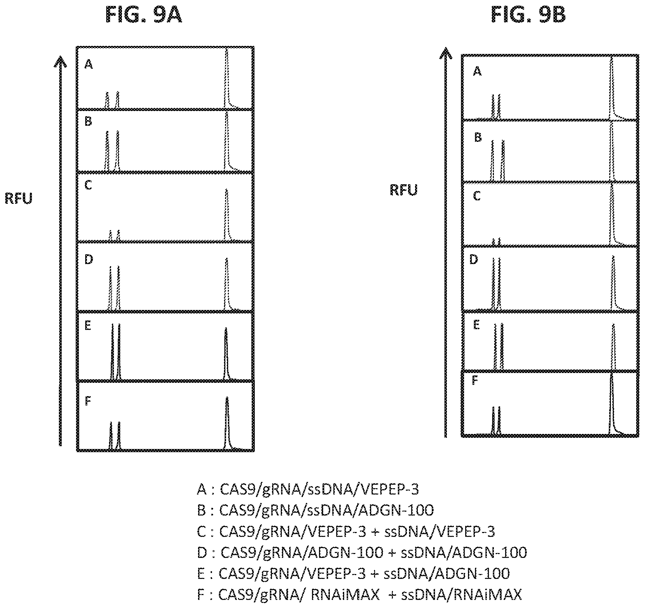

FIG. 9A shows analysis of the efficiency of HDR insertion of a HindIII and NheI restriction enzyme site in the EMX1 gene in U2OS cells. The percent HDR editing were calculated using an RFLP assay with NheI digestion of EMXI PCR amplicons.
FIG. 9B shows analysis of the efficiency of HDR insertion of a HindIII and NheI restriction enzyme site in the EMX1 gene in JURKAT cells. The percent HDR editing were calculated using an RFLP assay with NheI digestion of EMXI PCR amplicons.

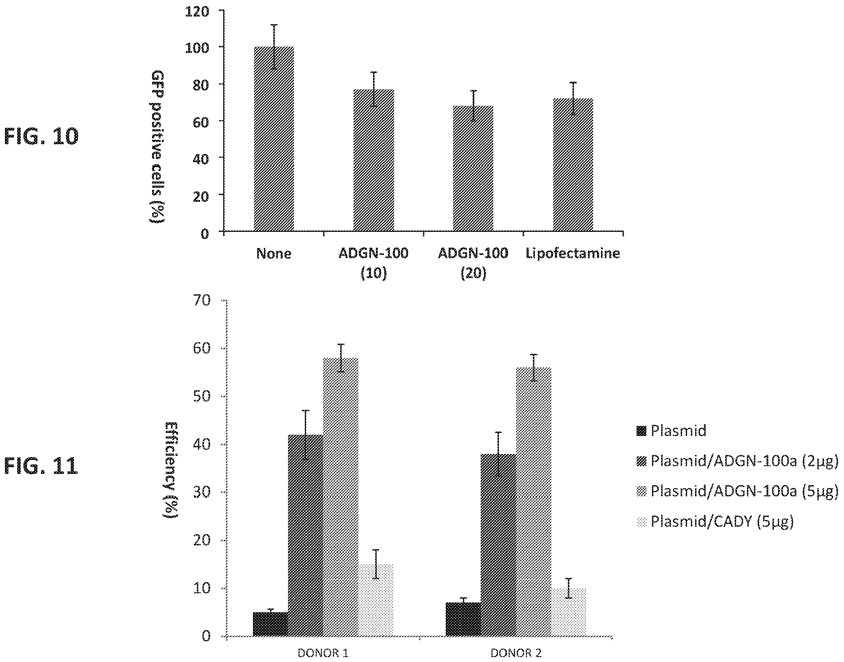

FIG. 10 shows gene disruption frequency of an EGFP reporter gene in U2OS cells from co delivery of CAS9-expresson plasmid and sgRNA using ADGN-100a peptide. CAS9 expression plasmid and sgRNA were associated with ADGN-100a at molar ratio 10:1 or 20:1 (indicated by values in parentheses) of ADGN-100a to each of the CAS9 expression plasmid and the sgRNA, and to lipofectamine 2000.
FIG. 11 shows the transfection efficiency of ADGN-100a/plasmid complexes. Primary T cells were transfected with ADGN-100a particles containing an anti-CD19 CAR-encoding plasmid at 2 concentrations (2µg and 5µg). At Day 4, expression of the anti-CD19-CAR was evaluated by flow cytometry using a Protein L assay. Data were normalized to untransfected cells and compared to cells incubated with free plasmid.

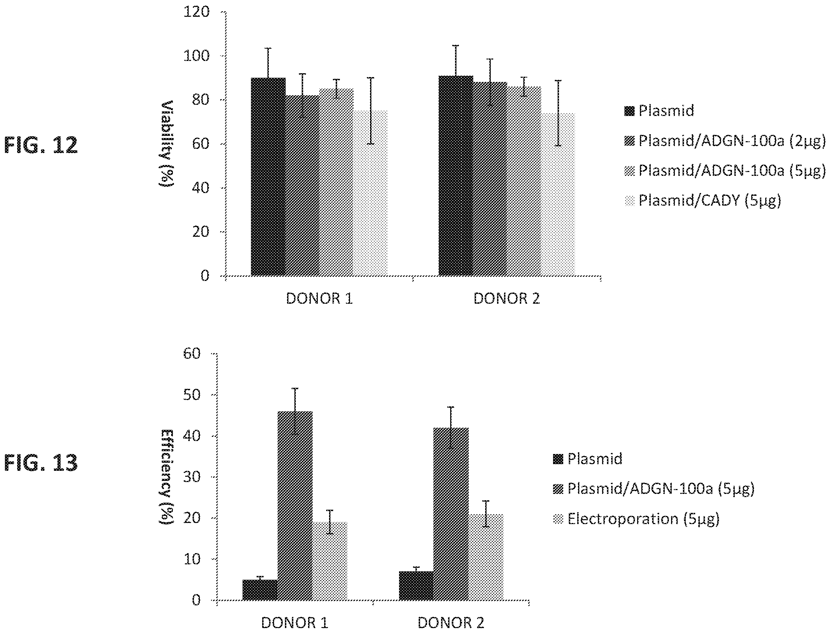

FIG. 12 shows the viability of primary T cells following transfection with ADGN-100a/plasmid complexes. Primary T cells were transfected with ADGN-100a particles containing an anti-CD19 CAR-encoding plasmid at 2 concentrations (2µg and 5µg). At Day 4, cell viability was quantified by flow cytometry using 7-AAD. Data were normalized to untransfected cells and compared to T cells incubated with free plasmid.
FIG. 13 shows the transfection efficiency of ADGN-100a/plasmid complexes used in combination with peptide/RGEN/gRNA complexes. Primary T cells were transfected with ADGN-100a particles containing an anti-CD19 CAR-encoding plasmid (5µg) and peptide particles containing gRNA/CAS9 complexes. At Day 6, expression of the anti-CD 19-CAR was evaluated by flow cytometry using a Protein L assay. Data were normalized to untransfected cells and compared to cells incubated with free plasmid.

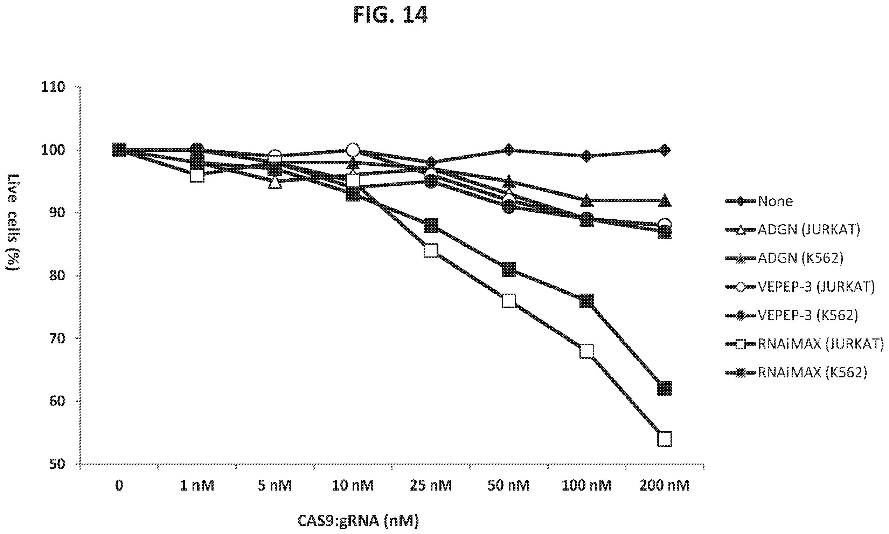

FIG. 14 shows toxicity evaluation of ADGN-100a/CAS9/gRNA and VEPEP-3a/CAS9/gRNA complexes on JURKAT and K562 cells as measured by MTT assay. Delivery of CAS9/gRNA complexes by RNAiMAX was included for comparison.

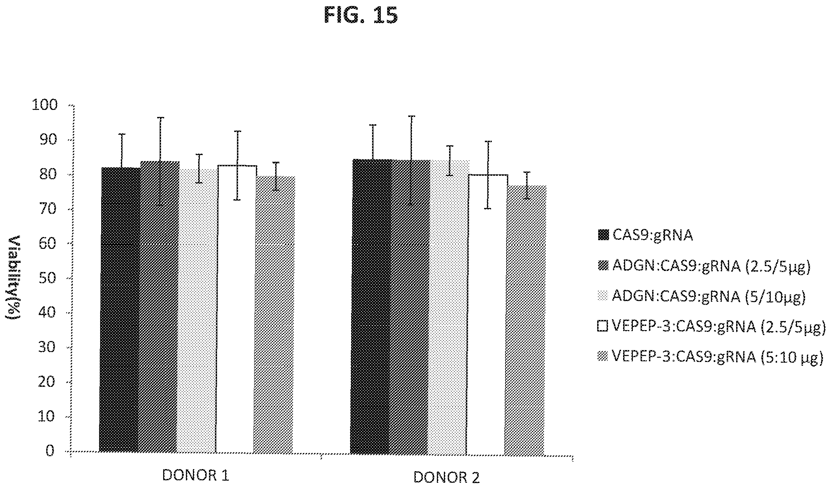

FIG. 15 shows the viability of primary T cells following transfection with ADGN-100a or VEPEP-3a particles containing CAS9/gRNA complexes. T cells were transfected with ADGN-100a or VEPEP-3a particles containing CAS9/gRNA particles at 2 concentrations (5µg/10µg CAS9/gRNA and 2.5µg/5µg CAS9/gRNA). After 48h, cell viability was quantified by flow cytometry using 7-AAD. Data were normalized to untransfected cells and compared to T cells incubated with free CAS9/gRNA.

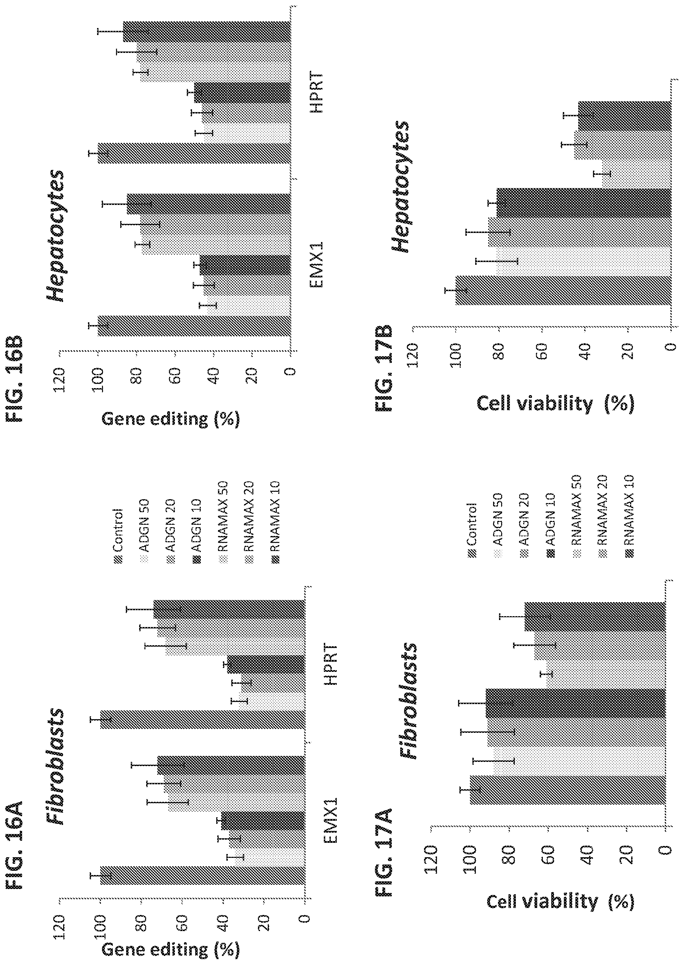

FIGS. 16A and 16B show the gene disruption frequency of *EMX1* and *HPRT* endogenous genes in primary human fibroblasts (FIG. 16A) or primary human hepatocytes (FIG. 16B) mediated by CAS9/gRNA, as quantified by T7E1 assay. CAS9/gRNA complexes (10 nM, 20 nM, or 50 nM) were delivered either by ADGN-100a (molar ratio of 20:1 for peptide to complex) or by RNAiMAX.
FIGS. 17A and 17B show the toxicity evaluation of ADGN-100a/CAS9/gRNA complexes on human primary fibroblasts (FIG. 17A) or human primary hepatocytes (FIG. 17B) as measured by MTT assay. Peptide-mediated delivery was compared to delivery by RNAiMAX.

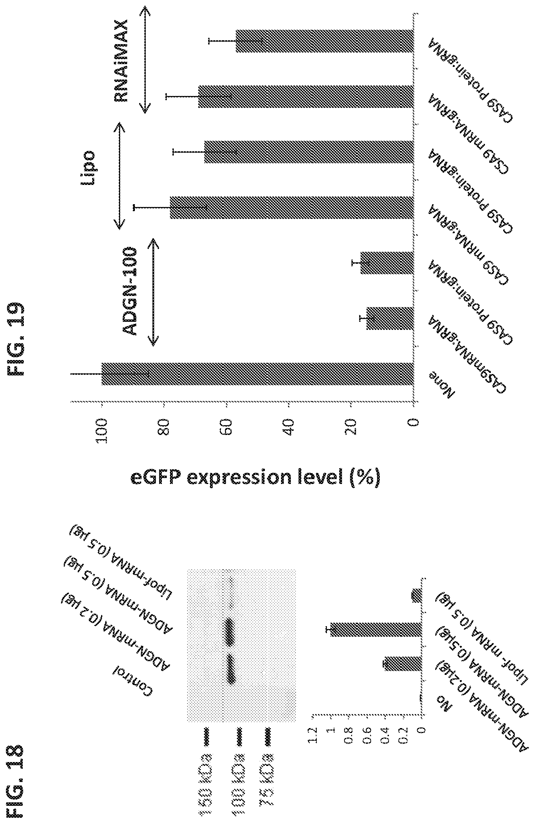

FIG. 18 shows western blot analysis and quantification for CAS9 protein expression in U2OS cells transfected with CSA9 mRNA (0.2 µg or 0.5 µg) using either ADGN-100a or Lipofectamine 2000.
FIG. 19 shows the gene disruption frequency of an EGFP reporter gene in U2OS cells by ADGN-100a-mediated delivery of CAS9/gRNA or CAS9 mRNA/gRNA, as quantified by flow cytometry. CAS9/gRNA (2.5 µM/5 µM) or CAS9 mRNA/gRNA (0.5 µg/5 µg) complexes were associated with ADGN-100a at a molar ratio of 20:1 (peptide to complex) for peptide-mediated delivery, and compared to delivery by lipofectamine 2000 or RNAiMAX.

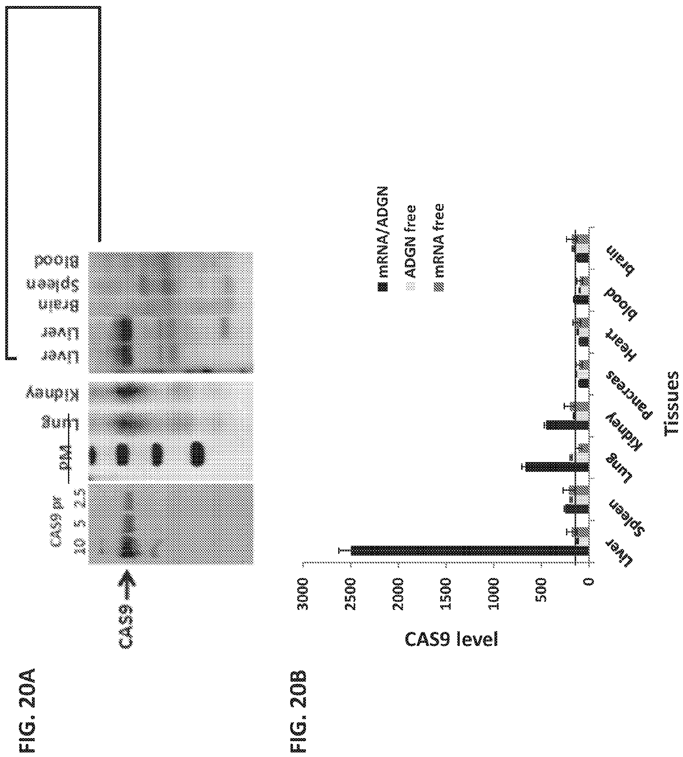

FIGS. 20A and 20B show quantification of CAS9 protein expression by western blot (FIG. 20A) or ELISA (FIG. 20B) in different tissues after *in vivo* administration of CAS9 mRNA (10 µg) associated with ADGN-100a peptide at a 20:1 molar ratio of peptide to mRNA. Tissues were homogenized and CAS9 expression was analyzed by western blot or ELISA on protein extract using antibody against CRISPR/Cas9.

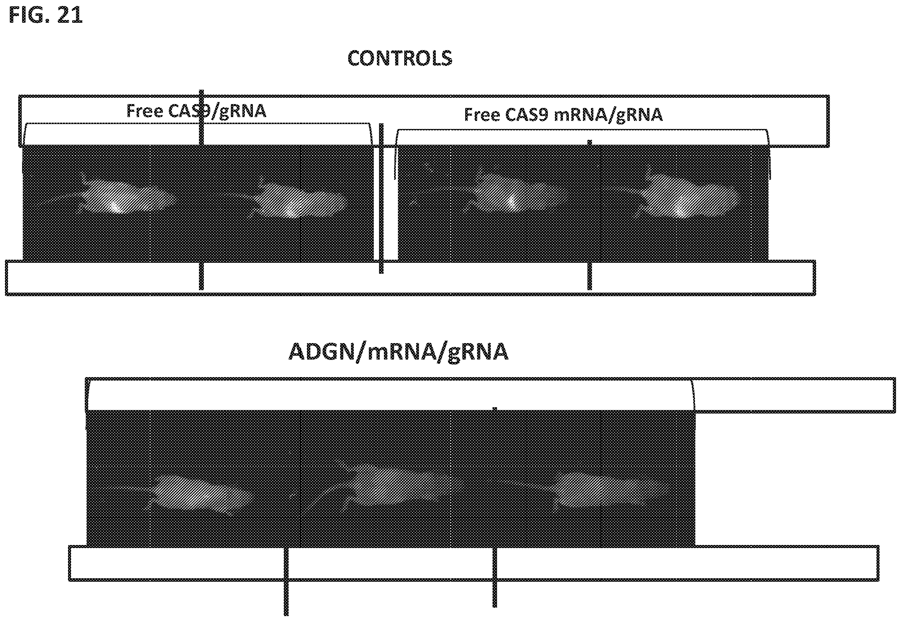

FIG. 21 shows whole animal fluorescence imaging for luciferase expression 72 hours after injection of free ADGN-100a peptide (2 animals/group), free CAS9 mRNA (2 animals/group), or CAS9 mRNA/gRNA/ADGN-100a (3 animals per group).

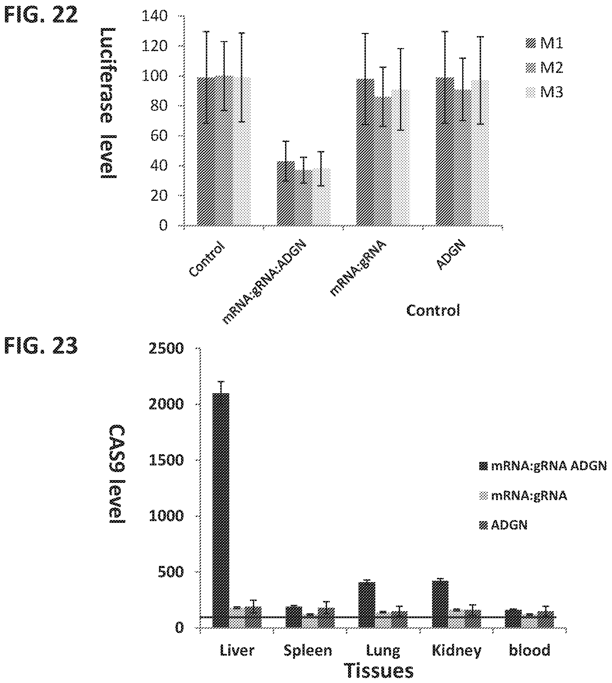

FIG. 22 shows the *in vivo* gene disruption frequency of a Luciferase reporter gene in the liver resulting from ADGN-100a-mediated delivery of mRNA CAS9/sgRNA, as quantified by fluorescence imaging. Mice received a single intravenous injection of free ADGN-100a peptide, free CAS9 mRNA, or CAS9 mRNA/gRNA/ADGN-100a 3 animals per group (labeled M1, M2, and M3).
FIG. 23 shows ELISA analysis for CAS9 protein expression in different tissues after *in vivo* administration of CAS9 mRNA/gRNA (10 µg) associated with ADGN-100a peptide at a 20:1 molar ratio of peptide to mRNA/gRNA. Tissues were homogenized and CAS9 expression was analyzed by ELISA on protein extract using antibody against CRISPR/Cas9.

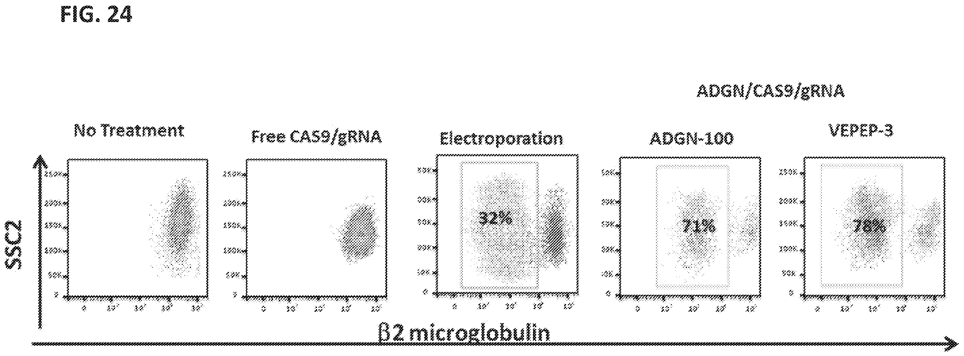

FIG. 24 shows FACS analysis of β2 microglobulin gene editing in T cells. T cells were transfected with CAS9/gRNA (5 µg/10 µg CAS9/gRNA) by association with either ADGN-100a or VEPEP-3a, or by electroporation. Free CAS9/gRNA and no treatment conditions were included as controls. β2 microglobulin expression was evaluated by FACS after 72 hours.

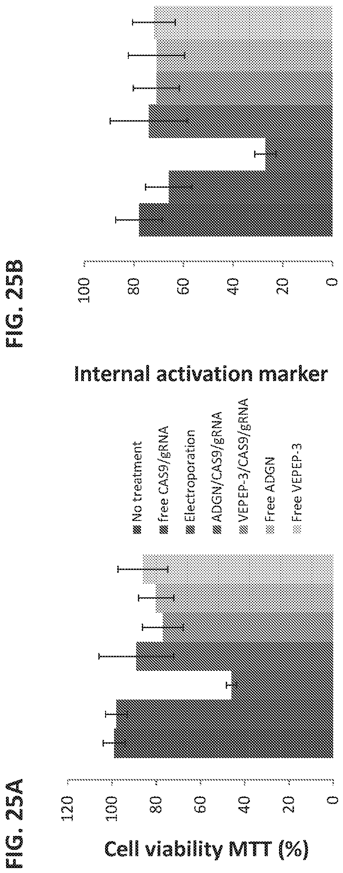

FIGS. 25A and 25B show toxicity analysis of CPP/CAS9/gRNA complexes on T cells. PBMC-isolated T cells were treated with CAS9/gRNA complexes associated with ADGN-100a or VEPEP-3b, or electroporated with CAS9/gRNA complexes, and toxicity was assessed after 48 hours by MTT assay (FIG. 25A) and by monitoring activation damage-induced cell death (DICD) after 72 hours (FIG. 25B). No treatment, free CAS9/gRNA, free ADGN-100a, and free VEPEP-3b conditions were included as controls.

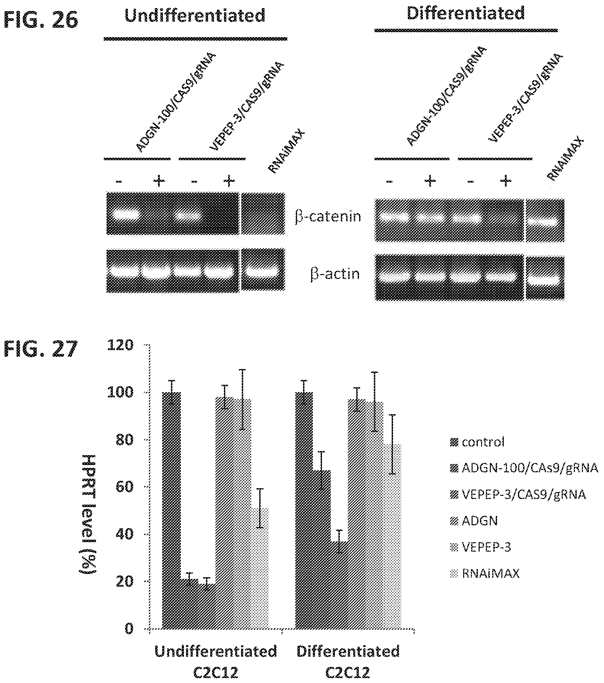

FIG. 26 shows gene disruption of the β-Catenin gene in undifferentiated and differentiated mouse muscle myoblast cells (C2C12) by VEPEP-3b- and ADGN-100a-mediated delivery of CAS9/gRNA as quantified by gel analysis of PCR. Cells were transfected with CAS9/gRNA (2.5 µM/5 µM) complexes associated with CPP (+) or not (-) at a molar ratio of 20:1 (peptide to complex) and compared to delivery by RNAiMAX for β-Catenin expression. β-actin expression was included as a loading control.
FIG. 27 shows the gene disruption efficiency of the HPRT gene in undifferentiated and differentiated mouse muscle myoblast cells (C2C12) by VEPEP-3b- and ADGN-100a-mediated delivery of CAS9/gRNA. Cell samples were analyzed by T7EI methods and indel results were determined by quantifying the amount of uncut vs cut DNA using a Fragment Analyzer System Delivery by RNAiMAX was included as a control.

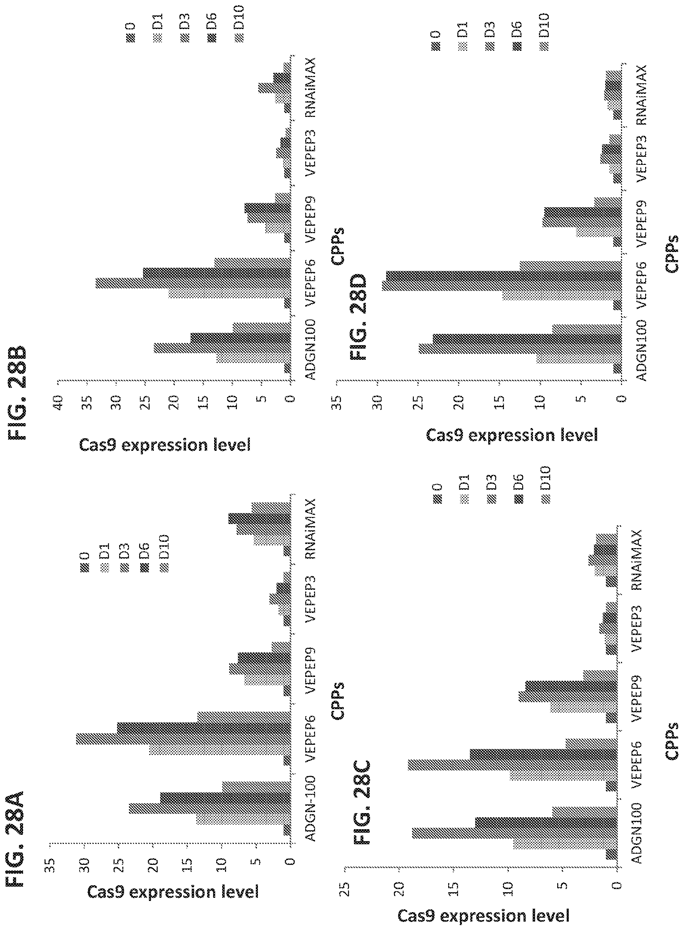

FIGS. 28A-28D show quantification of CAS9 protein expression by ELISA in different cell types transfected with CAS9 mRNA (0.5 µg) using ADGN-100a, VEPEP-6, VEPEP-9, or VEPEP-3a peptides or RNAiMAX. FIG. 28A shows results for U2OS cells. FIG. 28B shows results for HEPG2 cells. FIG. 28C shows results for primary human fibroblasts. FIG. 28D shows results for K562 cells.

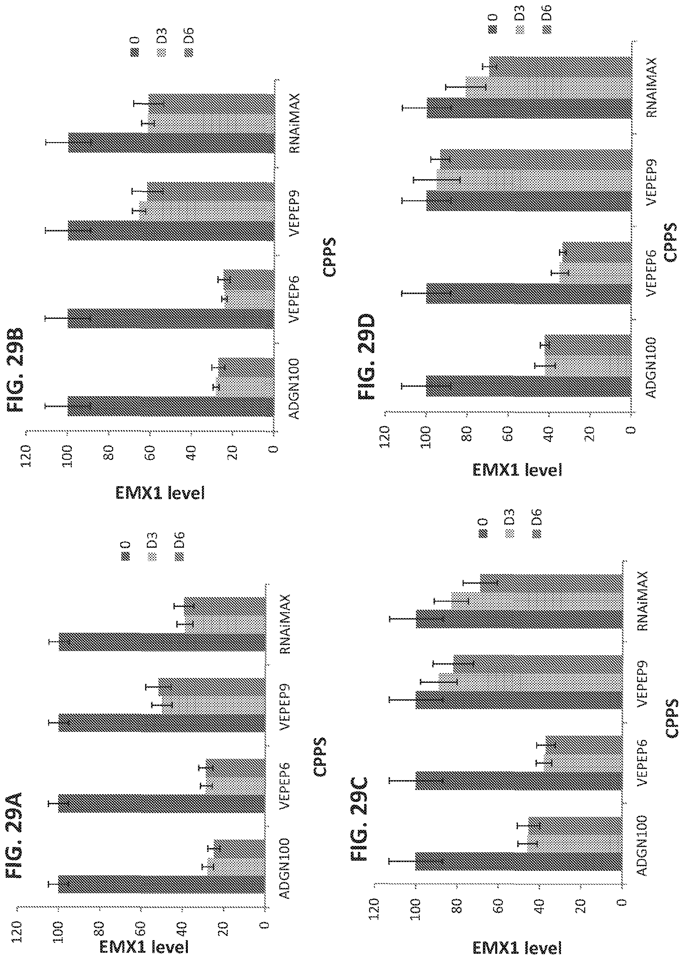

FIGS. 29A-29D show the gene disruption frequency of the endogenous *EMX1* gene in U2OS cells (FIG. 29A), HEPG2 cells (FIG. 29B), primary human fibroblasts (FIG. 29C), or K562 cells (FIG. 29D) mediated by CAS9 mRNA/gRNA, as quantified by T7E1 assay. CAS9 mRNA/gRNA (0.5 µg/2.5 µg) were delivered by ADGN-100a, VEPEP-6, VEPEP-9, or VEPEP-3a peptides (molar ratio of 20:1 for CPP to cargo) or by RNAiMAX.

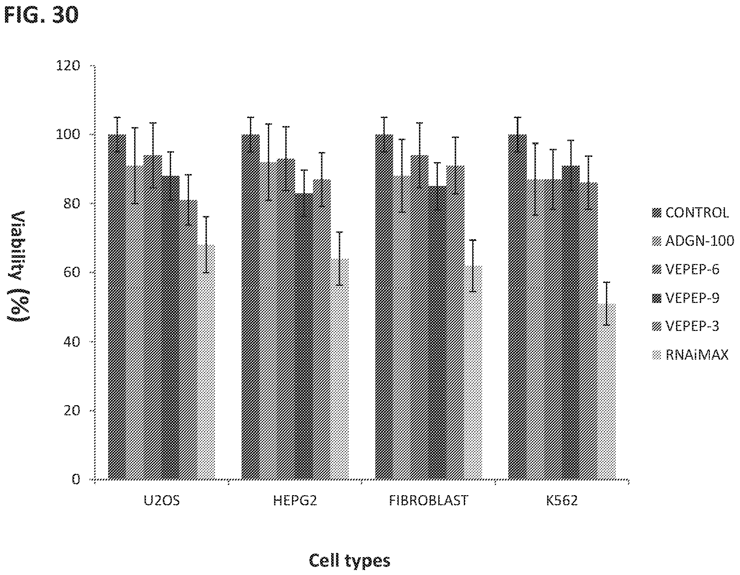

FIG. 30 shows the toxicity evaluation of CPP/CAS9 mRNA/gRNA complexes on U2OS, HEPG2, and K562 cell lines and human primary fibroblasts as measured by MTT assay. Peptide-mediated delivery was compared to delivery by RNAiMAX.

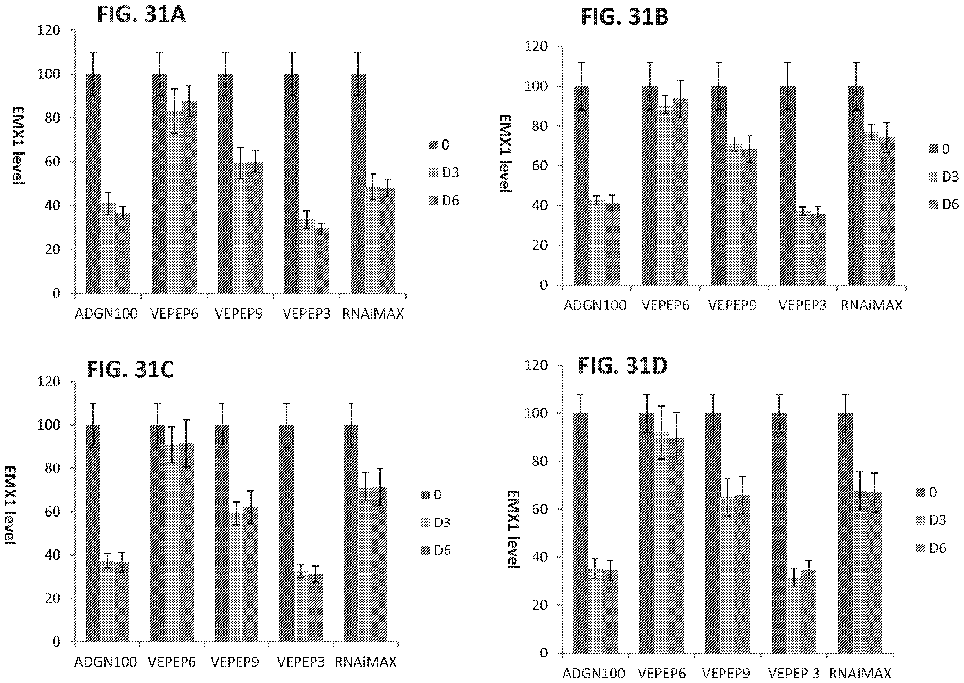

FIGS. 31A-31D show the gene disruption frequency of the endogenous *EMX1* gene in U2OS cells (FIG. 31A), HEPG2 cells (FIG. 31B), primary human fibroblasts (FIG. 31C), or K562 cells (FIG. 31D) mediated by CAS9 protein/gRNA, as quantified by T7E1 assay. The preloaded CAS9/gRNA complexes (2.5 µg/5 µg CAS9/gRNA) were mixed with CPP peptides at a 20:1 molar ratio of CPP to CAS9/gRNA complexes for peptide-mediated delivery, and compared to delivery by lipofectamine 2000 or RNAiMAX.

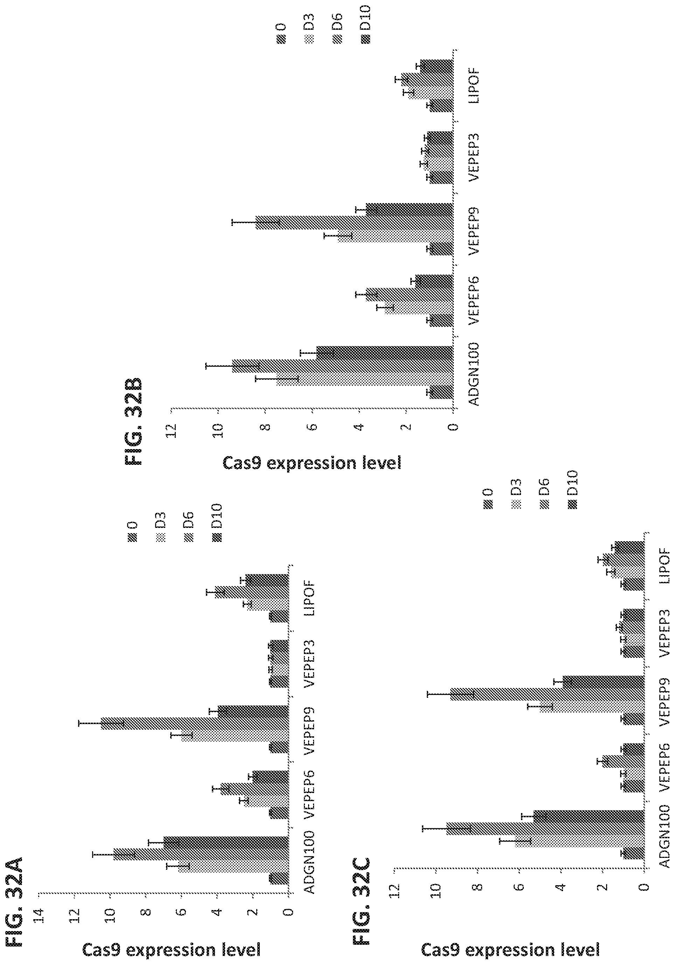

FIGS. 32A-32C show quantification of CAS9 protein expression by ELISA in different cell types transfected with a CAS9-expressing plasmid (0.5 µg) using ADGN-100a, VEPEP-6, VEPEP-9, or VEPEP-3a peptides, or using lipofectamine 2000. FIG. 32A shows results for U2OS cells. FIG. 32B shows results for HEPG2 cells. FIG. 32C shows results for primary human fibroblasts.

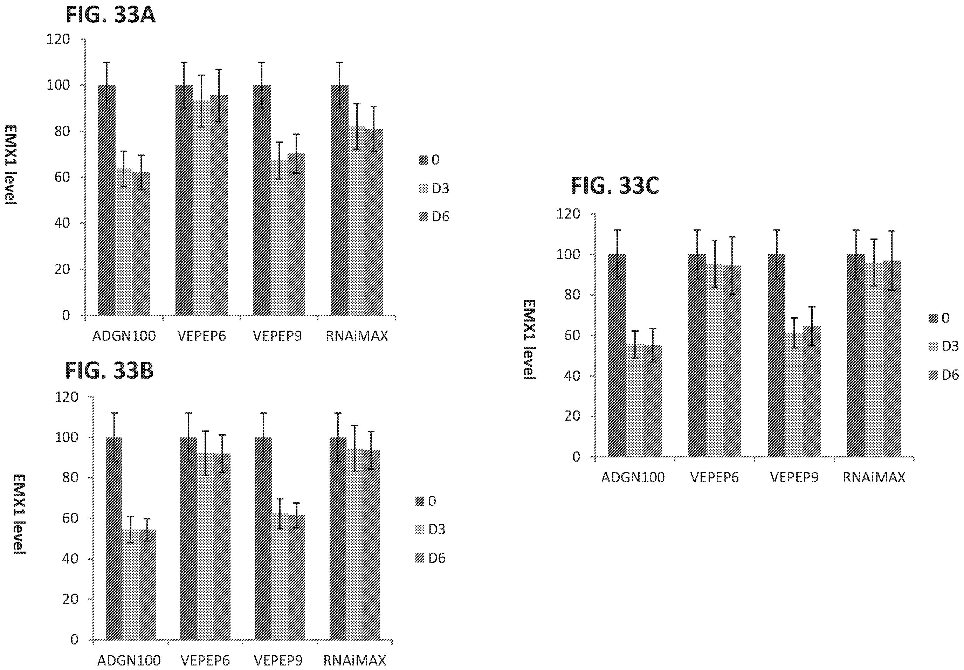

FIGS. 33A-33C show the gene disruption frequency of the endogenous *EMX1* gene in U2OS cells (FIG. 33A), HEPG2 cells (FIG. 33B), or primary human fibroblasts (FIG. 33C) mediated by a CAS9-expressing plasmid and gRNA, as quantified by T7E1 assay. The CAS9-expressing plasmid (0.5µg) and gRNA (5µg) were mixed with CPP peptides at a 20:1 molar ratio of CPP to nucleic acid (plasmid + gRNA). Peptide-mediated delivery of the CAS9-expressing plasmid/gRNA was compared to delivery by lipofectamine 2000 or RNAiMAX.

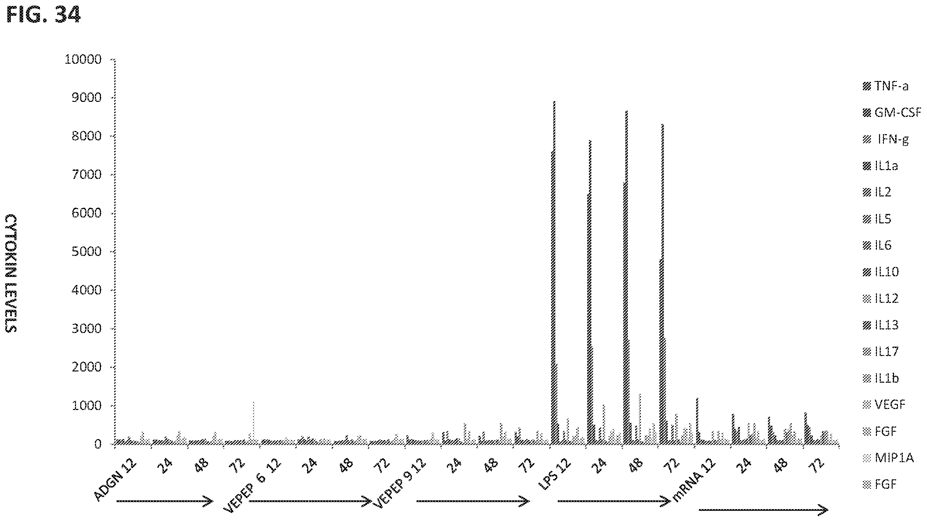

FIG. 34 shows evaluation of non-specific cytokine induction associated with *in vivo* administration of CPP/mRNA complexes. Mice were injected with a single intravenous injection of CAS9 mRNA/ADGN-100a, CAS9 mRNA/VEPEP-6, or CAS9 mRNA/VEPEP-9. Serum cytokine levels were evaluated at different time points using The Cytokine Mouse Magnetic 20-Plex Panel. Control included administration of LPS or free mRNA. For each time point, bars from left to right correspond to TNF-a, GM-CSF, IFN-γ, IL1α, IL2, IL5, IL6, IL10, IL12 (p40/p70), IL13, IL17, IL1β, VEGF, FGF, MIP-1a, and FGF, respectively.

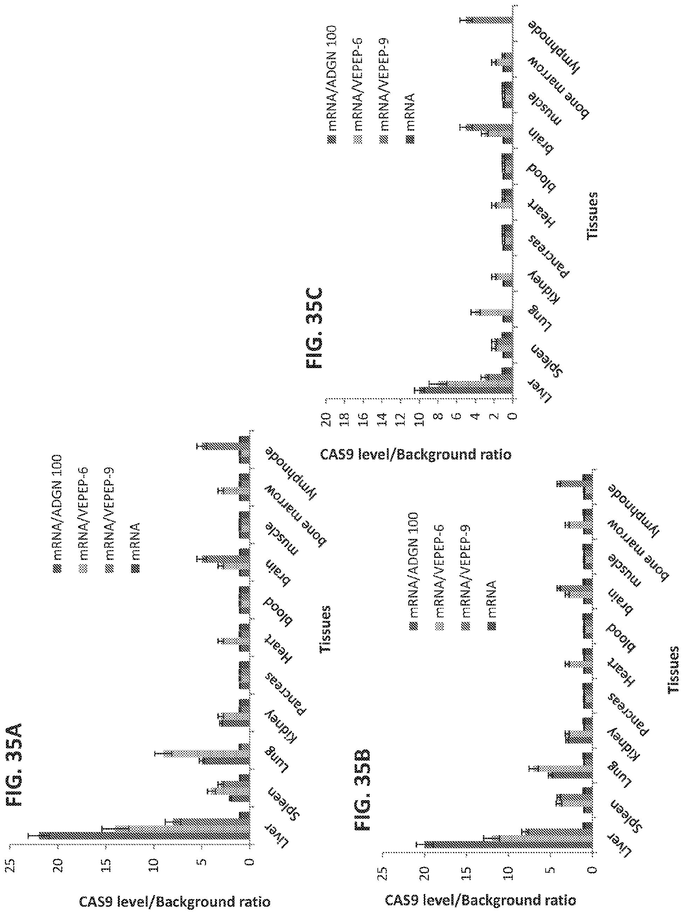

FIGS. 35A-35C show quantification of CAS9 protein expression by ELISA in various tissues at 3 days (FIG. 35A), 6 days (FIG. 35B), and 10 days (FIG. 35C) after *in vivo* administration of CAS9 mRNA (5 µg) associated with ADGN-100a, VEPEP-6, or VEPEP-9 peptides at a 20:1 molar ratio of CPP to mRNA. Tissues were homogenized and CAS9 expression was analyzed by ELISA on protein extract using an antibody against CRISPR/Cas9.

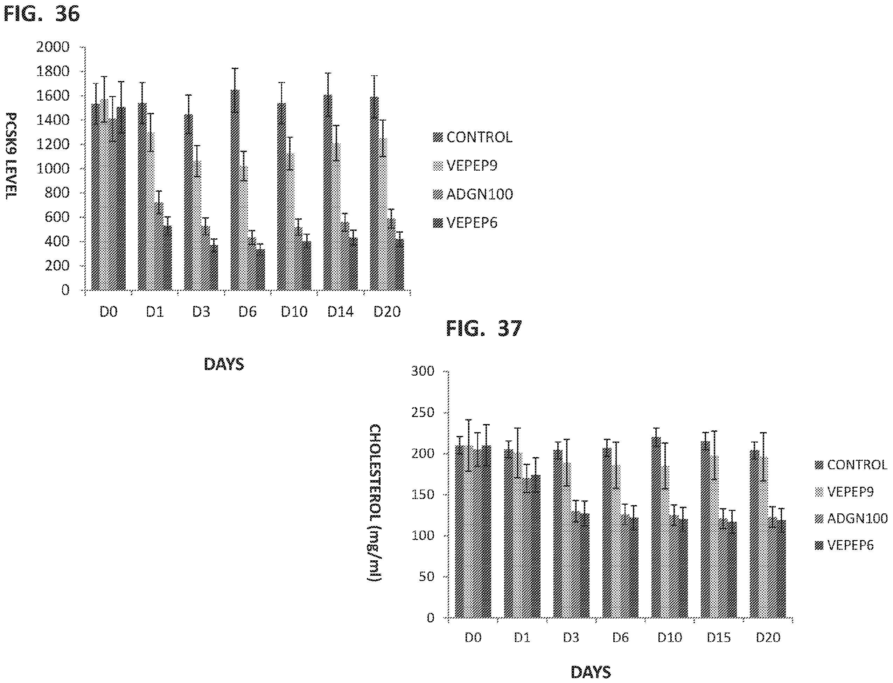

FIG. 36 shows quantification of serum PCSK9 protein levels at different time points after *in vivo* administration of CAS9 mRNA (5 µg)/gRNA targeting PCSK9 exon 1 associated with ADGN-100a, VEPEP-6, or VEPEP-9 peptides at a 20:1 molar ratio of CPP to nucleic acid (mRNA + gRNA). Serum levels of PCSK9 were determined by ELISA using the Mouse Proprotein Convertase 9/PCSK9 Quantikine ELISA Kit (MPC-900, R&DSystems).
FIG. 37 shows quantification of total serum cholesterol levels at different time points after *in vivo* administration of CAS9 mRNA (5 µg)/gRNA targeting PCSK9 exon 1 associated with ADGN-100a, VEPEP-6, or VEPEP-9 peptides at a 20:1 molar ratio of CPP to nucleic acid (mRNA+ gRNA). Total serum cholesterol levels were measured using the Infinity Cholesterol Reagent (Thermo Fisher).

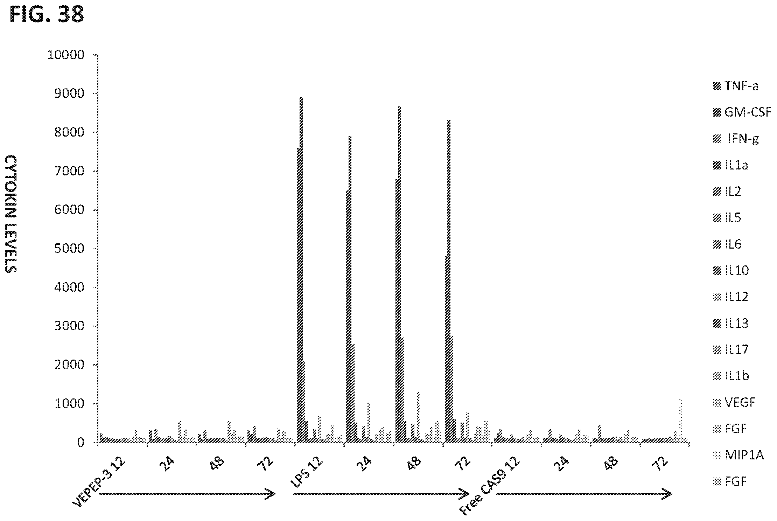

FIG. 38 shows evaluation of non-specific cytokine induction associated with *in vivo* administration of VEPEP-3a/CAS9 protein complexes. Mice were injected with a single intravenous injection of CAS9 /VEPEP-3a. Serum cytokine levels were evaluated at different time points using The Cytokine Mouse Magnetic 20-Plex Panel. Controls included administration of free cas9 protein or LPS. For each time point, bars from left to right correspond to TNF-a, GM-CSF, IFN-γ, IL1α, IL2, IL5, IL6, IL10, IL12 (p40/p70), IL13, IL17, IL1β, VEGF, FGF, MIP-1a, and FGF, respectively.

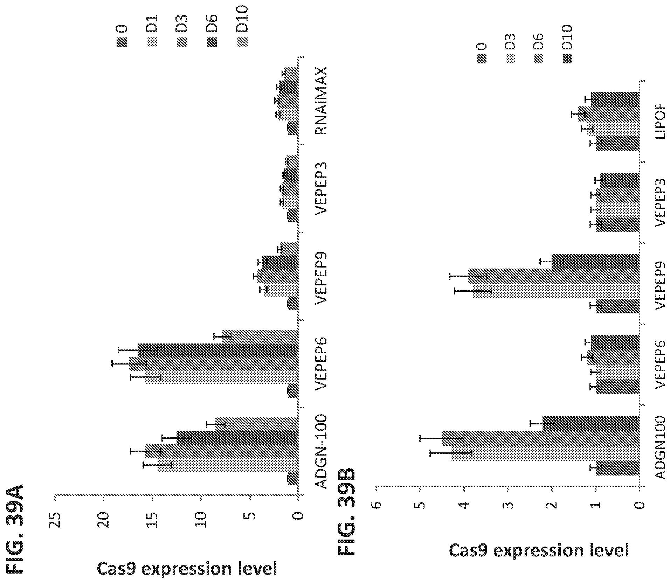

FIGS. 39A and 39B show quantification of CAS9 protein expression as determined by ELISA in primary human T cells. FIG. 39A corresponds to T cells transfected with CAS9 mRNA(0.5 µg) using ADGN-100a, VEPEP-6, VEPEP-9, or VEPEP-3a peptides, or RNAiMAX. FIG. 39B corresponds to T cells transfected with CAS9 expressing plasmid (0.5 µg) using ADGN-100a, VEPEP-6, VEPEP-9, or VEPEP-3a peptides, or lipofectamine 2000.

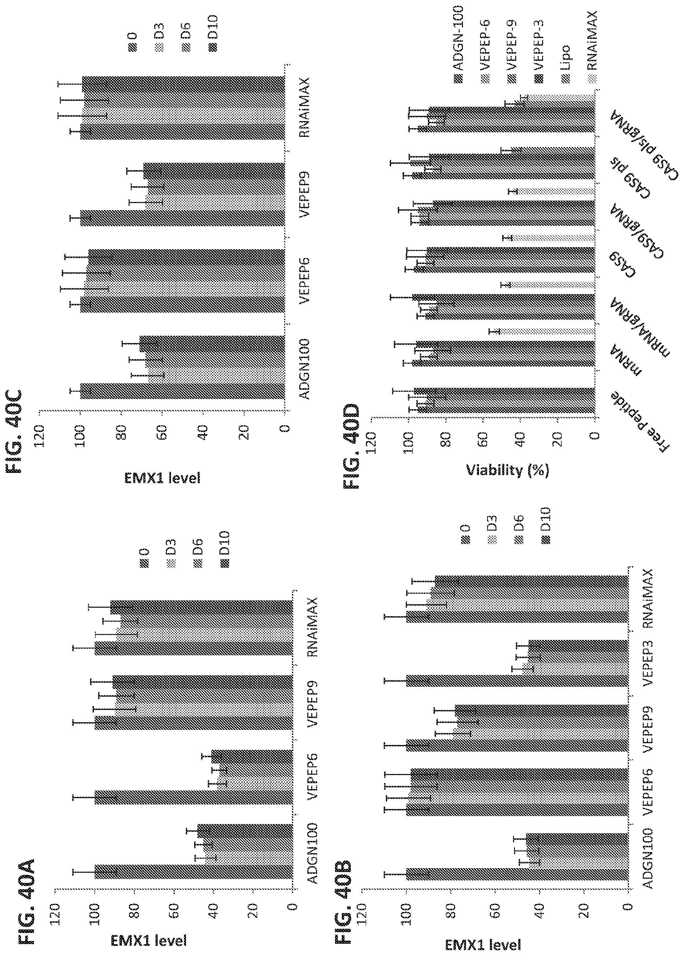

FIGS. 40A-40D show the gene disruption frequency of the endogenous *EMX1* gene in human primary T cells mediated by RGEN/gRNA, as quantified by T7E1 assay. FIG. 40A corresponds to T cells transfected with CAS9 mRNA/gRNA (0.5 µg/2.5 µg) using ADGN-100a, VEPEP-6, or VEPEP-9 (molar ratio of 20:1 for CPP to cargo), or by RNAiMAX. FIG. 40B corresponds to T cells transfected with preloaded CAS9/gRNA complexes (2.5 µg/5 µg CAS9/gRNA). The complexes were mixed with ADGN-100a, VEPEP-6, VEPEP-9, or VEPEP-3a (molar ratio of 20:1 for CPP to complex) and compared to delivery by RNAiMAX. FIG. 40C corresponds to T cells transfected with CAS9-expressing plasmid (0.5µg) and gRNA (5µg) using ADGN-100a, VEPEP-6, or VEPEP-9 (molar ratio of 20:1 for CPP to cargo), or by lipofectamine 2000/RNAiMAX. FIG 40D corresponds to the toxicity evaluation of CPP in complexes with CAS9 mRNA, CAS9 protein, or CAS9-expressing plasmid (CAS9 pls) either associated or not with a gRNA in human primary T cells as measured by MTT assay. Peptide-mediated delivery was compared to delivery by RNAiMAX or Lipofectamine 2000.

### DETAILED DESCRIPTION OF THE INVENTION

In order for genome-editing techniques (such as designer nuclease-based genome-modification, *e.g.,* CRISPR) to be therapeutically applicable, the genome-editing system molecules (such as CRISPR system molecules) must be efficiently delivered to their targets inside of cells. Adeno-associated viral particles have commonly been used as gene delivery agents, however due to safety issues and loading capacity limits, viral carriers are non-ideal delivery vehicles (Swiech et al., Nat. Biotechnol. 33:102-106, 2015). Non-viral means of delivering genome-editing system molecules include lipid-based vectors, lipid nanoparticles, polymeric vectors, polyethylenimine, and poly(L-lysine) to name a few, though application of genome editing (such as by using CRISPR tools) *in vivo* remains a challenge due to the limitations of the delivery approaches currently being used (Li et al. (2015). Human gene therapy, 26(7):452-462; Wang et al. (2016). Proceedings of the National Academy of Sciences, 113(11):2868-2873). Thus, there is a need for improved methods for simple and efficient delivery of genome-editing system molecules (such as CRISPR system molecules).

The present application provides complexes and nanoparticles comprising a cell-penetrating peptide (CPP) and one or more molecules of a genome-editing system, wherein the CPP is suitable for stabilizing and/or delivering into a cell the one or more genome-editing system molecules. The complexes and nanoparticles may comprise a plurality of genome-editing system molecules, and some of the plurality of genome-editing system molecules may be in pre-formed complexes prior to association with the CPP. The complexes and nanoparticles may comprise the entire genome-editing system (*e.g*., the complexes and nanoparticles may comprise molecules that when delivered to a cell are sufficient for effecting the modification to the genome for which the genome-editing system is designed). Cell-penetrating peptide technology is less complex, less toxic and easier to use than viral vectors or chemical transfection. Genome-editing system molecules are meant to include nucleic acids encoding a molecule of a genome-editing system. For example, the molecules of a genome-editing system may include, for example, a) an enzyme and an RNA, b) the RNA and a nucleic acid encoding the enzyme, c) the enzyme and a nucleic acid encoding the RNA, or d) nucleic acid encoding both the enzyme and the RNA. In some embodiments, the genome-editing system comprises a designer nuclease (or a nucleic acid encoding the designer nuclease, such as an mRNA or a DNA plasmid), such as a zinc-finger nuclease (ZFN), a transcription activator-like effector nuclease (TALEN), a homing endonuclease (such as an ARC nuclease™) or a nucleic acid-guided endonuclease (NGEN), such as an RNA-guided endonuclease (RGEN, *e.g*., Cas9) or a DNA-guided endonuclease (DGEN). In some embodiments, the genome-editing system further comprises a guide nucleic acid (gNA) (or a nucleic acid encoding the guide nucleic acid, such as an mRNA or a DNA plasmid), such as a guide RNA (gRNA) or a guide DNA (gDNA). In some embodiments, the genome-editing system is a clustered regularly interspaced short palindromic repeat (CRISPR) system (including, for example, CRISPR-associated proteins and/or nucleic acids, or nucleic acids encoding one or more of CRISPR-associated proteins and/or nucleic acids). In some embodiments, the genome-editing system comprises a ZFN. In some embodiments, the genome-editing system comprises a TALEN. In some embodiments, the genome-editing system comprises a homing endonuclease. In some embodiments, the genome-editing system comprises an integrase (or a nucleic acid encoding the integrase, such as an mRNA or a DNA plasmid). In some embodiments, the genome-editing system further comprises a donor nucleic acid comprising a recombination site recognized by the integrase.

Thus, the present application in one aspect provides novel genome-editing complexes and nanoparticles which are described further below in more detail.

In another aspect, there are provided methods of delivering genome-editing system molecules into a cell using the cell-penetrating peptides.

Also provided are pharmaceutical compositions comprising a cell-penetrating peptide and one or more molecules of a genome-editing system (for example in the forms of complexes and nanoparticles) and uses thereof for treating diseases.

### Definitions

In aspects of the invention the term "single guide RNA" or "sgRNA" refers to a polynucleotide sequence comprising a guide sequence, a tracr sequence and a tracr mate sequence. The term "guide sequence" refers to the about 20 bp sequence within the guide RNA that specifies the target site. The term "tracr mate sequence" may also be used interchangeably with the term "direct repeat(s)".

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%/, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993). Laboratory Techniques In Biochemistry And Molecular Biology-Hybridization With Nucleic Acid Probes Part I, Second Chapter "Overview of principles of hybridization and the strategy of nucleic acid probe assay". Elsevier, N.Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating" refers to an approach for obtaining beneficial or desired results including but not limited to a therapeutic benefit. By therapeutic benefit is meant any therapeutically relevant improvement in or effect on one or more diseases, conditions, or symptoms under treatment.

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

As used herein, the singular form "a", "an", and "the" includes plural references unless indicated otherwise.

Reference to "about" a value or parameter herein includes (and describes) embodiments that are directed to that value or parameter per se. For example, description referring to "about X" includes description of "X."

The compositions and methods of the present invention may comprise, consist of, or consist essentially of the essential elements and limitations of the invention described herein, as well as any additional or optional ingredients, components, or limitations described herein or otherwise useful.

Unless otherwise noted, technical terms are used according to conventional usage.

### Complexes and nanoparticles

In some aspects, the invention provides complexes and nanoparticles comprising cell-penetrating peptides for delivering one or more molecules of a genome-editing system into a cell. In some embodiments, cell-penetrating peptides are complexed with a genome-editing nuclease (or a nucleic acid encoding the genome-editing nuclease, such as an mRNA or DNA plasmid). In some embodiments, the genome-editing nuclease is a ZFN, TALEN, homing endonuclease, RGEN (*e.g.,* Cas9), or DGEN. In some embodiments, the genome-editing nuclease is a ZFN. In some embodiments, the genome-editing nuclease is a TALEN. In some embodiments, the genome-editing nuclease is a nucleic acid-guided nuclease (*e.g.*, an RGEN or DGEN), and the cell-penetrating peptides are complexed with the nucleic acid-guided nuclease (or a nucleic acid encoding the nucleic acid-guided nuclease) in combination with (such as complexed with) a guide sequence (or a nucleic acid encoding a guide sequence) to form a genome-editing complex or nanoparticle capable of being delivered to a cell. In some embodiments, the genome-editing complex or nanoparticle comprises a cell-penetrating peptide and a ZFN or TALEN, or a nucleic acid encoding the ZFN or TALEN. In some embodiments, the genome-editing complex or nanoparticle comprises a cell-penetrating peptide, an RGEN or a nucleic acid encoding the RGEN, and a guide RNA (gRNA) or a nucleic acid encoding the gRNA. In some embodiments, the genome-editing complex or nanoparticle comprises a cell-penetrating peptide, a DGEN or a nucleic acid encoding the DGEN, and a guide DNA (gDNA) or a nucleic acid encoding the gDNA. In some embodiments, the cell-penetrating peptide and the genome-editing nuclease are covalently attached. In some embodiments, the RGEN and gRNA or DGEN and gDNA are in a pre-formed complex prior to association with the cell-penetrating peptide to form the genome-editing complex. In some embodiments, cell-penetrating peptides are complexed with a genome-editing integrase (or a nucleic acid encoding the genome-editing integrase). In some embodiments, the genome-editing integrase is a bacteriophage integrase. In some embodiments, the cell-penetrating peptides are complexed with the genome-editing integrase (or a nucleic acid encoding the genome-editing integrase) in combination with (such as complexed with) a donor nucleic acid comprising a recombination site recognized by the integrase to form a genome-editing complex or nanoparticle capable of being delivered to a cell. The cell-penetrating peptides are capable of forming stable complexes and nanoparticles with genome-editing system molecules (*e.g*., a Cas nuclease and/or guide RNA, or nucleic acids encoding a Cas nuclease and/or guide RNA). In some embodiments, the genome-editing complex or nanoparticle comprises the entire genome-editing system (e.g., delivery of the molecules in the genome-editing complex or nanoparticle into a cell is sufficient to effect genome editing in the cell).

In some aspects, the invention provides complexes and nanoparticles comprising cell-penetrating peptides for delivering a ZFP or ZFN to a host cell. In some embodiments, cell-penetrating peptides are complexed with a ZFP or ZFN (or a nucleic acid encoding the ZFP or ZFN) to form a genome-editing complex or nanoparticle capable of being delivered to a cell. The cell-penetrating peptides are capable of forming stable complexes and nanoparticles with the ZFP or ZFN or nucleic acids encoding the ZFP or ZFN. In some embodiments, the genome-editing complex or nanoparticle comprises a cell-penetrating peptide and a ZFP or ZFN or a nucleic acid encoding the ZFP or ZFN. In some embodiments, the cell-penetrating peptide and the ZFP or ZFN are covalently attached.

In some aspects, the invention provides complexes and nanoparticles comprising cell-penetrating peptides for delivering a TALE or TALEN to a host cell. In some embodiments, cell-penetrating peptides are complexed with a TALE or TALEN (or a nucleic acid encoding the TALE or TALEN) to form a genome-editing complex or nanoparticle capable of being delivered to a cell. The cell-penetrating peptides are capable of forming stable complexes and nanoparticles with the TALE or TALEN or nucleic acids encoding the TALE or TALEN. In some embodiments, the genome-editing complex or nanoparticle comprises a cell-penetrating peptide and a TALE or TALEN or a nucleic acid encoding the TALE or TALEN. In some embodiments, the cell-penetrating peptide and the TALE or TALEN are covalently attached.

In some aspects, the invention provides complexes and nanoparticles comprising cell-penetrating peptides for delivering one or more CRISPR system molecules to a host cell. In some embodiments, cell-penetrating peptides are complexed with a CRISPR nuclease (e.g., Cas9), or a nucleic acid encoding the CRISPR nuclease, optionally in combination with (such as complexed with) a guide sequence (or a nucleic acid encoding the guide sequence) to form a genome-editing complex or nanoparticle capable of being delivered to a cell. The cell-penetrating peptides are capable of forming stable complexes and nanoparticles with CRISPR system molecules (*e.g.,* Cas nuclease and/or guide RNA) and/or nucleic acids encoding one or more of the CRISPR system molecules. In some embodiments, the genome-editing complex or nanoparticle comprises a cell-penetrating peptide and a) an RGEN (*e.g*., Cas9) or a nucleic acid encoding the RGEN and/or b) a gRNA or a nucleic acid encoding the gRNA. In some embodiments, the cell-penetrating peptide and the RGEN are covalently attached. In some embodiments, the RGEN and gRNA are in a pre-formed complex prior to association with the cell-penetrating peptide to form the genome-editing complex or nanoparticle. In some embodiments, the genome-editing complex or nanoparticle comprises the entire CRISPR system For example, in some embodiments, the genome-editing complex or nanoparticle comprises a cell-penetrating peptide, an RGEN (*e.g*., Cas9) or a nucleic acid encoding the RGEN, and a gRNA or a nucleic acid encoding the gRNA.

In some aspects, the invention provides complexes and nanoparticles comprising cell-penetrating peptides for delivering one or more molecules of a DGEN system to a host cell, such as the system described in Gao et al. (2016). Nature biotechnology*,* which uses a DNA-guided genome editing system comprising the *Natronobacterium gregoryi* Argonaute (NgAgo) and a guide DNA (gDNA). In some embodiments, cell-penetrating peptides are complexed with a DGEN (or a nucleic acid encoding the DGEN) in combination with (such as complexed with) a guide sequence (or a nucleic acid encoding the guide sequence) to form a genome-editing complex or nanoparticle capable of being delivered to a cell. The cell-penetrating peptides are capable of forming stable complexes and nanoparticles with DGEN system molecules (*e.g*., NgAgo nuclease and/or guide DNA) and/or nucleic acids encoding one or more of the DGEN system molecules. In some embodiments, the genome-editing complex or nanoparticle comprises a cell-penetrating peptide, a DGEN or a nucleic acid encoding the DGEN, and a gDNA or a nucleic acid encoding the gDNA. In some embodiments, the cell-penetrating peptide and the DGEN are covalently attached. In some embodiments, the DGEN and gDNA are in a pre-formed complex prior to association with the cell-penetrating peptide to form the genome-editing complex. In some embodiments, the genome-editing complex or nanoparticle comprises the entire DGEN system For example, in some embodiments, the genome-editing complex or nanoparticle comprises a cell-penetrating peptide, a DGEN or a nucleic acid encoding the DGEN, and a gDNA or a nucleic acid encoding the gDNA.

In some aspects, the invention provides complexes and nanoparticles comprising cell-penetrating peptides for delivering an integrase to a host cell. In some embodiments, cell-penetrating peptides are complexed with an integrase (or a nucleic acid encoding the integrase) to form a genome-editing complex or nanoparticle capable of being delivered to a cell. In some embodiments, the cell-penetrating peptides are complexed with the integrase (or a nucleic acid encoding the integrase) in combination with (such as complexed with) a donor nucleic acid comprising a recombination site recognized by the integrase to form a genome-editing complex or nanoparticle capable of being delivered to a cell. The cell-penetrating peptides are capable of forming stable complexes and nanoparticles with the integrase or nucleic acid encoding the integrase. In some embodiments, the genome-editing complex or nanoparticle comprises a cell-penetrating peptide and an integrase or a nucleic acid encoding the integrase. In some embodiments, the cell-penetrating peptide and the integrase are covalently attached. In some embodiments, the integrase and donor nucleic acid are in a pre-formed complex prior to association with the cell-penetrating peptide to form the genome-editing complex or nanoparticle.

### Cell-penetrating peptides

The cell-penetrating peptides in the genome-editing complexes or nanoparticles of the present invention are capable of forming stable complexes and nanoparticles with various molecules of a genome-editing system, such as nucleases (*e.g.,* ZFNs, TALENs, and CRISPR-associated nucleases (such as Cas9 and Cpfl)), integrases (such as bacteriophage integrases, *e.g*., ΦC31), and nucleic acids (*e.g.,* guide RNAs, guide DNAs, and donor nucleic acids). Any of the cell-penetrating peptides in any of the genome-editing complexes or nanoparticles described herein may comprise or consist of any of the cell-penetrating peptide sequences described in this section.

In some embodiments, a genome-editing complex or nanoparticle described herein comprises a cell-penetrating peptide selected from the group consisting of CADY, PEP-1, MPG, VEPEP-3 peptides, VEPEP-4 peptides, VEPEP-5 peptides, VEPEP-6 peptides, VEPEP-9 peptides, and ADGN-100 peptides. In some embodiments, the cell-penetrating peptide is present in a genome-editing complex. In some embodiments, the cell-penetrating peptide is present in a genome-editing complex present in the core of a nanoparticle. In some embodiments, the cell-penetrating peptide is present in the core of a nanoparticle. In some embodiments, the cell-penetrating peptide is present in the core of a nanoparticle and is associated with a ZFN, TALEN, homing endonuclease, RGEN (*e.g*., Cas9), DGEN, or integrase. In some embodiments, the cell-penetrating peptide is present in the core of a nanoparticle and is associated with a gRNA or gDNA. In some embodiments, the cell-penetrating peptide is present in the core of a nanoparticle and is associated with an RGEN/gRNA complex or a DGEN/gDNA complex. In some embodiments, the cell-penetrating peptide is present in the core of a nanoparticle and is associated with a donor nucleic acid. In some embodiments, the cell-penetrating peptide is present in an intermediate layer of a nanoparticle. In some embodiments, the cell-penetrating peptide is present in the surface layer of a nanoparticle. In some embodiments, the cell-penetrating peptide is linked to a targeting moiety. In some embodiments, the linkage is covalent. WO2014/053879 discloses VEPEP-3 peptides; WO2014/053881 discloses VEPEP-4 peptides; WO2014/053882 discloses VEPEP-5 peptides; WO2012/137150 discloses VEPEP-6 peptides; WO2014/053880 discloses VEPEP-9 peptides; WO 2016/102687 discloses ADGN-100 peptides; US2010/0099626 discloses CADY peptides; and. U.S. Pat. No. 7,514,530 discloses MPG peptides; the disclosures of which are hereby incorporated herein by reference in their entirety.

In some embodiments, a genome-editing complex or nanoparticle described herein comprises a VEPEP-3 cell-penetrating peptide comprising the amino acid sequence X₁X₂X₃X₄X₅X₂X₃X₄X₆X₇X₃X₈X₉X₁₀X₁₁X₁₂X₁₃ (SEQ ID NO: 1), wherein X₁ is beta-A or S, X₂ is K, R or L (independently from each other), X₃ is F or W (independently from each other), X₄ is F, W or Y (independently from each other), X₅ is E, R or S, X₆ is R, T or S, X₇ is E, R, or S, X₈ is none, F or W, X₉ is P or R, X₁₀ is R or L, X₁₁ is K, W or R, X₁₂ is R or F, and X₁₃ is R or K. In some embodiments, the VEPEP-3 peptide comprises the amino acid sequence X₁X₂WX₄EX₂WX₄X₆X₇X₃PRX₁₁RX₁₃ (SEQ ID NO: 2), wherein X₁ is beta-A or S, X₂ is K, R or L, X₃ is F or W, X₄ is F, W or Y, X₅ is E, R or S, X₆ is R, T or S, X₇ is E, R, or S, X₈ is none, F or W, X₉ is P or R, X₁₀ is R or L, X₁₁ is K, W or R, X₁₂ is R or F, and X₁₃ is R or K. In some embodiments, the VEPEP-3 peptide comprises the amino acid sequence X₁KWFERWFREWPRKRR (SEQ ID NO: 3), X₁KWWERWWREWPRKRR (SEQ ID NO: 4), X₁KWWERWWREWPRKRK (SEQ ID NO: 5), X₁RWWEKWWTRWPRKRK (SEQ ID NO: 6), or X₁RWYEKWYTEFPRRRR (SEQ ID NO: 7), wherein X₁ is beta-A or S. In some embodiments, the VEPEP-3 peptide comprises the amino acid sequence of any one of SEQ ID NOs: 1-7, wherein the cell-penetrating peptide is modified by replacement of the amino acid in position 10 by a non-natural amino acid, addition of a non-natural amino acid between the amino acids in positions 2 and 3, and addition of a hydrocarbon linkage between the two non-natural amino acids. In some embodiments, the VEPEP-3 peptide comprises the amino acid sequence X₁KX₁₄WWERWWRX₁₄WPRKRK (SEQ ID NO: 8), wherein X₁ is beta-A or S and X₁₄ is a non-natural amino acid, and wherein there is a hydrocarbon linkage between the two non-natural amino acids. In some embodiments, the VEPEP-3 peptide comprises the amino acid sequence X₁X₂X₃WX₅X₁₀X₃WX₆X₇WX₈X₉X₁₀WX₁₂R (SEQ ID NO: 9), wherein X₁ is beta-A or S, X₂ is K, R or L, X₃ is F or W, X₅ is R or S, X₆ is R or S, X₇ is R or S, X₈ is F or W, X₉ is R or P, X₁₀ is L or R, and X₁₂ is R or F. In some embodiments, the VEPEP-3 peptide comprises the amino acid sequence X₁RWWRLWWRSWFRLWRR (SEQ ID NO: 10), X₁LWWRRWWSRWWPRWRR (SEQ ID NO: 11), X₁LWWSRWWRSWFRLWFR (SEQ ID NO: 12), or X₁KFWSRFWRSWFRLWRR (SEQ ID NO: 13), wherein X₁ is beta-A or S. In some embodiments, the VEPEP-3 peptide comprises the amino acid sequence of any one of SEQ ID NOs: 1 and 9-13, wherein the cell-penetrating peptide is modified by replacement of the amino acids in position 5 and 12 by non-natural amino acids, and addition of a hydrocarbon linkage between the two non-natural amino acids. In some embodiments, the VEPEP-3 peptide comprises the amino acid sequence X₁RWWX₁₄ WX₁₄RLWRR (SEQ ID NO: 14), wherein X₁ is a beta-alanine or a serine and X₁₄ is a non-natural amino acid, and wherein there is a hydrocarbon linkage between the two non-natural amino acids. In some embodiments, the VEPEP-3 peptide is present in a genome-editing complex. In some embodiments, the VEPEP-3 peptide is present in a genome-editing complex in the core of a nanoparticle. In some embodiments, the VEPEP-3 peptide is present in the core of a nanoparticle. In some embodiments, the VEPEP-3 peptide is present in the core of a nanoparticle and is associated with a ZFN, TALEN, homing endonuclease, RGEN (*e.g*., Cas9), DGEN, or integrase. In some embodiments, the VEPEP-3 peptide is present in the core of a nanoparticle and is associated with a gRNA or gDNA. In some embodiments, the VEPEP-3 peptide is present in the core of a nanoparticle and is associated with an RGEN/gRNA complex or a DGEN/gDNA complex. In some embodiments, the VEPEP-3 peptide is present in the core of a nanoparticle and is associated with a donor nucleic acid. In some embodiments, the VEPEP-3 peptide is present in an intermediate layer of a nanoparticle. In some embodiments, the VEPEP-3 peptide is present in the surface layer of a nanoparticle. In some embodiments, the VEPEP-3 peptide is linked to a targeting moiety. In some embodiments, the linkage is covalent.

In some embodiments, a genome-editing complex or nanoparticle described herein comprises a VEPEP-6 cell-penetrating peptide. In some embodiments, the VEPEP-6 peptide comprises an amino acid sequence selected from the group consisting of X₁LX₂RALWX₉LX₃X₉X₄LWX₉LX₅X₆X₇X₈ (SEQ ID NO: 15), X₁LX₂LARWX₉LX₃X₉X₄LWX₉LX₅X₆X₇X₈ (SEQ ID NO: 16) and X₁LX₂ARLWX₉LX₃X₉X₄LWX₉LX₅X₆X₇X₈ (SEQ ID NO: 17), wherein X₁ is beta-A or S, X₂ is F or W, X₃ is L, W, C or I, X₄ is S, A,N orT, X₅ isL orW, X₆ isW orR, X₇ isK orR, X₈ isAor none, and X₉ is R or S. In some embodiments, the VEPEP-6 peptide comprises the amino acid sequence X₁LX₂RALWRLX₃RX₄LWRLX₅X₆X₇X₈ (SEQ ID NO: 18), wherein X₁ is beta-A or S, X₂ is F or W, X₃ is L, W, C or I, X₄ is S, A, N or T, X₅ is L or W, X₆ is W or R, X₇ is K or R, and X₈ is A or none. In some embodiments, the VEPEP-6 peptide comprises the amino acid sequence X₁LX₂RALWRLX₃RX₄LWRLX₅X₆KX₇ (SEQ ID NO: 19), wherein X₁ is beta-A or S, X₂ is F or W, X₃ is L or W, X₄ is S, A or N, X₅ is L or W, X₆ is W or R, X₇ is A or none. In some embodiments, the VEPEP-6 peptide comprises an amino acid sequence selected from the group consisting of X₁LFRALWRLLRX₂LWRLLWX₃ (SEQ ID NO: 20), X₁LWRALWRLWRX₂LWRLLWX₃A (SEQ ID NO: 21), X₁LWRALWRLX₄RX₂LWRLWRX₃A (SEQ ID NO: 22), X₁LWRALWRLWRX₂LWRLWRX₃A (SEQ ID NO: 23), X₁LWRALWRLX₂RALWRLLWX₃A (SEQ ID NO: 24), and X₁LWRALWRLX₄RNLWRLLWX₃A (SEQ ID NO: 25), wherein X₁ is beta-A or S, X₂ is S or T, X₃ is K or R, X₄ is L, C or I and X₅ is L or I. In some embodiments, the VEPEP-6 peptide comprises an amino acid sequence selected from the group consisting of Ac-X₁LFRALWRLLRSLWRLLWK-cysteamide (SEQ ID NO: 26), Ac-X₁LWRALWRLWRSLWRLLWKA-cysteamide (SEQ ID NO: 27), Ac-X₁LWRALWRLLRSLWRLWRKA-cysteamide (SEQ ID NO: 28), Ac-X₁LWRALWRLWRSLWRLWRKA-cysteamide (SEQ ID NO: 29), Ac-X₁LWRALWRLLRALWRLLWKA-cysteamide (SEQ ID NO: 30), and Ac-X₁LWRALWRLLRNLWRLLWKA-cysteamide (SEQ ID NO: 31), wherein X₁ is beta-A or S. In some embodiments, the VEPEP-6 peptide comprises the amino acid sequence of any one of SEQ ID NOs: 15-31, further comprising a hydrocarbon linkage between two residues at positions 8 and 12. In some embodiments, the VEPEP-6 peptide comprises an amino acid sequence selected from the group consisting of Ac-X₁LFRALWR_{S}LLRS_{S}LWRLLWK-cysteamide (SEQ ID NO: 32), Ac-X₁LFLARWR_{S}LLRS_{S}LWRLLWK-cysteamide (SEQ ID NO: 33), Ac-X₁LFRALWS_{S}LLRS_{S}LWRLLWK-cysteamide (SEQ ID NO: 34), Ac-X₁LFLARWS_{S}LLRS_{S}LWRLLWK-cysteamide (SEQ ID NO: 35), Ac-X₁LFRALWRLLR_{S}SLWS_{S}LLWK-cysteamide (SEQ ID NO: 36), Ac-X₁LFLARWRLLR_{S}SLWS_{S}LLWK-cysteamide (SEQ ID NO: 37), Ac-X₁LFRALWRLLS_{S}SLWS_{S}LLWK-cysteamide (SEQ ID NO: 38), Ac-X₁LFLARWRLLS_{S}SLWS_{S}LLWK-cysteamide (SEQ ID NO: 39), and Ac-X₁LFAR_{S}LWRLLRS_{S}LWRLLWK-cysteamide (SEQ ID NO: 40), wherein X₁ is beta-A or S and wherein the residues followed by an inferior "S" are those which are linked by said hydrocarbon linkage. In some embodiments, the VEPEP-6 peptide is present in a genome-editing complex. In some embodiments, the VEPEP-6 peptide is present in a genome-editing complex in the core of a nanoparticle. In some embodiments, the VEPEP-6 peptide is present in the core of a nanoparticle. In some embodiments, the VEPEP-6 peptide is present in the core of a nanoparticle and is associated with a ZFN, TALEN, homing endonuclease, RGEN (*e.g.*, Cas9), DGEN, or integrase. In some embodiments, the VEPEP-6 peptide is present in the core of a nanoparticle and is associated with a gRNA or gDNA. In some embodiments, the VEPEP-6 peptide is present in the core of a nanoparticle and is associated with an RGEN/gRNA complex or a DGEN/gDNA complex. In some embodiments, the VEPEP-6 peptide is present in the core of a nanoparticle and is associated with a donor nucleic acid. In some embodiments, the VEPEP-6 peptide is present in an intermediate layer of a nanoparticle. In some embodiments, the VEPEP-6 peptide is present in the surface layer of a nanoparticle. In some embodiments, the VEPEP-6 peptide is linked to a targeting moiety. In some embodiments, the linkage is covalent.

In some embodiments, a genome-editing complex or nanoparticle described herein comprises a VEPEP-9 cell-penetrating peptide comprising the amino acid sequence X₁X₂X₃WWX₄X₅WAX₆X₃X₇X₈X₉X₁₀X₁₁X₁₂WX₁₃R (SEQ ID NO: 41), wherein X₁ is beta-A or S, X₂ is L or none, X₃ is R or none, X₄ is L, R or G, X₅ is R, W or S, X₆ is S, P or T, X₇ is W or P, X₈ is F, A or R, X₉ is S, L, P or R, X₁₀ is R or S, X₁₁ is W or none, X₁₂ is A, R or none and X₁₃ is W or F, and wherein if X₃ is none, then X₂, X₁₁ and X₁₂ are none as well. In some embodiments, the VEPEP-9 peptide comprises the amino acid sequence X₁X₂RWWLRWAX₆RWX₈X₉X₁₀WX₁₂WX₁₃R (SEQ ID NO: 42), wherein X₁ is beta-A or S, X₂ is L or none, X₆ is S or P, X₈ is F or A, X₉ is S, L or P, X₁₀ is R or S, X₁₂ is A or R, and X₁₃ is W or F. In some embodiments, the VEPEP-9 peptide comprises an amino acid sequence selected from the group consisting of X₁LRWWLRWASRWFSRWAWWR (SEQ ID NO: 43), X₁LRWWLRWASRWASRWAWFR (SEQ ID NO: 44), X₁RWWLRWASRWALSWRWWR (SEQ ID NO: 45), X₁RWWLRWASRWFLSWRWWR (SEQ ID NO: 46), X₁RWWLRWAPRWFPSWRWWR (SEQ ID NO: 47), and X₁RWWLRWASRWAPSWRWWR (SEQ ID NO: 48), wherein X₁ is beta-A or S. In some embodiments, the VEPEP-9 peptide comprises the amino acid sequence of X₁WWX₄X₅WAX₆X₇X₈RX₁₀WWR (SEQ ID NO: 49), wherein X₁ is beta-A or S, X₄ is R or G, X₅ is W or S, X₆ is S, T or P, X₇ is W or P, X₈ is A or R, and X₁₀ is S or R. In some embodiments, the VEPEP-9 peptide comprises an amino acid sequence selected from the group consisting of X₁WWRWWASWARSWWR (SEQ ID NO: 50), X₁WWGSWATPRRRWWR (SEQ ID NO: 51), and X₁WWRWWAPWARSWWR (SEQ ID NO: 52), wherein X₁ is beta-A or S. In some embodiments, the VEPEP-9 peptide is present in a genome-editing complex. In some embodiments, the VEPEP-9 peptide is present in a genome-editing complex in the core of a nanoparticle. In some embodiments, the VEPEP-9 peptide is present in the core of a nanoparticle. In some embodiments, the VEPEP-9 peptide is present in the core of a nanoparticle and is associated with a ZFN, TALEN, homing endonuclease, RGEN (*e.g*., Cas9), DGEN, or integrase. In some embodiments, the VEPEP-9 peptide is present in the core of a nanoparticle and is associated with a gRNA or gDNA. In some embodiments, the VEPEP-9 peptide is present in the core of a nanoparticle and is associated with an RGEN/gRNA complex or a DGEN/gDNA complex. In some embodiments, the VEPEP-9 peptide is present in the core of a nanoparticle and is associated with a donor nucleic acid. In some embodiments, the VEPEP-9 peptide is present in an intermediate layer of a nanoparticle. In some embodiments, the VEPEP-9 peptide is present in the surface layer of a nanoparticle. In some embodiments, the VEPEP-9 peptide is linked to a targeting moiety. In some embodiments, the linkage is covalent.

In some embodiments, a genome-editing complex or nanoparticle described herein comprises an ADGN-100 cell-penetrating peptide comprising the amino acid sequence X₁KWRSX₂X₃X₄RWRLWRX₅X₆X₇X₈SR (SEQ ID NO: 53), wherein X₁ is any amino acid or none, and X₂-X₈ are any amino acid. In some embodiments, the ADGN-100 peptide comprises the amino acid sequence X₁KWRSX₂X₃X₄RWRLWRX₅X₆X₇X₈SR (SEQ ID NO: 54), wherein X₁ is βA, S, or none, X₂ is A or V, X₃ is or L, X₄ is W or Y, X₅ is V or S, X₆ is R, V, or A, X₇ is S or L, and X₈ is W or Y. In some embodiments, the ADGN-100 peptide comprises the amino acid sequence KWRSAGWRWRLWRVRSWSR (SEQ ID NO: 55), KWRSALYRWRLWRVRSWSR (SEQ ID NO: 56), KWRSALYRWRLWRSRSWSR (SEQ ID NO: 57), or KWRSALYRWRLWRSALYSR (SEQ ID NO: 58). In some embodiments, the ADGN-100 peptide comprises two residues separated by three or six residues that are linked by a hydrocarbon linkage. In some embodiments, the ADGN-100 peptide comprises the amino acid sequence KWRS_{S}AGWR_{S}WRLWRVRSWSR (SEQ ID NO: 59), KWR_{S}SAGWRWR_{S}LWRVRSWSR (SEQ ID NO: 60), KWRSAGWR_{S}WRLWRVR_{S}SWSR (SEQ ID NO: 61), KWRS_{S}ALYR_{S}WRLWRSRSWSR (SEQ ID NO: 62), KWR_{S}SALYRWR_{S}LWRSRSWSR (SEQ ID NO: 63), KWRSALYR_{S}WRLWRSR_{S}SWSR (SEQ ID NO: 64), KWRSALYRWR_{S}LWRS_{S}RSWSR (SEQ ID NO: 65), KWRSALYRWRLWRS_{S}RSWS_{S}R (SEQ ID NO: 66), KWR_{S}SALYRWR_{S}LWRSALYSR (SEQ ID NO: 67), KWRS_{S}ALYR_{S}WRLWRSALYSR (SEQ ID NO: 68), KWRSALYRWR_{S}LWRS_{S}ALYSR (SEQ ID NO: 69), or KWRSALYRWRLWRS_{S}ALYS_{S}R (SEQ ID NO: 70), wherein the residues marked with a subscript "S" are linked by a hydrocarbon linkage. In some embodiments, the ADGN-100 peptide is present in a genome-editing complex. In some embodiments, the ADGN-100 peptide is present in a genome-editing complex in the core of a nanoparticle. In some embodiments, the ADGN-100 peptide is present in the core of a nanoparticle. In some embodiments, the ADGN-100 peptide is present in the core of a nanoparticle and is associated with a ZFN, TALEN, homing endonuclease, RGEN (*e.g*., Cas9), DGEN, or integrase. In some embodiments, the ADGN-100 peptide is present in the core of a nanoparticle and is associated with a gRNA or gDNA. In some embodiments, the ADGN-100 peptide is present in the core of a nanoparticle and is associated with an RGEN/gRNA complex or a DGEN/gDNA complex. In some embodiments, the ADGN-100 peptide is present in the core of a nanoparticle and is associated with a donor nucleic acid. In some embodiments, the ADGN-100 peptide is present in an intermediate layer of a nanoparticle. In some embodiments, the ADGN-100 peptide is present in the surface layer of a nanoparticle. In some embodiments, the ADGN-100 peptide is linked to a targeting moiety. In some embodiments, the linkage is covalent.

In some embodiments, the CPP described herein (*e.g*., VEPEP-3 peptide, VEPEP-6 peptide, VEPEP-9 peptide, or ADGN-100 peptide) further comprises one or more moieties linked to the N-terminus of the CPP. In some embodiments, the one or more moieties is covalently linked to the N-terminus of the CPP. In some embodiments, the one or more moieties are selected from the group consisting of an acetyl group, a stearyl group, a fatty acid, a cholesterol, a poly-ethylene glycol, a nuclear localization signal, a nuclear export signal, an antibody or antibody fragment thereof, a peptide, a polysaccharide, and a targeting molecule. In some embodiments, the one or more moieties is an acetyl group and/or a stearyl group. In some embodiments, the CPP comprises an acetyl group and/or a stearyl group linked to its N-terminus. In some embodiments, the CPP comprises an acetyl group linked to its N-terminus. In some embodiments, the CPP comprises a stearyl group linked to its N-terminus. In some embodiments, the CPP comprises an acetyl group and/or a stearyl group covalently linked to its N-terminus. In some embodiments, the CPP comprises an acetyl group covalently linked to its N-terminus. In some embodiments, the CPP comprises a stearyl group covalently linked to its N-terminus.

In some embodiments, the CPP described herein (*e.g*., VEPEP-3 peptide, VEPEP-6 peptide, VEPEP-9 peptide, or ADGN-100 peptide) further comprises one or more moieties linked to the C-terminus of the CPP. In some embodiments, the one or more moieties is covalently linked to the C-terminus of the CPP. In some embodiments, the one or more moieties are selected from the group consisting of a cysteamide group, a cysteine, a thiol, an amide, a nitrilotriacetic acid, a carboxyl group, a linear or ramified C₁-C₆ alkyl group, a primary or secondary amine, an osidic derivative, a lipid, a phospholipid, a fatty acid, a cholesterol, a poly-ethylene glycol, a nuclear localization signal, a nuclear export signal, an antibody or antibody fragment thereof, a peptide, a polysaccharide, and a targeting molecule. In some embodiments, the one or more moieties is a cysteamide group. In some embodiments, the CPP comprises a cysteamide group linked to its C-terminus. In some embodiments, the CPP comprises a cysteamide group covalently linked to its C-terminus.

In some embodiments, the CPP described herein (*e.g*., VEPEP-3 peptide, VEPEP-6 peptide, VEPEP-9 peptide, or ADGN-100 peptide) is stapled. "Stapled" as used herein refers to a chemical linkage between two residues in a peptide. In some embodiments, the CPP is stapled, comprising a chemical linkage between two amino acids of the peptide. In some embodiments, the two amino acids linked by the chemical linkage are separated by 3 or 6 amino acids. In some embodiments, two amino acids linked by the chemical linkage are separated by 3 amino acids. In some embodiments, the two amino acids linked by the chemical linkage are separated by 6 amino acids. In some embodiments, each of the two amino acids linked by the chemical linkage is R or S. In some embodiments, each of the two amino acids linked by the chemical linkage is R In some embodiments, each of the two amino acids linked by the chemical linkage is S. In some embodiments, one of the two amino acids linked by the chemical linkage is R and the other is S. In some embodiments, the chemical linkage is a hydrocarbon linkage.

### Complexes

In some embodiments, there is provided a genome-editing complex for modifying a target polynucleotide comprising a cell-penetrating peptide and one or more molecules of a genome-editing system (including nucleic acids encoding any of the one or more genome-editing system molecules). In some embodiments, the genome-editing complex comprises a cell-penetrating peptide (*e.g*., a VEPEP-3, VEPEP-6, VEPEP-9, or ADGN-100 peptide) and a genome-editing nuclease (or a nucleic acid encoding the genome-editing nuclease). In some embodiments, the genome-editing nuclease is a ZFN, TALEN, homing endonuclease, RGEN (*e.g*., Cas9), or DGEN. In some embodiments, the genome-editing complex further comprises a gRNA or gDNA (or nucleic acid encoding the gRNA or gDNA), wherein the gRNA or gDNA comprises a guide sequence complementary to a target sequence in the target polynucleotide. In some embodiments, the genome-editing complex comprises the cell-penetrating peptide associated with a pre-formed complex comprising an RGEN and a gRNA or a pre-formed complex comprising a DGEN and a gDNA. In some embodiments, the genome-editing complex comprises a cell-penetrating peptide and an integrase (or a nucleic acid encoding the integrase). In some embodiments, the integrase is a bacteriophage integrase (*e.g*., ΦC31). In some embodiments, the genome-editing complex further comprises a donor nucleic acid comprising a recombination site recognized by the integrase. In some embodiments, at least some of the cell-penetrating peptides in the genome-editing complex are linked to a targeting moiety. In some embodiments, the linkage is covalent. In some embodiments, the molar ratio of cell-penetrating peptide to at least one of the one or more genome-editing system molecules (such as all of the one or more genome-editing system molecules) in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to at least one of the one or more genome-editing system molecules (such as all of the one or more genome-editing system molecules) in the genome-editing complex is between about 5:1 and about 20:1 (such as about any of 5:1, 6:1, 7:1, 8:1, 9:1,10:1,11:1,12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs or dDNAs comprising different guide sequences. In some embodiments, the genome-editing complex further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the genome-editing complex comprises the entire genome-editing system.

In some embodiments, there is provided a genome-editing complex for modifying a target polynucleotide comprising a cell-penetrating peptide (*e.g*., a VEPEP-3, VEPEP-6, VEPEP-9, or ADGN-100 peptide) and a ZFN or TALEN (or one or more nucleic acids encoding the ZFN or TALEN). In some embodiments, at least some of the cell-penetrating peptides in the genome-editing complex are linked to a targeting moiety. In some embodiments, the linkage is covalent. In some embodiments, the molar ratio of cell-penetrating peptide to ZFN or TALEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to ZFN or TALEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying a target polynucleotide comprising a cell-penetrating peptide (*e.g*., a VEPEP-3, VEPEP-6, VEPEP-9, or ADGN-100 peptide) and an integrase (or one or more nucleic acids encoding the integrase). In some embodiments, the integrase is a bacteriophage integrase (*e.g*., ΦC31). In some embodiments, the genome-editing complex further comprises a donor nucleic acid comprising a recombination site recognized by the integrase for introducing a specific modification to the target polynucleotide. In some embodiments, at least some of the cell-penetrating peptides in the genome-editing complex are linked to a targeting moiety. In some embodiments, the linkage is covalent. In some embodiments, the molar ratio of cell-penetrating peptide to integrase in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to integrase in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying a target polynucleotide comprising a cell-penetrating peptide (*e.g*., a VEPEP-3, VEPEP-6, VEPEP-9, or ADGN-100 peptide) and one or both of an RGEN (or nucleic acid encoding the RGEN) and a gRNA (or nucleic acid encoding the gRNA), wherein the gRNA comprises a guide sequence complementary to a target sequence in the target polynucleotide. In some embodiments, the genome-editing complex comprises the cell-penetrating peptide associated with a pre-formed complex comprising the RGEN and the gRNA. In some embodiments, at least some of the cell-penetrating peptides in the genome-editing complex are linked to a targeting moiety. In some embodiments, the linkage is covalent In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8,1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs comprising different guide sequences. In some embodiments, the genome-editing complex further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying a target polynucleotide comprising a cell-penetrating peptide (*e.g*., a VEPEP-3, VEPEP-6, VEPEP-9, or ADGN-100 peptide) and one or both of a DGEN (or nucleic acid encoding the DGEN) and a gDNA (or nucleic acid encoding the gDNA), wherein the gDNA comprises a guide sequence complementary to a target sequence in the target polynucleotide. In some embodiments, the genome-editing complex comprises the cell-penetrating peptide associated with a pre-formed complex comprising the DGEN and the gDNA. In some embodiments, at least some of the cell-penetrating peptides in the genome-editing complex are linked to a targeting moiety. In some embodiments, the linkage is covalent. In some embodiments, the DGEN is NgAgo. In some embodiments, the molar ratio of DGEN to gDNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of DGEN to gDNA in the genome-editing complex is about 1:1. In some embodiments, the molar ratio of cell-penetrating peptide to DGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to DGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gDNAs comprising different guide sequences. In some embodiments, the genome-editing complex further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying a target polynucleotide comprising a cell-penetrating peptide (*e.g*., a VEPEP-3, VEPEP-6, VEPEP-9, or ADGN-100 peptide) associated with a pre-formed RGEN/gRNA complex comprising an RGEN and a gRNA, wherein the gRNA comprises a guide sequence complementary to a target sequence in the target polynucleotide. In some embodiments, at least some of the cell-penetrating peptides in the genome-editing complex are linked to a targeting moiety. In some embodiments, the linkage is covalent In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs comprising different guide sequences. In some embodiments, the genome-editing complex further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for introducing a modification to a target polynucleotide comprising a cell-penetrating peptide (*e.g*., a VEPEP-3, VEPEP-6, VEPEP-9, or ADGN-100 peptide), a ZFN or TALEN, and a donor nucleic acid, wherein the ZFN or TALEN cleaves a target sequence in the target polynucleotide, and wherein the donor nucleic acid comprises a sequence corresponding to a portion of the target polynucleotide modified to comprise the modification. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, at least some of the cell-penetrating peptides in the genome-editing complex are linked to a targeting moiety. In some embodiments, the linkage is covalent. In some embodiments, the molar ratio of cell-penetrating peptide to ZFN or TALEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to ZFN or TALEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the genome-editing complex comprises a TALEN. In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional donor nucleic acids comprising different modifications. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for introducing an exogenous nucleic acid into a target polynucleotide comprising a cell-penetrating peptide (*e.g*., a VEPEP-3, VEPEP-6, VEPEP-9, or ADGN-100 peptide), an integrase, and a donor nucleic acid comprising the exogenous nucleic acid, wherein the integrase is capable of mediating recombination between a first recombination site in the target polynucleotide and a second recombination site in the donor nucleic acid. In some embodiments, at least some of the cell-penetrating peptides in the genome-editing complex are linked to a targeting moiety. In some embodiments, the linkage is covalent In some embodiments, the molar ratio of cell-penetrating peptide to integrase in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to integrase in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional donor nucleic acids comprising different exogenous nucleic acids. In some embodiments, the exogenous nucleic acid is inserted into a coding sequence of the target polynucleotide. In some embodiments, the exogenous nucleic acid is inserted into anon-coding sequence of the target polynucleotide. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the target polynucleotide is modified to express a product of the exogenous nucleic acid (such as a protein, *e.g*., an exogenous protein).

In some embodiments, there is provided a genome-editing complex for introducing a modification to a target polynucleotide comprising a cell-penetrating peptide (*e.g*., a VEPEP-3, VEPEP-6, VEPEP-9, or ADGN-100 peptide) and one or more of an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, and wherein the donor nucleic acid comprises a sequence corresponding to a portion of the target polynucleotide modified to comprise the modification. In some embodiments, the genome-editing complex comprises the cell-penetrating peptide associated with a) a pre-formed complex comprising the RGEN and the gRNA; and b) the donor nucleic acid. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, at least some of the cell-penetrating peptides in the genome-editing complex are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs comprising different guide sequences. In some embodiments, the genome-editing complex further comprises one or more additional donor nucleic acids comprising different modifications. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for introducing a modification to a target polynucleotide comprising a cell-penetrating peptide (*e.g.,* a VEPEP-3, VEPEP-6, VEPEP-9, or ADGN-100 peptide), an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is addition, deletion, or substitution of one or more nucleotides in the target nucleic acid. In some embodiments, the genome-editing complex comprises the cell-penetrating peptide associated with a) a pre-formed complex comprising the RGEN and the gRNA; and b) the donor nucleic acid. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length. In some embodiments, at least some of the cell-penetrating peptides in the genome-editing complex are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs comprising different guide sequences. In some embodiments, the genome-editing complex further comprises one or more additional donor nucleic acids comprising different modifications. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for introducing a modification to a target polynucleotide comprising a cell-penetrating peptide (*e.g*., a VEPEP-3, VEPEP-6, VEPEP-9, or ADGN-100 peptide), an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is insertion of a heterologous nucleic acid in the target nucleic acid. In some embodiments, the genome-editing complex comprises the cell-penetrating peptide associated with a) a pre-formed complex comprising the RGEN and the gRNA; and b) the donor nucleic acid. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length. In some embodiments, at least some of the cell-penetrating peptides in the genome-editing complex are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more additional gRNAs comprising different guide sequences. In some embodiments, the genome-editing complex further comprises one or more additional donor nucleic acids comprising different modifications. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying one or more target polynucleotides comprising a cell-penetrating peptide (*e.g.,* a VEPEP-3, VEPEP-6, VEPEP-9, or ADGN-100 peptide), an RGEN (or a nucleic acid encoding the RGEN), and a plurality of gRNAs (or one or more nucleic acids encoding the plurality of gRNAs), wherein each of the plurality of gRNAs individually comprises a different guide sequence complementary to a target sequence in one of the one or more target polynucleotides. In some embodiments, the genome-editing complex comprises the cell-penetrating peptide associated with pre-formed complexes comprising the RGEN and the plurality of gRNAs. In some embodiments, at least some of the cell-penetrating peptides in the genome-editing complex are linked to a targeting moiety. In some embodiments, the linkage is covalent. In some embodiments, each of the plurality of gRNAs is a single-guide RNA (sgRNA) comprising a specificity-determining CRISPR RNA (crRNA) fused to an auxiliary trans-activating crRNA (tracrRNA). In some embodiments, each of the plurality of gRNAs is an sgRNA comprising the guide sequence of the gRNA, a tracr mate sequence, a tracr sequence, and a tail sequence. In some embodiments, the RGEN is Cas9 or Cpfl. In some embodiments, the molar ratio of RGEN to gRNA in the genome-editing complex is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the genome-editing complex is about 1:1. In some embodiments, the molar ratio of cell-penetrating peptide to RGEN in the genome-editing complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the genome-editing complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the genome-editing complex further comprises one or more donor nucleic acids, wherein each of the one or more donor nucleic acids individually comprises a sequence for introducing a modification to one of the one or more target polynucleotides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product.

In some embodiments, there is provided a genome-editing complex for modifying a target polynucleotide comprising a cell-penetrating peptide associated with a genome-editing enzyme (or a nucleic acid encoding the genome-editing enzyme), such as a nuclease or integrase, wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing enzyme is a nuclease selected from a ZFN, TALEN, homing endonuclease, RGEN, or DGEN. In some embodiments, the genome-editing enzyme is Cas9. In some embodiments, the genome-editing complex further comprises a gRNA or a gDNA (or a nucleic acid encoding the gRNA or gDNA). In some embodiments, the genome-editing enzyme is a TALEN. In some embodiments, the genome-editing enzyme is an integrase. In some embodiments, the genome-editing complex further comprises a donor nucleic acid for introducing a specific modification to the target polynucleotide.

In some embodiments, there is provided a genome-editing complex for modifying a target polynucleotide comprising a cell-penetrating peptide associated with a pre-formed RGEN/gRNA complex comprising an RGEN and a gRNA, wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the RGEN is Cas9.

In some embodiments, there is provided a genome-editing complex for modifying a target polynucleotide comprising a cell-penetrating peptide, an RGEN (or a nucleic acid encoding the RGEN), and a gRNA (or a nucleic acid encoding the gRNA), wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the RGEN is Cas9.

In some embodiments, there is provided a genome-editing complex for introducing a modification to a target polynucleotide comprising a cell-penetrating peptide, an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, wherein the donor nucleic acid comprises a sequence corresponding to a portion of the target polynucleotide modified to comprise the modification, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising the RGEN and the gRNA; and b) the donor nucleic acid. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the RGEN is Cas9.

In some embodiments, there is provided a genome-editing complex for introducing a modification to a target polynucleotide comprising a cell-penetrating peptide, an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is addition, deletion, or substitution of one or more nucleotides in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising the RGEN and the gRNA; and b) the donor nucleic acid. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length. In some embodiments, the RGEN is Cas9.

In some embodiments, there is provided a genome-editing complex for introducing a modification to a target polynucleotide comprising a cell-penetrating peptide, an RGEN (or a nucleic acid encoding the RGEN), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is insertion of a heterologous nucleic acid in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising the RGEN and the gRNA; and b) the donor nucleic acid. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length. In some embodiments, the RGEN is Cas9.

In some embodiments, there is provided a genome-editing complex for modifying one or more target polynucleotides comprising a cell-penetrating peptide, an RGEN (or a nucleic acid encoding the RGEN), and a plurality of gRNAs (or one or more nucleic acids encoding the plurality of gRNAs), wherein each of the plurality of gRNAs individually comprises a different guide sequence complementary to a target sequence in one of the one or more target polynucleotides, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with pre-formed complexes comprising the RGEN and the plurality of gRNAs. In some embodiments, the RGEN is Cas9.

In some embodiments, there is provided a genome-editing complex for modifying a target polynucleotide comprising a cell-penetrating peptide associated with a pre-formed Cas9/gRNA complex comprising Cas9 and a gRNA, wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80.

In some embodiments, there is provided a genome-editing complex for modifying a target polynucleotide comprising a cell-penetrating peptide, Cas9 (or a nucleic acid encoding Cas9), and a gRNA (or a nucleic acid encoding the gRNA), wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80.

In some embodiments, there is provided a genome-editing complex for introducing a modification to a target polynucleotide comprising a cell-penetrating peptide, Cas9 (or a nucleic acid encoding Cas9), a gRNA (or a nucleic acid encoding the gRNA), and a donor nucleic acid, wherein the gRNA comprises a guide sequence complementary to a target sequence in the target polynucleotide, wherein the donor nucleic acid comprises a sequence corresponding to a portion of the target polynucleotide modified to comprise the modification, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising Cas9 and the gRNA; and b) the donor nucleic acid. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence.

In some embodiments, there is provided a genome-editing complex for introducing a modification to a target polynucleotide comprising a cell-penetrating peptide, Cas9 (or a nucleic acid encoding Cas9), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is addition, deletion, or substitution of one or more nucleotides in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising Cas9 and the gRNA; and b) the donor nucleic acid. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length.

In some embodiments, there is provided a genome-editing complex for introducing a modification to a target polynucleotide comprising a cell-penetrating peptide, Cas9 (or a nucleic acid encoding Cas9), a gRNA (or a nucleic acid encoding the gRNA), and a donor nucleic acid, wherein the gRNA comprises a guide sequence complementary to a target sequence in the target polynucleotide, the donor nucleic acid comprises a sequence corresponding to a portion of the target polynucleotide modified to comprise the modification, and the modification is insertion of a heterologous nucleic acid in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with a) a pre-formed complex comprising Cas9 and the gRNA; and b) the donor nucleic acid. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length.

In some embodiments, there is provided a genome-editing complex for modifying one or more target polynucleotides comprising a cell-penetrating peptide, Cas9 (or a nucleic acid encoding Cas9), and a plurality of gRNAs (or one or more nucleic acids encoding the plurality of gRNAs), wherein each of the plurality of gRNAs individually comprises a different guide sequence complementary to a target sequence in one of the one or more target polynucleotides, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing complex comprises the cell-penetrating peptide associated with pre-formed complexes comprising Cas9 and the plurality of gRNAs.

In some embodiments, the guide sequence of a gRNA contained in a genome-editing complex according to any of the embodiments described herein is complementary to a target sequence in a gene encoding a protein involved in regulating an immune response, including immune checkpoint regulators and proteins involved in antigen presentation. In some embodiments, the guide sequence of a gRNA contained in a genome-editing complex according to any of the embodiments described herein is complementary to a target sequence in a gene encoding a protein involved in regulating cholesterol transport and/or metabolism. In some embodiments, the guide sequence is complementary to a target sequence in a gene encoding a protein including, without limitation, PD-1, PD-L1, PD-L2, TIM-1, TIM-3, TIM-4, BTLA, VISTA, LAG-3, CTLA-4, TIGIT, 4-1BB, OX40, CD27, CD28, HVEM, GITR, ICOS, CD40, CD80, CD86, B7-H2, B7-H3, B7-H4, B7-H6, 2B4, CD160, gp49B, PIR-B, KIR family receptors, SIRPalpha (CD47), CD48, 2B4 (CD244), B7.1, B7.2, ILT-2, ILT-4, A2aR, toll-like receptors TLR-2, 3, 4, 6, 7, 8, and 9, granulocyte macrophage colony stimulating factor (GM-CSF), TNF, CD40L, FLT-3 ligand, cytokines such as IL-1, IL-2, IL-4, IL-7, IL-10, IL-12, IL-15, IL-21, and IL-35, FasL, TGF-β, indoleamine-2,3 dioxygenase (IDO), major histocompatibility complex (MHC) proteins, including beta-2 microglobulin (β2M), low-density lipoprotein (LDL) receptor (LDLR), apolipoprotein B (ApoB), low-density lipoprotein receptor adapter protein 1 (LDLRAP1), and proprotein convertase subtilisin kexin 9 (PCSK9).

In some embodiments, according to any of the genome-editing complexes described herein, the genome-editing complex further comprises a protein other than a genome-editing system molecule, or a nucleic acid molecule encoding the protein (such as a DNA plasmid or mRNA).

In some embodiments, the mean size (diameter) of a genome-editing complex described herein is between any of about 10 nm and about 10 microns, including for example between about 30 nm and about 1 micron, between about 50 nm and about 250 nm, between about 50 nm and about 180 nm, and between about 150 nm and about 200 nm. In some embodiments, the genome-editing complex is between about 10 nm and about 400 nm. In some embodiments, the genome-editing complex is between about 20 nm and about 400 nm. In some embodiments, the genome-editing complex is between about 30 nm and about 200 nm. In some embodiments, the genome-editing complex is between about 40 nm and about 200 nm. In some embodiments, the genome-editing complex is between about 40 nm and about 150 nm. In some embodiments, the genome-editing complex is between about 40 nm and about 100 nm. In some embodiments, the genome-editing complex is substantially non-toxic.

In some embodiments, the targeting moiety of a genome-editing complex described herein targets the genome-editing complex to a tissue or a specific cell type. In some embodiments, the tissue is a tissue in need of treatment In some embodiments, the targeting moiety targets the genome-editing complex to a tissue or cell that can be treated by the genome-editing system.

### Nanoparticles

In some embodiments, there is provided a nanoparticle for modifying a target polynucleotide comprising a core comprising one or more genome-editing complexes described herein. In some embodiments, the nanoparticle core comprises a plurality of genome-editing complexes. In some embodiments, the nanoparticle core comprises a plurality of genome-editing complexes present in a predetermined ratio. In some embodiments, the predetermined ratio is selected to allow the most effective use of the nanoparticle in any of the methods described below in more detail. In some embodiments, the nanoparticle core further comprises one or more additional cell-penetrating peptides, one or more additional genome-editing nucleases, one or more additional gNAs, and/or one or more additional donor nucleic acids. In some embodiments, the one or more additional cell-penetrating peptides do not comprise a cell-penetrating peptide found in any of the one or more genome-editing complexes. In some embodiments, the one or more additional genome-editing nucleases do not comprise a genome-editing nuclease found in any of the one or more genome-editing complexes. In some embodiments, the one or more additional gNAs do not comprise a gNA found in any of the one or more genome-editing complexes. In some embodiments, the one or more additional donor nucleic acids do not comprise a donor nucleic acid found in any of the one or more genome-editing complexes. In some embodiments, the one or more additional cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, at least some of the one or more additional cell-penetrating peptides are linked to a targeting moiety. In some embodiments, the linkage is covalent.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide) and a genome-editing enzyme (or a nucleic acid encoding the genome-editing enzyme), such as a nuclease or integrase. In some embodiments, at least some of the one or more cell-penetrating peptides in the nanoparticle are linked to a targeting moiety. In some embodiments, the linkage is covalent. In some embodiments, the molar ratio of cell-penetrating peptide to genome-editing enzyme associated with the cell-penetrating peptide in a complex present in the nanoparticle is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to genome-editing enzyme associated with the cell-penetrating peptide in a complex present in the nanoparticle is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the genome-editing enzyme is a nuclease selected from a ZFN, TALEN, homing endonuclease, RGEN, or DGEN. In some embodiments, the genome-editing enzyme is Cas9. In some embodiments, the nanoparticle core further comprises one or more gRNAs or gDNAs (or one or more nucleic acids encoding the one or more gRNAs or gDNAs). In some embodiments, the genome-editing enzyme is a TALEN. In some embodiments, the genome-editing enzyme is an integrase. In some embodiments, the nanoparticle core further comprises a donor nucleic acid for introducing a specific modification to the target polynucleotide. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), an RGEN (or a nucleic acid encoding the RGEN), and a gRNA (or a nucleic acid encoding the gRNA), wherein the gRNA comprises a guide sequence complementary to a target sequence in the target polynucleotide. In some embodiments, the nanoparticle core comprises one of the one or more cell-penetrating peptides associated with a pre-formed complex comprising the RGEN and the gRNA (RGEN/gRNA complex). In some embodiments, the nanoparticle core comprises a first complex comprising one of the one or more cell-penetrating peptides associated with the RGEN and a second complex comprising one of the one or more cell-penetrating peptides associated with the gRNA. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in a complex present in the nanoparticle; and/or b) cell-penetrating peptide to gRNA associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in a complex present in the nanoparticle; and/or b) cell-penetrating peptide to gRNA associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), a DGEN (or a nucleic acid encoding the DGEN), and a gDNA (or a nucleic acid encoding the gDNA), wherein the gDNA comprises a guide sequence complementary to a target sequence in the target polynucleotide. In some embodiments, the nanoparticle core comprises one of the one or more cell-penetrating peptides associated with a pre-formed complex comprising the DGEN and the gDNA (DGEN/gDNA complex). In some embodiments, the nanoparticle core comprises a first complex comprising one of the one or more cell-penetrating peptides associated with the DGEN and a second complex comprising one of the one or more cell-penetrating peptides associated with the gDNA. In some embodiments, at least some of the one or more cell-penetrating peptides in the nanoparticle are linked to a targeting moiety. In some embodiments, the linkage is covalent. In some embodiments, the DGEN is NgAgo. In some embodiments, the molar ratio of DGEN to gDNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of DGEN to gDNA in the nanoparticle is about 1:1. In some embodiments, the molar ratio of a) cell-penetrating peptide to DGEN associated with the cell-penetrating peptide in a complex present in the nanoparticle; and/or b) cell-penetrating peptide to gDNA associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to DGEN associated with the cell-penetrating peptide in a complex present in the nanoparticle; and/or b) cell-penetrating peptide to gDNA associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gDNAs comprising different guide sequences. In some embodiments, at least one of the one or more additional gDNAs is present in the pre-formed DGEN/gDNA complex. In some embodiments, at least one of the one or more additional gDNAs is present in the second complex. In some embodiments, at least one of the one or more additional gDNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed DGEN/gDNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide) and a pre-formed complex comprising an RGEN and a gRNA (RGEN/gRNA complex), wherein at least one of the one or more cell-penetrating peptides is associated with the pre-formed RGEN/gRNA complex, and wherein the gRNA comprises a guide sequence complementary to a target sequence in the target polynucleotide. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of cell-penetrating peptide to RGEN in the pre-formed RGEN/gRNA complex associated with the cell-penetrating peptide is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to RGEN in the pre-formed RGEN/gRNA complex associated with the cell-penetrating peptide is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), a first complex comprising one of the one or more cell-penetrating peptides associated with an RGEN (or a nucleic acid encoding the RGEN), and a second complex comprising one of the one or more cell-penetrating peptides associated with a gRNA (or a nucleic acid encoding the gRNA), wherein the gRNA comprises a guide sequence complementary to a target sequence in the target polynucleotide. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in the first complex; and/or b) cell-penetrating peptide to gRNA associated with the cell-penetrating peptide in the second complex, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in the first complex; and/or b) cell-penetrating peptide to gRNA associated with the cell-penetrating peptide in the second complex, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, and wherein the donor nucleic acid comprises a sequence corresponding to a portion of the target polynucleotide modified to comprise the modification. In some embodiments, the nanoparticle core comprises one of the one or more cell-penetrating peptides associated with a pre-formed complex comprising the RGEN and the gRNA (RGEN/gRNA complex). In some embodiments, the donor nucleic acid is present in a ternary complex comprising one of the one or more cell-penetrating peptides, a pre-formed RGEN/gRNA complex, and the donor nucleic acid. In some embodiments, the nanoparticle core comprises a first complex comprising one of the one or more cell-penetrating peptides associated with the RGEN and a second complex comprising one of the one or more cell-penetrating peptides associated with the gRNA. In some embodiments, the donor nucleic acid is present in the first complex. In some embodiments, the donor nucleic acid is present in the second complex. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity -determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, and wherein the donor nucleic acid comprises a sequence corresponding to a portion of the target polynucleotide modified to comprise the modification, and the modification is addition, deletion, or substitution of one or more nucleotides in the target nucleic acid. In some embodiments, the nanoparticle core comprises one of the one or more cell-penetrating peptides associated with a pre-formed complex comprising the RGEN and the gRNA (RGEN/gRNA complex). In some embodiments, the donor nucleic acid is present in a ternary complex comprising one of the one or more cell-penetrating peptides, a pre-formed RGEN/gRNA complex, and the donor nucleic acid. In some embodiments, the nanoparticle core comprises a first complex comprising one of the one or more cell-penetrating peptides associated with the RGEN and a second complex comprising one of the one or more cell-penetrating peptides associated with the gRNA. In some embodiments, the donor nucleic acid is present in the first complex. In some embodiments, the donor nucleic acid is present in the second complex. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising one or more cell-penetrating peptides (e.g., a VEPEP-3, VEPEP-6, VEPEP-9, or ADGN-100 peptide), an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, and wherein the donor nucleic acid comprises a sequence corresponding to a portion of the target polynucleotide modified to comprise the modification, and the modification is insertion of a heterologous nucleic acid in the target nucleic acid. In some embodiments, the nanoparticle core comprises one of the one or more cell-penetrating peptides associated with a pre-formed complex comprising the RGEN and the gRNA (RGEN/gRNA complex). In some embodiments, the donor nucleic acid is present in a ternary complex comprising one of the one or more cell-penetrating peptides, a pre-formed RGEN/gRNA complex, and the donor nucleic acid. In some embodiments, the nanoparticle core comprises a first complex comprising one of the one or more cell-penetrating peptides associated with the RGEN and a second complex comprising one of the one or more cell-penetrating peptides associated with the gRNA. In some embodiments, the donor nucleic acid is present in the first complex. In some embodiments, the donor nucleic acid is present in the second complex. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length. In some embodiments, at least some of the one or more cell-penetrating peptides in the nanoparticle are linked to a targeting moiety. In some embodiments, the linkage is covalent. In some embodiments, the gRNA is a single-guide RNA (sgRNA) comprising a specificity-determining CRISPR RNA (crRNA) fused to an auxiliary trans-activating crRNA (tracrRNA). In some embodiments, the gRNA is an sgRNA comprising the guide sequence, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in a complex present in the nanoparticle; b) cell-penetrating peptide to gRNA associated with the cell-penetrating peptide in a complex present in the nanoparticle; and/or c) cell-penetrating peptide to donor nucleic acid associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises one or more additional gRNAs comprising different guide sequences. In some embodiments, at least one of the one or more additional gRNAs is present in the pre-formed RGEN/gRNA complex. In some embodiments, at least one of the one or more additional gRNAs is present in the second complex. In some embodiments, at least one of the one or more additional gRNAs is present in an additional complex comprising one of the one or more cell-penetrating peptides. In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first or second complex. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying one or more target polynucleotides comprising a core comprising one or more cell-penetrating peptides (e.g., a VEPEP-3, VEPEP-6, VEPEP-9, or ADGN-100 peptide), an RGEN (or a nucleic acid encoding the RGEN), and a plurality of gRNAs (or one or more nucleic acids encoding the plurality of gRNAs), wherein each of the plurality of gRNAs individually comprises a different guide sequence complementary to a target sequence in one of the one or more target polynucleotides. In some embodiments, the nanoparticle core comprises one of the one or more cell-penetrating peptides associated with a pre-formed complex comprising the RGEN and at least one of the plurality of gRNAs (RGEN/gRNA complex). In some embodiments, the pre-formed RGEN/gRNA complex comprises each of the plurality of gRNAs. In some embodiments, the nanoparticle core comprises a first complex comprising one of the one or more cell-penetrating peptides associated with the RGEN and one or more complexes, each comprising one of the one or more cell-penetrating peptides associated with at least one of the plurality of gRNAs. In some embodiments, the one or more complexes is a second complex comprising each of the plurality of gRNAs. In some embodiments, at least some of the one or more cell-penetrating peptides in the nanoparticle are linked to a targeting moiety. In some embodiments, the linkage is covalent. In some embodiments, each of the plurality of gRNAs is a single-guide RNA (sgRNA) comprising a specificity-determining CRISPR RNA (crRNA) fused to an auxiliary trans-activating crRNA (tracrRNA). In some embodiments, each of the plurality of gRNAs is an sgRNA comprising the guide sequence of the gRNA, a tracr mate sequence, a tracr sequence, and a tail sequence. In some embodiments, the RGEN is Cas9 or Cpfl. In some embodiments, the molar ratio of RGEN to gRNA in the nanoparticle is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of RGEN to gRNA in the nanoparticle is about 1:1. In some embodiments, the molar ratio of a) cell-penetrating peptide to RGEN associated with the cell-penetrating peptide in a complex present in the nanoparticle; and/or b) cell-penetrating peptide to gRNA associated with the cell-penetrating peptide in a complex present in the nanoparticle, is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of a) cell-penetrating peptide to RGEN associated with the cell-penetrating peptide in a complex present in the nanoparticle; and/or b) cell-penetrating peptide to gRNA associated with the cell-penetrating peptide in a complex present in the nanoparticle, is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the nanoparticle core further comprises a donor nucleic acid for introducing a specific modification to the target polynucleotide. In some embodiments, the donor nucleic acid is present in the pre-formed RGEN/gRNA complex. In some embodiments, the donor nucleic acid is present in the first complex or the one or more complexes. In some embodiments, the donor nucleic acid is present in an additional complex comprising one of the one or more cell-penetrating peptides. In some embodiments, the target polynucleotide is modified in a coding sequence. In some embodiments, the target polynucleotide is modified in a non-coding sequence. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the one or more cell-penetrating peptides include, but are not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide.

In some embodiments, there is provided a nanoparticle for modifying a target polynucleotide comprising a core comprising a cell-penetrating peptide and a genome-editing enzyme (or a nucleic acid encoding the genome-editing enzyme), wherein the cell-penetrating peptide is associated with the genome-editing enzyme, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing enzyme is a nuclease selected from a ZFN, TALEN, homing endonuclease, RGEN, or DGEN. In some embodiments, the genome-editing enzyme is Cas9. In some embodiments, the nanoparticle core further comprises a gRNA or gDNA (or a nucleic acid encoding the gRNA or gDNA). In some embodiments, the genome-editing enzyme is a TALEN. In some embodiments, the genome-editing enzyme is an integrase. In some embodiments, the nanoparticle core further comprises a donor nucleic acid for introducing a specific modification to the target polynucleotide.

In some embodiments, there is provided a nanoparticle for modifying a target polynucleotide comprising a core comprising a cell-penetrating peptide, an RGEN (or a nucleic acid encoding the RGEN), and a gRNA (or a nucleic acid encoding the gRNA), wherein the gRNA comprises a guide sequence complementary to a target sequence in the target polynucleotide, and wherein the cell-penetrating peptide comprises the amino acid sequence of of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising the RGEN and the gRNA (RGEN/gRNA complex). In some embodiments, the nanoparticle core comprises a first complex comprising the cell-penetrating peptide associated with the RGEN and a second complex comprising the cell-penetrating peptide associated with the gRNA. In some embodiments, the RGEN is Cas9.

In some embodiments, there is provided a nanoparticle for modifying a target polynucleotide comprising a core comprising a cell-penetrating peptide and a pre-formed complex comprising an RGEN and a gRNA (RGEN/gRNA complex), wherein the cell-penetrating peptide is associated with the pre-formed RGEN/gRNA complex, wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the RGEN is Cas9.

In some embodiments, there is provided a nanoparticle for modifying a target polynucleotide comprising a core comprising a cell-penetrating peptide, a first complex comprising the cell-penetrating peptide associated with an RGEN (or a nucleic acid encoding the RGEN), and a second complex comprising the cell-penetrating peptide associated with a gRNA (or a nucleic acid encoding the gRNA), wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the RGEN is Cas9.

In some embodiments, there is provided a nanoparticle for modifying a target polynucleotide comprising a core comprising a cell-penetrating peptide, an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising the RGEN and the gRNA (RGEN/gRNA complex) and a separate complex comprising the cell-penetrating peptide associated with the donor nucleic acid. In some embodiments, the donor nucleic acid is present in a ternary complex comprising the cell-penetrating peptide, a pre-formed RGEN/gRNA complex, and the donor nucleic acid. In some embodiments, the nanoparticle core comprises a first complex comprising the cell-penetrating peptide associated with the RGEN and a second complex comprising the cell-penetrating peptide associated with the gRNA. In some embodiments, the donor nucleic acid is present in the first complex. In some embodiments, the donor nucleic acid is present in the second complex. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the RGEN is Cas9.

In some embodiments, there is provided a nanoparticle for modifying a target polynucleotide comprising a core comprising a cell-penetrating peptide, an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and the modification is addition, deletion, or substitution of one or more nucleotides in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising the RGEN and the gRNA (RGEN/gRNA complex) and a separate complex comprising the cell-penetrating peptide associated with the donor nucleic acid. In some embodiments, the donor nucleic acid is present in a ternary complex comprising the cell-penetrating peptide, a pre-formed RGEN/gRNA complex, and the donor nucleic acid. In some embodiments, the nanoparticle core comprises a first complex comprising the cell-penetrating peptide associated with the RGEN and a second complex comprising the cell-penetrating peptide associated with the gRNA. In some embodiments, the donor nucleic acid is present in the first complex. In some embodiments, the donor nucleic acid is present in the second complex. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length. In some embodiments, the RGEN is Cas9.

In some embodiments, there is provided a nanoparticle for modifying a target polynucleotide comprising a core comprising a cell-penetrating peptide, an RGEN (or a nucleic acid encoding the RGEN), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and the modification is insertion of a heterologous nucleic acid in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising the RGEN and the gRNA (RGEN/gRNA complex) and a separate complex comprising the cell-penetrating peptide associated with the donor nucleic acid. In some embodiments, the donor nucleic acid is present in a ternary complex comprising the cell-penetrating peptide, a pre-formed RGEN/gRNA complex, and the donor nucleic acid. In some embodiments, the nanoparticle core comprises a first complex comprising the cell-penetrating peptide associated with the RGEN and a second complex comprising the cell-penetrating peptide associated with the gRNA. In some embodiments, the donor nucleic acid is present in the first complex. In some embodiments, the donor nucleic acid is present in the second complex. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length. In some embodiments, the RGEN is Cas9.

In some embodiments, there is provided a nanoparticle for modifying one or more target polynucleotides comprising a core comprising a cell-penetrating peptide, an RGEN (or a nucleic acid encoding the RGEN), and a plurality of gRNAs (or one or more nucleic acids encoding the plurality of gRNAs), wherein each of the plurality of gRNAs individually comprises a different guide sequence complementary to a target sequence in one of the one or more target polynucleotides, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising the RGEN and at least one of the plurality of gRNAs (RGEN/gRNA complex). In some embodiments, the pre-formed RGEN/gRNA complex comprises each of the plurality of gRNAs. In some embodiments, the nanoparticle core comprises a first complex comprising the cell-penetrating peptide associated with the RGEN and one or more complexes, each comprising the cell-penetrating peptide associated with at least one of the plurality of gRNAs. In some embodiments, the one or more complexes is a second complex comprising each of the plurality of gRNAs. In some embodiments, the RGEN is Cas9.

In some embodiments, there is provided a nanoparticle for modifying a target polynucleotide comprising a core comprising a cell-penetrating peptide, Cas9 (or a nucleic acid encoding Cas9), and a gRNA (or a nucleic acid encoding the gRNA), wherein the gRNA comprises a guide sequence complementary to a target sequence in the target polynucleotide, and wherein the cell-penetrating peptide comprises the amino acid sequence of of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising Cas9 and the gRNA (Cas9/gRNA complex). In some embodiments, the nanoparticle core comprises a first complex comprising the cell-penetrating peptide associated with the RGEN and a second complex comprising the cell-penetrating peptide associated with the gRNA.

In some embodiments, there is provided a nanoparticle for modifying a target polynucleotide comprising a core comprising a cell-penetrating peptide and a pre-formed complex comprising Cas9 and a gRNA (Cas9/gRNA complex), wherein the cell-penetrating peptide is associated with the pre-formed Cas9/gRNA complex, wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80.

In some embodiments, there is provided a nanoparticle for modifying a target polynucleotide comprising a core comprising a cell-penetrating peptide, a first complex comprising the cell-penetrating peptide associated with Cas9 (or a nucleic acid encoding Cas9), and a second complex comprising the cell-penetrating peptide associated with a gRNA (or a nucleic acid encoding the gRNA), wherein the gRNA comprises a guide sequence complementary to a target sequence in the target polynucleotide, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80.

In some embodiments, there is provided a nanoparticle for modifying a target polynucleotide comprising a core comprising a cell-penetrating peptide, Cas9 (or a nucleic acid encoding Cas9), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising Cas9 and the gRNA (Cas9/gRNA complex) and a separate complex comprising the cell-penetrating peptide associated with the donor nucleic acid. In some embodiments, the donor nucleic acid is present in a ternary complex comprising the cell-penetrating peptide, a pre-formed Cas9/gRNA complex, and the donor nucleic acid. In some embodiments, the nanoparticle core comprises a first complex comprising the cell-penetrating peptide associated with Cas9 and a second complex comprising the cell-penetrating peptide associated with the gRNA. In some embodiments, the donor nucleic acid is present in the first complex. In some embodiments, the donor nucleic acid is present in the second complex. In some embodiments, the modification is addition, deletion, or substitution of one or more nucleotides in the target polynucleotide, and the donor nucleic acid is a single-stranded DNA oligonucleotide. In some embodiments, the modification is insertion of a heterologous nucleic acid in the target polynucleotide, and the donor nucleic acid is a double-stranded DNA molecule, such as a plasmid. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence.

In some embodiments, there is provided a nanoparticle for modifying a target polynucleotide comprising a core comprising a cell-penetrating peptide, Cas9 (or a nucleic acid encoding Cas9), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and the modification is addition, deletion, or substitution of one or more nucleotides in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising Cas9 and the gRNA (Cas9/gRNA complex) and a separate complex comprising the cell-penetrating peptide associated with the donor nucleic acid. In some embodiments, the donor nucleic acid is present in a ternary complex comprising the cell-penetrating peptide, a pre-formed Cas9/gRNA complex, and the donor nucleic acid. In some embodiments, the nanoparticle core comprises a first complex comprising the cell-penetrating peptide associated with Cas9 and a second complex comprising the cell-penetrating peptide associated with the gRNA. In some embodiments, the donor nucleic acid is present in the first complex. In some embodiments, the donor nucleic acid is present in the second complex. In some embodiments, the modification is addition, deletion, or substitution of between about 1 and about 50 (such as about any of 1, 2, 3, 4, 5, 6, 7, 8, 9, 10, 15, 20, 25, 30, 35, 40, 45, and 50, including any ranges between these values) nucleotides in the target polynucleotide. In some embodiments, the donor nucleic acid is a single-stranded DNA oligonucleotide. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually between about 20 and about 150 (such as about any of 20, 30, 40, 50, 60, 70, 80, 90, 100, 110, 120, 130, 140, and 150, including any ranges between these values) nucleotides in length.

In some embodiments, there is provided a nanoparticle for modifying a target polynucleotide comprising a core comprising a cell-penetrating peptide, Cas9 (or a nucleic acid encoding Cas9), a gRNA (or a nucleic acid encoding the gRNA), and a donor nucleic acid for introducing a specific modification to the target polynucleotide, wherein the gRNA comprises a guide sequence complementary to a target sequence in the target polynucleotide, wherein the donor nucleic acid comprises a sequence corresponding to a portion of the target polynucleotide modified to comprise the modification, and the modification is insertion of a heterologous nucleic acid in the target nucleic acid, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising Cas9 and the gRNA (Cas9/gRNA complex) and a separate complex comprising the cell-penetrating peptide associated with the donor nucleic acid. In some embodiments, the donor nucleic acid is present in a ternary complex comprising the cell-penetrating peptide, a pre-formed Cas9/gRNA complex, and the donor nucleic acid. In some embodiments, the nanoparticle core comprises a first complex comprising the cell-penetrating peptide associated with Cas9 and a second complex comprising the cell-penetrating peptide associated with the gRNA. In some embodiments, the donor nucleic acid is present in the first complex. In some embodiments, the donor nucleic acid is present in the second complex. In some embodiments, the modification is insertion of a heterologous nucleic acid greater than about 50 (such as greater than about any of 50, 100, 200, 300, 400, 500, 600, 700, 800, 900, 1000, 2000, 3000, 4000, 5000, or more, including any ranges between these values) nucleotides in length. In some embodiments, the donor nucleic acid is a double-stranded DNA molecule. The donor nucleic acid comprises a 5' homology arm that is homologous to a 5' target homology region comprising a portion of the target polynucleotide adjacent and 5' to the modification and a 3' homology arm that is homologous to a 3' target homology region comprising a portion of the target polynucleotide adjacent and 3' to the modification. In some embodiments, the 5' target homology region and/or the 3' target homology region overlap with at least about 1 (such as at least about any of 1, 2, 3, 4, 5, 6, 7, 8, 9, 10, 12, 14, 16, 18, 20, or more) nucleotide in the target sequence. In some embodiments, the 5' target homology region and/or the 3' target homology region are within about 1000 (such as within about any of 1000, 500, 400, 300, 200, 100, 75, 50, 25, 20, 15, 10, 5, or 1) nucleotides from the target sequence. In some embodiments, the 5' homology arm and the 3' homology arm are each individually greater than about 300 (such as greater than about any of 300, 400, 500, 600, 700, 800, 900, 1000, or more, including any ranges between these values) nucleotides in length.

In some embodiments, there is provided a nanoparticle for modifying one or more target polynucleotides comprising a core comprising a cell-penetrating peptide, Cas9 (or a nucleic acid encoding Cas9), and a plurality of gRNAs (or one or more nucleic acids encoding the plurality of gRNAs), wherein each of the plurality of gRNAs individually comprises a different guide sequence complementary to a target sequence in one of the one or more target polynucleotides, and wherein the cell-penetrating peptide comprises the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the nanoparticle core comprises the cell-penetrating peptide associated with a pre-formed complex comprising Cas9 and at least one of the plurality of gRNAs (Cas9/gRNA complex). In some embodiments, the pre-formed Cas9/gRNA complex comprises each of the plurality of gRNAs. In some embodiments, the nanoparticle core comprises a first complex comprising the cell-penetrating peptide associated with Cas9 and one or more complexes, each comprising the cell-penetrating peptide associated with at least one of the plurality of gRNAs. In some embodiments, the one or more complexes is a second complex comprising each of the plurality of gRNAs.

In some embodiments, the nanoparticle further comprises a surface layer comprising a peripheral CPP surrounding the core. In some embodiments, the peripheral CPP is the same as a CPP in the core. In some embodiments, the peripheral CPP is different than any of the CPPs in the core. In some embodiments, the peripheral CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the peripheral CPP is a VEPEP-3 peptide, a VEPEP-6 peptide, a VEPEP-9 peptide, or an ADGN-100 peptide. In some embodiments, at least some of the peripheral cell-penetrating peptides in the surface layer are linked to a targeting moiety. In some embodiments, the linkage is covalent. In some embodiments, the nanoparticle further comprises an intermediate layer between the core of the nanoparticle and the surface layer. In some embodiments, the intermediate layer comprises an intermediate CPP. In some embodiments, the intermediate CPP is the same as a CPP in the core. In some embodiments, the intermediate CPP is different than any of the CPPs in the core. In some embodiments, the intermediate CPP includes, but is not limited to, a PTD-based peptide, an amphipathic peptide, a poly-arginine-based peptide, an MPG peptide, a CADY peptide, a VEPEP peptide (such as a VEPEP-3, VEPEP-6, or VEPEP-9 peptide), an ADGN-100 peptide, a Pep-1 peptide, and a Pep-2 peptide. In some embodiments, the intermediate CPP is a VEPEP-3 peptide, a VEPEP-6 peptide, a VEPEP-9 peptide, or an ADGN-100 peptide.

In some embodiments, the guide sequence of a gRNA or gDNA contained in a nanoparticle according to any of the embodiments described herein is complementary to a target sequence in a gene encoding a protein involved in regulating an immune response, including immune checkpoint regulators and proteins involved in antigen presentation. In some embodiments, the guide sequence of a gRNA or gDNA contained in a nanoparticle according to any of the embodiments described herein is complementary to a target sequence in a gene encoding a protein involved in regulating cholesterol transport and/or metabolism In some embodiments, the guide sequence is complementary to a target sequence in a gene encoding a protein including, without limitation, PD-1, PD-L1, PD-L2, TIM-1, TIM-3, TIM-4, BTLA, VISTA, LAG-3, CTLA-4, TIGIT, 4-1BB, OX40, CD27, CD28, HVEM, GITR, ICOS, CD40, CD80, CD86, B7-H2, B7-H3, B7-H4, B7-H6, 2B4, CD160, gp49B, PIR-B, KIR family receptors, SIRPalpha (CD47), CD48, 2B4 (CD244), B7.1, B7.2, ILT-2, ILT-4, A2aR, toll-like receptors TLR-2, 3, 4, 6, 7, 8, and 9, granulocyte macrophage colony stimulating factor (GM-CSF), TNF, CD40L, FLT-3 ligand, cytokines such as IL-1, IL-2, IL-4, IL-7, IL-10, IL-12, IL-15, IL-21, and IL-35, FasL, TGF-β, indoleamine-2,3 dioxygenase (IDO), major histocompatibility complex (MHC) proteins, including beta-2 microglobulin (β2M), LDLR, ApoB, LDLRAP1, and PCSK9.

In some embodiments, according to any of the nanoparticles described herein, the nanoparticle further comprises a protein other than a genome-editing system molecule, or a nucleic acid molecule encoding the protein (such as a DNA plasmid or mRNA).

In some embodiments, according to any of the nanoparticles described herein, the mean size (diameter) of the nanoparticle is from about 10 nm to about 400 nm, including for example from about 50 nm to about 200 nm, from about 60 nm to about 180 nm, from about 80 nm to about 140 nm, and from about 90 nm to about 120 nm. In some embodiments, the mean size (diameter) of the nanoparticle is no greater than about 1000 nanometers (nm), such as no greater than about any of 900, 800, 700, 600, 500, 400, 300, 200, or 100 nm. In some embodiments, the average or mean diameter of the nanoparticle is no greater than about 200 nm. In some embodiments, the average or mean diameters of the nanoparticles is no greater than about 150 nm. In some embodiments, the average or mean diameter of the nanoparticle is no greater than about 100 nm. In some embodiments, the average or mean diameter of the nanoparticle is about 10 nm to about 400 nm. In some embodiments, the average or mean diameter of the nanoparticle is about 20 nm to about 400 nm. In some embodiments, the average or mean diameter of the nanoparticle is about 30 nm to about 200 nm. In some embodiments, the average or mean diameter of the nanoparticle is about 40 nm to about 200 nm. In some embodiments, the average or mean diameter of the nanoparticle is about 40 nm to about 150 nm. In some embodiments, the average or mean diameter of the nanoparticle is about 40 nm to about 100 nm. In some embodiments, the nanoparticles are sterile-filterable.

In some embodiments, the zeta potential of the nanoparticle is from about -30 mV to about 60 mV (such as about any of -30, -25, -20, -15, -10, -5, 0, 5, 10, 15, 20, 25, 30, 35, 40, 45, 50, 55, and 60 mV, including any ranges between these values). In some embodiments, the zeta potential of the nanoparticle is from about -30 mV to about 30 mV, including for example from about -25 mV to about 25 mV, from about -20 mV to about 20 mV, from about -15 mV to about 15 mV, from about -10 mV to about 10 mV, and from about -5 mV to about 10 mV. In some embodiments, the polydispersity index (PI) of the nanoparticle is from about 0.05 to about 0.6 (such as about any of 0.05, 0.1, 0.15, 0.2, 0.25, 0.3, 0.35, 0.4, 0.45, 0.5, 0.55, and 0.6, including any ranges between these values). In some embodiments, the nanoparticle is substantially non-toxic.

### Cargo

In some embodiments, a genome-editing system molecule (e.g. RGEN) of a genome-editing complex or nanoparticle described herein is a protein or polypeptide. For example, in some embodiments, a genome-editing complex or nanoparticle described herein comprises an RGEN (e.g., Cas9). In some embodiments, the protein or polypeptide is between about 10 kDa and about 200 kDa (such as about any of 10, 20, 30, 40, 50, 60, 70, 80, 90, 100, 110, 120, 130, 140, 150, 160, 170, 180, 190, and 200 kDa, including any ranges between these values). In some embodiments, the genome-editing complex or nanoparticle comprises a plurality of proteins or polypeptides, wherein each of the plurality of protein or polypeptides is between about 10 kDa and about 200 kDa (such as about any of 10, 20, 30, 40, 50, 60, 70, 80, 90, 100, 110, 120, 130, 140, 150, 160, 170, 180, 190, and 200 kDa, including any ranges between these values).

In some embodiments, a genome-editing system molecule (e.g. gRNA) of a genome-editing complex or nanoparticle described herein is a nucleic acid. In some embodiments, the nucleic acid is between about 20 nt and about 20 kb (such as about any of 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, and 20 kb, including any ranges between these values). For example, in some embodiments, a genome-editing complex or nanoparticle described herein comprises a gRNA (e.g., a Cas9 gRNA). In some embodiments, the gRNA is between about 20 nt and about 200 nt (such as about any of 20, 30, 40, 50, 60, 70, 80, 90, 100, 110, 120, 130, 140, 150, 160, 170, 180, 190, and 200 nt, including any ranges between these values). In some embodiments, the nucleic acid is DNA, such as a DNA plasmid encoding a genome-editing system molecule. In some embodiments, the DNA plasmid comprises an expression cassette for expressing the genome-editing system molecule. In some embodiments, the DNA plasmid is between about 1 kb and about 20 kb (such as about any of 1, 2, 3, 4, 5, 6, 7, 8, 9, 10, 11, 12, 13, 14, 15, 16, 17, 18, 19, and 20 kb, including any ranges between these values). In some embodiments, the nucleic acid is RNA, such as mRNA encoding a genome-editing system molecule. In some embodiments, the mRNA is between about 100 nt and about 10 kb (such as about any of 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, and 10 kb, including any ranges between these values). In some embodiments, the genome-editing complex or nanoparticle comprises a plurality of nucleic acids, such as any of the nucleic acids described herein. For example, in some embodiments, the genome-editing complex or nanoparticle comprises a gRNA and a nucleic acid encoding a genome-editing system molecule (e.g., a DNA plasmid or mRNA encoding the genome-editing system molecule). In some embodiments, the genome-editing complex or nanoparticle comprises nucleic acid encoding a plurality of genome-editing system molecules (e.g., one or more DNA plasmid encoding the plurality of genome-editing system molecules, or a plurality of mRNAs encoding the plurality of genome-editing system molecules).

In some embodiments, a genome-editing system molecule (e.g. RGEN or gRNA) of a genome-editing complex or nanoparticle described herein is replaced with a nucleic acid encoding the genome-editing system molecule. For example, in some embodiments, a genome-editing complex or nanoparticle described herein comprises a nucleic acid encoding an RGEN and/or a nucleic acid encoding a gRNA In some embodiments, the nucleic acid is DNA, such as a DNA plasmid encoding a genome-editing system molecule. In some embodiments, the DNA plasmid comprises an expression cassette for expressing the genome-editing system molecule. In some embodiments, the DNA plasmid is between about 1 kb and about 20 kb (such as about any of 1, 2, 3, 4, 5, 6, 7, 8, 9, 10, 11, 12, 13, 14, 15, 16, 17, 18, 19, and 20 kb, including any ranges between these values). In some embodiments, the nucleic acid is RNA, such as mRNA encoding a genome-editing system molecule. In some embodiments, the mRNA is between about 100 nt and about 10 kb (such as about any of 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, and 10 kb, including any ranges between these values).

"Polynucleotide," or "nucleic acid," as used interchangeably herein, refers to polymers of nucleotides of any length, and includes DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. The term "nucleic acid" as used herein refers to a polymer containing at least two deoxyribonucleotides or ribonucleotides in either single- or double-stranded form and includes DNA and RNA. DNA may be in the form of, e.g., antisense molecules, plasmid DNA, precondensed DNA, a PCR product, vectors (PAC, BAC, YAC, artificial chromosomes), expression cassettes, chimeric sequences, chromosomal DNA, or derivatives and combinations of these groups. RNA may be in the form of siRNA, asymmetrical interfering RNA (aiRNA), microRNA (miRNA), mRNA, tRNA, rRNA, RNA, viral RNA (vRNA), and combinations thereof. Nucleic acids include nucleic acids containing known nucleotide analogs or modified backbone residues or linkages, including for example locked nucleic acid (LNA), unlocked nucleic acid (UNA), and zip nucleic acid (ZNA), which can be synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer e al., Nucleic Acid Res., 19:5081 (1991); Ohtsuka et a., j . Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylases, and alkylhalides. "Oligonucleotide," as used herein, generally refers to short, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

In some embodiments, the nucleic acids are single stranded oligonucleotides. In some embodiments, the nucleic acids are double stranded oligonucleotides. The nucleic acids described herein may be any of a range of length of up to, but not necessarily 200 nucleotides in the case of antisense oligonucleotides, RNAi, siRNA, shRNA, iRNA, antagomirs or up to 1000 kilo bases in the case of plasmid DNA.

In some embodiments, the nucleic acids are plasmid DNA or DNA fragments (for example DNA fragments of lengths of up to about 1000 bp). In addition, the plasmid DNA or DNA fragments may be hypermethylated or hypomethylated. In some embodiments, the plasmid DNA or DNA fragments encode one or more genes, and may contain regulatory elements necessary for the expression of said one or more genes. In some embodiments, the plasmid DNA or DNA fragments may comprise one or more genes that encode a selectable marker, allowing for maintenance of the plasmid DNA or DNA fragment in an appropriate host cell.

### Modifications

In some embodiments, a genome-editing complex or nanoparticle as described herein comprises a targeting moiety, wherein the targeting moiety is a ligand capable of cell-specific and/or nuclear targeting. A cell membrane surface receptor and/or cell surface marker is a molecule or structure which can bind said ligand with high affinity and preferably with high specificity. Said cell membrane surface receptor and/or cell surface marker is preferably specific for a particular cell, *i.e.* it is found predominantly in one type of cell rather than in another type of cell (*e.g*. galactosyl residues to target the asialoglycoprotein receptor on the surface of hepatocytes). The cell membrane surface receptor facilitates cell targeting and internalization into the target cell of the ligand (*e.g.* the targeting moiety) and attached molecules (*e.g.* the complex or nanoparticle of the invention). A large number of ligand moieties/ligand binding partners that may be used in the context of the present invention are widely described in the literature. Such a ligand moiety is capable of conferring to the complex or nanoparticle of the invention the ability to bind to a given binding-partner molecule or a class of binding-partner molecules localized at the surface of at least one target cell. Suitable binding-partner molecules include without limitation polypeptides selected from the group consisting of cell-specific markers, tissue-specific markers, cellular receptors, viral antigens, antigenic epitopes and tumor-associated markers. Binding-partner molecules may moreover consist of or comprise, for example, one or more sugar, lipid, glycolipid, antibody molecules or fragments thereof, or aptamer. According to the invention, a ligand moiety may be for example a lipid, a glycolipid, a hormone, a sugar, a polymer (*e.g.* PEG, polylysine, PET), an oligonucleotide, a vitamin, an antigen, all or part of a lectin, all or part of a polypeptide, such as for example JTS1 (WO 94/40958), an antibody or a fragment thereof, or a combination thereof. In some embodiments, the ligand moiety used in the present invention is a peptide or polypeptide having a minimal length of 7 amino acids. It is either a native polypeptide or a polypeptide derived from a native polypeptide. "Derived" means containing (a) one or more modifications with respect to the native sequence (*e.g.* addition, deletion and/or substitution of one or more residues), (b) amino acid analogs, including non-naturally occurring amino acids, (c) substituted linkages, or (d) other modifications known in the art. The polypeptides serving as ligand moiety encompass variant and chimeric polypeptides obtained by fusing sequences of various origins, such as for example a humanized antibody which combines the variable region of a mouse antibody and the constant region of a human immunoglobulin. In addition, such polypeptides may have a linear or cyclized structure (*e.g.* by flanking at both extremities a polypeptide ligand by cysteine residues). Additionally, the polypeptide in use as a ligand moiety may include modifications of its original structure by way of substitution or addition of chemical moieties (*e.g.* glycosylation, alkylation, acetylation, amidation, phosphorylation, addition of sulfhydryl groups and the like). The invention further contemplates modifications that render the ligand moiety detectable. For this purpose, modifications with a detectable moiety can be envisaged (*i.e.* a scintigraphic, radioactive, or fluorescent moiety, or a dye label and the like). Such detectable labels may be attached to the ligand moiety by any conventional techniques and may be used for diagnostic purposes (*e.g.* imaging of tumoral cells). In some embodiments, the binding-partner molecule is an antigen (*e.g.* a target cell-specific antigen, a disease-specific antigen, an antigen specifically expressed on the surface of engineered target cells) and the ligand moiety is an antibody, a fragment or a minimal recognition unit thereof (*e.g.* a fragment still presenting an antigenic specificity) such as those described in detail in immunology manuals (see for example Immunology, third edition 1993, Roitt, Brostoff and Male, ed Gambli, Mosby). The ligand moiety may be a monoclonal antibody. Many monoclonal antibodies that bind many of these antigens are already known, and using techniques known in the art in relation to monoclonal antibody technology, antibodies to most antigens may be prepared. The ligand moiety may be a part of an antibody (for example a Fab fragment) or a synthetic antibody fragment (for example, ScFv). In some embodiments, the ligand moiety is selected among antibody fragments, rather than whole antibodies. Effective functions of whole antibodies, such as complement binding, are removed. ScFv and dAb antibody fragments may be expressed as a fusion with one or more other polypeptides. Minimal recognition units may be derived from the sequence of one or more of the complementary-determining regions (CDR) of the Fv fragment. Whole antibodies, and F(ab')2 fragments are "bivalent". By "bivalent" it is meant that said antibodies and F(ab')2 fragments have two antigen binding sites. In contrast, Fab, Fv, ScFv, dAb fragments and minimal recognition units are monovalent, having only one antigen binding sites. In some embodiments, the ligand moiety allows targeting to a tumor cell and is capable of recognizing and binding to a molecule related to the tumor status, such as a tumor-specific antigen, a cellular protein differentially or over-expressed in tumor cells or a gene product of a cancer-associated vims. Examples of tumor-specific antigens include but are not limited to MUC-1 related to breast cancer (Hareuven i et al., 990, Eur. J. Biochem 189, 475-486), the products encoded by the mutated BRCA1 and BRCA2 genes related to breast and ovarian cancers (Miki et al, 1994, Science 226, 66-7 1; Fuireal et al, 1994, Science 226, 120- 122; Wooster et al., 1995, Nature 378, 789-792), APC related to colon cancer (Poiakis, 1995, Curr. Opin. Genet. Dev. 5, 66-71), prostate specific antigen (PSA) related to prostate cancer, (Stamey et al., 1987, New England J. Med. 317, 909), carcinoma embryonic antigen (CEA) related to colon cancers (Schrewe et al., 1990, Mol. Cell. Biol. 10, 2738-2748), tyrosinase related to melanomas (Vile et al, 1993, Cancer Res. 53, 3860-3864), receptor for melanocyte-stimulating hormone (MSH) which is highly expressed in melanoma cells, ErbB-2 related to breast and pancreas cancers (Harris et al., 1994, Gene Therapy 1, 170-175), and alpha- foetoprotein related to liver cancers (Kanai et al., 1997, Cancer Res. 57, 46 1-465). In some embodiments, the ligand moiety is a fragment of an antibody capable of recognizing and binding to the MUC-1 antigen and thus targeting MUC-1 positive tumor cells. In some embodiments, the ligand moiety is the scFv fragment of the SM3 monoclonal antibody which recognizes the tandem repeat region of the MUC-1 antigen (Burshell et al., 1987, Cancer Res. 47, 5476-5482; Girling et al., 1989, Int. J. Cancer 43, 1072-1076; Dokurno et al., 1998, J. Mol. Biol. 284, 713-728). Examples of cellular proteins differentially or overexpressed in tumor cells include but are not limited to the receptor for interleukin 2 (IL-2) overexpressed in some lymphoid tumors, GRP (Gastrin Release Peptide) overexpressed in lung carcinoma cells, pancreas, prostate and stomach tumors (Michael et al., 1995, Gene Therapy 2, 660-668), TNF (Tumor Necrosis Factor) receptor, epidermal growth factor receptors, Fas receptor, CD40 receptor, CD30 receptor, CD27 receptor, OX-40, α-v integrins (Brooks et al, 994, Science 264, 569) and receptors for certain angiogenic growth factors (Hanahan, 1997, Science 277, 48). Based on these indications, it is within the scope of those skilled in the art to define an appropriate ligand moiety capable of recognizing and binding to such proteins. To illustrate, IL-2 is a suitable ligand moiety to bind to TL-2 receptor. In the case of receptors that are specific to fibrosis and inflammation, these include the TGFbeta receptors or the Adenosine receptors that are identified above and are suitable targets for invention compositions. Cell surface markers for multiple myeloma include, but are not limited to, CD56, CD40, FGFR3, CS1, CD138, IGF1R, VEGFR, and CD38, and are suitable targets for invention compositions. Suitable ligand moieties that bind to these cell surface markers include, but are not limited to, anti-CD56, anti-CD40, PRO-001, Chir-258, HuLuc63, anti-CD138-DM1, anti-IGF1R and bevacizumab.

### Targets

In some embodiments, a genome-editing complex or nanoparticle described herein comprises one or more molecules of a genome-editing system (such as the entire genome-editing system) targeting one or more genes including, but are not limited to, Adenosine receptor A2A, Adenosine receptor A2B, Adenylyl cyclase, Akt, ALK, ALK/Met, angiopoietin receptor, Angiotensin II, APC, AR, ARK5, arrestin, ATF1, ATF-2, B7-1, B7-h1 (pdl-1), β-catenin, Bcl-2, BCL2L12, Bcl6, Bcr-Abl, BRAF, BRCA1, BRCA2, BTK, caspase-2, caspase-9, CCL2, CCN1, Ccnd2, CDK-activating kinases, CEBPA, Chop, c-Jun, c-Myc, CREB, CREB1, CS1, CTGF, CTNNB1, CXCR4, Cyclin d1-2, cyclin-dependent kinases (Cdkl to 13), DEPTOR, DNMT3B, DPC4, EBOV polymerase L, EGF, EGFR, eIF5A, Elk-1, ER, Erbb4, ERK, ESR1, Etsl, EWSR1, FAK, FGF, FGFR, FOXO1, Frizzled family receptors (FZD-1 to 10), Fyn, GATA1, GATA3, GLI1, GM-CSF, GP/sGP, GSK, HBV conserved sequences, HDACs, HDGF, HDGFR, Her2, Her3, Hexokinase II, HGF, HGFR, HTF-1, HIF-1α, histone methyltransferase EZH2, HIV TAR RNA, HIV Tat, HLA-B7/Beta 2-Microglobulin, HMGA2, HNF1A, HNF1B, Hsp27, HSP47, human CCR5, Idh1, Idh2, IGF, IGFR, IKK, IKZF1, IL-12, IL-2, INK4, interferon-gamma, IRF1, IRF4, JAK, JNK, keratin 16, keratin 17, keratin K6A, keratin K6B, KGFR, KLF6, KRAS, LMO, LMO1, LMP2, LMP7, LOXL2, LPL, LYL1, MADR2, MAPK, Max, Mcl-1, MDA-7, MDM2, MDR-1, MDS1-EVI1, MECL-1, MEF2C, MEK, MEKK, MKK, MLH1, MLST8, MMP-2, MMP-9, MSFR, MSH2, MSH6, MSIN1, mTOR, MUC-1, mutant DDX3X, MYC, NCAP-D2, NCAP-D3, NCAP-G, NCAP-G2, NCAP-H, NCAP-H2, NF1, NF2, NFAT4, NF-κB, Notch1, NPC1, NR4A3, NRAS, Olig2, osteopontin, p53, PAI-1, PARP-1, patched, PAX3, PAX5, PAX7, PBX1, PDCD4, PDGF, PDGFR, PDK1, PHOX2B, PI3K, PKA, PKC, PKN3, PLK-1, PML, PR, PRAS40, PRDM16, Prdx1 and Prdx2 (burkitts lymphoma), Pre-gen/Pre-C, Pre-S1, Pre-S2/S, PTC, PTEN, Pyk2, Rad51, RAF, Raptor, Rb, RET, Rictor, RPN13, RRM2, RSV nucleocapsid, RUNX1, S6 kinase, Sapla, SETBP1, Shc, SLAMF7, Smad, Smad 3, Smad 4, Smad 7, SMC-2, SMC-4, smoothened, SOX9, SPARC, Spry2, Src, β-2 adrenergic receptors (ADRB2), β-Globin, STAT5B, STATs, survivin, Syk, Tal, TAL1, TGFR, TGF-α, TGF-β, TGFβ receptors 1, TGFβ receptors 2, TGFβ receptors 3, TGFβ1, thrombospondin, thymidine kinase, TIM-1, TIMP, TNF-α, TP53, Transthyretin, TRPV1, ubiquitin ligase, uPAR, VEGF, VEGFR, VEGFR1, VEGFR2, VEGFR3, VHL, VP24, VP30, VP35, VP40, wnt, WT1, WT2, XBP1 (spliced and unspliced), XIAP, and ZBTB16, including mutants thereof (e.g., mutant PTEN, mutant KRAS, mutant p53, and the like). For example, in some embodiments, the genome-editing complex or nanoparticle comprises one or more molecules of an RGEN-based genome-editing system (e.g., a CRISPR/Cas9 genome-editing system), wherein the RGEN-based genome-editing system comprises a gRNA targeting one of the genes described herein. In some embodiments, the genome-editing complex or nanoparticle comprises one or more molecules of a DGEN-based genome-editing system, wherein the DGEN-based genome-editing system comprises a gDNA targeting one of the genes described herein. In some embodiments, the genome-editing complex or nanoparticle comprises one or more molecules of a ZFN-based genome-editing system, wherein the ZFN targets one of the genes described herein. In some embodiments, the genome-editing complex or nanoparticle comprises one or more molecules of a TALEN-based genome-editing system, wherein the TALEN targets one of the genes described herein. In some embodiments, the genome-editing complex or nanoparticle comprises one or more molecules of a homing endonuclease-based genome-editing system, wherein the homing endonuclease targets one of the genes described herein. In some embodiments, the genome-editing complex or nanoparticle comprises one or more molecules of an integrase-based genome-editing system, wherein the integrase targets one of the genes described herein.

### Nucleic acid-guided endonucleases

In some embodiments, the nucleic acid-guided endonuclease is a CRISPR-associated nuclease. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella. Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to proteins, transcripts and other molecules involved in the activity of CRISPR-associated ("Cas") nucleases (such as RNA-guided endonucleases, or "RGENs"), including Cas gene products, Cas gene sequences, tracr (trans-activating CRISPR) sequences (e.g. tracrRNA or an active partial tracrRNA), tracr-mate sequences (including a "direct repeat" and a tracrRNA-processed partial direct repeat in the context of an endogenous CRISPR system), a guide sequence (also referred to as a "spacer" in the context of an endogenous CRISPR system), or other sequences, transcripts, and products derived from a CRISPR locus. In some embodiments, one or more molecules of a CRISPR system are derived from a type I, type II, or type III CRISPR system. In some embodiments, one or more molecules of a CRISPR system are derived from a particular organism comprising an endogenous CRISPR system, such as Streptococcus pyogenes. In general, a CRISPR system is characterized by molecules that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridization and promote formation of a CRISPR complex. A target sequence may comprise any polynucleotide, such as DNA or RNA polynucleotides. In some embodiments, a target sequence is present in the nucleus or cytoplasm of a cell. In some embodiments, the target sequence may be within an organelle of a eukaryotic cell, for example, mitochondrion or chloroplast A sequence or template that may be used for recombination into the targeted locus comprising the target sequences is referred to as an "editing template," "editing polynucleotide," "editing sequence," "donor sequence," or "donor nucleic acid". In aspects of the invention, an exogenous template polynucleotide may be referred to as an editing template. In an aspect of the invention the recombination is homologous recombination.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. The tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, the tracr sequence has sufficient complementarity to a tracr mate sequence to hybridize and participate in formation of a CRISPR complex. As with the target sequence, it is believed that complete complementarity is not needed, provided there is sufficient to be functional. In some embodiments, the tracr sequence has at least 50%, 60%, 70%, 80%, 90%, 95% or 99% of sequence complementarity along the length of the tracr mate sequence when optimally aligned. In some embodiments, one or more molecules of a CRISPR system are introduced into a host cell such that formation of a CRISPR complex at one or more target sites can occur. For example, a Cas nuclease, a guide sequence linked to a tracr-mate sequence, and a tracr sequence could each be introduced into a host cell to allow formation of a CRISPR complex at a target sequence in the host cell complementary to the guide sequence.

Non-limiting examples of Cas proteins include Cas1, Cas1B, Cas2, Cas3, Cas4, Cas5, Cas6, Cas7, Cas8, Cas9 (also known as Csn1 and Csx12), Cas10, Cpfl, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csxl7, Csxl4, Csx10, Csxl6, CsaX, Csx3, Csx1, Csx15, Csf1, Csf2, Csf3, Csf4, homologs thereof, or modified versions thereof, such as inducible, inactivated, or split Cas proteins (*see* for example Dominguez et al. (2015). Nature Reviews Molecular Cell Biology*;* Polstein, L. R, & Gersbach, C. A. (2015). Nature chemical biology, 11(3):198-200; Dow et al. (2015). Nature biotechnology, 33(4):390-394; Zetsche et al. (2015). Nature biotechnology, 33(2):139-142; Kleinstiver et al. (2015). Nature. 523:481-485; Bikard et al. (2013). Nucleic acids research, 41(15):7429-7437; Qi et al. (2013). Cell, 152(5):1173-1183). These enzymes are known to those of skill in the art; for example, the amino acid sequence of S. pyogenes Cas9 protein may be found in the SwissProt database under accession number Q99ZW2, and the amino acid sequence of Acidaminococcus sp. Cpf1 protein may be found in the SwissProt database under accession number U2UMQ6. In some embodiments, the unmodified CRISPR enzyme has DNA cleavage activity, such as Cas9. In some embodiments the CRISPR enzyme is Cas9, and may be Cas9 from S. pyogenes or S. pneumoniae. In some embodiments the CRISPR enzyme is Cpfl, and may be Cpf1 from Acidaminococcus or Lachnospiraceae. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, the CRISPR enzyme is mutated with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. In some embodiments, a Cas9 nickase may be used in combination with guide sequences, e.g., two guide sequences, which target respectively sense and antisense strands of the DNA target. This combination allows both strands to be nicked and used to induce NHEJ.

As a further example, two or more catalytic domains of Cas9 (RuvC I, RuvC II, and RuvC III) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form Other mutations may be useful; where the Cas9 or other CRISPR enzyme is from a species other than S. pyogenes, mutations in corresponding amino acids may be made to achieve similar effects.

In some embodiments, the Cas protein (such as Cas9) is a split Cas protein comprising an N-terminal Cas protein fragment, Cas(N), and a C-terminal Cas protein fragment, Cas(C), wherein Cas(N) is fused to a first dimerization domain and Cas(C) is fused to a second dimerization domain, and wherein the first and second dimerization domains facilitate dimerization of Cas(N) and Cas(C) to form a complex with a functional Cas nuclease activity. In some embodiments, dimerization of the first and second dimerization domains is sensitive to a dimerization agent. For example, in some embodiments, the first and second dimerization domains comprise the FK506 binding protein 12 (FKBP) and FKBP rapamycin binding (FRB) domains of the mammalian target of rapamycin (mTOR), and the dimerization agent is rapamycin.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. See Nakamura, Y, et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, Pa.), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a CRISPR enzyme comprises one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In some embodiments, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Typically, an NLS consists of one or more short sequences of positively charged lysines or arginines exposed on the protein surface, but other types of NLS are known. Non-limiting examples of NLSs include an NLS sequence derived from: the NLS of the SV40 virus large T-antigen, having the amino acid sequence PKKKRKV (SEQ ID NO: 83); the NLS from nucleoplasmin (e.g. the nucleoplasmin bipartite NLS with the sequence KRPAATKKAGQAKKKK (SEQ ID NO: 84)); the c-myc NLS having the amino acid sequence PAAKRVKLD (SEQ ID NO: 85) or RQRRNELKRSP (SEQ ID NO: 86); the hRNPA1 M9 NLS having the sequence NQSSNFGPMKGGNFGGRSSGPYGGGGQYFAKPRNQGGY (SEQ ID NO: 87); the sequence RMRIZFKNKGKDTAELRRRRVEVSVELRKAKKDEQILKRRNV (SEQ ID NO: 88) of the IBB domain from importin-alpha; the sequences VSRKRPRP (SEQ ID NO: 89) and PPKKARED (SEQ ID NO: 90) of the myoma T protein; the sequence PQPKKKPL (SEQ ID NO: 91) of human p53; the sequence SALIKKKKKMAP (SEQ ID NO: 92) of mouse c-ab1 IV; the sequences DRLRR (SEQ ID NO: 93) and PKQKKRK (SEQ ID NO: 94) of the influenza virus NS1; the sequence RKLKKKIKKL (SEQ ID NO: 95) of the Hepatitis virus delta antigen; the sequence REKKKFLKRR (SEQ ID NO: 96) of the mouse Mx1 protein; the sequence KRKGDEVDGVDEVAKKKSKK (SEQ ID NO: 97) of the human poly(ADP-ribose) polymerase; and the sequence RKCLQAGMNLEARKTKK (SEQ ID NO: 98) of the steroid hormone receptors (human) glucocorticoid.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, Calif.), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. In some embodiments, a guide sequence is selected to reduce the degree of secondary structure within the guide sequence. Secondary structure may be determined by any suitable polynucleotide folding algorithm Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A. R Gruber et al., 2008, Cell 106(1): 23-24; and P A Carr and G M Church, 2009, Nature Biotechnology 27(12): 1151-62). Further algorithms may be found in U.S. application Ser. No. 61/836,080; incorporated herein by reference.

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the guide sequence, tracr sequence and tracr mate sequence are contained within a single RNA (referred to herein as a "single-guide RNA," or "sgRNA"), such that hybridization between the tracr sequence and the tracr mate sequence produces a secondary structure, such as a hairpin. Preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In an embodiment of the invention, the sgRNA has at least two or more hairpins. In preferred embodiments, the sgRNA has two, three, four or five hairpins. In a further embodiment of the invention, the sgRNA has at most five hairpins. In some embodiments, the sgRNA further includes a transcription termination sequence; preferably this is a polyT sequence, for example six T nucleotides.

In some embodiments, a donor nucleic acid is also provided. In some embodiments, the donor nucleic acid is designed to serve as a template in homologous recombination, such as within or near a target sequence nicked or cleaved by a CRISPR enzyme as a part of a CRISPR complex. A donor nucleic acid may be of any suitable length, such as about or more than about 10, 15, 20, 25, 50, 75, 100, 150, 200, 500, 1000, or more nucleotides in length. In some embodiments, the donor nucleic acid comprises a sequence that is complementary to a portion of a polynucleotide comprising the target sequence. In some embodiments, when a donor nucleic acid and a polynucleotide comprising a target sequence are optimally aligned, the donor nucleic acid overlaps with one or more nucleotides of the target sequence (e.g. about or more than about 1, 5, 10, 15, 20, 25, 30, 35, 40, 45, 50, 60, 70, 80, 90, 100 or more nucleotides). In some embodiments, when a donor nucleic acid and a polynucleotide comprising a target sequence are optimally aligned, the nearest nucleotide of the donor nucleic acid in the region of complementarity is within about 1, 5, 10, 15, 20, 25, 50, 75, 100, 200, 300, 400, 500, 1000, 5000, 10000, or more nucleotides from the target sequence.

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502, incorporated herein by reference. In some embodiments, a tagged CRISPR enzyme is used to identify the location of a target sequence.

### ZFPs and ZFNs; TALs, TALEs, and TALENs

In some embodiments, the genome-editing system includes a DNA-binding protein such as one or more zinc finger protein (ZFP) or transcription activator-like protein (TAL), fused to an effector protein such as an endonuclease (or nucleic acid encoding the DNA-binding protein/effector protein fusion). Examples include ZFNs, TALEs, and TALENs. See Lloyd et al., Fronteirs in Immunology, 4(221), 1-7 (2013).

### ZFPs and ZFNs

In some embodiments, the genome-editing system comprises one or more zinc-finger proteins (ZFPs) or domains thereof that bind to DNA in a sequence-specific manner. AZFP or domain thereof is a protein or domain within a larger protein that binds DNA in a sequence-specific manner through one or more zinc fingers, regions of amino acid sequence within the binding domain whose structure is stabilized through coordination of a zinc ion. The term zinc finger DNA binding protein is often abbreviated as zinc finger protein or ZFP.

Among the ZFPs are artificial ZFP domains targeting specific DNA sequences, typically 9-18 nucleotides long, generated by assembly of individual fingers.

ZFPs include those in which a single finger domain is approximately 30 amino acids in length and contains an alpha helix containing two invariant histidine residues coordinated through zinc with two cysteines of a single beta turn, and having two, three, four, five, or six fingers. Generally, sequence-specificity of a ZFP may be altered by making amino acid substitutions at the four helix positions (-1, 2, 3 and 6) on a zinc finger recognition helix. Thus, in some embodiments, the ZFP or ZFP-containing molecule is non-naturally occurring, e.g., is engineered to bind to a target site of choice. See, for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416; U.S. Pat. Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061, all incorporated herein by reference in their entireties.

In some embodiments, the genome-editing system includes a zinc-finger DNA binding domain fused to a DNA cleavage domain to form a zinc-finger nuclease (ZFN). In some embodiments, fusion proteins comprise the cleavage domain (or cleavage half-domain) from at least one Type IIS restriction enzyme and one or more zinc finger binding domains, which may or may not be engineered. In some embodiments, the cleavage domain is from the Type IIS restriction endonuclease Fok I. Fok I generally catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982.]

In some embodiments, ZFNs target a gene present in a target cell. In some aspects, the ZFNs efficiently generate a double strand break (DSB), for example at a predetermined site in the coding region of the gene. Typical regions targeted include exons, regions encoding N-terminal regions, first exon, second exon, and promoter or enhancer regions. In some embodiments, transient expression of the ZFNs promotes highly efficient and permanent disruption of the target gene in target cells. In particular, in some embodiments, delivery of the ZFNs results in the permanent disruption of the gene with efficiencies surpassing 50%.

Many gene-specific engineered zinc fingers are available commercially. For example, Sangamo Biosciences (Richmond, CA, USA) has developed a platform (CompoZr) for zinc-finger construction in partnership with Sigma-Aldrich (St. Louis, MO, USA), allowing investigators to bypass zinc-finger construction and validation altogether, and provides specifically targeted zinc fingers for thousands of proteins. Gaj et al., Trends in Biotechnology, 2013, 31(7), 397-405. In some embodiments, commercially available zinc fingers are used or are custom designed. (See, for example, Sigma-Aldrich catalog numbers CSTZFND, CSTZFN, CTI1-1KT, and PZD0020).

### TALEs and TALENs

In some embodiments, the genome-editing system includes a naturally occurring or engineered (non-naturally occurring) transcription activator-like protein (TAL) DNA binding domain, such as in a transcription activator-like protein effector (TALE) protein, See, e.g., U.S. Patent Publication No. 20110301073, incorporated by reference in its entirety herein.

A TALE DNA binding domain or TALE is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein. Each TALE repeat unit includes 1 or 2 DNA-binding residues making up the Repeat Variable Diresidue (RVD), typically at positions 12 and/or 13 of the repeat The natural (canonical) code for DNA recognition of these TALEs has been determined such that an HD sequence at positions 12 and 13 leads to a binding to cytosine (C), NG binds to T, NI to A, NN binds to G or A, and NG binds to T and non-canonical (atypical) RVDs are also known. See, U.S. Patent Publication No. 20110301073. In some embodiments, TALEs may be targeted to any gene by design of TAL arrays with specificity to the target DNA sequence. The target sequence generally begins with a thymidine.

In some embodiments, the genome-editing system includes a DNA binding endonuclease, such as a TALE-nuclease (TALEN). In some aspects the TALEN is a fusion protein comprising a DNA-binding domain derived from a TALE and a nuclease catalytic domain to cleave a nucleic acid target sequence. In some embodiments, the TALE DNA-binding domain has been engineered to bind a target sequence within a target gene.

In some embodiments, the TALEN recognizes and cleaves the target sequence in the gene. In some aspects, cleavage of the DNA results in double-stranded breaks. In some aspects the breaks stimulate the rate of homologous recombination or non-homologous end joining (NHEJ). Generally, NHEJ is an imperfect repair process that often results in changes to the DNA sequence at the site of the cleavage. In some aspects, repair mechanisms involve rejoining of what remains of the two DNA ends through direct re-ligation (Critchlow and Jackson, Trends Biochem Sci. 1998 Oct;23(10):394-8) or via the so-called microhomology-mediated end joining. In some embodiments, repair via NHEJ results in small insertions or deletions and can be used to disrupt and thereby repress the gene. In some embodiments, the modification may be a substitution, deletion, or addition of at least one nucleotide. In some aspects, cells in which a cleavage-induced mutagenesis event, i.e. a mutagenesis event consecutive to an NHEJ event, has occurred can be identified and/or selected by well-known methods in the art

In some embodiments, TALE repeats are assembled to specifically target a gene. (Gaj et al., Trends in Biotechnology, 2013, 31(7), 397-405). A library of TALENs targeting 18,740 human protein-coding genes has been constructed (Kim et al., Nature Biotechnology. 31, 251-258 (2013)). Custom-designed TALE arrays are commercially available through Cellectis Bioresearch (Paris, France), Transposagen Biopharmaceuticals (Lexington, KY, USA), and Life Technologies (Grand Island, NY, USA).

In some embodiments the TALENs are introduced as transgenes encoded by one or more plasmid vectors. In some aspects, the plasmid vector can contain a selection marker which provides for identification and/or selection of cells which received said vector.

### Homing endonucleases

In some embodiments, the genome-editing system includes a homing nuclease (or nucleic acid encoding the homing nuclease, such as an mRNA or a DNA plasmid). Homing endonucleases are a collection of endonucleases encoded either as freestanding genes within introns, as fusions with host proteins, or as self-splicing inteins. They catalyze the hydrolysis of genomic DNA within the cells that synthesize them, but do so at very few, or even singular, locations. Homing endonucleases bind very long, and in many cases asymmetric, recognition sequences spanning 12 to 40 bp. Homing endonuclease recognition sequences are long enough to occur randomly only with a very low probability (approximately once every 7×10⁹ bp), and are normally found in one or very few instances per genome. Repair of the hydrolyzed DNA by the host cell frequently results in the gene encoding the homing endonuclease having been copied into the cleavage site, hence the term "homing" to describe the movement of these genes.

Homing endonucleases act as monomers or homodimers, yet often require associated proteins to regulate their activity, or form ribonucleoprotein complexes, wherein RNA is an integral component of the catalytic apparatus. Currently there are six known structural families. Their conserved structural motifs are: LAGLIDADG, GIY-YIG, His-Cys box, H-N-H, PD-(D/E)xK, and Vsr-like.

In some embodiments, the homing endonuclease is a synthetic enzyme (such as a fully synthetic enzyme) modified to target a specific DNA sequence. For example, in some embodiments, the homing endonuclease is an ARC nuclease™ (Precision BioSciences).

### Integrases

In some embodiments, the genome-editing system includes an integrase (or nucleic acid encoding the integrase). In some embodiments, the genome-editing system further includes a donor nucleic acid comprising a first recombination site recognized by the integrase. In some embodiments, a polynucleotide targeted for modification by the genome-editing system comprises a second recombination site recognized by the integrase, such that the integrase is capable of mediating recombination between the first recombination site and the second recombination site.

Many bacteriophage and integrative plasmids encode site-specific recombination systems that enable the stable incorporation of their genome into those of their hosts. In these systems, the minimal requirements for the recombination reaction are a recombinase enzyme, or integrase, which catalyzes the recombination event, and two recombination sites (Sadowski (1986) J. Bacteriol. 165: 341-347; Sadowski (1993) FASEB J. 7: 760-767). For phage integration systems, these are referred to as attachment (att) sites, with an attP element from phage DNA and the attB element encoded by the bacterial genome. The two attachment sites can share as little sequence identity as a few base pairs. The recombinase protein binds to both att sites and catalyzes a conservative and reciprocal exchange of DNA strands that result in integration of the circular phage or plasmid DNA into host DNA. Additional phage or host factors, such as the DNA binding protein IHF (integration host factor), may be required for an efficient reaction (Friedman (1988) Cell 55:545-554; Finkel & Johnson (1992) Mol. Microbiol. 6: 3257-3265). The reverse excision reaction sometimes requires an additional phage factor, such as the xis gene product of phage λ (Weisberg & Landy (1983) "Site-specific recombination in phage lambda." In Lambda II, eds. Hendrix et al (Cold Spring Harbor Laboratory, Cold Spring Harbor, NY) pp.211-250; Landy (1989) Ann. Rev. Biochem 58: 913-949).

The recombinases have been categorized into two groups, the λ integrase (Argos et al. (1986) EMBOJ. 5: 433-44; Voziyanov et al. (1999) Nucl. Acids Res. 27: 930- 941) and the resolvase/invertase (Hatfull & Grindley (1988) "Resolvases and DNA- invertases: a family of enzymes active in site-specific recombination" In Genetic Recombination, eds. Kucherhpati, R., & Smith, G. R. (Am. Soc. Microbiol., Washington DC), pp. 357-396) families. These vary in the structure of the integrase enzymes and the molecular details of their mode of catalysis (Stark et al. (1992) Trends Genetics 8: 432-439). The temperate Streptomyces phage ΦC31 encodes a 68 kD recombinase of the latter class. The ΦC31 integration reaction is simple in that it does not require a host factor and appears irreversible, most likely because an additional phage protein is required for excision. The phage and bacterial att sites share only three base pairs of homology at the point of cross-over. This homology is flanked by inverted repeats, presumably binding sites for the integrase protein. The minimal known functional size for both attB and attP is ∼50 bp. The Cre-lox system of bacteriophage PI, and the FLP-FRT system of Saccharomyces cerevisiae have been widely used for transgene and chromosome engineering in animals and plants (reviewed by Sauer (1994) Curr. Opin. Biotechnol. 5: 521- 527; Ow (1996) Curr. Opin. Biotechnol. 7: 181-186). Other systems that operate in animal or plant cells include the following: 1) the R-RS system from Zygosaccharomyces rouxii (Onouchi et al (1995) Mol Gen. Genet. 247: 653-660), 2) the Gin-gix system from bacteriophage Mu (Maeser & Kahmann (1991) Mol. Gen. Genet. 230: 170-176) and, 3) the β recombinase system from bacterial plasmid pSM19035 (Diaz et al. (1999) J. Biol. Chem. 21 A: 6634-6640). By using the site-specific recombinases, one can obtain a greater frequency of integration.

In some embodiments, the integrase is a prokaryotic recombinase, such as a bacteriophage integrase. In some embodiments, the integrase is unidirectional, in that it can catalyze recombination between two complementary recombination sites, but cannot catalyze recombination between the hybrid sites that are formed by this recombination. In some embodiments, the integrase is the ΦC31 integrase. The ΦC31 integrase, by itself, catalyzes only the attB x attP reaction, and cannot mediate recombination between the attL and attR sites that are formed upon recombination between attB and attP. Because recombinases such as the ΦC31 integrase cannot alone catalyze the reverse reaction, the attB x attP recombination formed by such recombinases is stable.

The methods involve contacting a pair of recombination sites (e.g., attB and attP) that are present in a eukaryotic cell with a corresponding recombinase. The recombinase then mediates recombination between the recombination sites. Depending upon the relative locations of the two recombination sites, any one of a number of events can occur as a result of the recombination. For example, if the two recombination sites are present on different nucleic acid molecules, the recombination can result in integration of one nucleic acid molecule into a second molecule. Thus, one can obtain integration of a plasmid that contains one recombination site into a eukaryotic cell chromosome that includes the corresponding recombination site. Because the recombinases used in the methods of the invention cannot catalyze the reverse reaction, the integration is stable. Such methods are useful, for example, for obtaining stable integration into the eukaryotic chromosome of a transgene that is present on the plasmid. The two recombination sites can also be present on the same nucleic acid molecule. In such cases, the resulting product typically depends upon the relative orientation of the sites. For example, recombination between sites that are in the direct orientation will generally result in excision of any DNA that lies between the two recombination sites. In contrast, recombination between sites that are in the reverse orientation can result in inversion of the intervening DNA. Again, the resulting rearranged nucleic acid is stable in that the recombination is irreversible in the absence of an additional factor, generally encoded by the particular bacteriophage from which the recombinase is derived, that is not normally found in eukaryotic cells. One example of an application for which this method is useful involves the placement of a promoter between the two recombination sites. If the promoter is initially in the opposite orientation relative to a coding sequence that is to be expressed by the promoter and the recombination sites that flank the promoter are in the inverted orientation, contacting the recombination sites will result in inversion of the promoter, thus placing the promoter in the correct orientation to drive expression of the coding sequence. Similarly, if the promoter is initially in the correct orientation for expression and the recombination sites are in the same orientation, contacting the recombination sites with the promoter can result in excision of the promoter fragment, thus stopping expression of the coding sequence.

### Nucleic adds

In some embodiments, the genome-editing system includes a nucleic acid. In some embodiments, the nucleic acid encodes a genome-editing enzyme, such as a nuclease or integrase. In some embodiments, the nucleic acid is a guide RNA or guide DNA (or a nucleic acid encoding a guide RNA or guide DNA, such as an mRNA or a DNA plasmid). In some embodiments, the nucleic acid is a donor nucleic acid, such as for introduction of a modification to a target polynucleotide by homologous recombination or insertion of an exogenous nucleic acid into a target polynucleotide by site-directed recombination. In some embodiments, the nucleic acid is RNA, such as an mRNA. In some embodiments, the nucleic acid is DNA, such as a DNA plasmid. In some embodiments, the nucleic acid of a genome-editing system comprises at least one modification, such as modifications that affect stability, subcellular targeting, protein binding affinity, complementary target sequence binding affinity, resistance to cellular degradation, and/or cellular permeability. In some embodiments, the at least one modification includes a label. Such modifications and methods for their introduction to nucleic acids are well known in the art and are contemplated for use with any of the nucleic acids described herein. *See* for example WO2011015347, WO2015026885, WO2015026887, WO2015026883, and WO2015026886.

In some embodiments, the genome-editing system comprises a nucleic acid comprising at least one modification selected from the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking, a modification or sequence that provides a binding site for proteins, a Locked Nucleic Acid (LNA), a 5-methyl dC nucleotide, a 2,6-Diaminopurine nucleotide, a 2'-Fluoro A nucleotide, a 2'-Fluoro U nucleotide; a 2-O-Methyl RNA nucleotide, a phosphorothioate bond, linkage to a cholesterol molecule, linkage to a polyethylene glycol molecule, linkage to a spacer 18 molecule, a 5' to 3' covalent linkage, and any combination thereof

### Compositions

In some embodiments, there is provided a composition comprising a genome-editing complex or nanoparticle as described herein. In some embodiments, the composition is a pharmaceutical composition comprising a genome-editing complex or nanoparticle as described herein and a pharmaceutically acceptable diluent, excipient and/or carrier. In some embodiments, the concentration of the complex or nanoparticle in the composition is from about 1 nM to about 100 mM, including for example from about 10 nM to about 50 mM, from about 25 nM to about 25 mM, from about 50 nM to about 10 mM, from about 100 nM to about 1 mM, from about 500 nM to about 750 µM, from about 750 nM to about 500 µM, from about 1 µM to about 250 µM, from about 10 µM to about 200 µM, and from about 50 µM to about 150 µM.

The term "pharmaceutically acceptable diluent, excipient, and/or carrier" as used herein is intended to include any and all solvents, dispersion media, coatings, antibacterial and antifungal agents, isotonic and absorption delaying agents, and the like, compatible with administration to humans or other vertebrate hosts. Typically, a pharmaceutically acceptable diluent, excipient, and/or carrier is a diluent, excipient, and/or carrier approved by a regulatory agency of a Federal, a state government, or other regulatory agency, or listed in the U.S. Pharmacopeia or other generally recognized pharmacopeia for use in animals, including humans as well as non-human mammals. The term diluent, excipient, and/or "carrier" refers to a diluent, adjuvant, excipient, or vehicle with which the pharmaceutical composition is administered. Such pharmaceutical diluent, excipient, and/or carriers can be sterile liquids, such as water and oils, including those of petroleum, animal, vegetable or synthetic origin. Water, saline solutions and aqueous dextrose and glycerol solutions can be employed as liquid diluents, excipients, and/or carriers, particularly for injectable solutions. Suitable pharmaceutical diluents and/or excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like, including lyophilization aids. The composition, if desired, can also contain minor amounts of wetting, bulking, emulsifying agents, or pH buffering agents. These compositions can take the form of solutions, suspensions, emulsion, sustained release formulations and the like. Examples of suitable pharmaceutical diluent, excipient, and/or carriers are described in "Remington's Pharmaceutical Sciences" by E. W. Martin. The formulation should suit the mode of administration. The appropriate diluent, excipient, and/or carrier will be evident to those skilled in the art and will depend in large part upon the route of administration.

In some embodiments, a pharmaceutical composition as described herein is formulated for intravenous, intratumoral, intraarterial, topical, intraocular, ophthalmic, intraportal, intracranial, intracerebral, intracerebroventricular, intrathecal, intravesicular, intradermal, subcutaneous, intramuscular, intranasal, intratracheal, pulmonary, intracavity, or oral administration.

In some embodiments, dosages of the pharmaceutical compositions of the present invention found to be suitable for treatment of human or mammalian subjects are in the range of about 0.001 mg/kg to about 100 mg/kg (such as about any of 0.001, 0.01, 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95, and 100 mg/kg, including any ranges between these values) of the genome-editing complexes or nanoparticles. In some embodiments, dosage ranges are about 0.1 mg/kg to about 20 mg/kg (such as about any of 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, and 20 mg/kg, including any ranges between these values). In some embodiments, dosage ranges are about 0.5 mg/kg to about 10 mg/kg.

Exemplary dosing frequencies include, but are not limited to, weekly without break; weekly, three out of four weeks; once every three weeks; once every two weeks; weekly, two out of three weeks. In some embodiments, the pharmaceutical composition is administered about once every 2 weeks, once every 3 weeks, once every 4 weeks, once every 6 weeks, or once every 8 weeks. In some embodiments, the pharmaceutical composition is administered at least about any of 1×, 2×, 3×, 4×, 5×, 6×, or 7× (i.e., daily) a week. In some embodiments, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week. In some embodiments, the schedule of administration of the pharmaceutical composition to an individual ranges from a single administration that constitutes the entire treatment to daily administration. The administration of the pharmaceutical composition can be extended over an extended period of time, such as from about a month up to about seven years. In some embodiments, the pharmaceutical composition is administered over a period of at least about any of 2, 3, 4, 5, 6, 7, 8, 9, 10, 11, 12, 18, 24, 30, 36, 48, 60, 72, or 84 months.

In some embodiments, there is provided a pharmaceutical composition comprising a genome-editing complex or nanoparticle as described herein and a pharmaceutically acceptable carrier, wherein the pharmaceutically acceptable carrier is a sugar or a protein. In some embodiments, the sugar is selected from the group consisting of sucrose, glucose, mannitol, and a combination thereof, and is present in the pharmaceutical composition at a concentration from about 5% to about 20%. In some embodiments, the sugar is sucrose. In some embodiments, the sugar is glucose. In some embodiments, the sugar is mannitol. In some embodiments, the protein is albumin. In some embodiments, the albumin is human serum albumin. In some embodiments, the pharmaceutical composition is lyophilized.

In some embodiments, a pharmaceutical composition as described herein further comprises an expression complex comprising a cell-penetrating peptide (such as any of the cell-penetrating peptides described herein) and a nucleic acid molecule encoding an exogenous construct. In some embodiments, the exogenous construct is an exogenous protein or nucleic acid. In some embodiments, the molar ratio of cell-penetrating peptide to nucleic acid molecule in the expression complex is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of cell-penetrating peptide to nucleic acid molecule in the expression complex is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the cell-penetrating peptide includes, without limitation, CADY, PEP-1, MPG, VEPEP-3, VEPEP-4, VEPEP,-5, VEPEP-6, VEPEP-9, and ADGN-100. In some embodiments, the cell-penetrating peptide in the expression complex comprises an amino acid sequence selected from the group consisting of SEQ ID NOs: 1-70 and 75-80. In some embodiments, the cell-penetrating peptide is the same as a cell-penetrating peptide in a genome-editing complex in the pharmaceutical composition. In some embodiments, the cell-penetrating peptide is not the same as a cell-penetrating peptide in a genome-editing complex in the pharmaceutical composition. In some embodiments, the exogenous protein is a therapeutic protein. In some embodiments, the exogenous protein is or comprises an antibody or antigen-binding fragment thereof. In some embodiments, the exogenous protein is a recombinant receptor, such as a recombinant receptor capable of being expressed on the surface of a cell. In some embodiments, the recombinant receptor comprises a ligand-binding domain, wherein upon ligand binding, the receptor is capable of transducing a signal through a signaling domain. In some embodiments, the recombinant receptor comprises the signaling domain. In some embodiments, the recombinant receptor is capable of associating with an endogenous protein comprising the signaling domain. In some embodiments, the ligand-binding domain is or comprises an antibody or antigen-binding fragment thereof. In some embodiments, the recombinant receptor is an antigen receptor, such as a chimeric antigen receptor (CAR).

Thus, in some embodiments, there is provided a composition, such as a pharmaceutical composition, comprising a genome-editing complex or nanoparticle as described herein and an expression complex as described herein. In some embodiments, such compositions, for example pharmaceutical compositions, can be used to simultaneously effect gene editing and gene expression in a cell.

Exemplary antigen receptors, including CARs, and methods for engineering such receptors, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

In some embodiments of a recombinant receptor comprising a ligand-binding domain described herein, the ligand-binding domain specifically binds to a ligand associated with a disease or condition. In some embodiments, the ligand-binding domain specifically binds to a cancer associated antigen or a pathogen-specific antigen. In some embodiments, the ligand-binding domain is specific for viral antigens (such as HIV, HCV, HBV, etc.), bacterial antigens, and/or parasitic antigens. In some embodiments, the ligand-binding domain specifically binds to a ligand including, without limitation, a orphan tyrosine kinase receptor ROR1, tEGFR, Her2, L1-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, OEPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, CS-1, c-Met, GD-2, and MAGE A3, CE7, Wilms Tumor 1 (WT-1), a cyclin, such as cyclin A1 (CCNA1), and/or biotinylated molecules, and/or molecules expressed by HIV, HCV, HBV or other pathogens.

### Methods of preparation

In some embodiments, there is provided a method of preparing a genome-editing complex or nanoparticle as described herein comprising combining a CPP with one or more molecules of a genome-editing system, thereby forming the genome-editing complex or nanoparticle. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (e.g., CRISPR/Cas9). In some embodiments, some of the one or more genome-editing system molecules are in a pre-formed complex prior to combination with the CPP. In some embodiments, the genome-editing system comprises an integrase (or a nucleic acid encoding the integrase). In some embodiments, the genome-editing system further comprises a donor nucleic acid comprising a recombination site recognized by the integrase.

Thus, in some embodiments, there is provided a method of preparing a genome-editing complex or nanoparticle as described herein comprising a) combining a CPP with an RGEN (or a nucleic acid encoding the RGEN) and a gRNA (or a nucleic acid encoding the gRNA); or b) combining a CPP with a pre-formed RGEN/gRNA complex, thereby forming the genome-editing complex or nanoparticle.

For example, in some embodiments, there is provided a method of preparing a genome-editing complex or nanoparticle as described herein comprising a) combining a first composition comprising one or more molecules of a genome-editing system (such as a genome-editing enzyme, or a nucleic acid encoding the genome-editing enzyme) with a second composition comprising a cell-penetrating peptide in an aqueous medium to form a mixture; and b) incubating the mixture to form a complex comprising the cell-penetrating peptide associated with the one or more genome-editing system molecules, thereby generating the genome-editing complex or nanoparticle. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (*e.g.,* CRISPR/Cas9). In some embodiments, some of the one or more genome-editing system molecules are in a pre-formed complex prior to combination with the CPP. In some embodiments, the genome-editing system comprises an integrase (or a nucleic acid encoding the integrase). In some embodiments, the genome-editing system further comprises a donor nucleic acid comprising a recombination site recognized by the integrase. In some embodiments, the aqueous medium is a buffer, including for example PBS, Tris, or any buffer known in the art for stabilizing nucleoprotein complexes. In some embodiments, the first composition comprising the one or more genome-editing system molecules is a solution comprising at least one of the one or more genome-editing system molecules (such as a genome-editing nuclease, a gRNA or gDNA, and/or a donor nucleic acid) at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the first composition comprising the one or more genome-editing system molecules is a solid comprising the one or more genome-editing system molecules in lyophilized form and a suitable carrier. In some embodiments, the second composition comprising the cell-penetrating peptide is a solution comprising the cell-penetrating peptide at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the second composition comprising the cell-penetrating peptide is a solid comprising the cell-penetrating peptide in lyophilized form and a suitable carrier. In some embodiments, the solutions are formulated in water. In some embodiments, the water is distilled water. In some embodiments, the solutions are formulated in a buffer. In some embodiments, the buffer is any buffer known in the art used for storing a nucleic acid or polypeptide. In some embodiments, the molar ratio of a genome-editing nuclease to a gRNA or gDNA in the first composition is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of a genome-editing nuclease to a gRNA or gDNA in the first composition is about 1:1. In some embodiments, the molar ratio of the cell-penetrating peptide to a genome-editing nuclease or complex comprising the genome-editing nuclease in the mixture is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of the cell-penetrating peptide a genome-editing nuclease or complex comprising the genome-editing nuclease in the mixture is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the mixture is incubated to form a complex or nanoparticle comprising the cell-penetrating peptide associated with the one or more genome-editing system molecules for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C, thereby resulting in a solution comprising the genome-editing complex or nanoparticle. In some embodiments, the solution comprising the genome-editing complex or nanoparticle remains stable for at least about three weeks, including for example for at least about any of 6 weeks, 2 months, 3 months, 4 months, 5 months, and 6 months at 4°C. In some embodiments, the solution comprising the genome-editing complex or nanoparticle is lyophilized in the presence of a carrier. In some embodiments, the carrier is a sugar, including for example, sucrose, glucose, mannitol and combinations thereof, and is present in the solution comprising the genome-editing complex or nanoparticle at from about 5% to about 20%, including for example from about 7.5% to about 17.5%, from about 10% to about 15%, and about 12.5%, weight per volume. In some embodiments, the carrier is a protein, including for example albumin, such as human serum albumin. In some embodiments, the cell-penetrating peptide is a VEPEP-3 peptide, a VEPEP-6 peptide, a VEPEP-9 peptide, or an ADGN-100 peptide as described herein. In some embodiments, the cell-penetrating peptide comprises the amino acid sequence of any one of SEQ ID NOs: 75-80.

In some embodiments, there is provided a method of preparing a genome-editing complex or nanoparticle as described herein comprising a) combining a composition comprising an RGEN with a composition comprising a gRNA in an aqueous medium to form a first mixture; b) incubating the first mixture to form an RGEN/gRNA complex comprising the RGEN and the gRNA; c) combining a composition comprising the RGEN/gRNA complex, such as the mixture of b), with a composition comprising a cell-penetrating peptide in an aqueous medium to form a second mixture; d) incubating the second mixture to form a complex comprising the cell-penetrating peptide associated with the RGEN/gRNA complex, thereby generating the genome-editing complex or nanoparticle. In some embodiments, step c) of the method comprises combining the composition comprising the RGEN/gRNA complex with the composition comprising the cell-penetrating peptide and a composition comprising a donor nucleic acid in an aqueous medium to form the second mixture. In some embodiments, the composition comprising the RGEN/gRNA complex and the composition comprising the donor nucleic acid are a single composition. In some embodiments, the aqueous medium is a buffer, including for example PBS, Tris, or any buffer known in the art for stabilizing nucleoprotein complexes. In some embodiments, the composition comprising the RGEN is a solution comprising the RGEN at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the RGEN is a solid comprising the RGEN in lyophilized form and a suitable carrier. In some embodiments, the composition comprising the gRNA is a solution comprising the gRNA at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the gRNA is a solid comprising the gRNA in lyophilized form and a suitable carrier. In some embodiments, the composition comprising the cell-penetrating peptide is a solution comprising the cell-penetrating peptide at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the cell-penetrating peptide is a solid comprising the cell-penetrating peptide in lyophilized form and a suitable carrier. In some embodiments, the composition comprising the donor nucleic acid is a solution comprising the donor nucleic acid at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the donor nucleic acid is a solid comprising the donor nucleic acid in lyophilized form and a suitable carrier. In some embodiments, the solutions are formulated in water. In some embodiments, the water is distilled water. In some embodiments, the solutions are formulated in a buffer. In some embodiments, the buffer is any buffer known in the art used for storing a nucleic acid or polypeptide. In some embodiments, the molar ratio of the RGEN to the gRNA in the first mixture is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of the RGEN to the gRNA in the first mixture is about 1:1. In some embodiments, the molar ratio of the cell-penetrating peptide to the RGEN/gRNA complex in the second mixture is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of the cell-penetrating peptide to the RGEN/gRNA complex in the second mixture is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the first mixture is incubated to form the RGEN/gRNA complex for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C, thereby resulting in a solution comprising the RGEN/gRNA complex. In some embodiments, the second mixture is incubated to form the complex comprising the cell-penetrating peptide associated with the RGEN/gRNA complex for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C, thereby resulting in a solution comprising the genome-editing complex or nanoparticle. In some embodiments, the solution comprising the genome-editing complex or nanoparticle remains stable for at least about three weeks, including for example for at least about any of 6 weeks, 2 months, 3 months, 4 months, 5 months, and 6 months at 4°C. In some embodiments, the solution comprising the genome-editing complex or nanoparticle is lyophilized in the presence of a carrier. In some embodiments, the carrier is a sugar, including for example, sucrose, glucose, mannitol and combinations thereof, and is present in the solution comprising the genome-editing complex or nanoparticle at from about 5% to about 20%, including for example from about 7.5% to about 17.5%, from about 10% to about 15%, and about 12.5%, weight per volume. In some embodiments, the carrier is a protein, including for example albumin, such as human serum albumin. In some embodiments, the cell-penetrating peptide is a VEPEP-3 peptide, a VEPEP-6 peptide, a VEPEP-9 peptide, or an ADGN-100 peptide as described herein. In some embodiments, the cell-penetrating peptide comprises the amino acid sequence of any one of SEQ ID NOs: 75-80.

In some embodiments, there is provided a method of preparing a genome-editing complex or nanoparticle as described herein comprising a) combining a composition comprising a cell-penetrating peptide, a composition comprising an RGEN (or a nucleic acid encoding the RGEN), and a composition comprising a gRNA (or a nucleic acid encoding the gRNA) in an aqueous medium to form a mixture; b) incubating the mixture to form a complex comprising the cell-penetrating peptide associated with the RGEN and the gRNA, thereby generating the genome-editing complex or nanoparticle. In some embodiments, step a) of the method comprises combining the composition comprising the cell-penetrating peptide, the composition comprising the RGEN, the composition comprising the gRNA, and a composition comprising a donor nucleic acid in an aqueous medium to form the mixture. In some embodiments, at least two of the composition comprising the RGEN, the composition comprising the gRNA, and the composition comprising the donor nucleic acid are a single composition (*e.g.,* a single composition comprises the gRNA and the donor nucleic acid). In some embodiments, the aqueous medium is a buffer, including for example PBS, Tris, or any buffer known in the art for stabilizing nucleoprotein complexes. In some embodiments, the composition comprising the RGEN is a solution comprising the RGEN at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the RGEN is a solid comprising the RGEN in lyophilized form and a suitable carrier. In some embodiments, the composition comprising the gRNA is a solution comprising the gRNA at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the gRNA is a solid comprising the gRNA in lyophilized form and a suitable carrier. In some embodiments, the composition comprising the cell-penetrating peptide is a solution comprising the cell-penetrating peptide at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the cell-penetrating peptide is a solid comprising the cell-penetrating peptide in lyophilized form and a suitable carrier. In some embodiments, the composition comprising the donor nucleic acid is a solution comprising the donor nucleic acid at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the donor nucleic acid is a solid comprising the donor nucleic acid in lyophilized form and a suitable carrier. In some embodiments, the solutions are formulated in water. In some embodiments, the water is distilled water. In some embodiments, the solutions are formulated in a buffer. In some embodiments, the buffer is any buffer known in the art used for storing a nucleic acid or polypeptide. In some embodiments, the molar ratio of the RGEN to the gRNA in the mixture is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of the RGEN to the gRNA in the mixture is about 1:1. In some embodiments, the molar ratio of the cell-penetrating peptide to the RGEN in the mixture is between about 1:1 and about 80:1 (such as about any of 1:1, 5:1,10:1, 20:1, 30:1, 40:1, 50:1, 60:1, 70:1, 80:1, including any ranges between these ratios). In some embodiments, the molar ratio of the cell-penetrating peptide to the RGEN in the mixture is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the mixture is incubated to form the complex comprising the cell-penetrating peptide associated with the RGEN and the gRNA for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C, thereby resulting in a solution comprising the genome-editing complex or nanoparticle. In some embodiments, the solution comprising the genome-editing complex or nanoparticle remains stable for at least about three weeks, including for example for at least about any of 6 weeks, 2 months, 3 months, 4 months, 5 months, and 6 months at 4°C. In some embodiments, the solution comprising the genome-editing complex or nanoparticle is lyophilized in the presence of a carrier. In some embodiments, the carrier is a sugar, including for example, sucrose, glucose, mannitol and combinations thereof, and is present in the solution comprising the genome-editing complex or nanoparticle at from about 5% to about 20%, including for example from about 7.5% to about 17.5%, from about 10% to about 15%, and about 12.5%, weight per volume. In some embodiments, the carrier is a protein, including for example albumin, such as human serum albumin. In some embodiments, the cell-penetrating peptide is a VEPEP-3 peptide, a VEPEP-6 peptide, a VEPEP-9 peptide, or an ADGN-100 peptide as described herein. In some embodiments, the cell-penetrating peptide comprises the amino acid sequence of any one of SEQ ID NOs: 75-80.

In some embodiments, there is provided a method of preparing a nanoparticle comprising a core and at least one additional layer as described herein, comprising a) combining a composition comprising an RGEN with a composition comprising a gRNA in an aqueous medium to form a first mixture; b) incubating the first mixture to form an RGEN/gRNA complex comprising the RGEN and the gRNA; c) combining a composition comprising the RGEN/gRNA complex, such as the mixture of b), with a composition comprising a first cell-penetrating peptide in an aqueous medium to form a second mixture; d) incubating the second mixture to form a core of the nanoparticle comprising the first cell-penetrating peptide associated with the RGEN/gRNA complex; e) combining a composition comprising the core of the nanoparticle, such as the mixture of d), with a composition comprising a second cell-penetrating peptide in an aqueous medium to form a third mixture, and f) incubating the third mixture to form a nanoparticle comprising a core and at least one additional layer. In some embodiments, the method further comprises g) combining a composition comprising the nanoparticle comprising a core and at least one additional layer and a composition comprising a third cell-penetrating peptide in an aqueous medium to form a fourth mixture, and h) incubating the fourth mixture to form a nanoparticle comprising a core and at least two additional layers. It is to be appreciated that the method can be adapted to form a nanoparticle comprising increasing numbers of layers. In some embodiments, at least one of the steps of combining (*e.g.* step a), c), e), or g)) further comprises combination with a composition comprising a donor nucleic acid. In some embodiments, the aqueous medium is a buffer, including for example PBS, Tris, or any buffer known in the art for stabilizing nucleoprotein complexes. In some embodiments, the composition comprising the RGEN is a solution comprising the RGEN at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the RGEN is a solid comprising the RGEN in lyophilized form and a suitable carrier. In some embodiments, the composition comprising the gRNA is a solution comprising the gRNA at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the gRNA is a solid comprising the gRNA in lyophilized form and a suitable carrier. In some embodiments, the compositions comprising the first, second, and/or third cell-penetrating peptides are each a solution comprising the cell-penetrating peptide at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the compositions comprising the first, second, and/or third cell-penetrating peptides are each a solid comprising the cell-penetrating peptide in lyophilized form and a suitable carrier. In some embodiments, the composition comprising the donor nucleic acid is a solution comprising the donor nucleic acid at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the donor nucleic acid is a solid comprising the donor nucleic acid in lyophilized form and a suitable carrier. In some embodiments, the solutions are formulated in water. In some embodiments, the water is distilled water. In some embodiments, the solutions are formulated in a buffer. In some embodiments, the buffer is any buffer known in the art used for storing a nucleic acid or polypeptide. In some embodiments, the molar ratio of the RGEN to the gRNA in the first mixture is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of the RGEN to the gRNA in the first mixture is about 1:1. In some embodiments, the molar ratio of the first cell-penetrating peptide to the RGEN/gRNA complex in the second mixture is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of the first cell-penetrating peptide to the RGEN/gRNA complex in the second mixture is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the first, second, third, and/or fourth mixtures are each incubated for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C. In some embodiments, the solution comprising the nanoparticle remains stable for at least about three weeks, including for example for at least about any of 6 weeks, 2 months, 3 months, 4 months, 5 months, and 6 months at 4°C. In some embodiments, the solution comprising the nanoparticle is lyophilized in the presence of a carrier. In some embodiments, the carrier is a sugar, including for example, sucrose, glucose, mannitol and combinations thereof, and is present in the solution comprising the genome-editing complex or nanoparticle at from about 5% to about 20%, including for example from about 7.5% to about 17.5%, from about 10% to about 15%, and about 12.5%, weight per volume. In some embodiments, the carrier is a protein, including for example albumin, such as human serum albumin. In some embodiments, the first, second, and/or third cell-penetrating peptides are each a VEPEP-3 peptide, a VEPEP-6 peptide, a VEPEP-9 peptide, or an ADGN-100 peptide as described herein. In some embodiments, the first, second, and/or third cell-penetrating peptides each comprises the amino acid sequence of SEQ ID NO: 75, 76, 77, 78, 79, or 80.

In some embodiments, there is provided a method of preparing a nanoparticle comprising a core and at least one additional layer as described herein, comprising a) combining a composition comprising a first cell-penetrating peptide, a composition comprising an RGEN (or a nucleic acid encoding the RGEN), and a composition comprising a gRNA in an aqueous medium to form a first mixture; b) incubating the first mixture to form a core of the nanoparticle comprising the first cell-penetrating peptide associated with the RGEN and the gRNA; c) combining a composition comprising the core of the nanoparticle, such as the mixture of b), with a composition comprising a second cell-penetrating peptide in an aqueous medium to form a second mixture, and d) incubating the second mixture to form a nanoparticle comprising a core and at least one additional layer. In some embodiments, the method further comprises e) combining a composition comprising the nanoparticle comprising a core and at least one additional layer and a composition comprising a third cell-penetrating peptide in an aqueous medium to form a third mixture, and f) incubating the third mixture to form a nanoparticle comprising a core and at least two additional layers. It is to be appreciated that the method can be adapted to form a nanoparticle comprising increasing numbers of layers. In some embodiments, at least one of the steps of combining (*e.g.* step a), c), or e)) further comprises combination with a composition comprising a donor nucleic acid. In some embodiments, the aqueous medium is a buffer, including for example PBS, Tris, or any buffer known in the art for stabilizing nucleoprotein complexes. In some embodiments, the composition comprising the RGEN is a solution comprising the RGEN at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 nM, 5 µM, 10 µM, 25 µM, 50 µM, 100 µM, 150 µM, or 200 µM, including any ranges between these values). In some embodiments, the composition comprising the RGEN is a solid comprising the RGEN in lyophilized form and a suitable carrier. In some embodiments, the composition comprising the gRNA is a solution comprising the gRNA at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the gRNA is a solid comprising the gRNA in lyophilized form and a suitable carrier. In some embodiments, the compositions comprising the first, second, and/or third cell-penetrating peptides are each a solution comprising the cell-penetrating peptide at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the compositions comprising the first, second, and/or third cell-penetrating peptides are each a solid comprising the cell-penetrating peptide in lyophilized form and a suitable carrier. In some embodiments, the composition comprising the donor nucleic acid is a solution comprising the donor nucleic acid at a concentration from about 1 nM to about 200 µM (such as about any of 2 nM, 5 nM, 10 nM, 25 nM, 50 nM, 100 nM, 200 nM, 300 nM, 400 nM, 500 nM, 600 nM, 700 nM, 800 nM, 900 nM, 1 µM, 2 µM, 5 µM, 10 µM, 25 µM, 50 µM, 100 µM, 150 µM, or 200 µM, including any ranges between these values). In some embodiments, the composition comprising the donor nucleic acid is a solid comprising the donor nucleic acid in lyophilized form and a suitable carrier. In some embodiments, the solutions are formulated in water. In some embodiments, the water is distilled water. In some embodiments, the solutions are formulated in a buffer. In some embodiments, the buffer is any buffer known in the art used for storing a nucleic acid or polypeptide. In some embodiments, the molar ratio of the RGEN to the gRNA in the first mixture is between about 10:1 and about 1:10 (such as about any of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, and 1:10, including any ranges between these ratios). In some embodiments, the molar ratio of the RGEN to the gRNA in the first mixture is about 1:1. In some embodiments, the molar ratio of the first cell-penetrating peptide to the RGEN in the first mixture is between about 1:1 and about 80:1 (such as about any of 1:1, 5:1, 10:1, 20:1, 30:1, 40:1, 50:1, 60:1, 70:1, 80:1, including any ranges between these ratios). In some embodiments, the molar ratio of the first cell-penetrating peptide to the RGEN in the first mixture is between about 5:1 and about 20:1 (such as about any of 5:1, 6:1, 7:1, 8:1, 9:1, 10:1, 11:1, 12:1, 13:1, 14:1, 15:1, 16:1, 17:1, 18:1, 19:1, and 20:1, including any ranges between these ratios). In some embodiments, the first, second, and/or third mixtures are each incubated for from about 10 min to 60 min, including for example for about any of 20 min, 30 min, 40min, and 50 min, at a temperature from about 2°C to about 50°C, including for example from about 2°C to about 5°C, from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, and from about 45°C to about 50°C. In some embodiments, the solution comprising the nanoparticle remains stable for at least about three weeks, including for example for at least about any of 6 weeks, 2 months, 3 months, 4 months, 5 months, and 6 months at 4°C. In some embodiments, the solution comprising the nanoparticle is lyophilized in the presence of a carrier. In some embodiments, the carrier is a sugar, including for example, sucrose, glucose, mannitol and combinations thereof, and is present in the solution comprising the genome-editing complex or nanoparticle at from about 5% to about 20%, including for example from about 7.5% to about 17.5%, from about 10% to about 15%, and about 12.5%, weight per volume. In some embodiments, the carrier is a protein, including for example albumin, such as human serum albumin. In some embodiments, the first, second, and/or third cell-penetrating peptides are each a VEPEP-3 peptide, a VEPEP-6 peptide, a VEPEP-9 peptide, or an ADGN-100 peptide as described herein. In some embodiments, the first, second, and/or third cell-penetrating peptides each comprises the amino acid sequence of SEQ ID NO: 75, 76, 77, 78, 79, or 80.

In some embodiments, for a stable composition comprising a genome-editing complex or nanoparticle of the invention, the average diameter of the complex or nanoparticle does not change by more than about 10%, and the polydispersity index does not change by more than about 10%.

### Methods of use

### Methods of disease treatment

In some embodiments, there is provided a method of treating a disease or condition in an individual comprising administering to the individual an effective amount of a pharmaceutical composition comprising a genome-editing complex or nanoparticle as described herein for modifying a target polynucleotide and a pharmaceutically acceptable carrier, wherein the genome-editing complex or nanoparticle comprises one or more molecules of a genome-editing system useful for the treatment of the disease or condition. In some embodiments, the genome-editing complex or nanoparticle comprises a CPP comprising the amino acid sequence of a VEPEP-3 peptide, a VEPEP-6 peptide, a VEPEP-9 peptide, or an ADGN-100 peptide. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (*e.g.,* CRISPR/Cas9). In some embodiments, the genome-editing system comprises an integrase (or a nucleic acid encoding the integrase). In some embodiments, the genome-editing system further comprises a donor nucleic acid comprising a recombination site recognized by the integrase. In some embodiments, the disease or condition to be treated includes, but is not limited to, cancer, diabetes, inflammatory diseases, fibrosis, viral infectious diseases, hereditary diseases, ocular diseases, aging and degenerative diseases, and diseases characterized by cholesterol level abnormality. In some embodiments, the genome-editing system is capable of modifying the sequence of one or more genes. In some embodiments, the genome-editing system is capable of modulating the expression of one or more genes. In some embodiments, the one or more genes encode proteins including, but not limited to, growth factors and cytokines, cell surface receptors, signaling molecules and kinases, transcription factors and other modulators of transcription, regulators of protein expression and modification, tumor suppressors, and regulators of apoptosis and metastasis. In some embodiments, the genome-editing complex or nanoparticle comprises the entire genome-editing system, such that administration of the pharmaceutical composition to the individual is sufficient to modify the target polynucleotide in a cell of the individual. In some embodiments, the pharmaceutical composition further comprises one or more additional genome-editing complexes or nanoparticles as described herein, wherein the plurality of genome-editing complexes or nanoparticles in the pharmaceutical composition comprise the entire genome-editing system, such that administration of the pharmaceutical composition to the individual is sufficient to modify the target polynucleotide in a cell of the individual. In some embodiments, the method further comprises administering to the individual an effective amount of one or more additional pharmaceutical compositions comprising one or more additional genome-editing complexes or nanoparticles as described herein, wherein the plurality of genome-editing complexes or nanoparticles in the plurality of pharmaceutical compositions comprise the entire genome-editing system, such that administration of the plurality of pharmaceutical composition to the individual is sufficient to modify the target polynucleotide in a cell of the individual. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the pharmaceutical composition further comprises an expression complex according to any of the embodiments described herein. In some embodiments, the pharmaceutical composition further comprises an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease or condition.

"Modulation" of activity or expression used herein means regulating or altering the status or copy numbers of a gene or mRNA or changing the amount of gene product such as a protein that is produced. In some embodiments, the genome-editing system inhibits the expression of a target gene. In some embodiments, the genome-editing system inhibits the expression of the gene or gene product by at least about any of 0%, 20%, 30%, 40%, 60%, 70%, 80%, 90%, or 100%.

In some embodiments, there is provided a method of treating a disease or condition in an individual comprising administering to the individual an effective amount of a pharmaceutical composition comprising a genome-editing complex or nanoparticle as described herein for modifying a target polynucleotide and a pharmaceutically acceptable carrier, wherein the genome-editing complex or nanoparticle comprises one or more molecules of a genome-editing system useful for the treatment of the disease or condition and a cell-penetrating peptide comprising the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (*e.g.,* CRISPR/Cas9). In some embodiments, the genome-editing system comprises an integrase (or a nucleic acid encoding the integrase). In some embodiments, the genome-editing system further comprises a donor nucleic acid comprising a recombination site recognized by the integrase. In some embodiments, the disease or condition to be treated includes, but is not limited to, cancer, diabetes, inflammatory diseases, fibrosis, viral infectious diseases, hereditary diseases, ocular diseases, aging and degenerative diseases, and cholesterol level abnormality. In some embodiments, the genome-editing complex or nanoparticle in the pharmaceutical composition comprises one or more molecules of a genome-editing system for modifying the sequence of one or more genes in the individual. In some embodiments, the genome-editing complex or nanoparticle in the pharmaceutical composition comprises one or more molecules of a genome-editing system for modulating the expression of one or more genes in the individual. In some embodiments, the one or more genes encode proteins including, but not limited to, growth factors and cytokines, cell surface receptors, signaling molecules and kinases, transcription factors and other modulators of transcription, regulators of protein expression and modification, tumor suppressors, and regulators of apoptosis and metastasis. In some embodiments, the genome-editing complex or nanoparticle comprises the entire genome-editing system, such that administration of the pharmaceutical composition to the individual is sufficient to modify the target polynucleotide in a cell of the individual. In some embodiments, the pharmaceutical composition further comprises one or more additional genome-editing complexes or nanoparticles as described herein, wherein the plurality of genome-editing complexes or nanoparticles in the pharmaceutical composition comprise the entire genome-editing system, such that administration of the pharmaceutical composition to the individual is sufficient to modify the target polynucleotide in a cell of the individual. In some embodiments, the method further comprises administering to the individual an effective amount of one or more additional pharmaceutical compositions comprising one or more additional genome-editing complexes or nanoparticles as described herein, wherein the plurality of genome-editing complexes or nanoparticles in the plurality of pharmaceutical compositions comprise the entire genome-editing system, such that administration of the plurality of pharmaceutical composition to the individual is sufficient to modify the target polynucleotide in a cell of the individual. In some embodiments, the target polynucleotide is modified to inactivate a target gene, such as by decreasing expression of the target gene or resulting in a modified target gene that expresses an inactive product. In some embodiments, the target polynucleotide is modified to activate a target gene, such as by increasing expression of the target gene or resulting in a modified target gene that expresses an active target gene product. In some embodiments, the pharmaceutical composition further comprises an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a chimeric antigen receptor. In some embodiments, the chimeric antigen receptor specifically binds to an antigen associated with the disease or condition.

In some embodiments of the methods described herein, the genome-editing complex or nanoparticle comprises one or more molecules of a genome-editing system (such as the entire genome-editing system) targeting one or more genes including, but are not limited to, Adenosine receptor A2A, Adenosine receptor A2B, Adenylyl cyclase, Akt, ALK, ALK/Met, angiopoietin receptor, Angiotensin II, APC, AR, ARK5, arrestin, ATF1, ATF-2, B7-1, B7-hl (pdl-1), β-catenin, Bcl-2, BCL2L12, Bcl6, Bcr-Abl, BRAF, BRCA1, BRCA2, BTK, caspase-2, caspase-9, CCL2, CCN1, Ccnd2, CDK-activating kinases, CEBPA, Chop, c-Jun, c-Myc, CREB, CREB1, CS1, CTGF, CTNNB1, CXCR4, Cyclin dl-2, cyclin-dependent kinases (Cdkl to 13), DEPTOR, DNMT3B, DPC4, EBOV polymerase L, EGF, EGFR, eIF5A, Elk-1, ER, Erbb4, ERK, ESR1, Etsl, EWSR1, FAK, FGF, FGFR, FOXO1, Frizzled family receptors (FZD-1 to 10), Fyn, GATA1, GATA3, GLI1, GM-CSF, GP/sGP, GSK, HBV conserved sequences, HDACs, HDGF, HDGFR, Her2, Her3, Hexokinase II, HGF, HGFR, HIF-1, HIF-1α, histone methyltransferase EZH2, HIV TAR RNA, HIV Tat, HLA-B7/Beta 2-Microglobulin, HMGA2, HNF1A, HNF1B, Hsp27, HSP47, human CCR5, Idh1, Idh2, IGF, IGFR, IKK, IKZF1, IL-12, IL-2, INK4, interferon-gamma, IRF1, IRF4, JAK, JNK, keratin 16, keratin 17, keratin K6A, keratin K6B, KGFR, KLF6, KRAS, LMO, LMO1, LMP2, LMP7, LOXL2, LPL, LYL1, MADR2, MAPK, Max, Mcl-1, MDA-7, MDM2, MDR-1, MDS1-EVI1, MECL-1, MEF2C, MEK, MEKK, MKK, MLH1, MLST8, MMP-2, MMP-9, MSFR, MSH2, MSH6, MSIN1, mTOR, MUC-1, mutant DDX3X, MYC, NCAP-D2, NCAP-D3, NCAP-G, NCAP-G2, NCAP-H, NCAP-H2, NF1, NF2, NFAT4, NF-κB, Notch1, NPC1, NR4A3, NRAS, Olig2, osteopontin, p53, PAI-1, PARP-1, patched, PAX3, PAX5, PAX7, PBX1, PDCD4, PDGF, PDGFR, PDK1, PHOX2B, PI3K, PKA, PKC, PKN3, PLK-1, PML, PR, PRAS40, PRDM16, Prdx1 and Prdx2 (burkitts lymphoma), Pre-gen/Pre-C, Pre-S1, Pre-S2/S, PTC, PTEN, Pyk2, Rad51, RAF, Raptor, Rb, RET, Rictor, RPN13, RRM2, RSV nucleocapsid, RUNX1, S6 kinase, Sap1a, SETBP1, Shc, SLAMF7, Smad, Smad 3, Smad 4, Smad 7, SMC-2, SMC-4, smoothened, SOX9, SPARC, Spry2, Src, B-2 adrenergic receptors (ADRB2), β-Globin, STAT5B, STATs, survivin, Syk, Tal, TAL1, TGFR, TGF-α, TGF-β, TGFβ receptors 1, TGFβ receptors 2, TGFβ receptors 3, TGFβ1, thrombospondin, thymidine kinase, TIM-1, TIMP, TNF-α, TP53, Transthyretin, TRPV1, ubiquitin ligase, uPAR, VEGF, VEGFR, VEGFR1, VEGFR2, VEGFR3, VHL, VP24, VP30, VP35, VP40, wnt, WT1, WT2, XBP1 (spliced and unspliced), XIAP, and ZBTB16, including mutants thereof (e.g., mutant PTEN, mutant KRAS, mutant p53, and the like).

### Diseases and conditions

In some embodiments of the methods described herein, the disease to be treated is cancer. In some embodiments, the cancer is a solid tumor, and the pharmaceutical composition comprises a genome-editing complex or nanoparticle comprising one or more molecules of a genome-editing system that modulates the expression of one or more genes that encode proteins including, but not limited to, growth factors and cytokines, cell surface receptors, signaling molecules and kinases, transcription factors and other modulators of transcription, regulators of protein expression and modification, tumor suppressors, and regulators of apoptosis and metastasis. In some embodiments, the growth factors or cytokines include, but are not limited to, EGF, VEGF, FGF, HGF, HDGF, IGF, PDGF, TGF-α, TGF-β, TNF-α, and wnt, including mutants thereof. In some embodiments, the cell surface receptors include, but are not limited to, ER, PR, Her2, Her3, angiopoietin receptor, EGFR, FGFR, HGFR, HDGFR, IGFR, KGFR, MSFR, PDGFR, TGFR, VEGFR1, VEGFR2, VEGFR3, Frizzled family receptors (FZD-1 to 10), smoothened, patched, and CXCR4, including mutants thereof. In some embodiments, the signaling molecules or kinases include, but are not limited to, KRAS, NRAS, RAF, MEK, MEKK, MAPK, MKK, ERK, JNK, JAK, PKA, PKC, PI3K, Akt, mTOR, Raptor, Rictor, MLST8, PRAS40, DEPTOR, MSIN1, S6 kinase, PDK1, BRAF, FAK, Src, Fyn, Shc, GSK, IKK, PLK-1, cyclin-dependent kinases (Cdkl to 13), CDK-activating kinases, ALK/Met, Syk, BTK, Bcr-Abl, RET, β-catenin, Mcl-1, and PKN3, including mutants thereof. In some embodiments, the transcription factors or other modulators of transcription include, but are not limited to, AR, ATF1, CEBPA, CREB1, ESR1, EWSR1, FOXO1, GATA1, GATA3, HNF1A, HNF1B, IKZF1, IRF1, IRF4, KLF6, LMO1, LYL1, MYC, NR4A3, PAX3, PAX5, PAX7, PBX1, PHOX2B, PML, RUNX1, SMAD4, SMAD7, STAT5B, TALI, TP53, WT1, ZBTB16, ATF-2, Chop, c-Jun, c-Myc, DPC4, Elk-1, Etsl, Max, MEF2C, NFAT4, Sapla, STATs, Tal, p53, CREB, NF-κB, HDACs, HIF-1α, and RRM2, including mutants thereof. In some embodiments, the regulators of protein expression or modification include, but are not limited to, ubiquitin ligase, LMP2, LMP7, and MECL-1, including mutants thereof. In some embodiments, the tumor suppressors include, but are not limited to, APC, BRCA1, BRCA2, DPC4, INK4, MADR2, MLH1, MSH2, MSH6, NF1, NF2, p53, PTC, PTEN, Rb, VHL, WT1, and WT2, including mutants thereof. In some embodiments, the regulators of apoptosis or metastasis include, but are not limited to, XIAP, Bcl-2, osteopontin, SPARC, MMP-2, MMP-9, uPAR, including mutants thereof.

In some embodiments of the methods described herein, the disease to be treated is cancer, wherein the cancer is a solid tumor, and the pharmaceutical composition comprises a genome-editing complex or nanoparticle comprising one or more molecules of a genome-editing system (such as the entire genome-editing system) targeting one or more genes encoding proteins involved in tumor development and/or progression. In some embodiments, the one or more genes encoding proteins involved in tumor development and/or progression include, but are not limited to, IL-2, IL-12, interferon-gamma, GM-CSF, B7-1, caspase-9, p53, MUC-1, MDR-1, HLA-B7/Beta 2-Microglobulin, Her2, Hsp27, thymidine kinase, and MDA-7, including mutants thereof. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (*e.g.,* CRISPR/Cas9). In some embodiments, the genome-editing system comprises an integrase (or a nucleic acid encoding the integrase). In some embodiments, the genome-editing system further comprises a donor nucleic acid comprising a recombination site recognized by the integrase.

In some embodiments of the methods described herein, the disease to be treated is cancer, wherein the cancer is liver cancer, and the pharmaceutical composition comprises a genome-editing complex or nanoparticle comprising one or more molecules of a genome-editing system (such as the entire genome-editing system) targeting one or more genes encoding proteins involved in liver cancer development and/or progression. In some embodiments, the liver cancer is hepatocellular carcinoma, cholangiocarcinoma, angiosarcoma of the liver, or hemangiosarcoma of the liver. In some embodiments, the one or more genes encoding proteins involved in liver cancer development and/or progression include, but are not limited to, CCND2, RAD23B, GRP78, CEP164, MDM2, and ALDH2, including mutants thereof.

In some embodiments, according to any of the methods described herein, the cancer is hepatocellular carcinoma (HCC). In some embodiments, the HCC is early stage HCC, non-metastatic HCC, primary HCC, advanced HCC, locally advanced HCC, metastatic HCC, HCC in remission, or recurrent HCC. In some embodiments, the HCC is localized resectable (*i.e.,* tumors that are confined to a portion of the liver that allows for complete surgical removal), localized unresectable (*i.e.,* the localized tumors may be unresectable because crucial blood vessel structures are involved or because the liver is impaired), or unresectable (*i.e.,* the tumors involve all lobes of the liver and/or has spread to involve other organs (*e.g.,* lung, lymph nodes, bone). In some embodiments, the HCC is, according to TNM classifications, a stage I tumor (single tumor without vascular invasion), a stage II tumor (single tumor with vascular invasion, or multiple tumors, none greater than 5 cm), a stage III tumor (multiple tumors, any greater than 5 cm, or tumors involving major branch of portal or hepatic veins), a stage IV tumor (tumors with direct invasion of adjacent organs other than the gallbladder, or perforation of visceral peritoneum), N1 tumor (regional lymph node metastasis), or M1 tumor (distant metastasis). In some embodiments, the HCC is, according to AJCC (American Joint Commission on Cancer) staging criteria, stage T1, T2, T3, or T4 HCC. In some embodiments, the HCC is any one of liver cell carcinomas, fibrolamellar variants of HCC, and mixed hepatocellular cholangiocarcinomas. In some embodiments, the individual may be a human who has a gene, genetic mutation, or polymorphism associated with hepatocellular carcinoma (e.g., mutation or polymorphism in CCND2, RAD23B, GRP78, CEP164, MDM2, and/or ALDH2) or has one or more extra copies of a gene associated with hepatocellular carcinoma.

In some embodiments of the methods described herein, the disease to be treated is cancer, wherein the cancer is lung cancer, and the pharmaceutical composition comprises a genome-editing complex or nanoparticle comprising one or more molecules of a genome-editing system (such as the entire genome-editing system) targeting one or more genes encoding proteins involved in lung cancer development and/or progression. In some embodiments, the one or more genes encoding proteins involved in lung cancer development and/or progression include, but are not limited to, SASH1, LATS1, IGF2R, PARK2, KRAS, PTEN, Kras2, Krag, Pas1, ERCC1, XPD, IL8RA, EGFR, Ot₁-AD, EPHX, MMP1, MMP2, MMP3, MMP12, IL1 β, RAS, and AKT, including mutants thereof.

In some embodiments, according to any of the methods described herein, the cancer is lung cancer. In some embodiments, the lung cancer is a non-small cell lung cancer (NSCLC). Examples of NSCLC include, but are not limited to, large-cell carcinoma (e.g., large-cell neuroendocrine carcinoma, combined large-cell neuroendocrine carcinoma, basaloid carcinoma, lymphoepithelioma-like carcinoma, clear cell carcinoma, and large-cell carcinoma with rhabdoid phenotype), adenocarcinoma (e.g., acinar, papillary (e.g., bronchioloalveolar carcinoma, nonmucinous, mucinous, mixed mucinous and nonmucinous and indeterminate cell type), solid adenocarcinoma with mucin, adenocarcinoma with mixed subtypes, well-differentiated fetal adenocarcinoma, mucinous (colloid) adenocarcinoma, mucinous cystadenocarcinoma, signet ring adenocarcinoma, and clear cell adenocarcinoma), neuroendocrine lung tumors, and squamous cell carcinoma (e.g., papillary, clear cell, small cell, and basaloid). In some embodiments, the NSCLC is, according to TNM classifications, a stage T tumor (primary tumor), a stage N tumor (regional lymph nodes), or a stage M tumor (distant metastasis). In some embodiments, the lung cancer is a carcinoid (typical or atypical), adenosquamous carcinoma, cylindroma, or carcinoma of the salivary gland (e.g., adenoid cystic carcinoma or mucoepidermoid carcinoma). In some embodiments, the lung cancer is a carcinoma with pleomorphic, sarcomatoid, or sarcomatous elements (e.g., carcinomas with spindle and/or giant cells, spindle cell carcinoma, giant cell carcinoma, carcinosarcoma, or pulmonary blastoma). In some embodiments, the cancer is small cell lung cancer (SCLC; also called oat cell carcinoma). The small cell lung cancer may be limited-stage, extensive stage or recurrent small cell lung cancer. In some embodiments, the individual may be a human who has a gene, genetic mutation, or polymorphism suspected or shown to be associated with lung cancer (e.g., mutation or polymorphism in SASH1, LATS1, IGF2R, PARK2, KRAS, PTEN, Kras2, Krag, Pas1, ERCC1, XPD, IL8RA, EGFR, Ot₁-AD, EPHX, MMP1, MMP2, MMP3, MMP12, IL1 β, RAS, and/or AKT) or has one or more extra copies of a gene associated with lung cancer.

In some embodiments of the methods described herein, the disease to be treated is cancer, wherein the cancer is renal cell carcinoma (RCC), and the pharmaceutical composition comprises a genome-editing complex or nanoparticle comprising one or more molecules of a genome-editing system (such as the entire genome-editing system) targeting one or more genes encoding proteins involved in RCC development and/or progression. In some embodiments, the one or more genes encoding proteins involved in RCC development and/or progression include, but are not limited to, VHL, TSC1, TSC2, CUL2, MSH2, MLH1, INK4a/ARF, MET, TGF- α, TGF-β1, IGF-I, IGF-IR, AKT, and PTEN, including mutants thereof.

In some embodiments, according to any of the methods described above, the cancer is renal cell carcinoma. In some embodiments, the renal cell carcinoma is an adenocarcinoma. In some embodiments, the renal cell carcinoma is a clear cell renal cell carcinoma, papillary renal cell carcinoma (also called chromophilic renal cell carcinoma), chromophobe renal cell carcinoma, collecting duct renal cell carcinoma, granular renal cell carcinoma, mixed granular renal cell carcinoma, renal angiomyolipomas, or spindle renal cell carcinoma. In some embodiments, the individual may be a human who has a gene, genetic mutation, or polymorphism associated with renal cell carcinoma (e.g., mutation or polymorphism in VHL, TSC1, TSC2, CUL2, MSH2, MLH1, INK4a/ARF, MET, TGF- α, TGF-β1, IGF-I, IGF-IR, AKT, and/or PTEN) or has one or more extra copies of a gene associated with renal cell carcinoma. In some embodiments, the renal cell carcinoma is associated with (1) von Hippel-Lindau (VHL) syndrome, (2) hereditary papillary renal carcinoma (HPRC), (3) familial renal oncocytoma (FRO) associated with Birt-Hogg-Dube syndrome (BHDS), or (4) hereditary renal carcinoma (HRC). In some embodiments, the renal cell carcinoma is at any of stage I, II, III, or IV, according to the American Joint Committee on Cancer (AJCC) staging groups. In some embodiments, the renal cell carcinoma is stage IV renal cell carcinoma.

In some embodiments of the methods described herein, the disease to be treated is cancer, wherein the cancer is a central nervous system (CNS) tumor, and the pharmaceutical composition comprises a genome-editing complex or nanoparticle comprising one or more molecules of a genome-editing system (such as the entire genome-editing system) targeting one or more genes encoding proteins involved in the CNS tumor development and/or progression. In some embodiments, the pharmaceutical composition is administered during or after (such as immediately following) a surgical procedure on the CNS tumor. In some embodiments, the surgical procedure is resection of the CNS tumor. In some embodiments, the pharmaceutical composition is administered into a surgical cavity resulting from the surgical procedure. In some embodiments, the one or more genes encoding proteins involved in the CNS tumor development and/or progression include, but are not limited to, NF1, NF2, SMARCB1, pVHL, TSC1, TSC2, p53, CHK2, MLH1, PMS2, PTCH, SUFU, and XRCC7, including mutants thereof.

In some embodiments, according to any of the methods described herein, the cancer is a CNS tumor. In some embodiments, the CNS tumor is a glioma (e.g., brainstem glioma and mixed gliomas), glioblastoma (also known as glioblastoma multiforme), astrocytoma (such as high-grade astrocytoma), pediatric glioma or glioblastoma (such as pediatric high-grade glioma (HGG) and diffuse intrinsic pontine glioma (DIPG)), CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma, or brain metastasis. In some embodiments, the individual may be a human who has a gene, genetic mutation, or polymorphism suspected or shown to be associated with the CNS tumor (e.g., mutation or polymorphism in NF1, NF2, SMARCB1, pVHL, TSC1, TSC2, p53, CHK2, MLH1, PMS2, PTCH, SUFU, and XRCC7) or has one or more extra copies of a gene associated with the CNS tumor.

In some embodiments of the methods described herein, the disease to be treated is a hematologic disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in hematologic disease development and/or progression. In some embodiments, the hematologic disease is a hemoglobinopathy, such as sickle-cell disease, thalassemia, or methemoglobinemia, an anemia, such as megaloblastic anemia, hemolytic anemia (*e.g*., hereditary spherocytosis, hereditary elliptocytosis, congenital dyserythropoietic anemia, glucose-6-phosphate dehydrogenase deficiency, pyruvate kinase deficiency, immune mediated hemolytic anemia, autoimmune hemolytic anemia, warm antibody autoimmune hemolytic anemia, systemic lupus erythematosus, Evans' syndrome, cold autoimmune hemolytic anemia, cold agglutinin disease, paroxysmal cold hemoglobinuria, infectious mononucleosis, alloimmune hemolytic anemia, hemolytic disease of the newborn, or paroxysmal nocturnal hemoglobinuria), aplastic anemia (*e.g.,* Fanconi anemia, Diamond-Blackfan anemia, or acquired pure red cell aplasia), myelodysplastic syndrome, myelofibrosis, neutropenia, agranulocytosis, Glanzmann's thrombasthenia, thrombocytopenia, a myeloproliferative disorder, such as polycethemia vera, erythrocytosis, leukocytosis, or thrombocytosis, or a coagulopathy, such as recurrent thrombosis, disseminated intravascular coagulation, hemophilia (*e.g.,* hemophilia A, hemophilia B, or hemophilia C), Von Willebrand disease, protein S deficiency, antiphospholipid syndrome, or Wiskott-Aldrich syndrome. In some embodiments, the one or more genes encoding proteins involved in hematologic disease development and/or progression include, but are not limited to, HBA1, HBA2, HBB, PROC, ALAS2, ABCB7, SLC25A38, MTTP, FANCA, FANCB, FANCC, FANCD1 (BRCA2), FANCD2, FANCE, FANCF, FANCG, FANCI, FANCJ (BRIP1), FANCL, FANCM, FANCN (PALB2), FANCP (SLX4), FANCS (BRCA1), RAD51C, XPF, ANK1, SPTB, SPTA, SLC4A1, EPB42, and TPI1, including mutants thereof.

In some embodiments of the methods described herein, the disease to be treated is an organ-based disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the organ-based disease development and/or progression. In some embodiments, the organ-based disease is a disease of the eye, liver, lung, kidney, heart, nervous system, muscle, or skin. In some embodiments, the disease is a cardiovascular disease, such as coronary heart disease, hypertension, atrial fibrillation, peripheral arterial disease, Marfan syndrome, long QT syndrome, or a congenital heart defect. In some embodiments, the disease is a digestive disease, such as irritable bowel syndrome, ulcerative colitis, Crohn's disease, celiac disease, peptic ulcer disease, gastroesophageal reflux disease, or chronic pancreatitis. In some embodiments, the disease is a urologic disease, such as chronic prostatitis, benign prostatic hyperplasia, or interstitial cystitis. In some embodiments, the disease is a musculoskeletal disease, such as osteoarthritis, osteoporosis, osteogenesis imperfecta, or Paget's disease of bone. In some embodiments, the disease is a skin disease, such as eczema, psoriasis, acne, rosacea, or dermatitis. In some embodiments, the disease is a dental or craniofacial disorder, such as periodontal disease or temporomandibular joint and muscle disorder (TMJD).

In some embodiments of the methods described herein, the disease to be treated is an ocular disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in ocular disease development and/or progression. In some embodiments, the ocular disease is age-related macular degeneration or the like, retinopathy of prematurity, polypoidal choroidal vasculopathy, diabetic retinopathy, diabetic macular edema, ischemic proliferative retinopathy, retinitis pigmentosa, cone dystrophy, proliferative vitreoretinopathy, retinal artery occlusion, retinal vein occlusion, keratitis, conjunctivitis, uveitis, Leber's disease, retinal detachment, retinal pigment epithelial detachment, neovascular glaucoma, corneal neovascularization, retinochoroidal neovascularization. In some embodiments, the one or more genes encoding proteins involved in hematologic disease development and/or progression include, but are not limited to, Rho, PDE6β, ABCA4, RPE65, LRAT, RDS/Peripherin, MERTK, CNGA1, RPGR, IMPDH1, ChR2, GUCY2D, RDS/Peripherin, AIPL1, ABCA4, RPGRIP1, IMPDH1, AIPL1, GUCY2D, LRAT, MERTK, RPGRIP1, RPE65, CEP290, ABCA4, DFNB31, MYO7A, USH1C, CDH23, PCDH15, USH1G, CLRN1, GNAT2, CNGA3, CNGB3, Rs1, OA1, MT-ND4, (OCA1), tyrosinase, p21 WAF-1/OCipl, REP-1, PDGF, Endostatin, Angiostatin, VEGF inhibitor, Opsin, OPN1LW, arylsulfatase B, and β-glucuronidase, including mutants thereof.

In some embodiments of the methods described herein, the disease to be treated is a liver disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in liver disease development and/or progression. In some embodiments, the liver disease is hepatitis, fatty liver disease (alcoholic and nonalcoholic), hemochromatosis, Wilson's disease, progressive familial intrahepatic cholestasis type 3, hereditary fructose intolerance, glycogen storage disease type IV, tyrosinemia type 1, argininosuccinate lyase deficiency, citrin deficiency (CTLN2, NICCD), cholesteryl ester storage disease, cystic fibrosis, Alström syndrome, congenital hepatic fibrosis, alpha 1-antitrypsin deficiency, glycogen storage disease type II, transthyretin-related hereditary amyloidosis, Gilbert's syndrome, cirrhosis, primary biliary cirrhosis, or primary sclerosing cholangitis. In some embodiments, the one or more genes encoding proteins involved in liver disease development and/or progression include, but are not limited to, ATP7B, ABCB4, ALDOB, GBE1, FAH, ASL, SLC25A13, LIPA, CFTR, ALMS1, HFE, HFE2, HFDE2B, HFE3, SLC11A3/SLC40A1, ceruloplasmin, transferrin, A1AT, BCS1L, B3GAT1, B3GAT2, B3GAT3, UGT1A1, UGT1A3, UGT1A4, UGT1A5, UGT1A6, UGT1A7, UGT1A8, UGT1A9, UGT1A10, UGT2A1, UGT2A2, UGT2A3, UGT2B4, UGT2B7, GT2B10, UGT2B11, UGT2B15, UGT2B17, and UGT2B28, including mutants thereof.

In some embodiments of the methods described herein, the disease to be treated is a lung disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in lung disease development and/or progression. In some embodiments, the lung disease is chronic obstructive pulmonary disease (COPD), asthma, cystic fibrosis, primary ciliary dyskinesia, pulmonary fibrosis, Birt Hogg Dube syndrome, tuberous sclerosis, Kartagener syndrome, α₁-antitrypsin deficiency, pulmonary capillary hemangiomatosis (PCH), or hereditary heamorrhagic telangiectasia. In some embodiments, the one or more genes encoding proteins involved in lung disease development and/or progression include, but are not limited to, EIF2AK4, IREB2, HHIP, FAM13A, IL1RL1, TSLP, IL33, IL25, CFTR, DNAI1, DNAH5, TXNDC3, DNAH11, DNAI2, KTU, RSPH4A, RSPH9, LRRC50, TERC, TERT, SFTPC, SFTPA2, FLCN, TSC1, TSC2, A1AT, ENG, ACVRL1, and MADH4, including mutants thereof.

In some embodiments of the methods described herein, the disease to be treated is a kidney disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in kidney disease development and/or progression. In some embodiments, the kidney disease is cystic kidney disease (*e.g.,* autosomal dominant polycystic kidney disease, autosomal recessive polycystic kidney disease, nephronophthisis, or medullary sponge kidney), Alport's syndrome, Bartter's syndrome, congenital nephrotic syndrome, nail-patella syndrome, primary immune glomerulonephritis, reflux nephropathy, or haemolytic uraemic syndrome. In some embodiments, the one or more genes encoding proteins involved in kidney disease development and/or progression include, but are not limited to, PKD1, PKD2, PKD3, fibrocystin, NPHP1, NPHP2, NPHP3, NPHP4, NPHP5, NPHP6, NPHP7, NPHP8, NPHP9, NPHP11, NPHP11, NPHPL1, GDNF, COL4A5, COL4A3, COL4A4, SLC12A2 (NKCC2), ROMK/KCNJ1, CLCNKB, BSND, CASR, SLC12A3 (NCCT), and ADAMTS13, including mutants thereof.

In some embodiments of the methods described herein, the disease to be treated is a muscle disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in muscle disease development and/or progression. In some embodiments, the muscle disease is myopathy (*e.g.,* mitochondrial myopathy), muscular dystrophy *(e.g.,* Duchenne, Becker, Emery-Dreifuss, facioscapulohumeral, myotonic, congenital, distal, limb-girdle, and oculopharyngeal), cerebral palsy, fibrodysplasia ossificans progressiva, dermatomyositis, compartment syndrome, myasthenia gravis, amyotrophic lateral sclerosis, rhabdomyolysis, polymyositis, fibromyalgia, myotonia, myofascial pain syndrome,. In some embodiments, the one or more genes encoding proteins involved in muscle disease development and/or progression include, but are not limited to, DMD, LAMA2, collagen VI (COL6A1, COL6A2, or COL6A3), POMT1, POMT2, FKTN, FKRP, LARGE1, POMGNT1, ISPD, SEPN1, LMNA, DYSF, EMD, DUX4, DMPK, ZNF9, PABPN1, CAV3, CAPN3, SGCA, SGCB, SGCG, SGCD, TTN, ANO5, DNAJB6, HNRNPDL, MYOT, TCAP, TNPO3, TRAPPC11, and TRIM32, including mutants thereof.

In some embodiments of the methods described herein, the disease to be treated is a nervous system disease (such as a central nervous system disease), and the pharmaceutical composition comprises a genome-editing complex or nanoparticle comprising one or more molecules of a genome-editing system (such as the entire genome-editing system) that modifies the sequence and/or expression of one or more genes encoding proteins involved in nervous system disease development and/or progression. In some embodiments, the nervous system disease is adrenoleukodystrophy, Angelman syndrome, ataxia telangiectasia, Charcot-Marie-Tooth syndrome, Cockayne syndrome, essential tremor, fragile X syndrome, Friedreich's ataxia, Gaucher disease, Lesch-Nyhan syndrome, maple syrup urine disease, Menkes syndrome, narcolepsy, neurofibromatosis, Niemann-Pick disease, phenylketonuria, Prader-Willi syndrome, Refsum disease, Rett syndrome, spinal muscular atrophy, spinocerebellar ataxia, Tangier disease, Tay-Sachs disease, tuberous sclerosis, Von Hippel-Lindau syndrome, Williams syndrome, Wilson's disease, Zellweger syndrome, attention deficit/hyperactivity disorder (ADHD), autism, bipolar disorder, depression, epilepsy, migraine, multiple sclerosis, myelopathy, Alzheimer's, Huntington's, Parkinson's, or Tourette's. In some embodiments, the one or more genes encoding proteins involved in nervous system disease development and/or progression include, but are not limited to, ALD, PS1 (AD3), PS2 (AD4), SOD1, UBE3A, ATM, PMP22, MPZ, LITAF, EGR2, MFN2, KIF1B, RAB7A, LMNA, TRPV4, BSCL2, GARS, NEFL, HSPB1, GDAP1, HSPB8, MTMR2, SBF2, SH3TC2, NDRG1, PRX, FGD4, FIG4, DNM2, YARS, GJB1, PRPS1, CSA, CSB, Cx26, EPM2A, ETM1 (FET1), ETM2, FMR1, frataxin, GBA, HTT, HPRT1, BCKDH, ATP7A, ATP7B, HLA-DQB1, HLA-DQA1, HLA-DRB1, NF1, NF2, SMPD1, NPC1, NPC2, LRRK2, PARK7, PINK1, PRKN, SNCA, UCHL1, PAH, PHYH, PEX7, MeCP2, SMN1, ATXN genes (*e.g.,* ATXN1, ATXN2, etc), ABC1, HEXA, TSC1, TSC2, VHL, CLIP2, ELN, GTF2I, GTF2IRD1, LIMK1, and PXR1, including mutants thereof.

In some embodiments of the methods described herein, the disease to be treated is cancer, wherein the cancer is a hematological malignancy, and the pharmaceutical composition comprises a genome-editing complex or nanoparticle comprising one or more molecules of a genome-editing system (such as the entire genome-editing system) that modifies the sequence and/or expression of one or more genes encoding proteins involved in hematological malignancy development and/or progression. In some embodiments, the one or more genes encoding proteins involved in hematological malignancy development and/or progression include, but are not limited to, GLI1, CTNNB1, eIF5A, mutant DDX3X, Hexokinase II, histone methyltransferase EZH2, ARK5, ALK, MUC1, HMGA2, IRF1, RPN13, HDAC11, Rad51, Spry2, mir-146a, mir-146b, survivin, MDM2, MCL1, CMYC, XBP1 (spliced and unspliced), SLAMF7, CS1, Erbb4, Cxcr4 (waldenstroms macroglobulinemia), Myc, Bcl2, Prdx1 and Prdx2 (burkitts lymphoma), Bc16, Idh1, Idh2, Smad, Ccnd2, Cyclin d1-2, B7-hl (pdl-1), and Pyk2, including mutants thereof.

In some embodiments of the methods described herein, the disease to be treated is a viral infectious disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the viral infectious disease development and/or progression. In some embodiments, the viral infectious disease is characterized by infection with hepatitis virus, human immunodeficiency virus (HIV), picornavirus, poliovirus, enterovirus, human Coxsackie virus, influenza virus, rhinovirus, echovirus, rubella virus, encephalitis virus, rabies virus, herpes virus, papillomavirus, polyoma virus, RSV, adenovirus, yellow fever virus, dengue virus, parainfluenza virus, hemorrhagic virus, pox virus, varicella zoster virus, parainfluenza virus, reovirus, orbivirus, rotavirus, parvovirus, African swine fever virus, measles, mumps or Norwalk virus. In some embodiments, the viral infectious disease is characterized by infection with an oncogenic virus including, but not limited to, CMV, EBV, HBV, KSHV, HPV, MCV, HTLV-1, HIV-1, and HCV In some embodiments, the one or more genes encoding proteins involved in the viral infectious disease development and/or progression include, but are not limited to, genes encoding RSV nucleocapsid, Pre-gen/Pre-C, Pre-S1, Pre-S2/S,X, HBV conserved sequences, HIV Gag polyprotein (p55), HIV Pol polyprotein, HIV Gag-Pol precursor (p160), HIV matrix protein (MA, p17), HIV capsid protein (CA, p24), HIV spacer peptide 1 (SP1, p2), HIV nucleocapsid protein (NC, p9), HIV spacer peptide 2 (SP2, p1), HIV P6 protein, HIV reverse transcriptase (RT, p50), HIV RNase H (p15), HIV integrase (IN, p31), HIV protease (PR, p10), HIV Env (gp160), gp120, gp41, HIV transactivator (Tat), HIV regulator of expression of virion proteins (Rev), HIV lentivirus protein R (Vpr), HIV Vif, HIV negative factor (Nef), HIV virus protein U (Vpu), human CCR5, miR-122, EBOV polymerase L, VP24, VP40, GP/sGP, VP30, VP35, NPC1, and TIM-1, including mutants thereof.

In some embodiments of the methods described herein, the disease or condition to be treated is an autoimmune or inflammatory disease or condition, and the pharmaceutical composition comprises a genome-editing complex or nanoparticle comprising one or more molecules of a genome-editing system (such as the entire genome-editing system) that modifies the sequence and/or expression of one or more genes encoding proteins involved in the autoimmune or inflammatory disease or condition development and/or progression. In some embodiments, the autoimmune or inflammatory disease or condition is acne, allergies, anaphylaxis, asthma, celiac disease, diverticulitis, glomerulonephritis, inflammatory bowel disease, interstitial cystitis, lupus, otitis, pelvic inflammatory disease, rheumatoid arthritis, sarcoidosis, or vasculitis. In some embodiments, the one or more genes encoding proteins involved in the autoimmune or inflammatory disease or condition development and/or progression include, but are not limited to, genes encoding molecules of the complement system (CD46, CD59, CFB, CFD, CFH, CFHR1, CFHR2, CFHR3, CFHR4, CFHR5, CFI, CFP, CR1, CR1L, CR2, C1QA, C1QB, C1QC, C1R, C1S, C2, C3, C3AR1, C4A, C4B, C5, C5AR1, C6, C7, C8A, C8B, C8G, C9, ITGAM, ITGAX, and ITGB2), including mutants thereof.

In some embodiments of the methods described herein, the disease or condition to be treated is a lysosomal storage disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the lysosomal storage disease development and/or progression. In some embodiments, the lysosomal storage disease is Sphingolipidoses (*e.g*., Farber disease, Krabbe disease (infantile onset, late onset), galactosialidosis, gangliosidoses (*e.g.,* Fabry disease, Schindler disease, GM1 gangliosidosis (Infantile, Juvenile, Adult/Chronic), GM2 gangliosidosis (e.g., Sandhoff disease (Infantile, Juvenile, Adult onset), Tay-Sachs)), Gaucher Disease (Type I, Type II, Type III), Lysosomal acid lipase deficiency (Early onset, Late onset), Niemann-Pick disease (Type A, Type B), Sulfatidosis (*e.g.,* Metachromatic Leukodystrophy (MLD), Multiple sulfatase deficiency)), Mucopolysaccharidoses (*e.g.,* MPS I Hurler Syndrome, MPS I S Scheie Syndrome, MPS I H-S Hurler-Scheie Syndrome, Type II (Hunter syndrome), Type III (Sanfilippo syndrome), Type IV (Morquio), Type VI (Maroteaux-Lamy syndrome), Type VII (Sly Syndrome), Type IX (Hyaluronidase deficiency)), Mucolipidosis (*e.g.*,. Type I (Sialidosis), Type II (I-cell disease), Type III (Pseudo-Hurler Polydystrophy / Phosphotransferase deficiency), Type IV (Mucolipidin 1 deficiency)), Lipidoses (*e.g.,* Niemann-Pick disease (Type C, Type D), Neuronal Ceroid Lipofuscinoses, Wolman disease), Alpha-mannosidosis, Beta-mannosidosis, Aspartylglucosaminuria, Fucosidosis, Lysosomal Transport Diseases (*e.g.,* Cystinosis, Pycnodysostosis, Salla disease/Sialic Acid Storage Disease, Infantile Free Sialic Acid Storage Disease (ISSD)), Cholesteryl ester storage disease. In some embodiments, the one or more genes encoding proteins involved in the lysosomal storage disease development and/or progression include, but are not limited to, genes encoding ceramidase, Alpha-galactosidase (A, B), Beta-galactosidase, Hexosaminidase A, Sphingomyelinase, Lysosomal acid lipase, Saposin B, sulfatase, Hyaluronidase, Phosphotransferase, Mucolipidin 1, aspartylglucosaminidase, alpha-D-mannosidase, beta-mannosidase, alpha-L-fucosidase, cystinosin, cathepsin K, sialin, SLC17A5, acid alpha-glucosidase, LAMP2, including mutants thereof.

In some embodiments of the methods described herein, the disease or condition to be treated is a glycogen storage disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the glycogen storage disease development and/or progression. In some embodiments, the glycogen storage disease is von Gierke's disease, Pompe's disease, Cori's disease or Forbes' disease, Andersen disease, McArdle disease, Hers' disease, Tarui's disease, Fanconi-Bickel syndrome, or Red cell aldolase deficiency. In some embodiments, the one or more genes encoding proteins involved in the glycogen storage disease development and/or progression include, but are not limited to, genes encoding glycogen synthase, glucose-6-phosphatase, acid alpha-glucosidase, glycogen debranching enzyme, glycogen branching enzyme, muscle glycogen phosphorylase, liver glycogen phosphorylase, muscle phosphofructokinase, Phosphorylase kinase, glucose transporter, GLUT2, Aldolase A, and β-enolase, including mutants thereof.

In some embodiments of the methods described herein, the disease or condition to be treated is an immunodeficiency disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the glycogen storage disease development and/or progression. In some embodiments, the glycogen storage disease is von Gierke's disease, Pompe's disease, Cori's disease or Forbes' disease, Andersen disease, McArdle disease, Hers' disease, Tarui's disease, Fanconi-Bickel syndrome, or Red cell aldolase deficiency. In some embodiments, the one or more genes encoding proteins involved in the glycogen storage disease development and/or progression include, but are not limited to, genes encoding glycogen synthase, glucose-6-phosphatase, acid alpha-glucosidase, glycogen debranching enzyme, glycogen branching enzyme, muscle glycogen phosphorylase, liver glycogen phosphorylase, muscle phosphofructokinase, Phosphorylase kinase, glucose transporter, GLUT2, Aldolase A, and β-enolase, including mutants thereof.

In some embodiments of the methods described herein, the condition to be treated is characterized by abnormal cholesterol levels (such as abnormally high LDL levels, e.g., LDL above about 100 mg/dL, and/or abnormally low HDL levels, e.g., HDL below about 40-50 mg/dL), including, e.g., familial hypercholesterolemia, and the pharmaceutical composition comprises a genome-editing complex or nanoparticle comprising one or more molecules of a genome-editing system (such as the entire genome-editing system) that modifies the sequence and/or expression of one or more genes encoding proteins involved in cholesterol transport and/or metabolism. In some embodiments, the one or more genes encoding proteins involved in cholesterol transport and/or metabolism include, but are not limited to, low-density lipoprotein (LDL) receptor (LDLR), apolipoprotein B (ApoB), low-density lipoprotein receptor adapter protein 1 (LDLRAP1), and PCSK9, including mutants thereof.

In some embodiments, a genome-editing complex or nanoparticle as described herein is used to activate LDLR expression. Methods of using such systems to activate cell expression of LDLR are well within the level of a skilled artisan. Exemplary LDLR-specific target sequences for guide RNA/DNA sequences can include any set forth in SEQ ID NOS: 110-112. Commercially available reagents, such as nuclease dead Cas9 (dCas9) and gRNA vectors, are readily available, *e.g.* from Santa Cruz Biotechnology *(see, e.g.,* catalog number sc-400645-ACT and sc-400645-ACT-2).

In some embodiments, a genome-editing complex or nanoparticle as described herein is used to correct a mutation in a gene encoding LDLR. Methods of using such systems with template DNA corresponding to wild-type LDLR sequences are well within the level of a skilled artisan. Methods using CRISPR systems, *e*.*g*., for introducing double-strand DNA breaks in an LDLR gene are known in the art, and can be combined, *e.g.,* with donor nucleic acids for homology-directed DNA repair (HDR) to replace mutant sequences in the LDLR gene with wild-type sequences. For example, exemplary target sequences for guide RNA sequences can include any set forth in SEQ ID NOS: 104-109. Commercially available kits, gRNA vectors, and donor vectors for introducing double-strand DNA breaks in an LDLR gene and correcting mutations by HDR are readily available, *e.g.,* from Santa Cruz Biotechnology *(see, e.g.,* catalog number sc-400645 and sc-400645-HDR) or GeneCopoeia *(see, e.g.,* catalog number HTN210577, HTN261606, HTN261605, HTN261608, and HTN261607).

In some embodiments, a genome-editing complex or nanoparticle as described herein is used to introduce a gene encoding LDLR. Methods of using such systems with template DNA comprising a gene encoding LDLR are well within the level of a skilled artisan. Methods using CRISPR systems, *e.g.,* for introducing double-strand DNA breaks in a genome are known in the art, and can be combined, *e.g.,* with donor nucleic acids for HDR-mediated insertion of a gene encoding LDLR into the genome.

In some embodiments, a genome-editing complex or nanoparticle as described herein is used to activate ApoB expression. Methods of using such systems to activate cell expression of ApoB are well within the level of a skilled artisan. Exemplary ApoB-specific target sequences for guide RNA/DNA sequences can include any set forth in SEQ ID NOS: 119-121. Commercially available reagents, such as nuclease dead Cas9 (dCas9) and gRNA vectors, are readily available, *e.g.* from Santa Cruz Biotechnology *(see, e.g.,* catalog number sc-400705-ACT and sc-400705-ACT-2).

In some embodiments, a genome-editing complex or nanoparticle as described herein is used to correct a mutation in a gene encoding ApoB. Methods of using such systems with template DNA corresponding to wild-type ApoB sequences are well within the level of a skilled artisan. Methods using CRISPR systems, *e*.*g*., for introducing double-strand DNA breaks in an ApoB gene are known in the art, and can be combined, *e.g.,* with donor nucleic acids for homology-directed DNA repair (HDR) to replace mutant sequences in the ApoB gene with wild-type sequences. For example, exemplary target sequences for guide RNA sequences can include any set forth in SEQ ID NOS: 113-118. Commercially available kits, gRNA vectors, and donor vectors for introducing double-strand DNA breaks in an ApoB gene and correcting mutations by HDR are readily available, *e.g.,* from Santa Cruz Biotechnology *(see, e.g.,* catalog number sc-400705-KO-2 and sc-400705-HDR-2) or GeneCopoeia *(see, e.g.,* catalog number HTN209218).

In some embodiments, a genome-editing complex or nanoparticle as described herein is used to introduce a gene encoding ApoB. Methods of using such systems with template DNA comprising a gene encoding ApoB are well within the level of a skilled artisan. Methods using CRISPR systems, *e.g.,* for introducing double-strand DNA breaks in a genome are known in the art, and can be combined, *e.g.,* with donor nucleic acids for HDR-mediated insertion of a gene encoding ApoB into the genome.

In some embodiments, a genome-editing complex or nanoparticle as described herein is used to activate LDLRAP1 expression. Methods of using such systems to activate cell expression of LDLRAP1 are well within the level of a skilled artisan. Exemplary LDLRAP1-specific target sequences for guide RNA/DNA sequences can include any set forth in SEQ ID NOS: 128-130. Commercially available reagents, such as nuclease dead Cas9 (dCas9) and gRNA vectors, are readily available, *e.g.* from Santa Cruz Biotechnology *(see, e.g.,* catalog number sc-406114-ACT and sc-406114-ACT-2).

In some embodiments, a genome-editing complex or nanoparticle as described herein is used to correct a mutation in a gene encoding LDLRAP1. Methods of using such systems with template DNA corresponding to wild-type LDLRAP1 sequences are well within the level of a skilled artisan. Methods using CRISPR systems, *e.g.,* for introducing double-strand DNA breaks in an LDLRAP1 gene are known in the art, and can be combined, *e.g.,* with donor nucleic acids for homology-directed DNA repair (HDR) to replace mutant sequences in the LDLRAP1 gene with wild-type sequences. For example, exemplary target sequences for guide RNA sequences can include any set forth in SEQ ID NOS: 122-127. Commercially available kits, gRNA vectors, and donor vectors for introducing double-strand DNA breaks in an LDLRAP1 gene and correcting mutations by HDR are readily available, *e.g.,* from Santa Cruz Biotechnology (*see, e.g.,* catalog number sc-406114, sc-406114-KO-2, and sc-406114-HDR-2) or GeneCopoeia (*see, e.g.,* catalog number HTN207062).

In some embodiments, a genome-editing complex or nanoparticle as described herein is used to introduce a gene encoding LDLRAP1. Methods of using such systems with template DNA comprising a gene encoding LDLRAP1 are well within the level of a skilled artisan. Methods using CRISPR systems, *e.g.,* for introducing double-strand DNA breaks in a genome are known in the art, and can be combined, *e.g.,* with donor nucleic acids for HDR-mediated insertion of a gene encoding LDLRAP1 into the genome.

In some embodiments, a genome-editing complex or nanoparticle as described herein is used to repress PCSK9 expression, such as by gene knockout. Methods of using such systems to repress cell expression of PCSK9 are well within the level of a skilled artisan. Exemplary PCSK9-specific target sequences for guide RNA/DNA sequences can include any set forth in SEQ ID NOS: 131-136. Commercially available kits, gRNA vectors, and donor vectors for introducing double-strand DNA breaks in a PCSK9 gene are readily available, *e.g.,* from Santa Cruz Biotechnology *(see, e.g.,* catalog number sc-402445) or GeneCopoeia *(see, e.g.,* catalog number HTN206606).

In some embodiments of the methods described herein, the disease to be treated is a genetic disease, such as a hereditary disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the genetic disease development and/or progression. In some embodiments, the genome-editing system corrects a mutation in one or more genes encoding proteins involved in the genetic disease development and/or progression. In some embodiments, the genetic disease includes, but is not limited to, 22q11.2 deletion syndrome, achondroplasia, Alpha-1 Antitrypsin Deficiency, Angelman syndrome, Autosomal dominant polycystic kidney disease, breast cancer, Canavan disease, Charcot-Marie-Tooth disease, colon cancer, Color blindness, Cystic fibrosis, Duchenne muscular dystrophy, Factor V Leiden thrombophilia, Familial Mediterranean Fever, Fragile X syndrome, Gaucher disease, Haemochromatosis, Haemophilia, Huntington's disease, Marfan syndrome, Myotonic dystrophy, Osteogenesis imperfecta, Parkinson's disease, Phenylketonuria, Polycystic kidney disease, porphyria, Prader-Willi syndrome, progeria, SCID, Sickle-cell disease, Spinal muscular atrophy, Tay-Sachs disease, thalassemia, Trimethylamine, and Wilson's disease. In some embodiments, the genes involved in the genetic disease development and/or progression include, but are not limited to, AAT, ADA, ALAD, ALAS2, APC, ASPM, ATP7B, BDNF, BRCA1, BRCA2, CFTR, COL1A1, COL1A2, COMT, CNBP, CPOX, CREBBP, CRH, CRTAP, CXCR4, DHFR, DMD, DMPK, F5, FBN1, FECHFGFR3, FGR3, FIX, FVIII, FMO3, FMR1, GARS, GBA, HBB, HEXA, HFE, HMBS, HTT, IL2RG, KRT14, KRT5, LMNA, LRRK2, MEFV, MLH1, MSH2, MSH6, PAH, PARK2, PARK3, PARK7, PGL2, PHF8, PINK1, PKD1, PKD2, PMS1, PMS2, PPOX, RHO, SDHB, SDHC, SDHD, SMNI, SNCA, SRY, TSC1, TSC2, UCHL1, UROD, UROS, MEFV, APP, GAST, INS, LCK, LEP, LIF, MCM6, MYH7, MYOD1, NPPB, OSM, PKC, PIP, SLC18A2, TBX1, Transthyretin, MDS1-EVI1, PRDM16, SETBP1, β-Globin, and LPL, including mutants thereof.

In some embodiments of the methods described herein, the disease to be treated is an aging or degenerative disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the aging or degenerative disease development and/or progression. In some embodiments, the one or more genes encoding proteins involved in the aging or degenerative disease development and/or progression include, but are not limited to, keratin K6A, keratin K6B, keratin 16, keratin 17, p53, β-2 adrenergic receptors (ADRB2), TRPV1, VEGF, VEGFR, HIF-1, and caspase-2, including mutants thereof.

In some embodiments of the methods described herein, the disease to be treated is a fibrotic or inflammatory disease, and the pharmaceutical composition comprises a genome-editing complex or nanoparticle comprising one or more molecules of a genome-editing system (such as the entire genome-editing system) that modifies the sequence and/or expression of one or more genes encoding proteins involved in the fibrotic or inflammatory disease development and/or progression. In some embodiments, the one or more genes encoding proteins involved in the fibrotic or inflammatory disease development and/or progression are selected from the group consisting of SPARC, CTGF, TGFβ1, TGFβ receptors 1, TGFβ receptors 2, TGFβ receptors 3, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-κB, and PARP-1, including mutants thereof.

In some embodiments of the methods described herein, the pharmaceutical composition comprises a genome-editing complex or nanoparticle comprising one or more molecules of a genome-editing system (such as the entire genome-editing system) that modulates the expression of one or more miRNAs involved in a disease or condition. In some embodiments, the disease or conditoin includes, but is not limited to, hepatitis B, hepatitis C, polycystic liver and kidney disease, cancer, cardiovascular disease, cardiac failure, cardiac hypertrophy, neurodevelopmental disease, fragile X syndrome, Rett syndrome, Down syndrome, Alzheimer's disease, Huntington's disease, schizophrenia, inflammatory disease, rheumatoid arthritis, systemic lupus erythematosus, psoriasis, and skeletal muscle disease. In some embodiments, the one or more miRNAs include, but are not limited to, has-mir-126*, Has-miR-191, has-mir-205, has-mir-21, hsa-let-7a-2, let-7 family, let-7c, let-7f-1, miR-1, miR-100, miR-103, miR-103-1, miR-106b-25, miR-107, miR-10b, miR-112, miR-122, miR-125b, miR-125b-2, miR125bl, miR-126, miR-128a, mIR-132, miR-133, miR-133b, miR135, miR-140, miR-141, miR-142-3p, miR143, miR-143, miR145, miR-145, miR-146, miR-146b, miR150, miR-155, miR-15a, miR-15b, miR16, miR-16, miR-17-19 family, miR-173p, miR17-5p, miR-17-5p, miR-17-92, miR-181a, miR-181b, miR-184, miR-185, miR-189, miR-18a, miR-191, miR-192, miR-193a, miR-193b, miR-194, miR-195, miR-196a, miR-198, miR-199, miR-199a, miR-19a, miR-19b-1, miR200a, miR-200a, miR-200b, miR200c, miR-200c, miR-203, miR-205, miR-208, miR-20a, miR-21, miR-214, miR-221, miR-222, miR-223, miR-224, miR-23, miR-23a, miR-23b, miR-24, miR-26a, miR-26b, miR-27b, miR-29, miR-298, miR-299-3p, miR-29c, miR-30a-5p, miR-30c, miR-30d, miR-30e-5p, miR31, miR-34, miR342, miR-381, miR-382, miR-383, miR-409-3p, miR-45, miR-61, miR-78, miR-802, miR-9, miR-92a-1, miR-99a, miR-let7, miR-let7a, and miR-let7g.

In some embodiments of the methods described herein, the pharmaceutical composition is administered to the individual by any of intravenous, intratumoral, intraarterial, topical, intraocular, ophthalmic, intraportal, intracranial, intracerebral, intracerebroventricular, intrathecal, intravesicular, intradermal, subcutaneous, intramuscular, intranasal, intratracheal, pulmonary, intracavity, or oral administration.

In some embodiments of the methods described herein, the individual is a mammal. In some embodiments, the individual is human.

### Methods of cell delivery

In some embodiments, there is provided a method of delivering one or more molecules of a genome-editing system into a cell comprising contacting the cell with a genome-editing complex or nanoparticle as described herein, wherein the complex or nanoparticle comprises the one or more genome-editing system molecules. In some embodiments, the complex or nanoparticle comprises a CPP comprising the amino acid sequence of a VEPEP-3 peptide, a VEPEP-6 peptide, a VEPEP-9 peptide, or an ADGN-100 peptide. In some embodiments, the contacting of the cell with the complex or nanoparticle is carried out *in vivo.* In some embodiments, the contacting of the cell with the complex or nanoparticle is carried out *ex vivo.* In some embodiments, the contacting of the cell with the complex or nanoparticle is carried out *in vitro.* In some embodiments, the cell is an immortalized cell, such as a cell from a cell line. In some embodiments, the cell is a primary cell, such as a cell from an individual. In some embodiments, the cell is an immune cell, such as a granulocyte, a mast cell, a monocyte, a dendritic cell, a B cell, a T cell, or a natural killer cell. In some embodiments, the cell is a peripheral blood-derived T cell, a central memory T cell, a cord blood-derived T cell, or a hematopoietic stem cell or other precursor cell. In some embodiments, the T cell is an immortalized T cell, such as a T cell from a T cell line. In some embodiments, the T cell is a primary T cell, such as a T cell of an individual. In some embodiments, the cell is a T cell, and the contacting is carried out after activating the T cell. In some embodiments, the cell is a T cell, and the contacting is carried out at least 12 hours (such as at least about any of 12 hours, 1 day, 2 days, 3 days, 4 days, 5 days, 6 days, or more) after activating the T cell. In some embodiments, the T cell is activated using an anti-CD3/CD28 reagent (such as microbeads). In some embodiments, the cell is a fibroblast. In some embodiments, the fibroblast is a primary fibroblast, such as a fibroblast of an individual. In some embodiments, the cell is a hepatocyte. In some embodiments, the hepatocyte is a primary hepatocyte, such as a hepatocyte of an individual. In some embodiments, the cell is a human lung progenitor cell (LPC). In some embodiments, the cell is a neuronal cell. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing nuclease is a TALEN. In some embodiments, the genome-editing system is a CRISPR system In some embodiments, the one or more CRISPR system molecules are selected from the group consisting of CRISPR-associated proteins (*e.g.,* an RGEN, such as Cas9, or a nucleic acid encoding the RGEN) and nucleic acids (*e.g.,* gRNAs and donor nucleic acid). In some embodiments, the genome-editing system comprises an integrase (or a nucleic acid encoding the integrase). In some embodiments, the genome-editing system further comprises a donor nucleic acid comprising a recombination site recognized by the integrase. In some embodiments, the genome-editing system is useful for the treatment of a disease, such as any of the diseases to be treated described herein *(e.g.,* cancer, diabetes, inflammatory diseases, fibrosis, viral infectious diseases, hereditary diseases, ocular diseases, and aging and degenerative diseases). In some embodiments, the complex or nanoparticle further comprises one or more molecules of an additional genome-editing system In some embodiments, the additional genome-editing system is useful for the treatment of the disease. In some embodiments, the method further comprises contacting the cell with an expression complex according to any of the embodiments described herein. In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease.

Thus, in some embodiments, there is provided a method of delivering one or more molecules of a genome-editing system into a cell comprising contacting the cell with a genome-editing complex or nanoparticle as described herein, wherein the genome-editing complex or nanoparticle comprises the one or more genome-editing system molecules and a CPP comprising the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the contacting of the cell with the complex or nanoparticle is carried out *in vivo.* In some embodiments, the contacting of the cell with the complex or nanoparticle is carried out *ex vivo.* In some embodiments, the contacting of the cell with the complex or nanoparticle is carried out *in vitro.* In some embodiments, the cell is an immortalized cell, such as a cell from a cell line. In some embodiments, the cell is a primary cell, such as a cell from an individual. In some embodiments, the cell is an immune cell, such as a granulocyte, a mast cell, a monocyte, a dendritic cell, a B cell, a T cell, or a natural killer cell. In some embodiments, the T cell is an immortalized T cell, such as a T cell from a T cell line. In some embodiments, the T cell is a primary T cell, such as a T cell of an individual. In some embodiments, the cell is a fibroblast. In some embodiments, the fibroblast is a primary fibroblast, such as a fibroblast of an individual. In some embodiments, the cell is a hepatocyte. In some embodiments, the hepatocyte is a primary hepatocyte, such as a hepatocyte of an individual. In some embodiments, the cell is a human lung progenitor cell (LPC). In some embodiments, the cell is a neuronal cell. In some embodiments, the genome-editing system comprises a genome-editing nuclease (or a nucleic acid encoding the genome-editing nuclease). In some embodiments, the genome-editing nuclease is a ZFN, TALEN, homing endonuclease, RGEN, or DGEN. In some embodiments, the genome-editing system further comprises a gRNA or gDNA (or a nucleic acid encoding the gRNA or gDNA). In some embodiments, the genome-editing system further comprises a donor nucleic acid for introducing a specific modification to the target polynucleotide. In some embodiments, the genome-editing nuclease is a TALEN. In some embodiments, the genome-editing system is a CRISPR system (e.g., CRISPR/Cas9). In some embodiments, the genome-editing system comprises an integrase (or a nucleic acid encoding the integrase). In some embodiments, the genome-editing system further comprises a donor nucleic acid comprising a recombination site recognized by the integrase. In some embodiments, the genome-editing system is useful for the treatment of a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the genome-editing system is useful for modulating a protein involved in a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease. In some embodiments, the cell-penetrating peptide is an ADGN-100 peptide or a VEPEP-3 peptide.

In some embodiments, there is provided a method of delivering one or more CRISPR system (*e.g*., CRISPR/Cas9) molecules into a cell comprising contacting the cell with a genome-editing complex or nanoparticle as described herein, wherein the genome-editing complex or nanoparticle comprises the one or more CRISPR system molecules and a CPP selected from the group consisting of VEPEP-3 peptides, VEPEP-6 peptides, VEPEP-9 peptides, and ADGN-100 peptides. In some embodiments, the contacting of the cell with the complex or nanoparticle is carried out *in vivo.* In some embodiments, the contacting of the cell with the complex or nanoparticle is carried out *ex vivo.* In some embodiments, the contacting of the cell with the complex or nanoparticle is carried out *in vitro.* In some embodiments, the cell is an immortalized cell, such as a cell from a cell line. In some embodiments, the cell is a primary cell, such as a cell from an individual. In some embodiments, the cell is an immune cell, such as a granulocyte, a mast cell, a monocyte, a dendritic cell, a B cell, a T cell, or a natural killer cell. In some embodiments, the T cell is an immortalized T cell, such as a T cell from a T cell line. In some embodiments, the T cell is a primary T cell, such as a T cell of an individual. In some embodiments, the cell is a fibroblast. In some embodiments, the fibroblast is a primary fibroblast, such as a fibroblast of an individual. In some embodiments, the cell is a hepatocyte. In some embodiments, the hepatocyte is a primary hepatocyte, such as a hepatocyte of an individual. In some embodiments, the cell is a human lung progenitor cell (LPC). In some embodiments, the cell is a neuronal cell. In some embodiments, the CRISPR system is useful for the treatment of a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the CRISPR system is useful for modulating a protein involved in a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease. In some embodiments, the cell-penetrating peptide is an ADGN-100 peptide or a VEPEP-3 peptide. In some embodiments, the CRISPR system molecules include Cas9 (or a nucleic acid encoding Cas9) and a gRNA (or a nucleic acid encoding the gRNA), and optionally a donor nucleic acid for introducing a modification.

In some embodiments, there is provided a method of delivering one or more CRISPR system molecules into a cell comprising contacting the cell with a genome-editing complex or nanoparticle as described herein, wherein the genome-editing complex or nanoparticle comprises the one or more CRISPR system molecules and a CPP comprising the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14,75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the contacting of the cell with the complex or nanoparticle is carried out *in vivo.* In some embodiments, the contacting of the cell with the complex or nanoparticle is carried out *ex vivo.* In some embodiments, the contacting of the cell with the complex or nanoparticle is carried out *in vitro.* In some embodiments, the cell is an immortalized cell, such as a cell from a cell line. In some embodiments, the cell is a primary cell, such as a cell from an individual. In some embodiments, the cell is an immune cell, such as a granulocyte, a mast cell, a monocyte, a dendritic cell, a B cell, a T cell, or a natural killer cell. In some embodiments, the T cell is an immortalized T cell, such as a T cell from a T cell line. In some embodiments, the T cell is a primary T cell, such as a T cell of an individual. In some embodiments, the cell is a fibroblast. In some embodiments, the fibroblast is a primary fibroblast, such as a fibroblast of an individual. In some embodiments, the cell is a hepatocyte. In some embodiments, the hepatocyte is a primary hepatocyte, such as a hepatocyte of an individual. In some embodiments, the cell is a human lung progenitor cell (LPC). In some embodiments, the cell is a neuronal cell. In some embodiments, the CRISPR system is useful for the treatment of a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the CRISPR system is useful for modulating a protein involved in a disease, such as any of the diseases to be treated described herein (e.g., cancer, diabetes, inflammatory diseases, fibrosis, viral infectious diseases, hereditary diseases, ocular diseases, and aging and degenerative diseases). In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease. In some embodiments, the cell-penetrating peptide is an ADGN-100 peptide or a VEPEP-3 peptide. In some embodiments, the CRISPR system molecules include Cas9 (or a nucleic acid encoding Cas9) and a gRNA (or a nucleic acid encoding the gRNA), and optionally a donor nucleic acid for introducing a modification.

In some embodiments, there is provided a method of delivering one or more molecules of a genome-editing system into a T cell comprising contacting the cell with a genome-editing complex or nanoparticle as described herein, wherein the complex or nanoparticle comprises the one or more genome-editing system molecules and a CPP selected from the group consisting of VEPEP-3 peptides, VEPEP-6 peptides, VEPEP-9 peptides, and ADGN-100 peptides. In some embodiments, the contacting of the T cell with the complex or nanoparticle is carried out *in vivo.* In some embodiments, the contacting of the T cell with the complex or nanoparticle is carried out *ex vivo.* In some embodiments, the contacting of the T cell with the complex or nanoparticle is carried out *in vitro.* In some embodiments, the T cell is an immortalized T cell, such as a T cell from a T cell line. In some embodiments, the T cell is a primary T cell, such as a T cell of an individual. In some embodiments, the one or more genome-editing system molecules comprise a genome-editing nuclease (or a nucleic acid encoding the genome-editing nuclease, such as an mRNA or DNA plasmid). In some embodiments, the genome-editing nuclease is a ZFN, TALEN, homing endonuclease, RGEN, or DGEN. In some embodiments, the genome-editing nuclease is a nucleic acid-guided nuclease (e.g., an RGEN or DGEN), and the cell-penetrating peptides are complexed with the nucleic acid-guided nuclease (or a nucleic acid encoding the nucleic acid-guided nuclease) in combination with (such as complexed with) a guide sequence (or a nucleic acid encoding a guide sequence) to form a genome-editing complex or nanoparticle capable of being delivered to a cell. In some embodiments, the one or more genome-editing system molecules further comprises a donor nucleic acid as a template for homology-directed repair. In some embodiments, the one or more genome-editing system molecules comprise a genome-editing integrase (or a nucleic acid encoding the genome-editing integrase), optionally in combination with (such as complexed with) a donor nucleic acid comprising a recombination site recognized by the integrase to form a genome-editing complex or nanoparticle capable of being delivered to a cell. In some embodiments, the genome-editing system is useful for the treatment of a disease, such as any of the diseases to be treated described herein. In some embodiments, the complex or nanoparticle further comprises one or more molecules of an additional genome-editing system. In some embodiments, the additional genome-editing system is useful for the treatment of the disease. In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease.

In some embodiments, there is provided a method of delivering one or more CRISPR system molecules into a T cell comprising contacting the cell with a genome-editing complex or nanoparticle as described herein, wherein the complex or nanoparticle comprises the one or more CRISPR system molecules and a CPP comprising the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the contacting of the T cell with the complex or nanoparticle is carried out *in vivo.* In some embodiments, the contacting of the T cell with the complex or nanoparticle is carried out *ex vivo.* In some embodiments, the contacting of the T cell with the complex or nanoparticle is carried out *in vitro.* In some embodiments, the T cell is an immortalized T cell, such as a T cell from a T cell line. In some embodiments, the T cell is a primary T cell, such as a T cell of an individual. In some embodiments, the one or more CRISPR system molecules are selected from the group consisting of CRISPR-associated proteins (e.g., RGENs, such as Cas9, including nucleic acids encoding the RGEN) and nucleic acids (e.g., gRNAs and donor nucleic acid). In some embodiments, the CRISPR system is useful for the treatment of a disease, such as any of the diseases to be treated described herein. In some embodiments, the complex or nanoparticle further comprises one or more molecules of an additional CRISPR system In some embodiments, the additional CRISPR system is useful for the treatment of the disease. In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease. In some embodiments, the cell-penetrating peptide is an ADGN-100 peptide or a VEPEP-3 peptide.

In some embodiments, there is provided a method of delivering one or more molecules of a genome-editing system into a fibroblast comprising contacting the fibroblast with a genome-editing complex or nanoparticle as described herein, wherein the complex or nanoparticle comprises the one or more genome-editing system molecules and a CPP selected from the group consisting of VEPEP-3 peptides, VEPEP-6 peptides, VEPEP-9 peptides, and ADGN-100 peptides. In some embodiments, the contacting of the fibroblast with the complex or nanoparticle is carried out *in vivo.* In some embodiments, the contacting of the fibroblast with the complex or nanoparticle is carried out *ex vivo.* In some embodiments, the contacting of the fibroblast with the complex or nanoparticle is carried out *in vitro.* In some embodiments, the fibroblast is an immortalized fibroblast, such as a fibroblast from a fibroblast line. In some embodiments, the fibroblast is a primary fibroblast, such as a fibroblast of an individual. In some embodiments, the one or more genome-editing system molecules comprise a genome-editing nuclease (or a nucleic acid encoding the genome-editing nuclease, such as an mRNA or DNA plasmid). In some embodiments, the genome-editing nuclease is a ZFN, TALEN, homing endonuclease, RGEN, or DGEN. In some embodiments, the genome-editing nuclease is a nucleic acid-guided nuclease (*e.g.,* an RGEN or DGEN), and the cell-penetrating peptides are complexed with the nucleic acid-guided nuclease (or a nucleic acid encoding the nucleic acid-guided nuclease) in combination with (such as complexed with) a guide sequence (or a nucleic acid encoding a guide sequence) to form a genome-editing complex or nanoparticle capable of being delivered to a cell. In some embodiments, the one or more genome-editing system molecules further comprises a donor nucleic acid as a template for homology-directed repair. In some embodiments, the one or more genome-editing system molecules comprise a genome-editing integrase (or a nucleic acid encoding the genome-editing integrase), optionally in combination with (such as complexed with) a donor nucleic acid comprising a recombination site recognized by the integrase to form a genome-editing complex or nanoparticle capable of being delivered to a cell. In some embodiments, the genome-editing system is useful for the treatment of a disease, such as any of the diseases to be treated described herein. In some embodiments, the complex or nanoparticle further comprises one or more molecules of an additional genome-editing system. In some embodiments, the additional genome-editing system is useful for the treatment of the disease. In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease.

In some embodiments, there is provided a method of delivering one or more molecules of a genome-editing system into a hepatocyte comprising contacting the hepatocyte with a genome-editing complex or nanoparticle as described herein, wherein the complex or nanoparticle comprises the one or more genome-editing system molecules and a CPP selected from the group consisting of VEPEP-3 peptides, VEPEP-6 peptides, VEPEP-9 peptides, and ADGN-100 peptides. In some embodiments, the contacting of the hepatocyte with the complex or nanoparticle is carried out *in vivo.* In some embodiments, the contacting of the hepatocyte with the complex or nanoparticle is carried out *ex vivo.* In some embodiments, the contacting of the hepatocyte with the complex or nanoparticle is carried out *in vitro.* In some embodiments, the hepatocyte is an immortalized hepatocyte, such as a hepatocyte from a hepatocyte line. In some embodiments, the hepatocyte is a primary hepatocyte, such as a hepatocyte of an individual. In some embodiments, the one or more genome-editing system molecules comprise a genome-editing nuclease (or a nucleic acid encoding the genome-editing nuclease, such as an mRNA or DNA plasmid). In some embodiments, the genome-editing nuclease is a ZFN, TALEN, homing endonuclease, RGEN, or DGEN. In some embodiments, the genome-editing nuclease is a nucleic acid-guided nuclease (*e.g.,* an RGEN or DGEN), and the cell-penetrating peptides are complexed with the nucleic acid-guided nuclease (or a nucleic acid encoding the nucleic acid-guided nuclease) in combination with (such as complexed with) a guide sequence (or a nucleic acid encoding a guide sequence) to form a genome-editing complex or nanoparticle capable of being delivered to a cell. In some embodiments, the one or more genome-editing system molecules further comprises a donor nucleic acid as a template for homology-directed repair. In some embodiments, the one or more genome-editing system molecules comprise a genome-editing integrase (or a nucleic acid encoding the genome-editing integrase), optionally in combination with (such as complexed with) a donor nucleic acid comprising a recombination site recognized by the integrase to form a genome-editing complex or nanoparticle capable of being delivered to a cell. In some embodiments, the genome-editing system is useful for the treatment of a disease, such as any of the diseases to be treated described herein. In some embodiments, the complex or nanoparticle further comprises one or more molecules of an additional genome-editing system In some embodiments, the additional genome-editing system is useful for the treatment of the disease. In some embodiments, the method further comprises contacting the cell with an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a recombinant receptor, such as chimeric antigen receptor. In some embodiments, the recombinant receptor is a chimeric antigen receptor that specifically binds to an antigen associated with the disease.

In some embodiments, there is provided a method of delivering one or more molecules of a genome-editing system into a cell in an individual comprising administering to the individual a composition comprising a genome-editing complex or nanoparticle as described herein, wherein the complex or nanoparticle comprises the one or more genome-editing system molecules and a CPP selected from the group consisting of VEPEP-3 peptides, VEPEP-6 peptides, VEPEP-9 peptides, and ADGN-100 peptides. In some embodiments, the composition is administered to the individual via an intravenous, intraarterial, intraperitoneal, intravesicular, subcutaneous, intrathecal, intracranial, intracerebral, intracerebroventricular, intrapulmonary, intramuscular, intratracheal, intraocular, ophthalmic, intraportal, transdermal, intradermal, oral, sublingual, topical, or inhalation route. In some embodiments, the composition is administered to the individual via an intravenous route. In some embodiments, the composition is administered to the individual via a subcutaneous route. In some embodiments, the cell is present in an organ or tissue including lung, liver, brain, kidney, heart, spleen, blood, pancreas, muscle, bone marrow, and intestine. In some embodiments, the cell is present in the lung, liver, kidney, or spleen of the individual. In some embodiments, the one or more genome-editing system molecules comprise a genome-editing nuclease (or a nucleic acid encoding the genome-editing nuclease, such as an mRNA or DNA plasmid). In some embodiments, the genome-editing nuclease is a ZFN, TALEN, homing endonuclease, RGEN, or DGEN. In some embodiments, the genome-editing nuclease is a nucleic acid-guided nuclease (e.g., an RGEN or DGEN), and the cell-penetrating peptides are complexed with the nucleic acid-guided nuclease (or a nucleic acid encoding the nucleic acid-guided nuclease) in combination with (such as complexed with) a guide sequence (or a nucleic acid encoding a guide sequence) to form a genome-editing complex or nanoparticle capable of being delivered to a cell. In some embodiments, the one or more genome-editing system molecules further comprises a donor nucleic acid as a template for homology-directed repair. In some embodiments, the one or more genome-editing system molecules comprise a genome-editing integrase (or a nucleic acid encoding the genome-editing integrase), optionally in combination with (such as complexed with) a donor nucleic acid comprising a recombination site recognized by the integrase to form a genome-editing complex or nanoparticle capable of being delivered to a cell. In some embodiments, the cell is an immune cell, such as a granulocyte, a mast cell, a monocyte, a dendritic cell, a B cell, a T cell, or a natural killer cell. In some embodiments, the cell is a fibroblast. In some embodiments, the cell is a hepatocyte. In some embodiments, the cell is a human lung progenitor cell (LPC). In some embodiments, the cell is a neuronal cell. In some embodiments, the individual has, or is at risk of developing, a disease, and the CRISPR system is useful for the treatment of the disease. In some embodiments, the composition further comprises an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a chimeric antigen receptor. In some embodiments, the chimeric antigen receptor specifically binds to an antigen associated with the disease. In some embodiments, the composition is a pharmaceutical composition, and further comprises a pharmaceutically acceptable carrier. In some embodiments, the individual is a mammal. In some embodiments, the individual is human.

In some embodiments, there is provided a method of delivering one or more CRISPR system molecules into a cell in an individual comprising administering to the individual a composition comprising a genome-editing complex or nanoparticle as described herein, wherein the complex or nanoparticle comprises the one or more CRISPR system molecules and a CPP comprising the amino acid sequence of a VEPEP-3 peptide, a VEPEP-6 peptide, a VEPEP-9 peptide, or an ADGN-100 peptide. In some embodiments, the VEPEP-3 peptide comprises the amino acid sequence of any one of SEQ ID NOs: 1-14, 75, and 76. In some embodiments, the VEPEP-6 peptide comprises the amino acid sequence of any one of SEQ ID NOs: 15-40, and 77. In some embodiments, the VEPEP-9 peptide comprises the amino acid sequence of any one of SEQ ID NOs: 41-52, and 78. In some embodiments, the ADGN-100 peptide comprises the amino acid sequence of any one of SEQ ID NOs: 53-70, 79, and 80. In some embodiments, the composition is administered to the individual via an intravenous, intraarterial, intraperitoneal, intravesicular, subcutaneous, intrathecal, intracranial, intracerebral, intracerebroventricular, intrapulmonary, intramuscular, intratracheal, intraocular, ophthalmic, intraportal, transdermal, intradermal, oral, sublingual, topical, or inhalation route. In some embodiments, the composition is administered to the individual via an intravenous route. In some embodiments, the composition is administered to the individual via a subcutaneous route. In some embodiments, the one or more CRISPR system molecules are selected from the group consisting of CRISPR-associated proteins (*e.g.,* RGENs, such as Cas9, including nucleic acids encoding the RGEN) and nucleic acids (*e.g.,* gRNAs and donor nucleic acid). In some embodiments, the cell is an immune cell, such as a granulocyte, a mast cell, a monocyte, a dendritic cell, a B cell, a T cell, or a natural killer cell. In some embodiments, the cell is a fibroblast. In some embodiments, the cell is a hepatocyte. In some embodiments, the cell is a human lung progenitor cell (LPC). In some embodiments, the cell is a neuronal cell. In some embodiments, the individual has, or is at risk of developing, a disease, and the CRISPR system is useful for the treatment of the disease. In some embodiments, the composition further comprises an expression complex comprising a cell-penetrating peptide (such as any of the cell-penetrating peptides described herein, including VEPEP-3 peptides, VEPEP-6 peptides, VEPEP-9 peptides, and ADGN-100 peptides) and a nucleic acid molecule encoding a chimeric antigen receptor. In some embodiments, the chimeric antigen receptor specifically binds to an antigen associated with the disease. In some embodiments, the cell-penetrating peptide is an ADGN-100 peptide or a VEPEP-3 peptide. In some embodiments, the composition is a pharmaceutical composition, and further comprises a pharmaceutically acceptable carrier. In some embodiments, the individual is a mammal. In some embodiments, the individual is human.

### Methods of cell engineering

In some embodiments, there is provided a method of producing an engineered cell, such as an engineered T cell, comprising a method described herein for delivering one or more molecules of a genome-editing system into a cell. In some embodiments, the method is an improvement over previous methods of producing an engineered cell, such as methods involving the use of electroporation or viral transfection. In some embodiments, the improvement includes, without limitation, increasing the efficiency of the method, reducing costs associated with the method, reducing cellular toxicity of the method, and/or reducing the complexity of the method (such as by eliminating the need for precautions necessary when using viral methods for cells intended for use in humans).

### Methods of genome-editing system stabilization

In another aspect of the present application, there is provided a method of stabilizing one or more molecules of a genome-editing system (such as CRISPR system molecules), comprising combining the one or more genome-editing system molecules with a CPP as described herein, thereby stabilizing the one or more genome-editing system molecules. In some embodiments, the one or more genome-editing system molecules and the CPP form a genome-editing complex or nanoparticle as described herein. In some embodiments, the one or more genome-editing system molecules includes a nucleic acid and the CPP stabilizes the supercoil structure of the nucleic acid. In some embodiments, the one or more genome-editing system molecules are susceptible to degradation (for example by serum components or nucleases *in vitro* or *in vivo),* and the CPP protects the one or more genome-editing system molecules from the degradation.

It is to be understood that any of the methods described herein can be combined. Thus, for example, a first set of one or more molecules of a genome-editing system (such as CRISPR system molecules) and a second set of one or more molecules of a genome-editing system can be delivered into a cell by combining any of the methods described herein for delivering a plurality of genome-editing system molecules into a cell. Possible combinations contemplated include combinations of two or more of any of the methods described herein. Kits

Also provided herein are kits, reagents, and articles of manufacture useful for the methods described herein. Such kits may contain vials containing the CPPs, assembly molecules and/or other cell-penetrating peptides, separately from vials containing the one or more genome-editing system molecules (such as CRISPR system molecules). At the time of patient treatment, it is first determined what particular pathology is to be treated based on for example, gene expression analysis or proteomic or histological analysis of patient samples. Having obtained those results, the CPPs and any optional assembly molecules and/or cell-penetrating peptides are combined accordingly with the appropriate one or more molecules of a genome-editing system to result in complexes or nanoparticles that can be administered to the patient for an effective treatment. Thus, in some embodiments, there is provided a kit comprising: 1) a CPP, and optionally 2) one or more molecules of a genome-editing system. In some embodiments, the kit further comprises assembly molecules and/or other cell-penetrating peptides. In some embodiments, the kit further comprises a nucleic acid molecule encoding an exogenous molecule and optionally a cell-penetrating peptide, as described herein for use in an expression complex. In some embodiments, the kit further comprises agents for determining gene expression profiles. In some embodiment, the kit further comprises a pharmaceutically acceptable carrier.

The kits described herein may further comprise instructions for using the components of the kit to practice the subject methods (for example instructions for making the pharmaceutical compositions described herein and/or for use of the pharmaceutical compositions). The instructions for practicing the subject methods are generally recorded on a suitable recording medium. For example, the instructions may be printed on a substrate, such as paper or plastic, etc. As such, the instructions may be present in the kits as a package insert, in the labeling of the container of the kits or components thereof (i.e., associated with the packaging or sub packaging) etc. In some embodiments, the instructions are present as an electronic storage data file present on a suitable computer readable storage medium, e.g., CD-ROM, diskette, etc. In yet other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable substrate

The various components of the kit may be in separate containers, where the containers may be contained within a single housing, e.g., a box.

### EXEMPLARY EMBODIMENTS

Embodiment 1. In some embodiments, there is provided a genome-editing complex for modifying a target polynucleotide comprising a cell-penetrating peptide and one or more molecules of a genome-editing system, wherein the cell-penetrating peptide is selected from the group consisting of VEPEP-3 peptides, VEPEP-6 peptides, VEPEP-9 peptides, and ADGN-100 peptides, and wherein the one or more genome-editing system molecules are selected from the group consisting of:
   a) one or both of an RNA-guided endonuclease (RGEN) and a guide RNA (gRNA), wherein the gRNA comprises a guide sequence complementary to a target sequence in the target polynucleotide;
   b) one or both of a DNA-guided endonuclease (DGEN) and a guide DNA (gDNA), wherein the gDNA comprises a guide sequence complementary to a target sequence in the target polynucleotide;
   c) a zinc finger protein (ZFP), wherein the ZFP recognizes a target sequence in the target polynucleotide;
   d) a transcription activator-like effector nuclease (TALEN), wherein the TALEN recognizes a target sequence in the target polynucleotide;
   e) a homing endonuclease, wherein the homing endonuclease recognizes a target sequence in the target polynucleotide; and
   f) an integrase, wherein the integrase recognizes a recombination site in the target polynucleotide.
Embodiment 2. The genome-editing complex of embodiment 1, wherein the cell-penetrating peptide is a VEPEP-3 peptide.
Embodiment 3. The genome-editing complex of embodiment 2, wherein the cell-penetrating peptide comprises an amino acid sequence selected from the group consisting of SEQ ID NOs: 1-14.
Embodiment 4. The genome-editing complex of embodiment 2, wherein the cell-penetrating peptide comprises the amino acid sequence of SEQ ID NO: 75 or 76.
Embodiment 5. The genome-editing complex of embodiment 1, wherein the cell-penetrating peptide is a VEPEP-6 peptide.
Embodiment 6. The genome-editing complex of embodiment 5, wherein the cell-penetrating peptide comprises an amino acid sequence selected from the group consisting of SEQ ID NOs: 15-40.
Embodiment 7. The genome-editing complex of embodiment 5, wherein the cell-penetrating peptide comprises the amino acid sequence of SEQ ID NO: 77.
Embodiment 8. The genome-editing complex of embodiment 1, wherein the cell-penetrating peptide is a VEPEP-9 peptide.
Embodiment 9. The genome-editing complex of embodiment 8, wherein the cell-penetrating peptide comprises an amino acid sequence selected from the group consisting of SEQ ID NOs: 41-52.
Embodiment 10. The genome-editing complex of embodiment 8, wherein the cell-penetrating peptide comprises the amino acid sequence of SEQ ID NO: 78.
Embodiment 11. The genome-editing complex of embodiment 1, wherein the cell-penetrating peptide is an ADGN-100 peptide.
Embodiment 12. The genome-editing complex of embodiment 11, wherein the cell-penetrating peptide comprises an amino acid sequence selected from the group consisting of SEQ ID NOs: 53-70.
Embodiment 13. The genome-editing complex of embodiment 11, wherein the cell-penetrating peptide comprises the amino acid sequence of SEQ ID NO: 79 or 80.
Embodiment 14. The genome-editing complex of any one of embodiments 1-13, wherein the cell-penetrating peptide further comprises one or more moieties covalently linked to the N-terminus of the cell-penetrating peptide, and wherein the one or more moieties are selected from the group consisting of an acetyl, a fatty acid, a cholesterol, a poly-ethylene glycol, a nuclear localization signal, nuclear export signal, an antibody, a polysaccharide and a targeting molecule.
Embodiment 15. The genome-editing complex of embodiment 14, wherein the cell-penetrating peptide comprises an acetyl group covalently linked to its N-terminus.
Embodiment 16. The genome-editing complex of any one of embodiments 1-15, wherein the cell-penetrating peptide further comprises one or more moieties covalently linked to the C-terminus of the cell-penetrating peptide, and wherein the one or more moieties are selected from the group consisting of a cysteamide, a cysteine, a thiol, an amide, a nitrilotriacetic acid optionally substituted, a carboxyl, a linear or ramified C₁-C₆ alkyl optionally substituted, a primary or secondary amine, an osidic derivative, a lipid, a phospholipid, a fatty acid, a cholesterol, a poly-ethylene glycol, a nuclear localization signal, nuclear export signal, an antibody, a polysaccharide and a targeting molecule.
Embodiment 17. The genome-editing complex of embodiment 16, wherein the cell-penetrating peptide comprises a cysteamide group covalently linked to its C-terminus.
Embodiment 18. The genome-editing complex of any one of embodiments 1-17, wherein at least some of the cell-penetrating peptides in the genome-editing complex are linked to a targeting moiety by a linkage.
Embodiment 19. The genome-editing complex of embodiment 18, wherein the linkage is covalent.
Embodiment 20. The genome-editing complex of any one of embodiments 1-19, wherein the genome-editing complex comprises one or both of an RGEN and a gRNA.
Embodiment 21. The genome-editing complex of embodiment 20, wherein the genome-editing complex comprises an RGEN and a gRNA.
Embodiment 22. The genome-editing complex of embodiment 20 or 21, wherein the gRNA is a single guide RNA (sgRNA).
Embodiment 23. The genome-editing complex of any one of embodiments 20-22, wherein the RGEN is Cas9.
Embodiment 24. The genome-editing complex of any one of embodiments 20-23, wherein the molar ratio of RGEN to gRNA is between about 1:10 and about 10:1.
Embodiment 25. The genome-editing complex of any one of embodiments 20-24, wherein the molar ratio of the cell-penetrating peptide to the RGEN is between about 1:1 and about 80:1.
Embodiment 26. The genome-editing complex of embodiment 25, wherein the molar ratio of the cell-penetrating peptide to the RGEN is between about 5:1 and about 20:1.
Embodiment 27. The genome-editing complex of any one of embodiments 20-26, further comprising one or more additional gRNAs comprising different guide sequences.
Embodiment 28. The genome-editing complex of embodiment 27, wherein the target sequence is present in a target gene, and the one or more additional gRNAs individually comprise a guide sequence complementary to a sequence in the target gene.
Embodiment 29. The genome-editing complex of embodiment 27, wherein the target sequence is present in a first target gene, and the one or more additional gRNAs individually comprise a guide sequence complementary to a sequence present in one or more additional target genes.
Embodiment 30. The genome-editing complex of any one of embodiments 1-19, wherein the genome-editing complex comprises one or both of a DGEN and a gDNA.
Embodiment 31. The genome-editing complex of any one of embodiments 20, wherein the genome-editing complex comprises a DGEN and a gDNA.
Embodiment 32. The genome-editing complex of any one of embodiments 1-19, wherein the genome-editing complex comprises a ZFP.
Embodiment 33. The genome-editing complex of any one of embodiments 1-19, wherein the genome-editing complex comprises a TALEN.
Embodiment 34. The genome-editing complex of any one of embodiments 1-19, wherein the genome-editing complex comprises a homing nuclease.
Embodiment 35. The genome-editing complex of any one of embodiments 20-34, further comprising a donor nucleic acid for introducing a modification to the target polynucleotide, wherein the donor nucleic acid comprises a sequence corresponding to a portion of the target polynucleotide modified to comprise the modification.
Embodiment 36. The genome-editing complex of embodiment 35, wherein the modification is addition, deletion, or substitution of one or more nucleotides in the target polynucleotide.
Embodiment 37. The genome-editing complex of embodiment 35 or 36, wherein the donor nucleic acid is a single-stranded DNA oligonucleotide.
Embodiment 38. The genome-editing complex of embodiment 35, wherein the modification is insertion of a heterologous nucleic acid in the target polynucleotide.
Embodiment 39. The genome-editing complex of embodiment 35 or 38, wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified.
Embodiment 40. The genome-editing complex of any one of embodiments 35-39, further comprising one or more additional donor nucleic acids for introducing modifications to one or more additional target polynucleotides.
Embodiment 41. The genome-editing complex of any one of embodiments 1-19, wherein the genome-editing complex comprises an integrase.
Embodiment 42. The genome-editing complex of embodiment 41, further comprising a donor nucleic acid for insertion into the target polynucleotide, wherein the integrase recognizes a recombination site in the donor nucleic acid and is capable of mediating recombination between the recombination site in the target polynucleotide and the recombination site in the donor nucleic acid to insert the donor nucleic acid into the target polynucleotide.
Embodiment 43. The genome-editing complex of any one of embodiments 1-42, wherein the average diameter of the genome-editing complex is between about 10 nm and about 300 nm.
Embodiment 44. In some embodiments, there is provided a nanoparticle comprising a core comprising the genome-editing complex of any one of embodiments 1-43.
Embodiment 45. The nanoparticle of embodiment 44, wherein the core further comprises one or more additional genome-editing complexes of any one of embodiments 1-43.
Embodiment 46. The nanoparticle of embodiment 44 or 45, wherein the core further comprises a donor nucleic acid for introducing a modification to the target polynucleotide comprising a sequence corresponding to a portion of the target polynucleotide modified to comprise the modification, wherein the donor nucleic acid is complexed with a second cell-penetrating peptide.
Embodiment 47. The nanoparticle of embodiment 46, wherein the second cell-penetrating peptide is selected from the group consisting of VEPEP-3 peptides, VEPEP-6 peptides, VEPEP-9 peptides, and ADGN-100 peptides.
Embodiment 48. The nanoparticle of embodiment 47, wherein the second cell-penetrating peptide comprises an amino acid sequence selected from the group consisting of SEQ ID NOs: 1-70 and 75-80.
Embodiment 49. The nanoparticle of any one of embodiments 46-48, wherein the modification is addition, deletion, or substitution of one or more nucleotides in the target polynucleotide.
Embodiment 50. The nanoparticle of embodiment 49, wherein the donor nucleic acid is a single-stranded DNA oligonucleotide.
Embodiment 51. The nanoparticle of any one of embodiments 46-48, wherein the modification is insertion of a heterologous nucleic acid in the target polynucleotide.
Embodiment 52. The nanoparticle of embodiment 51, wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified.
Embodiment 53. The nanoparticle of any one of embodiments 46-52, wherein the core further comprises one or more additional donor nucleic acids for introducing modifications to one or more additional target polynucleotides.
Embodiment 54. The nanoparticle of any one of embodiments 44-53, wherein at least some of the cell-penetrating peptides in the nanoparticle are linked to a targeting moiety by a linkage.
Embodiment 55. The nanoparticle of any one of embodiments 44-53, wherein the core is coated by a shell comprising a peripheral cell-penetrating peptide.
Embodiment 56. The nanoparticle of embodiment 55, wherein the peripheral cell-penetrating peptide is selected from the group consisting of VEPEP-3 peptides, VEPEP-6 peptides, VEPEP-9 peptides, and ADGN-100 peptides.
Embodiment 57. The nanoparticle of embodiment 56, wherein the peripheral cell-penetrating peptide comprises an amino acid sequence selected from the group consisting of SEQ ID NOs: 1-70 and 75-80.
Embodiment 58. The nanoparticle of any one of embodiments 55-57, wherein at least some of the peripheral cell-penetrating peptides in the shell are linked to a targeting moiety by a linkage.
Embodiment 59. The nanoparticle of embodiment 54 or 58, wherein the linkage is covalent.
Embodiment 60. The nanoparticle of any one of embodiments 44-59, wherein the average diameter of the nanoparticle is between about 10 nm and about 400 nm.
Embodiment 61. In some embodiments, there is provided a pharmaceutical composition comprising the genome-editing complex of any one of embodiments 1-43 or the nanoparticle of any one of embodiments 44-60, and a pharmaceutically acceptable carrier.
Embodiment 62. The pharmaceutical composition of embodiment 61, further comprising an expression complex comprising a third cell-penetrating peptide and a nucleic acid molecule encoding an exogenous protein.
Embodiment 63. The pharmaceutical composition of embodiment 62, wherein the exogenous protein is a recombinant receptor capable of being expressed on the surface of a cell.
Embodiment 64. The pharmaceutical composition of embodiment 63, wherein the recombinant receptor is a chimeric antigen receptor (CAR).
Embodiment 65. The pharmaceutical composition of any one of embodiments 62-64, wherein the third cell-penetrating peptide is selected from the group consisting of VEPEP-3 peptides, VEPEP-6 peptides, VEPEP-9 peptides, and ADGN-100 peptides.
Embodiment 66. The pharmaceutical composition of embodiment 65, wherein the third cell-penetrating peptide comprises an amino acid sequence selected from the group consisting of SEQ ID NOs: 1-70 and 75-80.
Embodiment 67. In some embodiments, there is provided a method of preparing the genome-editing complex of any one of embodiments 1-19, comprising combining the cell-penetrating peptide with the one or more genome-editing system molecules, thereby forming the genome-editing complex.
Embodiment 68. The method of embodiment 67, wherein the one or more genome-editing system molecules include an RGEN and a gRNA, and wherein the cell-penetrating peptide and the RGEN are combined at a ratio from about 1:1 to about 80:1, respectively, and the RGEN and the gRNA are combined at a ratio from about 10:1 to about 1:10, respectively.
Embodiment 69. The method of embodiment 68, wherein the cell-penetrating peptide and the RGEN are combined at a ratio from about 5:1 to about 20:1, respectively.
Embodiment 70. The method of embodiment 68 or 69, wherein the RGEN and the gRNA are combined at a ratio of about 1:1.
Embodiment 71. In some embodiments, there is provided a method of delivering one or more molecules of a genome-editing system into a cell, comprising contacting the cell with the genome-editing complex of any one of embodiments 1-43 or the nanoparticle of any one of embodiments 44-60, wherein the genome-editing complex or the nanoparticle comprises the one or more genome-editing system molecules.
Embodiment 72. The method of embodiment 71, wherein the contacting of the cell with the genome-editing complex or nanoparticle is carried out *in vivo.*
Embodiment 73. The method of embodiment 71, wherein the contacting of the cell with the genome-editing complex or nanoparticle is carried out *ex vivo.*
Embodiment 74. The method of embodiment 71, wherein the contacting of the cell with the genome-editing complex or nanoparticle is carried out *in vitro.*
Embodiment 75. The method of any one of embodiments 71-74, wherein the cell is a granulocyte, a mast cell, a monocyte, a dendritic cell, a B cell, a T cell, a natural killer cell, a fibroblast, a hepatocyte, a lung progenitor cell, or a neuronal cell.
Embodiment 76. The method of embodiment 75, wherein the cell is a T cell.
Embodiment 77. The method of embodiment 75 or 76, wherein the genome-editing system targets a sequence in a gene selected from the group consisting of PD-1, PD-L1, PD-L2, TIM-3, BTLA, VISTA, LAG-3, CTLA-4, TIGIT, 4-1BB, OX40, CD27, TIM-1, CD28, HVEM, GITR, and ICOS.
Embodiment 78. The method of any one of embodiments 71-77, further comprising contacting the cell with an expression complex comprising a fourth cell-penetrating peptide and a nucleic acid molecule encoding an exogenous protein.
Embodiment 79. The method of embodiment 78, wherein the exogenous protein is a recombinant receptor capable of being expressed on the surface of a cell.
Embodiment 80. The method of embodiment 79, wherein the recombinant receptor is a chimeric antigen receptor (CAR).
Embodiment 81. The method of any one of embodiments 78-80, wherein the fourth cell-penetrating peptide is selected from the group consisting of VEPEP-3 peptides, VEPEP-6 peptides, VEPEP-9 peptides, and ADGN-100 peptides.
Embodiment 82. The method of embodiment 81, wherein the fourth cell-penetrating peptide comprises an amino acid sequence selected from the group consisting of SEQ ID NOs: 1-70 and 75-80.
Embodiment 83. In some embodiments, there is provided a method of modifying a target polynucleotide in a cell, comprising contacting the cell with the genome-editing complex of any one of embodiments 1-43 or the nanoparticle of any one of embodiments 44-60, wherein the genome-editing complex or the nanoparticle comprises one or more molecules of a genome-editing system that targets a sequence in the target polynucleotide.
Embodiment 84. In some embodiments, there is provided a method of treating a disease in an individual comprising administering to the individual an effective amount of the pharmaceutical composition of any one of embodiments 61-66.
Embodiment 85. The method of embodiment 84, wherein the disease is selected from the group consisting of cancer, diabetes, inflammatory diseases, fibrosis, viral infectious diseases, hereditary diseases, ocular diseases, liver diseases, lung diseases, kidney diseases, aging and degenerative diseases, and diseases characterized by cholesterol level abnormality.
Embodiment 86. The method of embodiment 85, wherein the disease is cancer.
Embodiment 87. The method of embodiment 86, wherein the cancer is a solid tumor, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis.
Embodiment 88. The method of embodiment 87, wherein the cancer is cancer of the liver, lung, or kidney.
Embodiment 89. The method of embodiment 86, wherein the cancer is a hematological malignancy, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis.
Embodiment 90. The method of embodiment 85, wherein the disease is a viral infection disease, and wherein the pharmaceutical composition comprises a genome-editing complex or nanoparticle comprising one or more molecules of a genome-editing system that modulates the expression of one or more proteins involved in the viral infectious disease development and/or progression.
Embodiment 91. The method of embodiment 85, wherein the disease is a hereditary disease, and wherein the pharmaceutical composition comprises a genome-editing complex or nanoparticle comprising one or more molecules of a genome-editing system that modulates the expression of one or more proteins involved in the hereditary disease development and/or progression.
Embodiment 92. The method of embodiment 85, wherein the disease is an aging or degenerative disease, and wherein the pharmaceutical composition comprises a genome-editing complex or nanoparticle comprising one or more molecules of a genome-editing system that modulates the expression of one or more proteins involved in the aging or degenerative disease development and/or progression.
Embodiment 93. The method of embodiment 85, wherein the disease is a fibrotic or inflammatory disease, and wherein the pharmaceutical composition comprises a genome-editing complex or nanoparticle comprising one or more molecules of a genome-editing system that modulates the expression of two or more proteins involved in the fibrotic or inflammatory disease development and/or progression.
Embodiment 94. The method of any one of embodiments 84-93, wherein the individual is human.
Embodiment 95. In some embodiments, there is provided a kit comprising a composition comprising the genome-editing complex of any one of embodiments 1-43 and/or the nanoparticle of any one of embodiments 44-60.

### EXAMPLES

Those skilled in the art will recognize that several embodiments are possible within the scope and spirit of this invention. The invention will now be described in greater detail by reference to the following non-limiting examples. The following examples further illustrate the invention but, of course, should not be construed as in any way limiting its scope.

### Materials and Methods

Lipofectamine 2000, RNAiMAX, TranscriptAid T7 transcription kit, MEGAclear transcription Clean Up kit, GeneArt Genomic Cleavage Detection kit, and Platinum Green Hot Start PCR mix were obtained from Thermo Fisher Life Science (France). CRISPR/Cas9 antibodies were obtained from Diagenode (Diagenode cat. No. C15200223) and ABCAM (clone 7A9- ab191468). HiScribe™ T7 ARCA mRNA Kit was obtained from New England Biolab. All oligonucleotides were obtained from Eurogentec (Belgium).

### gRNA

gRNAs were obtained by *in vitro* transcription. Generation of sgRNA was performed using a generic sgRNA expression plasmid containing a T7 promoter adapter sequence as template for a PCR product, which can be *in vitro* transcribed. Linear DNA fragments containing the T7 promoter binding site followed by the 20-bp sgRNA target sequence were transcribed *in vitro* using TranscriptAid T7 high Yield transcription Kit (Thermo Fisher life science, France) following the manufacturer's instructions. *In vitro* transcribed gRNAs were precipitated with ethanol and further purified using MEGAclear transcription clean up Kit (Thermo Fisher life Science). Stock solutions of sgRNAs were solubilized in water, quantified by UV absorbance and stored at -80 °C.

EGFP gRNA was obtained by *in vitro* transcription using an sgRNA expression plasmid (Addgene 47511, plasmid pFYF1320 EGFP) according to Fu et al. (2013). Nature biotechnology, 31(9):822-826. EGFP target site: GGGCACGGGCAGCTTGCCGG (SEQ ID NO: 71).

EMX1 gRNA was obtained by *in vitro* transcription using an sgRNA expression plasmid (Addgene #47508, plasmid pFYF1548 EMX1) according to Fu *et al.,* 2013 (*supra*). EMX1 target site: GAGTCCGAGCAGAAGAAGAA (SEQ ID NO: 72).

HPRT gRNA was obtained by *in vitro* transcription using sgRNA expression plasmid (Addgene #69539, plasmid HPRT2b-pGEM) according to Liao et al., (2015). Nucleic acids research*,* gkv675. HPRT target site: GACTGTAAGTGAATTACTT (SEQ ID NO: 73).

AAVS gRNA was obtained by *in vitro* transcription using an sgRNA expression plasmid (Addgene #41818, plasmid gRNA_AAVSI-T2) according to Mali et al., (2013). Science, 339(6121):823-826. AAVS target site: GGGGCCACTAGGGACAGGAT (SEQ ID NO: 74).

Luciferase gRNA was obtained by *in vitro* transcription using an sgRNA expression plasmid (Addgene #74190, plasmid pLCKO_Luciferase_sgRNA) according to Hart, T., et al. (2015). Cell, 163(6), 1515-1526. Luciferase target site: ACAACTTTACCGACCGCGCC (SEQ ID NO: 103).

hPD1 gRNA was obtained by *in vitro* transcription using sgRNA expression plasmids gRN AhPD1a and gRNAhPDlb obtained from Eurogentec (Belgium). hPD1sgA1:
ACCGCGTGACTTCCACATGAGCG (SEQ ID NO: 99). hPD1sgB1:
AAACCGCTCATGTGGAAGTCACG (SEQ ID NO: 100). hPD1sgC1f:
ACCGCAGTTGTGTGACACGGAAG (SEQ ID NO: 101). hPD1sgD2:
AAACCTTCCGTGTCACACAACTG (SEQ ID NO: 102).

### CAS9 mRNA

CAS9 mRNA was prepared using the HiScribe™ T7 ARCA mRNA Kit (New England Biolab) with linearized pCS2-CAS9 vector (Addgene # 47322) for the DNA template. Synthesized mRNA was purified by LiCl precipitation, phenol:chloroform extraction, and ethanol precipitation. mRNA concentration was determined by measuring the ultraviolet light absorbance at 260 nm, and samples were stored at -20°C. For in vivo studies, polyadenylated, capped CAS9 mRNA was obtained from ThermoFisher.

### CAS9 Protein expression and purification

*S. pyogenes* HIS-NLS-CAS9 was expressed in *E. coli* and purified as previously described by Zuris et al., 2015. Nature biotechnology, 33(1):73-80. S. pyogenes HIS-CAS9-NLS pET29 expression vector (Addgene #62933) was transformed in BL21 STAR (DE3) competent cells. Cells were grown in luria Bertani (LB) Medium containing 100 mg/ml ampicillin at 37 °C up to about 0.5 OD₅₉₅. Cell cultures were then cooled down to 20 °C and protein expression was induced overnight with 0.5 mM Isopropl-1-thio-β-D-galactopyranoside. Cells were harvested by centrifugation and lysed by sonication in lysis buffer containing 50 mM HEPES (pH 8.0), 0.5 mM NaCl, 50 mM KCl, 20% Glycerol and 0.5 mM Tris (2-carboxyethyl)phosphine (TCEP). Soluble lysate containing HIS-NLS-CAS9 was obtained by centrifugation at 20,000 x g for 30 min and incubated for 1 hr at 4 °C with 2 ml of NI-NTA (nitriloacetic acid) Agarose Resin (Thermo Fisher Life Science). Resin was then washed twice with lysis buffer and HIS-NLS-CAS9 protein eluted with HEPES (pH 8.0), 0.1 M NaCl, 20% glycerol, 10 mM TCEP and 300 mM imidazole. Protein solution was applied to a HITrap Desalting column (GE Healhcare Life Science) and further purified on a cation exchange HITrap SP HP column (GE Healhcare Life Science) equilibrated in buffer containing 50 mM HEPES (pH 8.0), 0.1 mM NaCl, 20 % glycerol and 10 mM TCEP. HIS-CAS9-NLS was eluted with a linear NaCl gradient, concentrated up to 5 mg/ml, and stored at -80 °C. For some studies, CAS9 protein was obtained from Thermo Fisher Life Science (FRANCE).

### Cell Penetrating Peptides:

Peptides were obtained from GENEPEP Montpellier (FRANCE) in acetate salt form. The following peptides were used:
VEPEP-3a: βAKWFERWFREWPRKRR (SEQ ID NO: 75)
VEPEP-3b: βAKWWERWWREWPRKRR (SEQ ID NO: 76)
VEPEP-6: βALWRALWRLWRSLWRLLWKA (SEQ ID NO: 77)
VEPEP-9: βALRWWLRWASRFSRWAWWR (SEQ ID NO: 78)
ADGN-100a: βAKWRSAGWRWRLWRVRSWSR (SEQ ID NO: 79)
ADGN-100b: βAKWRSALYRWRLWRVRSWSR (SEQ ID NO: 80)
CADY: GLWRALWRLLRSLWRLLWKV (SEQ ID NO: 81)

Stock solutions of peptides were prepared at 2 mg/mL in distilled water or 5% DMSO and sonicated for 10 min in a water bath sonicator then diluted just before use.

### Cell lines

Several cell lines were used, including stable EGFP expressing cell lines (GFP-U2OS, EGFP-JURKAT T, EGFP-HEK) as well as U2OS (ATCC® HTB-96™), primary human fibroblasts, Hep G2 (ATCC® HB-8065™), Human Embryonic Kidney (HEK293) (ATCC® CRL-1573™), Human Myelogenous Leukemia K562 cells (ATCC® CCL243™), Jurkat T cells (ATCC® TIB-152™), human ESCs (H9), and mouse ESCs (ESF 158). Cells were obtained from the American Type Culture Collection [ATCC].

### Example 1: Cell-penetrating peptide-mediated functional delivery of RGEN/gRNA protein-RNA complex for gene disruption

Five cell-penetrating peptides (VEPEP-6, VEPEP-9, VEPEP-3, ADGN-100 and CADY) were evaluated for the functional cellular delivery of RGEN/gRNA complexes in different cell lines. Cell-penetrating peptides were associated with genome editing RGEN/gRNA complexes comprising the RNA-guided endonuclease (RGEN) CAS9 and a guide RNA (gRNA), wherein the gRNA comprises a guide sequence complementary to a nucleic acid sequence target. CPP-mediated functional delivery of RGEN/gRNA complex was investigated by monitoring gene disruption of an exogenous *EGFP* gene in three stable EGFP expressing cell lines (U2OS, Jurkat T, and HEK) and/or gene disruption of the endogenous *EMX1, AAYS,* or *HPRT* endogenous genes in mouse and human cell lines (U2OS, HEK and K562), including human (H9) and mouse (ESF-158) embryonic stem (ES) cells.

### CPPs form stable complexes with RGEN (CAS9) and RGEN/gRNA (CAS9/sgRNA) complex

Formation of CPP/CAS9 and CPP/CAS9/sgRNA complexes was monitored by fluorescence spectroscopy using CY5 fluorescently labeled CAS9 protein. CAS9 protein was labeled with CY5 dye on accessible cysteine residues using maleimide-CY5. As shown in FIG. 1A and 1B, a fixed 5 nM concentration of CY5-CAS9 (FIG. 1A) or CY5-CAS9:sgRNA (at 1:1 molar ratio) (FIG. 1B) was titrated with increasing concentration of CPPs (VEPEP-3a/b, VEPEP-6, VEPEP-9, ADGN-100a/b, CADY). Curve fitting demonstrated that VEPEP-3a/b, ADGN-100a/b and VEPEP-9 interact with both CY5-CAS9 and CY5-CAS9/sgRNA complex, whereas, VEPEP-6 and CADY only bind CY5-CAS9:sgRNA complexes. As shown in Table 1, VEPEP-3a/b and ADGN-100a/b peptides constitute the most potent peptides and exhibit a very high affinity for RGEN/gRNA complex in the low nanomolar range, VEPEP-9 and CADY are moderate binders, and VEPEP-6 poorly interacts with both CY5-CAS9 and CY5-CAS9/sgRNA complex.

**Table 1: CPP/CAS9, and CPP/CAS9-sgRNA binding parameters.**

| | **Kd (nM)** | |
|---|---|---|
| **Peptides** | **Cas9** | **Cas9/sgRNA** |
| ADGN-100a | 54 ± 10 | 12 ± 5 |
| ADGN-100b | 72 ± 10 | 11 ± 5 |
| VEPEP-3a | 17 ± 5 | 11 ± 5 |
| VEPEP-3b | 15 ± 5 | 13 ± 5 |
| VEPEP-6 | > 100 | 46 ± 10 |
| VEPEP-9 | 62 ± 15 | 28 ± 5 |
| CADY | > 100 | 21 ± 5 |

The CPP/CAS9 and CPP/CAS9/sgRNA particles were further characterized; the mean size, polydispersity and zeta potential were measured with Zetasizer 4 apparatus (Malvem Ltd). CAS9 and preloaded CAS9/gRNA were mixed with VEPEP-3b, VEPEP-9, ADGN-100a and CADY peptides at 10:1 and 20:1 molar ratio of CPP to cargo in physiological conditions (0.9% NaCl) at 20 °C and incubated for 30 min at 37 °C prior to analysis. The mean size and the polydispersity of the CCP/CAS9 and CCP/CAS9:gRNA complexes were determined at 25 °C for 3 min per measurement and zeta potential was measured with Zetasizer 4 apparatus (Malvem Ltd). Data are shown in Table 2 for a mean of 3 separate experiments.

At molar ratio 10:1 or 20:1, both VEPEP-3b and ADGN-100a peptides were able to form stable nanoparticles with CAS9 and CAS9:gRNA complex. Mean diameters of about 50 nm and 80 nm and polydispersity index (PI) of 0.25 and 0.35 were obtained for VEPEP-3b and ADGN-100a, respectively. ADGN-100a/CAS9 and ADGN-100a/CAS9:gRNA particle charge obtained by zeta potential were similar for 10:1 and 20:1 molar ratios, with mean values of 6.0 ± 0.8 mV and 5.3 ± 1.0 mV, respectively, at 10:1 molar ratio and of 7.2 ± 1 mV and 4.8 ± 0.3 mV, respectively, at 20:1 molar ratio. VEPEP-3b/CAS9 and VEPEP-3b/CAS9:gRNA particle charge obtained by zeta potential were similar for 10:1 and 20:1 molar ratios, with mean values ranging between of 17.0 ± 0.5 mV and 27.0 ± 4 mV at 10:1.

In contrast, CADY did not form any complex with CAS9, and at molar ratio of 10:1, neither CADY nor VEPEP-9 formed stable particles with preloaded CAS9:gRNA complex. At a molar ratio 20:1, particle mean size of 170 ± 10 and 350 ± 20 nm and polydispersity index of 0.58 and 0.6 were obtained for VEPEP-9 and CADY, respectively.

**Table 2: CPP/RGEN and CPP/RGEN:gRNA nanoparticle characterizations**

| **Peptides (molar ratio)** | **Cas9** | | | **Cas9:gRNA** | | |
|---|---|---|---|---|---|---|
| | **Size (nm)** | **PI** | **zeta (mV)** | **Size (nm)** | **PI** | **zeta (mV)** |
| VEPEP-3b (10/1) | 45 ± 5 | 0.33 ± 0.1 | 21.0 ± 5 | 38 ± 4 | 0.35 ± 0.1 | 27.0 ± 4 |
| VEPEP-3b (20/1) | 52 ± 5 | 0.35 ± 0.1 | 17.0 ± 5 | 51 ± 5 | 0.37 ± 0.1 | 25.3 ± 7 |
| ADGN-100a (10/1) | 82 ± 5 | 0.25 ± 0.1 | 6.0 ± 0.8 | 91 ± 5 | 0.27 ± 0.1 | 5.3 ± 0.7 |
| ADGN-100a (20/1) | 78 ± 7 | 0.21 ± 0.1 | 7.2 ± 1 | 87 ± 6 | 0.28 ± 0.1 | 4.8 ± 0.3 |
| VEPEP-9 (20/1) | 280 ± 20 | 0.45 ± 0.1 | 32 ± 5 | 170 ± 10 | 0.58 ± 0.1 | 39 ± 8 |
| CADY (20/1) | - | - | - | 350 ± 20 | 0.6 ± 0.2 | 45 ± 8 |

### CPP-mediated Junctional delivery of RGEN/gRNA complex targeting EGFP reporter gene

Five CPPs (VEPEP-3b, VEPEP-6, VEPEP-9, CADY and ADGN-100a) were evaluated for cellular delivery of RGEN/gRNA complexes. Efficacy of CPP-mediated delivery was quantified using a well-established CAS9-induced gene disruption method (Fu et al, 2013, *supra*), by targeting a specific site in the exogenous EGFP gene reporter in three human cell lines expressing EGFP (GFP-U2OS, EGFP-HEK, EGFP-JURKAT).

Stock solutions of amphipathic peptides were prepared at 1 mg/mL in distilled water and sonicated for 10 min. CAS9 protein was preloaded with purified EGFP sgRNA at a molar ratio of 1:1 and incubated for 10 min prior to forming complexes with the peptides. Preloaded CAS9/gRNA complexes (10 nM, 25 nM, and 50 nM) were mixed with CPPs at 5:1, 10:1 and 20:1 molar ratios of CPP to cargo and incubated for 30 min at 37 °C prior to cell transfection.

Cell line containing a single integrated *EGFP* reporter gene (GFP-U2OS, EGFP-HEK and EGFP-JURKAT) were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS) at 37°C in a humidified atmosphere containing 5% CO₂. A total of 150,000 cells seeded in a 35 mm dish the day prior to transfection were grown to 50-60% confluence and overlaid with 200 µl of preformed complexes, incubated for 3-5 min, then 400 µl of DMEM was added. After 30 min incubation at 37°C, 1 mL of fresh DMEM containing 16% FCS was added to reach a final FCS concentration of 10%, without removing the overlay of CPP/CAS9-gRNA complexes. Cells were returned to the incubator for 72 hours.

CAS9-induced double-strand break formation was analyzed using flow cytometry by monitoring level of EGFP expression. Gene disruption frequency data shown in FIGS. 2-5 are reported as averages from 3 separate experiments. Both lipofectamine 2000 and RNAiMAX delivery methods were used as positive controls.

As shown in FIGS. 2A-2D and FIGS. 3A-3D, all CPPs promoted functional delivery of CAS9/gRNA and induced CAS9/gRNA concentration-dependent loss of EGFP expression in both EGFP-U2OS and EGFP-HEK cells. Optimal double strand break formation was obtained with CPP/CAS9/gRNA complexes formed at a 20:1 molar ratio of CPP to preloaded CAS9/gRNA, and no difference was observed using preloaded CAS9/gRNA formed at 1:1 or 1:2 molar ratios, respectively.

In U20S cells, decreases in EGFP expression of 92%, 87%, 55%, 33% and 25% were obtained with CAS9:gRNA (50 nM) associated with VEPEP-3b, ADGN-100a, VEPEP-9, CADY and VEPEP-6 (molar ratio of 20:1 of CPP to cargo), respectively. As a comparison, a decrease of 22% or 71% of EGFP expression was obtained using Lipofectamine 2000 or RNAiMAX as the delivery vector, respectively (FIG. 2C).

In HEK cells, decreases in EGFP expression of 93%, 86%, 58%, 42% and 30% were obtained with CAS9:gRNA (50 nM) associated with VEPEP-3b, ADGN-100a, VEPEP-9, CADY and VEPEP-6 (molar ratio of 20:1 of CPP to cargo), respectively. As a comparison, a decrease of 35% or 78% of EGFP expression was obtained using Lipofectamine 2000 or RNAiMAX as the delivery vector, respectively (FIG. 3C).

For both cell lines, greater double strand break formation was obtained with ADGN-100a and VEPEP-3b peptides, leading to a reduction of more than 85% for EGFP-positive cells. A dose response investigation (FIGS. 2D and 3D) demonstrated that CAS9:gRNA can be efficient at low nanomolar concentrations when associated with ADGN-100a or VEPEP-3b. The number of EGFP-expressing U20S cells was reduced by 68% or 57 % using InM CAS9:gRNA associated with VEPEP-3b or ADGN-100a, respectively. The number of EGFP-expressing HEK cells was reduced by 71% or 62 % using InM CAS9:gRNA associated with VEPEP-3b or ADGN-100a, respectively. In contrast, for both cell lines, using InM CAS9:gRNA resulted in no change in EGFP expression when using VEPEP-6, CADY and lipofectamine 2000, and only 18% or 5% loss of EGFP expression was obtained using RNAiMIX or VEPEP-9, respectively. Therefore, ADGN-100a and VEPEP-3b are 7- to 10-fold more efficient than lipofectamine and CADY, and 2- to 4-fold more efficient than RNAiMAX, for delivering a CRISPR complex.

As shown in FIGS. 4A-4D and FIGS. 5A and 5B, ADGN-100 and VEPEP-3 peptides promoted functional delivery CAS9/gRNA and induced concentration-dependent loss of EGFP expression in EGFP-JURKAT cells. Both peptides led to a reduction of EGFP-positive cells by more than 80% (81% for ADGN-100a and 87% for VEPEP-3b). Only 22% or 18% reduction of EGFP-positive cells was obtained with VEPEP-9 or CADY, respectively, and no significant change in the number of EGFP cells was observed with VEPEP-6 or Lipofectamine 2000. ADGN-100a and VEPEP-3b peptides are 12- to 20-fold more efficient than lipofectamine and CADY for delivering a CRISPR complex. Dose response analysis (FIG. 4D) demonstrated, as for other cell lines, that in JURKAT, CAS9:gRNA can be efficient at low nanomolar concentrations when associated with ADGN-100a and VEPEP-3b. The number of EGFP-expressing cells was reduced by 55% or 51 % using InM CAS9:gRNA associated with VEPEP-3b or ADGN-100a, respectively. In contrast, at the same CAS9:gRNA concentration no change in EGFP expression was observed using VEPEP-6, CADY, VEPEP-9 or lipofectamine 2000, and only 18% loss of EGFP expression was obtained using RNAiMAX.

### CPP mediated cellular delivery of CAS9/sgRNA complex do not induce toxicity

The robust EGFP disruption observed in the three cell lines was found not to be the result of cellular toxicity. The toxicity of CPP/CAS9/gRNA complexes was investigated in the three cell lines (EGFP-U2OS, EGFP-HEK, EGFP-JURKAT). A total of 30,000 cells seeded in 24-well plates the day prior to transfection were incubated with increasing concentrations of CPP/CAS9/gRNA (complexed at a 10:1 or 20:1 molar ratio of CPP to preloaded CAS9/gRNA complex) ranging from 1 to 200 nM for 30 min prior to addition of medium to reach a final 10% concentration of FCS. Cytotoxic response was measured 24 hours later by colorimetric MTT assay (Sigma, Germany). For MTT assay, cell culture medium was removed and replaced with PBS containing 2.5 mg/ml MTT for 4 hours. Results correspond to the average of 3 separate experiments. As shown in FIGS. 6A-6C, for concentrations up to 100 nM, no toxicity was observed for VEPEP-3b or ADGN-100a peptides in the three cell lines. Viability was reduced by less than 10% at 200 nM. In contrast, at 200 mM for Lipofectamine 2000 or RNAiMAX, between 60-70% and 20-25% cell death was observed, depending on cell line. Moreover, both lipid methods appeared to be highly toxic on JURKAT cells, inducing 75% (lipofectamine 2000) or 45% (RNAiMAX) cell death.

### CPP mediated functional delivery of RGEN/gRNA complex targeting EMX1 endogenous gene

VEPEP-3b, VEPEP-9 and ADGN-100a peptides were evaluated for cellular delivery of RGEN/gRNA complexes targeting the endogenous *EMX1* gene in three human cell lines (U2OS, JURKAT and K562).

Preloaded CAS9/gRNA (25 nM, 50 nM) complexes were mixed with VEPEP-3b, VEPEP-9 and ADGN-100a peptides at 20:1 molar ratio of CPP to cargo and incubated for 30 min at 37 °C prior to cell transfection. RNAiMAX delivery was used as a positive control as it was previously reported to show better results than Lipofectamine 2000 (Zuris *et al.,* 2015, *supra*). Cell lines (U2OS, JURKAT and K562) were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS), at 37 °C in a humidified atmosphere containing 5% CO₂. A total of 150,000 cells seeded in a 35 mm dish the day prior to transfection were grown to 50-60% confluence and overlaid with 200 µl of preformed complexes, incubated for 3-5 min, then 400 µl of DMEM was added. After 30 min incubation at 37 °C, 1 mL of fresh DMEM containing 16% FCS was added to reach a final FCS concentration of 10%, without removing the overlay of CPP/CAS9-gRNA complexes. Cells were returned to the incubator for 72 hours. CAS9-induced double-strand break formation followed by repair by non-homologous end joining (NHEJ) was analyzed by either the T7E1 assay or Genome Cleavage Detection kit (GCD Thermo Fisher) and quantified using a Fragment Analyzer System The indel frequency in the target gene was quantified using the T7E1 assay.

As shown in FIGS. 7A-7C and summarized in Table 3, both VEPEP-3b- and ADGN-100a-mediated RGEN/gRNA delivery induced a robust editing of the *EMX-1* gene in all three cell lines. In the three cell lines, RGEN/gRNA induced NHEJ-mediated indel mutation at the intended target site. Using a 25 nM concentration of CAS9-gRNA, mean indel frequencies of 67%, 71%, and 65% were obtained when delivered with ADGN-100a in U20S, K562 and JURKAT cells, respectively. Mean indel frequencies of 75%, 79%, and 69% were obtained using VEPEP-3b in U20S, K562 and JURKAT cells, respectively. Mean indel frequencies of 27%, 29%, and 21 % were obtained using VEPEP-9 in U20S, K562, and JURKAT cells, respectively. VEPEP-3b and ADGN-100a efficiency was at least 2-fold higher in comparison to RNAiMAX, which exhibited indel frequencies of 31%, 39%, and 22% in the same cell lines.

**TABLE 3: Comparison of different peptides to RNAiMAX for RGEN/gRNA delivery and resulting editing efficiencies as measured by T7E1 assay in different cell lines.**

| | **Mean indel Frequency (%) ± S.E.M** | | | | | |
|---|---|---|---|---|---|---|
| | **CAS9/sgRNA (25 nM)** | | | **CAS9/sgRNA (50 nM)** | | |
| | **U20S** | **K562** | **JURKAT** | **U20S** | **K562** | **JURKAT** |
| ADGN-100a | 67 ± 5 | 71 ± 8 | 65 ± 5 | 72 ± 8 | 74 ± 4 | 68 ± 6 |
| VEPEP-3b | 75 ± 7 | 79 ± 5 | 69 ± 8 | 77 ± 5 | 81 ± 7 | 71 ± 5 |
| VEPEP-9 | 27 ± 3 | 29 ± 7 | 21 ± 7 | 30 ± 6 | 25 ± 5 | 21 ± 5 |
| RNAiMAX | 31 ± 6 | 39 ± 6 | 22 ± 5 | 51 ± 7 | 58 ± 8 | 47 ± 5 |

### Example 2: CPP-mediated functional delivery of RGEN/gRNA complex for editing multiple genes

### Human cell lines

VEPEP-3, VEPEP-9 and ADGN-100 peptides were then evaluated for cellular delivery of multiple RGEN/gRNA complexes, targeting endogenous *EMX1* and exogenous *EGFP* reporter genes in two human cell lines expressing EGFP (GFP-U2OS, EGFP-JURKAT). The two preloaded CAS9/gRNA complexes (25 nM) were mixed with VEPEP-3b, VEPEP-9 and ADGN-100a peptides at 20:1 molar ratio of CPP to cargo and incubated for 30 min at 37 °C prior to cell transfection. RNAiMAX delivery was used as positive control. Cells were treated as described previously and CAS9-induced NHEJ analyzed 72 hr after transfection. CAS9-induced double-strand break formation followed by repair by non-homologous end joining (NHEJ) was analyzed by either the T7E1 assay or Genome Cleavage Detection kit (GCD Thermo Fisher) and quantified using a Fragment Analyzer System. The deletion (indel) frequency in the *EMX1* gene was quantified using the T7E1 assay and loss of EGFP expression was monitored by flow cytometry. Data for on-target mutations induced by RGEN/gRNA are reported as an average of 3 separate experiments.

As shown in FIGS. 8A-8C and summarized in Table 4, both VEPEP-3b- and ADGN-100a-mediated RGEN/gRNA delivery induced a robust editing of both *EMX-1* and *EGFP* genes in both cell lines. In the two cell lines, RGEN/gRNA induced NHEJ-mediated indel mutation at the intended target site.

Using a 25 nM concentration of CAS9-gRNA, mean indel frequencies of more than 70% for both genes were obtained with either ADGN-100a or VEPEP-3b in U20S and JURKAT cells. Mean indel frequencies of about 30% and 15% were obtained for VEPEP-9 in U20S and JURKAT cells, respectively. Gene disruption efficiency of 40% in U20S cells and of 28% in JURKAT cells was obtained with lipid based vector RNAiMAX. VEPEP-3b and ADGN-100a peptides were each about 2-fold and 2.8-fold more efficient than RNAiMAX in U20S and JURKAT cells, respectively.

**TABLE 4: comparison of different peptides to RNAiMAX for multiple RGEN/gRNA delivery and resulting editing efficiencies as measured by T7E1 assay in different cell lines**

| | **Mean indel Frequency (%) ± S.E.M** | | | |
|---|---|---|---|---|
| | **U20S** | | **JURKAT** | |
| | **EGFP** | **EMX1** | **EGFP** | **EMX1** |
| ADGN-100a | 77 ± 6 | 72 ± 8 | 78 ± 4 | 76 ± 7 |
| VEPEP-3b | 80 ± 5 | 82 ± 6 | 84 ± 7 | 79 ± 7 |
| VEPEP-9 | 32 ± 4 | 31 ± 5 | 15 ± 8 | 10 ± 2 |
| RNAiMAX | 33 ± 7 | 41 ± 4 | 28 ± 7 | 27 ± 5 |

Taken together; these results demonstrate that both VEPEP-3 and ADGN-100 peptides constitute potent methods for functional delivery of multiple RGEN/gRNA complexes in different cell lines, including hard to transfect cell lines such as JURKAT, without inducing any toxic response.

### Embryonic stem cells

VEPEP-3 and ADGN-100 peptides were then evaluated for cellular delivery of multiple RGEN:gRNA complexes, targeting three endogenous *EMX1, AAVS* and *HPRT* genes in U20S and in Human (H9) and Mouse (ESF-158) embryonic stem cells. The three preloaded CAS9/gRNA complexes (20 nM each) were mixed with VEPEP-3b and ADGN-100a peptides at 20:1 molar ratio of CPP to cargo and incubated for 30 min at 37 °C prior to cell transfection. RNAiMAX delivery method was used as a positive control.

U2OS cells were treated as described previously. H9 embryonic stem cells were cultured in Dulbecco's modified Eagle's medium with 4 mM L-glutamine adjusted to contain 1.5 g/L sodium bicarbonate and 4.5 g/L glucose and supplemented with 0.1 mM 2-mercaptoethanol, 85%; and 15% ES-Cell Qualified fetal bovine serum Mouse ESF-158 embryonic stem cells were cultured in Mouse ES Cell Basal Medium supplemented with 0.1 mM 2-mercaptoethanol, 1,000 U/mL mouse leukemia inhibitory factor (LIF) and 15% ES-Cell Qualified FBS. CAS9-induced double-strand break formation followed by repair through NHEJ was analyzed 72 hr after transfection by T7E1 assay and quantified using Fragment Analyzer System The deletion (indel) frequencies in *EMX1, AAVS* and *HPRT* genes were quantified using T7E1 assay. Data for on-target mutations induced by RGEN:gRNA are reported as an average of 3 separate experiments.

As shown in Table 5, both VEPEP-3b and ADGN-100a-mediated RGEN/gRNA delivery induced robust editing of *EMX-1, AAVS* and *HPRT* genes in the two ES cell lines tested. In all three cell lines, RGEN/gRNA induced NHEJ-mediated indel mutation at the intended on-target site. In contrast, no or negligible gene editing was observed in ES cell lines using RNAiMAX delivery methods.

Using a 20 nM concentration for the 3 CAS9/gRNA complexes, mean indel frequencies of more than 70% for the three genes were obtained with either ADGN-100a or VEPEP-3b, in U20S and mouse ES cells. Mean indel frequencies of more than 60% were obtained in H9 cells. These results further demonstrated that VEPEP-3 and ADGN-100 peptides are potent vehicles for the delivery of multiple RGEN:gRNA complexes.

**Table 5: Comparison of different peptides to RNAiMAX for multiple RGEN:gRNA delivery in ES Cells**

| | **Mean indel Frequency (%) ± S.E.M** | | | | | | | | |
|---|---|---|---|---|---|---|---|---|---|
| **Cell line** | **U20S** | | | **H9** | | | **ESF-158** | | |
| **Gene** | **EMX1** | **AAVS** | **HPRT** | **EMX1** | **AAVS HPRT** | | **EMX1** | **AAVS** | **HPRT** |
| ADGN-100a | 78 ± 7 | 72 ± 4 | 78 ± 6 | 61 ± 6 | 60 ± 5 | 61 ± 5 | 75 ± 5 | 71 ± 5 | 70 ± 4 |
| VEPEP-3b | 82 ± 5 | 75 ± 7 | 84 ± 6 | 67 ± 6 | 71 ± 7 | 70 ± 4 | 84 ± 6 | 79 ± 5 | 85 ± 7 |
| RNAiMAX | 44 ± 5 | 47 ± 6 | 51 ± 5 | 0 ± 0 | 0 ± 0 | 0 ± 0 | 5 ± 5 | 11 ± 5 | 7 ± 5 |

### Example 3: CPP-mediated functional delivery of RGEN/gRNA complex and gene editing via homology directed repair (HDR)

VEPEP-3 and ADGN-100 peptides were evaluated for the delivery of a ternary RGEN/gRNA/ssDNA complex for genome engineering using precise Homology Direct Repair (HDR). In the ternary complex, CAS9/gRNA targeting the *EMX1* gene was associated with an ssDNA donor allowing the insertion of 10 -12 nucleotide into a gene of interest by HDR in U20S and JUKAT cells.
ssDonor template: 5'-GGGGAGGACATCGATGTCACCTCCAATGAC **AAGCTTGCTAGC** GGTGGGCAACCACAAACCCACGAGGGCAGA-3' (SEQ ID NO: 82)

The ssDNA contains 2 homology arms of 30 nt surrounding the 12 nucleotide insert sequence (insert shown in bold in SEQ ID NO: 82). The 12 nucleotide insert contains specific HindIII and NheI restriction sites, which were used to quantify HDR efficiency.

Genome editing experiments at the *EMX1* locus were performed as follows: experiments were optimized for high levels of double strand break formation, using a concentration of 50 nM preloaded RGEN/gRNA associated with VEPEP-3b or ADGN-100a peptides at a 20:1 molar ratio of CPP to cargo. Such conditions led to more than 80% double-strand break efficiency on both JURKAT T and U20S cell lines as reported in FIG. 2C and FIG. 4C. 50 nM of both RGEN/gRNA and ssDNA-donor were associated with VEPEP-3b or ADGN-100a peptides at 20:1 molar ratio of CPP to cargo either by combining all component together to form a ternary complex (RGEN/gRNA/ssDNA/CPP) or by separately combining the CPP with the RGEN/gRNA complex in one step and combining the CPP with the ssDNA-donor in another step to form two separate complexes (RGEN/gRNA/CPP and ssDNA/CPP), as shown in Table 4. RNAiMAX was used as control.

Cells were grown to 50-60% confluence and overlaid with 200 µl of preformed complexes, incubated for 3-5 min, then 400 µl of DMEM was added. After 30 min incubation at 37 °C, 1 mL of fresh DMEM containing 16% FCS was added to reach a final FCS concentration of 10%, without removing the overlay of complexes. Cells were returned to the incubator for 72 hours. Efficiency of HDR insertion of HindIII and NheI restriction enzyme site was analyzed using Restriction Fragment Length Polymorphisms (RFLP) assay with NheI digestion of EMX1 PCR amplicons.

As shown in FIGS. 9A and 9B and summarized in Table 6, in both cell lines, high efficiency of HDR was obtained with ADGN-100a using either the single or double complex protocol. Higher HDR insertions up to 47% were obtained by combining the potency of VEPEP-3b to deliver CAS9/gRNA complex with that of ADGN-100a to deliver ssDNA. In some configurations with ADGN-100a and VEPEP-3b peptides, efficiency was increased 3-fold or more compared to RNAiMAX.

**TABLE 6: efficiency of HDR insertion as monitored by RFLP assay.**

| | **HDR (%)** | |
|---|---|---|
| **Condtions** | **U20S** | **JURKAT** |
| CAS9/gRNA/ssDNANEPEP-3b | 17 ± 4 | 11 ± 5 |
| CAS9/gRNA/ssDNA/ADGN-100a | 32 ± 5 | 34 ± 7 |
| CAS9/gRNA/VEPEP-3b + ssDNA/VEPEP-3b | 2 ± 2 | 4 ± 4 |
| CAS9/gRNA/ADGN-100a + ssDNA/ADGN-100a | 38 ± 6 | 44 ± 5 |
| CAS9/gRNA/VEPEP-3b + ssDNA/ADGN-100a | 44 ± 6 | 47 ± 9 |
| CAS9/gRNA/ RNAiMAX + ssDNA/RNAiMAX | 12 ± 6 | 17 ± 5 |

### Example 4: ADGN-100-mediated delivery of CAS9-expressing plasmid and gRNA targeting EGFP reporter gene

ADGN-100 was evaluated for cellular delivery of a plasmid encoding CAS9 and gRNA targeting a specific site in the exogenous *EGFP* gene reporter in U20S cells (EGFP-U2OS).

Stock solutions of amphipathic peptides were prepared at 1 mg/mL in distilled water and sonicated for 10 min. CAS9 expressing plasmid (Addgene MLM3639-Plasmid #42252) and gRNA (50 nM) were mixed with ADGN-100a at 10:1 and 20:1 molar ratios of ADGN-100a to each plasmid and incubated for 30 min at 37°C prior to cell transfection.

EGFP-U2OS cells containing a single integrated *EGFP* reporter gene were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS), at 37 °C in a humidified atmosphere containing 5% CO₂. A total of 150,000 cells seeded in a 35 mm dish the day prior to transfection were grown to 50-60% confluence and overlaid with 200 µl of preformed complexes, incubated for 3-5 min, then 400 µl of DMEM was added. After 30 min incubation at 37 °C, 1 mL of fresh DMEM containing 16% FCS was added to reach a final FCS concentration of 10%, without removing the overlay of CPP/CAS9-gRNA complexes. Cells were returned to the incubator for 72 hours.

CAS9-induced double-strand break formation was analyzed using flow cytometry by monitoring the level of EGFP expression. Gene disruption frequency data reported in FIG. 10 show an average of 3 separate experiments. Lipofectamine 2000 delivery was used as positive control.

As shown in FIG. 10, ADGN-100a promoted delivery of both CAS9-expressing plasmid and gRNA in human EGFP-U2OS cells. Optimal double strand break formation was obtained with ADGN-100a/CAS9 plasmid/gRNA complexes formed at molar ratio of 20:1 of ADGN-100a to each nucleic acid. A decrease in EGFP expression of 23% or 38% was obtained at molar ratios of 10:1 or 20:1, respectively. As a comparison, a decrease of 20% in EGFP expression was obtained using Lipofectamine 2000.

### Example 5: CPP-mediated CAR T cell engineering

The potency of ADGN-peptide technology for CAR-T cell engineering was evaluated. Three cell penetrating peptides (ADGN-100a, VEPEP-3a, and CADY) were evaluated for both functional cellular delivery of RGEN/gRNA complexes targeting the PD-1 receptor and delivery of a DNA plasmid encoding an anti-CD 19 CAR into two human cell lines (JURKAT and K562) and T cells isolated from peripheral blood mononuclear cell (PBMC) preparations from different donors. We demonstrated that both ADGN-100a and VEPEP-3a peptides can be efficiently used to reprogram primary human T cells by disruption of PD-1. One negative regulator of T-cell activity is the ligand PD-L1, which functions through binding of the programmed death-1 (PD-1) receptor on activated T cells. Moreover, ADGN-100a peptide alone or combined with VEPEP-3a was found to be a potent transfection vector for efficient delivery of RGEN/gRNA complex and CAR-encoding plasmid into T cells isolated from PBMCs to produce stable anti-CD19 CAR T cells with efficient down regulation of the hPD1 receptor expression.

### Protocol for CAS9/gRNAlpeptide particle formation

Stock solutions of ADGN-100a and VEPEP-3a peptides were prepared at 2 mg/mL in distilled water and sonicated for 10 min. CAS9 protein was preloaded with purified sgRNA at a molar ratio of 1:1 and incubated for 10 min prior to forming complexes with peptides. Preloaded CAS9/gRNA (2.5 µg and 5 µg) was mixed with ADGN-100a or VEPEP-3a at 20:1 molar ratio (peptide to CAS9/gRNA) and incubated for 30 min at 37°C prior to cell transfection.

### Protocol for plasmid DNA/peptide particle formation

Plasmid encoding a CD19-specific chimeric antigen receptor (CAR) under the control of a CMV promoter consisted of a CD19-specific scFv linked to CD28 and CD3ζ signaling moieties. Plasmid encoding luciferase (6.2 Kb) was obtained from New England BioLabs and recloned. Stock solutions of plasmid were prepared at 100 µM concentrations in 50 mM Tris, 0.5 mM EDTA. Nanoparticles were prepared by mixing ADGN-100a or CADY peptide (diluted to 400 µM) and plasmid (100 µM stock solution) for 30 min at 37°C with a final molar ratio of peptide to plasmid of 20:1, using 1 ng or 5 ng plasmid DNA. Complex solutions were then centrifuged for 5 min at 9000 rpm to remove any precipitation and diluted in 50% PBS solution just prior to transfection.

### Cell lines

K562 and Jurkat T cell lines were cultured in 75 cm² flasks and 6 well dishes in Dulbecco's Modified Eagle's Medium (DMEM) containing fetal bovine serum (FBS) at a final concentration of 10%. Cells were passaged every 4 days and freshly passaged 24hr prior to transfection.

### T cell isolation and activation

T cells were isolated from PBMCs (2 different donors) using anti-CD3/anti-CD28 antibodies bound to paramagnetic beads (Dynabeads ClinExVivo CD3/CD28, Invitrogen,) at a ratio of 3:1 (beads:cells). The cells and beads were co-incubated for 2 hours at room temperature and CD3⁺ cell enrichment was performed using Dynal ClinExVIVO MPC magnet (Thermo Fisher). The anti-CD3/anti-CD28 paramagnetic beads were removed, washed and concentrated. Cells in the CD3⁺ fraction were resuspended in activation media at a concentration of 1×10⁶ cells/ml with AIM-V medium (Thermo Fishers) supplemented with 10% fetal calf serum, 300 IU/ml IL2 and 50 ng/ml OKT3 (MuromonAB-CD3) for two days.

### Electroporation protocol

Cells were transfected by electroporation with CD-19 plasmid or gRNA/CAS9 (5µg/10µg) using a Nucleofector 2b (Lonza, Germany) with the Amaxa Human T cells Nucleofector Kit, VPA-1002 (Lonza, Germany). 5 × 10⁶ cells were washed twice with DPBS by centrifuging at 800 rpm for 5 minutes and resuspended in 100 µl transfection buffer, and then transferred into the electroporation cuvette. An optimized program selected for both high transfection and high efficiency on T cells was used. After electroporation, cells were resuspended in 500 µl pre-warmed AIM-V medium containing 10% FBS and incubated at 37 °C in 5% CO₂. The transfection efficiency was evaluated after 48 hr by flow cytometry or T7E1 assay.

### CPP-mediated delivery of gRNA/CAS9 complex in T-cell lines

VEPEP-3a, ADGN-100a and CADY peptides were evaluated for cellular delivery of RGEN/gRNA complexes targeting endogenous *hPD1* gene in two human T cell lines (JURKAT and K562).

Cell lines (JURKAT, K562) were cultured in DMEM supplemented with 2 mM glutamine, 1% antibiotics (streptomycin 10,000 µg/mL, penicillin, 10,000 IU/ mL) and 10% (w/v) foetal calf serum (FCS), at 37°C in a humidified atmosphere containing 5% CO₂. A total of 150,000 cells seeded in a 35 mm dish the day prior to transfection were grown to 50-60% confluence. CAS9 preloaded with two different gRNAs targeting Exon 2 of hPD1 (5 µg/10µg CAS9/gRNA and 2.5 µg/5µg CAS9/gRNA) were mixed with VEPEP-3a, ADGN-100a or CADY peptides at 20:1 molar ratio (peptide to CAS9/gRNA) and incubated for 30 min at 37°C prior to cell transfection. RNAiMAX delivery method was used as positive control. Cells were either resuspended in or overlaid with 100 µl of preformed complexes, incubated for 3-5 min, and then 300 µl of DMEM was added. After 30 min incubation at 37°C, 1 mL of fresh DMEM containing 16% FCS was added in order to reach a final FCS concentration of 10%, without removing the overlay of CPP/CAS9-gRNA complexes, and cells were returned to the incubator. CAS9-induced double-strand break followed by repair through NHEJ was analyzed by either the T7E1 assay or using the Genome Cleavage Detection kit (GCD Thermo Fisher) and quantified using Fragment Analyzer System. The indel frequency in the *hPD1* gene was quantified using the T7E1 assay.

As reported in Table 7, both VEPEP-3a and ADGN-100a-mediated RGEN/gRNA delivery induced a robust editing of the *hPD1* gene in the two cell lines. In both cell lines, RGEN/gRNA induced NHEJ-mediated indel mutation at the intended on-target site. Using 5 µg/10 µg CAS9/gRNA, mean indel frequencies of 71% and 72% were obtained when delivered with ADGN-100a in K562 and JURKAT cells, respectively. Mean indel frequencies of 75% and 78% were obtained when using VEPEP-3a in K562 and JURKAT cells, respectively. Mean indel frequencies of 31% and 35 % were obtained using RNAiMAX in K562 and JURKAT cells, respectively. No gene editing in the *hPD1* gene was observed using 5 µg/10 µg CAS9/gRNA negative control targeting GFP. In comparison, using electroporation-mediated delivery of 5 µg/10 µg CAS9/gRNA, mean indel frequencies of 65% and 72% were obtained in K562 and JURKAT cells, respectively. In cultured T cells, ADGN-100a and VEPEP-3a were at least 2-fold more efficient for PD1 knockdown as compared to RNAiMAX, and up to 50% more efficient than electroporation at lower amounts of CAS9/sgRNA. Data reported for on-target mutations induced by RGEN:gRNA are an average of 3 independent experiments.

**Table 7**

| | **CAS9/sgRNA (2.5µg/5µg)** | | **CAS9/sgRNA (5µg/10µg)** | |
|---|---|---|---|---|
| | **Mean indel Frequency (%) ± s.e.m** | | | |
| | **K562** | **JURKAT** | **K562** | **JURKAT** |
| ADGN-100a | 68±2 | 70±5 | 71±5 | 72±2 |
| VEPEP-3a | 71±6 | 72±5 | 75±6 | 78±7 |
| CADY | - | - | 15±9 | 12±9 |
| RNAiMAX | 23±4 | 20±9 | 31±8 | 35±5 |
| Electroporation | 55±5 | 44±6 | 65±7 | 72±6 |

### CPP-mediated delivery of plasmid DNA in T cell lines

Cell lines (Jurkat, K562) were freshly passed 24 hr prior to transfection. Culture medium was removed and cells were washed twice with PBS. Cells were either resuspended or incubated for 5 min with 100 µl of plasmid/peptide solutions, then 0.3 mL of serum free medium was added. After 30 min, 1.2 mL of full medium was added and serum level was adjusted to 10%. Cultures were incubated at 37°C for 48 hr, and then YFP was monitored by FACS analysis and anti-CD 19-CAR T expression was evaluated by flow cytometry using a Protein L assay.

### ADGN-100 mediated delivery of plasmid encoding and-CD19 CAR in human T cells

Activated T cells (1 x 10⁶) isolated from two donors were transfected with ADGN-100a- or CADY-particles (molar ratio 20:1 of peptide to plasmid) containing anti-CD19 CAR-expressing plasmid at 2 concentrations (2 µg and 5 µg) for 48 hours. All conditions tested are listed in Table 8, and each experiment was performed in triplicate. The plasmid (8.2 Kb) encoded a CD19-specific chimeric antigen receptor (CAR) under control of the CMV promoter, and consisted of a CD19-specific scFv linked to CD28 and CD3ζ signaling moieties. As control, similar experiment were performed using plasmid encoding luciferase (6.2 Kb). T cells were resuspended in 100 µl of plasmid/ADGN-100a- or plasmid/CADY-particles and incubated for 5 min, then 300 µl of serum free RPMI-1640 medium was added. After 30 min, 1.2 mL of full medium was added and serum level was adjusted to 10%.

On day 5, the level of transduced and untransduced cells was analyzed, and cells were transferred to fresh T cell expansion medium T cell expansion medium contained AIM V medium, supplemented with 5% heat-inactivated human AB Serum, 1% Gluta-Max, and 300 IU/mL IL-2. The cultures were expanded until day 12 and analyzed for viability and anti-CD19 CAR expression by flow cytometry. The expression of the anti-CD 19 CAR in the T cells was evaluated by flow cytometry using a Protein L assay. Biotinylated Protein L was purchased from ThermoScientific, reconstituted in sterile water at a stock concentration of 50ng/µl, and stored at 4°C. 7-AAD (Sigma) was used to determine viability.

As shown in FIG. 11, ADGN-100a promoted anti-CD19 CAR plasmid transfection in isolated T cells from PBMCs with efficiencies of 62% and 55% at 20:1 molar ratio (peptide to plasmid), using 2µg and 5µg plasmid, respectively. Stable expression was maintained over 6 days with a final transduction efficiency of 58% and 42% using 2µg and 5 µg plasmid, respectively. No cytotoxicity associated with ADGN-100a or transduction was observed as indicated by the average viability of approximately 80% or higher obtained in all conditions (*see* **FIG. 12****).** The results demonstrate that ADGN-100 transfection technology with a non-viral vector can be effectively used to engineer tumor-targeting T-cells, such as for use in adoptive cell therapy.

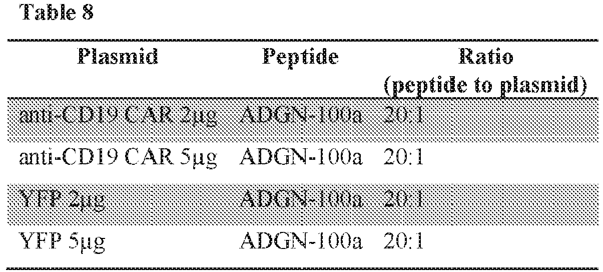

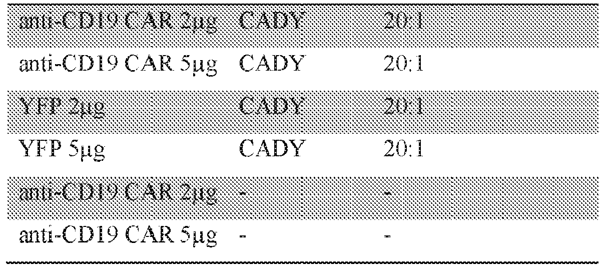

### ADGN-10Oa/VEPEP-3a-mediated functional delivery of RGENlgRNA complex targeting hPD1 in human T cells

Activated T cells (1 x 10⁶) isolated from two different donors were transfected with ADGN-100a, VEPEP-3a, or CADY particles using gRNAhPD1/CAS9 complexes at 2 concentrations (5 µg/10µg CAS9/gRNA and 2.5 µg/5µg CAS9/gRNA) for 48 hours. All of the conditions tested are listed in Table 9, and each experiment was performed in triplicate. T cells were resuspended in 100 µl of ADGN-100a, VEPEP-3a, or CADY particles containing gRNA/CAS9 complexes and incubated for 5 min, then 1 ml of full medium was added and serum level was adjusted to 10%. As control, CAS9 loaded with gRNA targeting GFP was used.

Cells were returned to the incubator for 72 hours. CAS9-induced double-strand break followed by repair through non-homologous end joining (NHEJ) was analyzed by either the T7E1 assay or using the Genome Cleavage Detection kit (GCD Thermo Fisher) and quantified using Fragment Analyzer System The indel frequency in the target gene was quantified using the T7E1 assay. The cultures were expanded and analyzed for viability and PD1 levels every 10 days. T cell expansion medium contained AIM V medium, supplemented with 5% heat-inactivated human AB Serum, 1% Gluta-Max, and 300 IU/mL IL-2.

As reported in Table 10, both VEPEP-3a- and ADGN-100a-mediated RGEN/gRNA delivery induced a robust editing of the *hPD1* gene in T Cells from the two donors. Using 5µg/10µg CAS9/gRNA, mean indel frequencies of 58% and 66% were obtained when delivered with ADGN-100a and VEPEP-3a, respectively. No editing of the *hPD1* gene in T cells was obtained using either CADY peptide or RNAiMAX. In comparison, using electroporation-mediated delivery with 5µg/10µg CAS9/gRNA, a mean indel frequency of 25% was obtained. These results suggest that ADGN-100a and VEPEP-3a constitute a highly potent strategy for gRNA/CAS9 complex delivery in T cells. ADGN-100a and VEPEP-3a were at least 2-fold more efficient for PD1 knockdown as compared to electroporation. Data reported for on-target mutations induced by RGEN:gRNA are an average of 3 independent experiments.

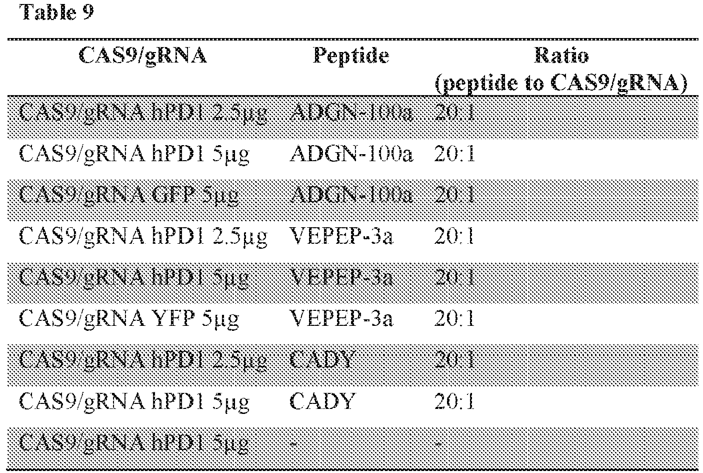

**Table 10**

| | **CAS9/sgRNA (2.5µg/5µg)** | | **CAS9/sgRNA (5µg/10µg)** | |
|---|---|---|---|---|
| | **Mean indel Frequency (%) ± s.e.m** | | | |
| | **Donor 1** | **Donor 2** | **Donor 1** | **Donor 2** |
| ADGN-100a | 48±6 | 52±5 | 56±4 | 61±8 |
| VEPEP-3a | 62±5 | 67±8 | 65±5 | 70±5 |
| CADY | 0 | 0 | 0 | 0 |
| RNAiMAX | 0 | 0 | 0 | 0 |
| Electroporation | 15±4 | 20±4 | 23±5 | 26±8 |

### ADGN-100/VEPEP-3-mediated simultaneous delivery of RGEN/gRNA complex targeting hPD1 and anti-CD19 CAR-expressing plasmld in human T cells

Activated T cells (1 x 10⁶) from two donors were simultaneously transfected with ADGN-100a or VEPEP-3a particles containing an anti-CD19 CAR-expressing plasmid (5 µg) and ADGN-100a, VEPEP-3a, or CADY particles containing gRNA hPD1/CAS9 complexes at 2 concentrations (5 µg/10µg CAS9/gRNA and 2.5 µg/5µg CAS9/gRNA) for 48 hours. All of the conditions tested are listed in Table 11, and each experiment was performed in triplicate. T-Cells were resuspended in 100 µl of serum free media containing ADGN-100a, VEPEP-3a, or CADY particles containing gRNAhPD1/CAS9 complexes and ADGN-100a or VEPEP-3a particles containing the anti-CD19 CAR-expressing plasmid. Cells were incubated for 5 min, then 300 µl of serum free RPMI-1640 medium was added. After 30 min, 1.2 mL of full medium was added and serum level was adjusted to 10%. As control, CAS9 loaded with gRNA targeting GFP was used. For comparison with electroporation methods, cells were transfected with the anti-CD-19 CAR plasmid (5µg) and gRNA/CAS9 (5µg/10µg) using Nucleofector 2b (Lonza, Germany) with the Amaxa Human T cells Nucleofector Kit, VPA-1002 (Lonza, Germany).

On day 5, the level of transduced and untransduced cells was analyzed, and cells were transferred to fresh T cell expansion medium The cultures were analyzed for viability and anti-CD19 CAR expression by flow cytometry. The expression of the anti-CD19 CAR in the T cells was evaluated by flow cytometry using a Protein L assay. Biotinylated Protein L was purchased from ThermoScientific, reconstituted in sterile water at a stock concentration of 50ng/µl, and stored at 4°C. 7-AAD (Sigma) was used to determine viability. CAS9-induced double-strand break followed by repair through NHEJ system was analyzed by either the T7E1 assay or using the Genome Cleavage Detection kit (GCD Thermo Fisher) and quantified using Fragment Analyzer System. The indel frequency in the target gene was quantified using the T7E1 assay. The cultures were expanded and analyzed for viability, anti-CD 19 expression and PD1 level every 10 days. T cell expansion medium contained AIM V medium, supplemented with 5% heat-inactivated human AB Serum, 1% Gluta-Max, and 300 IU/mL IL-2.

As shown in **FIG. 13****,** ADGN-100a promotes anti-CD 19 CAR plasmid transfection in isolated T cells from PBMC with efficiencies between 40% and 45% at 20/1 molar ratio (peptide to plasmid), using 5 µg plasmid. Stable expression was maintained at least over 6 days. In comparison, only 20% of T cells expressed the anti-CD 19 CAR when transfected by electroporation, showing that ADGN-100a gene delivery efficiency was at least 2-fold higher than electroporation in primary T cells.

As reported in Table 12, both VEPEP-3a and ADGN-100a-mediated RGEN/gRNA delivery induced a robust editing of the *hPD1* gene in T cells from the two donors. Using 5µg/10µg CAS9/gRNA, mean indel frequencies of approximately 50% and 57% were obtained when delivered with ADGN-100a and VEPEP-3a, respectively. In comparison, using electroporation-mediated delivery of 5µg/10µg CAS9/gRNA, a mean indel frequency of 15% was obtained. ADGN-100a and VEPEP-3a were at least 3-fold more efficient for PD1 knockdown as compared to electroporation. Using 2.5µg/5µg CAS9/gRNA, mean indel frequencies of approximately 42% and 50% were obtained when delivered with ADGN-100a and VEPEP-3a, respectively. At that CAS9/gRNA concentration, no significant PD1 knockdown was obtained by electroporation.

VEPEP-3a appeared to be more efficient than ADGN-100a for CAS9/gRNA induced PD1 knockdown. Data reported for on-target mutations induced by RGEN/gRNA are an average of 3 independent experiments.

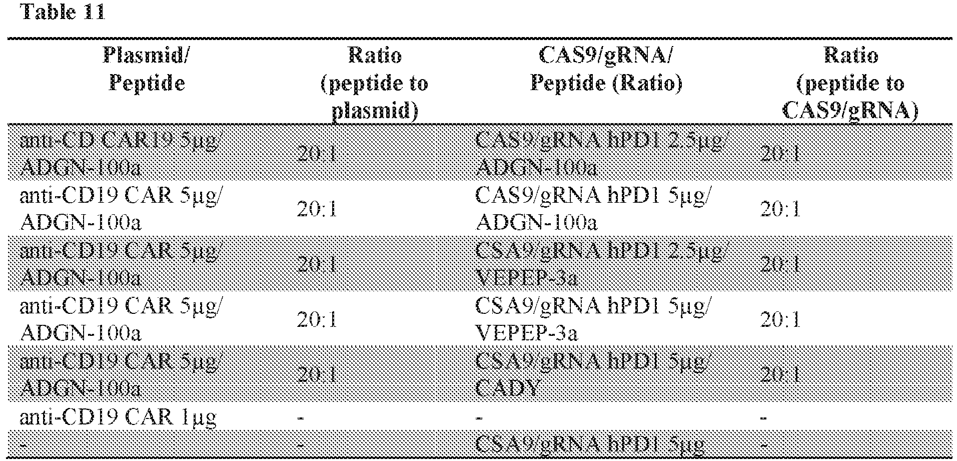

**Table 12**

| | **CAS9/sgRNA (2.5µg/5µg)** | | **CAS9/sgRNA (5µg/10µg)** | |
|---|---|---|---|---|
| | **Mean indel Frequency (%) ± s.e.m** | | | |
| | **Donor 1** | **Donor 2** | **Donor 1** | **Donor** 2 |
| ADGN-100a | 44±5 | 42±3 | 50±3 | 51±8 |
| VEPEP-3a | 51±5 | 53±5 | 57±2 | 58±8 |
| Electroporation | 5±5 | 5±5 | 16±2 | 15±6 |

### CPP mediated delivery of CAS9/sgRNA complex does not induce toxicity in T Cells

The robust hPD1 disruption observed in the two cell lines as well as in primary T cells isolated from PBMCs was not associated with cellular toxicity. The toxicity of CPP/ CAS9/gRNA complexes was investigated in the two cell lines (JURKAT and K562) and in PBMC-isolated T cells. Cell viability was determined using either MTT assay or 7-AAD.

A total of 30,000 JURKAT or K562 cells seeded in 24-well plates the day prior to transfection were incubated with increasing concentrations of CPP/CAS9/gRNA complexed at 20:1 molar ratio (CPP to CAS9/gRNA) ranging from 1 nM to 200 nM for 30 min prior to addition of medium to reach a final 10% concentration of FCS. Cytotoxic response was measured 24 hours later by colorimetric MTT assay (Sigma, Germany). For MTT assay, cell culture medium was removed and replaced with PBS containing 2.5 mg/ml of MTT for 4 hours. Results correspond to the average of 3 independent experiments. As shown in **FIG. 14****,** no toxicity for either VEPEP-3a or ADGN-100a peptides was observed up to a concentration of 100 nM for the two cell lines, and viability was reduced by less than 10% at 200 nM. In contrast, at 200 mM, between 40-45 % cell death was observed with RNAiMAX, depending on the cell line.

Primary T cells from the two different donors were transfected with ADGN-100a or VEPEP-3a particles containing CAS9/gRNA at 2 concentrations (5 µg/10µg CAS9/gRNA and 2.5 µg/5µg CAS9/gRNA) for 48 hours, then cell viability was quantified over a period of 12 days by flow cytometry using 7-AAD. Data were normalized to untransfected cells and compared to T cells incubated with free CAS9/gRNA. As shown in **FIG. 15****,** no cytotoxicity was observed with either ADGN-100a or VEPEP-3a, as indicated by the average viability of approximately 80% or higher in all conditions.

### Comparison of electroporation with peptide-based delivery of CAS9/gRNA in T cells

The knockdown of beta 2 microglobulin (β2M) using CAS9 with a gRNA containing a guide sequence targeting the gene encoding β2M in primary T cells is tested using electroporation-mediated delivery and compared to delivery mediated by complexation of the CAS9/gRNA with peptides VEPEP3 or ADGN100. T cells from 2 donors are used, for example, with CAS9/gRNA at a 5µg/10µg ratio, respectively, for electroporation and ratios of 5µg/10µg and 2.5µg/5µg, respectively, for the peptides. In each case, 5 x 10⁶ T cells, for example, are treated. Transfections with the peptides are performed, for example, in 5% serum or in serum free medium. Cell viability at 24 hours, knockdown efficiency at 3 days, and the cell number at the end of the expansion phase are assessed.

### Comparison of electroporation with peptide-based delivery of plasmid DNA in T cells

The expression of GFP from the plasmid pTRPE GFP (10 Kb) in primary T cells is tested using electroporation-mediated delivery of the plasmid and compared to delivery mediated by complexation of the plasmid with peptides ADGN100 or VEPEP9. T cells from 2 donors are used, for example, with 3 µg plasmid for electroporation and 2µg and 5µg plasmid for the peptides. In each case, 5 x 10⁶ T cells, for example, are treated. Transfections with the peptides are performed, for example, in 5% serum or in serum free medium. Cell viability at 24 hours, knockdown efficiency at 3 days, and the cell number at the end of the expansion phase are assessed.

### Example 6: ADGN-100-mediated functional delivery of RGEN/gRNA complex in primary human cardiac fibroblasts and primary human hepatocytes

ADGN-100 peptides were also evaluated for cellular delivery of multiple RGEN:gRNA complexes targeting endogenous *EMX1* and *HPRT* genes in primary human cardiac fibroblasts and primary human hepatocytes. The CAS9/gRNA complexes contained either CAS9 protein or mRNA encoding the CAS9 protein and a gRNA targeting either *EMX1* or *HPRT.* Human primary cardiac fibroblasts and human primary hepatocytes were freshly obtained and grown in flasks pre-coated with a gelatin-based coating solution and incubated in Culture Complete Growth Medium for 3 days before expansion and treatment. Cells were then treated with different concentrations of either ADGN-100a:CAS9 protein:gRNA complex or ADGN-100a: CAS9 mRNA:gRNA complex. Non-Homologous End Joining (NHEJ) was analyzed 72 hours after transfection by the T7E1 assay and compared to delivery using RNAiMAX lipid methods. The toxicity was analyzed for all conditions using the MTT assay.

CAS9 mRNA was obtained using HiScribe™ T7 ARCA mRNA Kit (New England Biolab). mRNA was synthesized using linearized plasmid DNA and purified by phenol:chloroform extraction. Synthesized mRNA was purified by LiCl precipitation, phenol: chloroform extraction followed by ethanol precipitation, then quantified by UV Light Absorbance. RNA concentration was determined by measuring the ultraviolet light absorbance at 260nm. 18 µg of capped mRNA was obtained using 1 µg of DNA template and stored at - 20°C. S. pyogenes HIS-NLS-CAS9 was either expressed in *E. coli* and purified as previously described by *Zuris et al, 2015,* using the HIS-CAS9-NLS pET29 expression vector (Addgene #62933), or obtained from Thermo Fisher Life Science. Lipofectamine 2000, RNAiMAX, TranscriptAid T7 transcription and MEGAclear transcription Clean Up kits, GeneArt Genomic Cleavage Detection kit, and Platinium Green Hot Start PCR mix were obtained from Thermo Fisher life Science. All oligonucleotides were obtained from Eurogentec.

Stock solutions of ADGN-100a peptide were prepared at 1 mg/mL in distilled water and sonicated for 10 min. CAS9 protein was preloaded with purified gRNA-GFP at a molar ratio of 1:1 and incubated for 10 min prior to forming complexes with ADGN peptides. Preloaded CAS9/gRNA (10 nM, 20 nM, 50 nM) was mixed with ADGN-100a at a 20:1 molar ratio (peptide to complex) and incubated for 30 min at 37°C prior to cell transfection.

The following protocol was used for both cell types. Cells were cultured to about 70% confluence at the time of transfection. Cells were maintained in high glucose DMEM media with 10% serum. Before transfection, cells were washed with DMEM twice and resuspended in 0.2 ml of complex solution (16 µl complex + 184 µl OPTiMEM) mixed gently and incubated for 10 min at 37°C. 0.8 mL of fresh OPTiMEM (high glucose) was added and cells were incubated for 20 min at 37°C. 2 mL of complete OPTiMEM (high glucose) containing 11.4% FCS was added in order to reach a final FCS concentration of 10%, without removing the overlay of ADGN-100a/CAS9/gRNA complexes. Cells were returned to the incubator at 37°C and assayed at 72 hours post transfection.

As a positive control, complexes were delivered using RNAiMAX (Invitrogen Thermo Fisher). CAS9-induced double-strand break formation followed by repair throughout the non-homologous end joining (NHEJ) system was analyzed at 72 hours by the T7E1 assay and quantified using a Fragment Analyzer System. The indel frequencies reported for the *EMX1* and *HPRT* genes are an average of 3 separate experiments. Cytotoxicity was analyzed using the MTT assay.

The toxicity of ADGN-100a/CAS9/gRNA complexes was investigated for the two primary cell types. A total of 30,000 cells seeded in 35-well plates the day prior to transfection were incubated with increasing concentrations of ADGN-100a/CAS9/gRNA complex (10 nM, 20 nM, and 50 nM) at 20:1 molar ratio (peptide to complex) for 30 min prior to addition of medium to reach a final 10% concentration of FCS. Cytotoxic response was measured 24 hours later by colorimetric MTT assay (Sigma, Germany). For MTT assay, cell culture medium was removed and replaced with PBS containing 2.5 mg/ml of MTT for 4 hours.

Data are reported in FIG. 16A for primary fibroblasts and FIG. 16B for primary hepatocytes, and in Table 13. As reported, ADGN-100 mediated RGEN/gRNA delivery induces a robust editing of both *EMX-1* and *HPRT* genes in the two primary cell types. Using a 20 nM concentration of CAS9-gRNA; mean indel frequencies of more than 60% and 55% for both genes were obtained in primary fibroblasts and primary hepatocytes, respectively. Mean indel frequencies of about 30% in primary human fibroblasts and of 20% in primary human hepatocytes cells were obtained with RNAiMAX using 50 nM RGEN/gRNA. These results demonstrated than ADGN-100 peptide is at least 3-4 folds more potent than RNAiMAX.

**Table 13: Comparison of ADGN-100a to RNAiMAX for RGEN:gRNA delivery and resulting editing efficiencies as measured by T7E1 assay in primary human fibroblasts and primary human hepatocytes**

| **Delivery Method** | **Mean indel Frequency (%) ± S.E.M** | | | | | |
|---|---|---|---|---|---|---|
| | **CAS9/sgRNA (10 nM)** | | **CAS9/sgRNA (20 nM)** | | **CAS9/sgRNA (50 nM)** | |
| | **Fibroblasts** | **Hepatocytes** | **Fibroblasts** | **Hepatocytes** | **Fibroblasts** | **Hepatocytes** |
| **ADGN-100a** | | | | | | |
| *EMX1* | 61 ± 5 | 55 ± 6 | 65 ± 4 | 58 ± 5 | 67 ± 5 | 61 ± 5 |
| *HPR* | 62 ± 5 | 54 ± 6 | 68 ± 4 | 56 ± 5 | 70 ± 5 | 57 ± 5 |

| **RNAiMAX** | | | | | | |
|---|---|---|---|---|---|---|
| *EMX1* | 16 ± 4 | 15 ± 6 | 22 ± 5 | 21 ± 4 | 28 ± 7 | 23 ± 6 |
| *HPRT* | 18 ± 7 | 16 ± 6 | 22 ± 5 | 19 ± 5 | 25 ± 6 | 21 ± 8 |

The robust *EMX1* and *HPRT* gene disruption observed in the two primary cell types was not associated with cellular toxicity. As shown in FIGS. 17A and 17B, less than 10% toxicity was observed using ADGN-100a peptide for both primary cell types. By contrast, with RNAiMAX between 60-65 % cell death in primary human hepatocytes and between 35-40% cell death in primary human fibroblasts was observed. These results demonstrate that ADGN-100a is a potent, non-toxic delivery technology useful for genome-editing, such as by CRISPR, in primary human cells.

### Example 7: ADGN-100-mediated in vitro delivery of CAS9-expressing mRNA and gRNA in cultured U20S cells

In this experiment, ADGN-1 00a peptide was evaluated for cellular delivery of CAS9 mRNA and CAS9 mRNA/gRNA in U20S cells. CAS9 mRNA was obtained using HiScribe™ T7 ARCA mRNA Kit (New England Biolab using linear pCS2-CAS9 vector as DNA template (Addgene # 47322).

CAS9 mRNA (0.2 µg and 0.5 µg) was mixed with ADGN-100a at a 20:1 molar ratio (peptide to mRNA) and incubated for 30 min at 37°C prior to cell transfection. U20S cells were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS), at 37°C in a humidified atmosphere containing 5% CO₂. A total of 150,000 cells seeded in a 35 mm dish the day prior to transfection were grown to 50-60% confluence and overlaid with 200 µl of preformed ADGN-100a/CAS9 mRNA complexes, incubated for 5 min, then 400 µl of DMEM was added. After 20 min incubation at 37°C, 1 mL of fresh DMEM containing 16% FCS was added in order to reach a final FCS concentration of 10%, without removing the overlay of complexes. Cells were returned to the incubator for 72 hours. CAS9 expression level was quantified by western blot on protein extract using antibody against CRISPR/Cas9 (Diagenode cat. No. C15200223).

As shown in FIG. 18, western blot analysis revealed dose-dependent expression of CAS9 protein in transfected U2OS cells. These results suggest that ADGN-100a promotes functional delivery of CAS9 mRNA followed by CAS9 protein expression. As a comparison, the level of CAS9 expression was about 10-fold lower using Lipofectamine 2000 or RNAiMAX as the mRNA delivery vector.

### Example 8: In vitro gene-editing of eGFP-U2OS cells using ADGN-100-complexed with CAS9 protein or CAS9-expressing mRNA and gRNA

Gene editing using either CAS9 protein or CAS9 mRNA using ADGN-100a was evaluated by targeting a specific site in the exogenous eGFP gene reporter in the eGFP-U2OS cell line. Premixed CAS9/gRNA (2.5 µM/5 µM) or CAS9 mRNA/gRNA (0.5 µg/5 µg) were mixed with ADGN-100a at a 20:1 molar ratio (peptide to complex) and incubated for 30 min at 37°C prior to cell transfection. Both lipofectamine 2000 and RNAiMAX delivery methods were included as controls. eGFP-U₂OS cells were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS), at 37°C in a humidified atmosphere containing 5% CO₂. A total of 150,000 cells seeded in a 35 mm dish the day prior to transfection were grown to 50-60% confluence and overlaid with 200 µl of preformed complexes, incubated for 5 min, then 400 µl of DMEM was added. After 20 min incubation at 37°C, 1 mL of fresh DMEM containing 16% FCS was added in order to reach a final FCS concentration of 10%, without removing the overlay of complexes. Cells were returned to the incubator for 72 hours.

CAS9-induced double-strand break formation was analyzed using flow cytometry, by monitoring the level of eGFP expression after 72 hours, as shown in FIG. 19. A decrease in eGFP expression of 87% or 85 % was obtained with CAS9/gRNA or CAS9 mRNA/gRNA associated with ADGN-100a (molar ratio of 20:1, peptide to complex), respectively. These results demonstrate that ADGN-100a delivers both functional CAS9 protein and CAS9 mRNA leading to similar gene editing efficiencies. As a comparison, a decrease of 22% or 31% in eGFP expression was obtained using Lipofectamine 2000 or RNAiMAX as delivery vector for CAS9 mRNA/gRNA, respectively. A decrease of 35% or 45% in eGFP expression was obtained using Lipofectamine 2000 or RNAiMAX as delivery vector for CAS9 protein/gRNA, respectively.

### Example 9: ADGN-100-mediated in vivo delivery of CAS9-expressing mRNA in mice

ADGN-100a peptide was evaluated for *in vivo* delivery of CAS9 mRNA in Swiss mice. CAS9 mRNA (10 µg) was associated with ADGN-100a peptide at a 20:1 ratio of peptide to mRNA. Mice received a single intravenous injection of free ADGN-100a peptide (2 animals/group), free CAS9 mRNA (2 animals/group), or CAS9 mRNA/ADGN-100a (3 animals per group). At 72 hours following injection, animals were sacrificed and tissues including lung, liver, brain, kidney, heart, spleen, blood, and pancreas were collected. Tissues were homogenized and CAS9 expression was analyzed by western blot on protein extract using antibody against CRISPR/Cas9 (Diagenode cat. No. C15200223) or by ELISA using Cas9 monoclonal antibody (SBI clone 7A9). As shown in FIGS. 20A and 20B, high expression of CAS9 protein was observed in the liver, at a level greater than 15-fold that of background. CAS9 expression was also observed in lung and kidney, with levels about 2- to 3-fold that of background. No significant increase in CAS9 protein expression was detected in the other tissues examined.

### Example 10: ADGN-100-mediated in vivo delivery of CAS9-expressing mRNA and gRNA targeting luciferase in mice expressing luciferase in the liver

ADGN-100a peptide was evaluated for *in vivo* delivery of CAS9 mRNA/gRNA in Swiss mice expressing luciferase in the liver. CAS9 mRNA and gRNA targeting luciferase (10 µg) were associated with ADGN-100a peptide at molar ratio of 20:1 (peptide to nucleic acid). Mice received a single intravenous injection of free ADGN peptide (3 animals/group), free CAS9 mRNA (3 animals/group) or CAS9 mRNA/gRNA/ADGN-100a (3 animals per group). Luciferase level was quantified by whole animal fluorescence imaging just after injection and at 72 hours after injection, as shown in FIG. 21.

At 72 hours post injection, animals were sacrificed and the following tissues were collected: liver, lung, kidney, spleen, and blood. Luciferase was quantified by fluorescence in the liver. Tissues were homogenized and CAS9 expression was analyzed by ELISA using Cas9 monoclonal antibody (SBI clone 7A9).

As shown in FIGS. 21 and 22, ADGN-100a-mediated delivery of CAS9 mRNA/gRNA induced between 60% to 65% knockdown of luciferase expression in the liver. By contrast, no change in luciferase expression was observed with either free ADGN-100a or free CAS9 mRNA/gRNA. In agreement with the previous results for CAS9 mRNA delivery, as shown in FIG. 23, ADGN-100a mediated CAS9 mRNA/gRNA delivery mainly in the liver (at about 20-fold background level) and at lower levels in the lung and kidney (at about 2-fold background level). These results demonstrate that ADGN-100a peptide constitutes a potent method for *in vivo* functional delivery of CRISPR components, including CAS9 mRNA/gRNA.

### Example 11: ADGN-100a- and VEPEP-3a-mediated functional delivery of CAS9/gRNA complex targeting β2-microglobufin in human primary T cells: assessment of gene-editing efficiency and toxicity

T cells (5 x 10⁶) isolated from a single donor were transfected using ADGN-100a or VEPEP-3a associated with CAS9/gRNA complexes (5 µg/10µg CAS9/gRNA) targeting β2-microglobulin. Briefly, T cells were resuspended in 100 µl of a solution containing CPP/CAS9/gRNA particles and incubated for 5 min, then 1 ml of full medium was added and serum level was adjusted to 10%. As controls, 5 x 10⁶ cells were transfected with CAS9/gRNA complexes by electroporation, treated with free CAS9/gRNA, or left untreated. Cells were returned to the incubator for 72 hours. The level of β2-microglobulin was then quantified by FACS analysis.

As shown in FIG. 24, both VEPEP-3a- and ADGN-100a-mediated RGEN/gRNA delivery induced a robust editing of the β2-microglobulin gene in T cells, with 71 % and 78% efficiency, respectively. By contrast, electroporation-mediated delivery with 5µg/10µg CAS9/gRNA resulted in an efficiency of 32%. No editing of the β2-microglobulin gene in T cells was observed using free complex. These results suggest that ADGN-100a and VEPEP-3a can mediate highly potent CAS9/gRNA complex delivery in T cells. ADGN-100a and VEPEP-3a were at least 2-fold more efficient for β2-microglobulin knockdown as compared to electroporation. Data reported for on-target mutations induced by RGEN:gRNA are an average of 3 independent experiments.

The toxicity of CPP/CAS9/gRNA complexes was investigated in PBMC-isolated T cells using MTT assay after 48 hours and by monitoring activation damage-induced cell death (DICD) after 72 hours. The robust β2-microglobulin knockdown observed in primary T cells was not associated with cellular toxicity, as shown in FIGS. 25A and 25B. No toxicity associated with CPP/CAS9/gRNA complexes or free peptides was observed by MTT assay. Moreover, no delay in T cell activation or activation damage-induced cell death was observed with CPP/CAS9/gRNA complexes or CPP peptides. In contrast, 55% toxicity was observed after electroporation along with more than 70% reduction in T cell activation and DICD activation.

### Example 1.2: ADGN-100a- and VEPEP-3a-mediated functional delivery of RGEN/gRNA complex targeting β-catenin (beta-catenin) or HPRT in C2C12 cells

VEPEP-3a and ADGN-100a peptides were evaluated for functional cellular delivery of RGEN/gRNA complexes targeting endogenous HPRT and β-Catenin genes in undifferentiated and differentiated mouse muscle myoblast cells (C2C12). In cardiac muscle, beta-catenin localizes to adherens junctions in intercalated disc structures, which are critical for electrical and mechanical coupling between adjacent cardiomyocytes. Preloaded CAS9/gRNA complexes (5 µg/10µg CAS9/gRNA) were mixed with VEPEP-3a and ADGN-100a peptides at 20:1 molar ratio of CPP to cargo and incubated for 30 min at 37 °C prior to cell transfection. Delivery by RNAiMAX was included as a positive control.

CAS9-induced NHEJ was analyzed 72 hours after transfection by PCR (FIG. 26) and T7E1 assay (FIG. 27). The deletion (indel) frequencies in the HPRT gene were quantified using a T7E1 assay by quantifying the amount of uncut vs cut DNA using a Fragment Analyzer System Data for on-target mutations induced by RGEN/gRNA are reported as an average of 3 separate experiments

C2C12 mouse adherent myoblasts were grown in Dulbecco's modified Eagle's medium (DMEM) supplemented with heat-inactivated 10% fetal bovine serum, 2mM glutamine, 1% antibiotics, 0.5% antimycoplasm and 25mM Hepes, pH 7.5. The cell line was maintained in 5% CO₂ at 37°C and cell viability was assessed by the Trypan Blue exclusion test. To induce myogenic differentiation, at about 80% cell confluence the medium containing 10% fetal calf serum was substituted with medium containing1 % fetal calf serum The cells were transfected at the undifferentiated stage or at day 7 after differentiation.

As shown in FIGS. 26 and 27, both VEPEP-3a- and ADGN-100a-mediated RGEN/gRNA delivery induced a robust editing of β-Catenin and HPRT genes in both undifferentiated and differentiated C2C12 cell lines.

Mean indel frequencies of 81% and 73% were obtained by VEPEP-3a-mediated delivery in undifferentiated and differentiated C2C12 cell lines, respectively. Mean indel frequencies of 79% and 47% were obtained by ADGN-100a-mediated delivery in undifferentiated and differentiated C2C12 cell lines, respectively. In contrast, RNAiMAX-mediated delivery resulted in indel frequencies of 46% and 18% in the undifferentiated and differentiated C2C12 cell lines, respectively. These results further demonstrate that VEPEP-3 and ADGN-100 peptides are potent vehicles for the delivery of multiple RGEN/gRNA complexes in differentiated muscle myoblast cells.

### Example 13a: CPP-mediated CAS9 mRNA delivery in multiple adherent and suspension cell lines

Cell-penetrating peptides (VEPEP-6, VEPEP-9, VEPEP-3a, and ADGN-100a) were evaluated for cellular delivery of CAS9 mRNA in multiple adherent (U2OS and primary human fibroblast) and suspension (HEPG2 and K562) cell lines. CAS9 mRNA was prepared using the HiScribe™ T7 ARCA mRNA Kit (New England Biolab) with linearized pCS2-CAS9 vector (Addgene # 47322) for the DNA template, and purified by phenol:chloroform extraction. CPP/CAS9 mRNA complexes were formed by mixing the CAS9 mRNA (0.5 µg) with CPP at a 20:1 molar ratio of CPP:CAS9 mRNA and incubating for 30 min at 37°C prior to cell transfection. Cells were transfected according to optimized protocols for either adherent or suspension cell lines.

### Adherent cell transfection

Human primary fibroblasts were freshly obtained and grown in flasks pre-coated with a gelatin-based coating solution and incubated in Culture Complete Growth Medium for 3 days before expansion and treatment. U2OS cells were cultured in Dulbecco's Modified Eagle's Medium (DMEM), supplemented with 2 mM glutamine, 1% antibiotics (streptomycin 10,000 µg/mL, penicillin, 10,000 IU/ mL) and 10% (w/v) foetal calf serum (FCS), at 37°C in a humidified atmosphere containing 5% CO₂. 35 mm dishes seeded with 150,000 cells the day prior to transfection were grown to 50-60% confluence and set up to be at about 70% confluences at the time of transfection. Before transfection, cells were washed with DMEM (2X) followed by PBS (1x). Cells were then overlaid with 0.2 ml of complex solution (16 µl CPP/Cargo complex in water + 184 µl OPTiMEM without antibiotics), mixed gently, and incubated for 10 min at 37°C. 0.8 mL of fresh OPTiMEM or DMEM was then added and cells were incubated for 20 min at 37°C. 2 mL of complete OPTiMEM (high glucose) or DMEM containing 11.4% FCS was then added in order to reach a final FCS concentration of 10%, without removing the overlay of CPP/Cargo complexes. Cells were returned to the incubator (37°C, 5% CO₂) and assayed at 72 hours post transfection.

### Suspension cell transfection

K562 and HEpG2 cells were set up to be at about 70% confluence at the time of transfection. The cells were maintained in high glucose DMEM media with 10% serum. Before transfection, cells were harvested by centrifugation, then washed with DMEM (2X) followed by PBS (1x). Cells were then resuspended in 0.2 ml of complex solution (16 µl CPP/Cargo complex in water + 184 µl OPTiMEM without antibiotics), mixed gently, and incubated for 10 min at 37°C. 0.8 mL of fresh OPTiMEM was then added and cells were incubated for 20 min at 37°C. 2 mL of complete OPTiMEM (high glucose) containing 11.4% FCS was then added in order to reach a final FCS concentration of 10%, without removing the overlay of CPP/Cargo complexes. Cells were returned to the incubator (37°C, 5% CO₂) and assayed at 72 hours post transfection.

RNAiMAX lipid-based delivery (Invitrogen Thermo Fisher France) was used as a control and transfections were performed following the manufacturer's instructions. CAS9 expression level was analyzed at multiple time points over a period of 10 days as determined by ELISA on protein extracts using antibodies against CRISPR/Cas9 obtained from Diagenode (Diagenode cat. No. C15200223) and ABCAM (clone 7A9- ab191468). CAS9 protein expression results are shown in FIGS. 28A-28D, and are reported as averages from 3 separate experiments.

As shown in FIGS. 28A-28D, VEPEP-6 and ADGN-100a CPPs promoted efficient delivery of mRNA, leading to robust CAS9 protein expression in each of the tested cell types. In U2OS, K562, and HepG2 cells, the ELISA signal for CAS9 protein expression at day 3 was increased to about 25 and 30 times the background levels (day 0 signal) using ADGN-100a and VEPEP-6, respectively. In primary human fibroblasts, the ELISA signal for CAS9 protein expression at day 3was increased to about 20 times the background using either ADGN-100a or VEPEP-6. CAS9 protein expression was optimal at day 3 after transfection, and decreased to about 50% of these levels by day 10. An increase in the ELISA signal for CAS9 protein expression at day 3 to about 5 to 10 times the background was obtained using VEPEP-9 for CAS9 mRNA delivery. In contrast, little to no increase in CAS9 protein expression at day 3 was observed with VEPEP-3a peptide. As a comparison, using RNAiMAX lipid formulation for CAS9 mRNA delivery, the ELISA signal for CAS9 protein expression at day 3 increased to about 5 to 10 times the background in U2OS cells, and little to no increase in CAS9 expression was observed in the other cell types (K562, HepG2, and primary human fibroblast). These results demonstrate that VEPEP-6 and ADGN-100a peptides are potent vehicles for the delivery of mRNA in hard to transfect cell types.

### Example 13b: CPP-mediated functional delivery of CAS9 mRNA and EMX1-targeting gRNA in multiple adherent and suspension cell types

Cell-penetrating peptides (VEPEP-6, VEPEP-9, and ADGN-100a) were evaluated for functional intracellular delivery of nucleoprotein complexes containing CAS9 mRNA and gRNA targeting the endogenous EMX1 gene in multiple adherent (U2OS and primary human fibroblast) and suspension (HEPG2, K562) cell types.

*EMX1*-targeting gRNAs and CAS9 mRNA were prepared as described above. CPP/CAS9 mRNA/gRNA complexes were formed by mixing the CAS9 mRNA (0.5 µg) and **gRNA** (5µg) with CPP at a 20:1 molar ratio of CPP:cargo and incubating for 30 min at 37°C prior to cell transfection. Suspension and adherent cells were transfected according to the protocols described in Example 13a. RNAiMAX-mediated delivery (Invitrogen Thermo Fisher France) was used as a control. *EMX1* gene editing was analyzed at day 3 and day 6 by T7E1 assay and quantified using a Fragment Analyzer System

Results for indel frequencies in the *EMX1* gene are shown in FIGS. 29A-29D (average of 3 separate experiments). VEPEP-6- and ADGN-100a-mediated CAS9 mRNA/gRNA delivery induced a robust editing of the *EMX-1* gene in each of the cell types tested. For both peptides, efficient functional intracellular delivery of complexes containing CAS9 mRNA and gRNA was observed after 72 hours. Mean indel frequencies of about 70-80% were obtained in U2OS and HepG2 cells, and of about 60% in primary human fibroblasts and K526 cells.

Using VEPEP-9 for delivery, mean indel frequencies of about 40-50% were obtained in U2OS and HepG2 cells, and of about 10% in primary human fibroblasts and K562 cells. As a comparison, using RNAiMAX for delivery, mean indel frequencies of about 40-50% were obtained in U2OS and HepG2 cells, and of about 10-15% in primary human fibroblast and K562 cells. These results demonstrate that VEPEP-6 and ADGN-100a peptides are about 3 to 5 times as potent as RNAiMAX for functional delivery of CAS9 mRNA and gRNA, depending on cell type.

The robust CPP-mediated *EAM1* gene disruption observed in the four cell types was not associated with cellular toxicity. As shown in FIG. 30, less than 10% toxicity was observed using either ADGN-100a or VEPEP-6 peptides. By contrast, with RNAiMAX between 40-50% cell death in primary human fibroblasts and K562 cells and between 35-40% cell death in U2OS and HepG2 cells was observed. These results demonstrate that both VEPEP-6 and ADGN-100a are potent, non-toxic cell-penetrating peptides useful for mRNA/gRNA delivery in multiple human cell types.

### Example 13c: CPP-mediated functional delivery of CAS9 protein/EMX1-targeting gRNA complexes in multiple adherent and suspension cell types

CPPs (ADGN-100a, VEPEP-9, VEPEP-6 and VEPEP-3a) were then evaluated for cellular delivery of CAS9 protein/gRNA complexes targeting the endogenous *FMX1* gene in multiple adherent (U2OS and primary human fibroblast) and suspension (HEPG2 and K562) cell types.

HIS-NLS-CAS9 protein and *EMX1*-targeting gRNAs were prepared as described above. CPP/CAS9/gRNA complexes were prepared by mixing preloaded CAS9/gRNA complexes (2.5 µg/5 µg CAS9/gRNA) with CPP peptides at a 20:1 molar ratio of CPP to cargo and incubating for 30 min at 37 °C prior to cell transfection. Cells were transfected according to the protocols described in Example 13a, and RNAiMAX-mediated delivery (Invitrogen Thermo Fisher France) was used as a control. *EMX1* gene editing was analyzed at day 3 and day 6 by T7E1 assay and quantified using a Fragment Analyzer System.

Data for on-target mutations induced by RGEN/gRNA are shown in FIGS. 31A-31D (average of 3 separate experiments). Both VEPEP-3a- and ADGN-100a-mediated RGEN/gRNA delivery induced a robust editing of the *EMX1* gene in each of the cell types tested. Mean indel frequencies of about 75-70% and about 60-65% were obtained with delivery by VEPEP-3a and ADGN-100a, respectively. Mean indel frequencies of 35-40% were obtained using VEPEP-9. In contrast, no significant *EMX1* gene editing was observed with VEPEP-6 peptide. For comparison, RNAiMAX delivery resulted in indel frequencies of 40% in U2OS and K562 cells and 25-30% in HEPG2 cells and primary human fibroblasts.

### Example 13d: CPP-mediated CAS9-expressing plasmid delivery in multiple adherent and suspension cell types

CPPs (ADGN-100a, VEPEP-9, VEPEP-3a, and VEPEP-6) were evaluated for cellular delivery of a plasmid encoding CAS9 protein in U2OS and HEPG2 cell lines and primary human fibroblasts.

CPP/plasmid complexes were prepared by mixing 0.5 µg of a CAS9-expressing plasmid (Addgene MLM3639-Plasmid #42252) with CPP at a 20:1 molar ratio of CPP:cargo and incubating for 30 min at 37°C prior to cell transfection. Cells were transfected according to the protocols for adherent and suspension cells described in Example 13a Lipofectamine 2000 was used as lipid-based delivery control. CAS9 protein expression level was analyzed at multiple time points over a period of 10 days as determined by ELISA on protein extracts using antibodies against CRISPR/Cas9 obtained from Diagenode (Diagenode cat. No. C15200223) and ABCAM (clone 7A9- abl91468).

CAS9 protein expression results are shown in FIGS. 32A-32C (averages from 3 separate experiments). ADGN-100a and VEPEP-9 CPPs promoted efficient delivery of the CAS9-expressing plasmid, as indicated by the robust CAS9 protein expression in each of the cell types tested. For both peptides, protein expression was optimal at day 6 after transfection and decreased by about 50% by day 10. The ELISA signal for CAS9 protein expression was increased at day 6 to about 9 to 10 times the background using ADGN-100a and VEPEP-9 in U2OS and HEPG2 cells and primary human fibroblasts, to about 4 times the background using VEPEP-6 in U2OS and HEPG2 cells, and to about 2 times the background using VEPEP-6 in primary human fibroblasts. In contrast, little to no increase in CAS9 expression was observed with VEPEP-3a peptide. As a comparison, using lipofectime 2000 for CAS9-expressing plasmid delivery, the ELISA signal for CAS9 protein expression at day 6 was increased to about 4 times the background in U2OS cells, with no significant (lower than 2 times the background) CAS9 protein expression observed in HepG2 cells and primary human fibroblasts. These results demonstrate that ADGN-100a and VEPEP-9 peptides are potent vehicles for the delivery of CAS9-expressing plasmids in hard to transfect cell types.

### Example 13e: CPP-mediated functional delivery of CAS9-expressing plasmid/EMX1-targeting gRNA in multiple adherent and suspension cell types

Cell-penetrating peptides (VEPEP-6, VEPEP-9, and ADGN-100a) were evaluated for functional intracellular delivery of a plasmid encoding CAS9 and gRNA targeting the endogenous *EMX1* gene in multiple adherent (U2OS and primary human fibroblast) and suspension (HEPG2) cell types.

*EMX1*-targeting gRNAs were prepared as described above. CPP/plasmid/gRNA complexes were prepared by mixing 0.5 µg of a CAS9-expressing plasmid (Addgene MLM3639-Plasmid #42252) and 5 µg gRNA with CPP at a 20:1 molar ratio of CPP to nucleic acid (plasmid + gRNA) and incubating for 30 min at 37°C prior to cell transfection. Suspension and adherent cells were transfected according to the protocols described in Example 13a. Lipofectamine 2000- and RNAiMAX-mediated delivery methods (Invitrogen Thermo Fisher France) were used as controls. *EMX1* gene editing was analyzed at day 3 and day 6 by T7E1 assay and quantified using a Fragment Analyzer System

The indel frequencies in the *EMX1* gene are shown in FIGS. 33A-33C (average of 3 separate experiments). ADGN-100a and VEPEP-9 promoted delivery of both CAS9-expressing plasmid and gRNA, inducing robust editing of the *EMX-1* gene in each of the three cell types. Optimal double strand break formation was obtained using ADGN-100a peptides. For both peptides, efficient functional intracellular delivery of CAS9 expressing plasmid and gRNA was observed at day 3. Mean indel frequencies of about 45-50% and about 35-40% were obtained in the three cell types using ADGN-lOOa and VEPEP-9, respectively.

As a comparison, mean indel frequencies of about 20% in U2OS cells and less than 10% in HepG2 cells and primary human fibroblasts were obtained using lipofectamine 2000 and RNAiMAX for delivery. These results demonstrate that VEPEP-9 and ADGN-100a peptides are about 2 to 5 times as potent as RNAiMAX for delivering both plasmid and gRNA, depending on the cell type.

### Example 14: CPP-mediated in vivo delivery of CAS9-expressing mRNA in mice

CPPs including ADGN-100a, VEPEP-6, and VEPEP-9 were evaluated for *in vivo* delivery of CAS9-expressing mRNA in Swiss mice. Polyadenylatyed and capped CAS9 mRNA was obtained from ThermoFisher. CPP/CAS9 mRNA complexes were prepared by mixing CAS9 mRNA (5 µg) with ADGN-100a, VEPEP-6, or VEPEP-9 peptides at a 20:1 ratio of CPP to mRNA. Mice received a single intravenous injection (100 µl) of CAS9 mRNA/ADGN-100a (6 animals/group), CAS9 mRNA/VEPEP-6 (6 animals per group), or CAS9 mRNA/VEPEP-9 (6 animals per group) complexes in physiological buffer. Free CAS9 mRNA in physiological buffer was injected as a control.

To evaluate CPP/mRNA delivery-associated *in vivo* cytokine induction, blood samples were collected by saphenous vein bleeds at multiple time points (12 hr, 24 hr, 48 hr and 72 hr), collecting no more than 100 µl or 10% of total blood volume per week. Serum cytokine levels were measured using The Cytokine Mouse Magnetic 20-Plex Panel from Invritrogen (LMC 0006M). The Cytokine Mouse Magnetic 20-Plex Panel for the Luminex™ platform is specifically designed for quantifying mouse cytokines, chemokines, and growth factors in serum, plasma, and tissue culture supernatant. The 20-plex panel allowed for the simultaneous quantification of mouse GM-CSF, IFN-g, EL1a, Il1b, Il2,IL4, Il5, Il6, 1110, Il12 (p40/p70), IL13, IL17, TNF-a, IP-10, KC, MCP-1, MIG; MIP-1a, FGF-basic, and VEGF in serum or tissue culture supernatant. Administration of lipopolysaccharides (LPS) was included as a control, and results are reported in FIG. 34. Little to no impact of the different CPPs on non-specific cytokine induction was observed at the different time points, in contrast to administration of LPS.

At day 3, day 6, and day 10 following injection, animals were sacrificed and tissues including lung, liver, brain, kidney, heart, spleen, blood, pancreas, muscle, bone marrow, and intestine were collected. Tissues were homogenized and CAS9 protein levels in protein extracts were analyzed by ELISA using an antibody against CRISPR/Cas9 (SBI clone 7A9). As shown in FIGS. 35A (day 3) and 35B (day 6), robust expression of CAS9 protein in the liver was observed using the three CPPs (ADGN-100a, VEPEP-6, and VEPEP-9), with ELISA signal for CAS9 protein expression greater than 20 times, 15 times, and 8 times that of background, respectively.

Using ADGN-100a for delivery, CAS9 expression was also observed in lung and kidney, with levels about 2 to 3 times that of background. Little to no increase in CAS9 protein expression was detected in the other tissues examined.

Using VEPEP-6 for delivery, robust CAS9 expression was also observed in lung (about 8 times that of background), as well as in kidney, heart, brain and bone marrow (about 3 to 4 times that of background). Little to no increase in CAS9 protein expression was detected in the other tissues examined.

Using VEPEP-9 for delivery, robust CAS9 expression was also observed in the spleen, brain and lymph node (about 4 to 5 times that of background). Little to no increase in CAS9 protein expression was detected in the other tissues examined.

For all three CPPs, the levels of CAS9 expression in the different tissues were similar at day 3 and day 6, suggesting long term persistence of CAS9 protein expression. As shown in FIG. 35C, expression of CAS9 protein was maintained 10 days following injection. At day 10, CAS9 expression was observed in the liver with the three CPPs (ADGN-100a, VEPEP-6, and VEPEP-9), in the brain with VEPEP-6 and VEPEP-9, in the lung with VEPEP-6, and in lymph node with VEPEP-9.

### Example 15: CPP-mediated in vivo delivery of CAS9-expressing mRNA and gRNA targeting PCSK9 in mice

CPPs including ADGN-100a, VEPEP-6 and VEPEP-9 were evaluated for *in vivo* delivery in Swiss mice of CAS9-expressing mRNA and gRNA targeting proprotein convertase subtilisin/kexin type 9 (PCSK9), a therapeutically relevant gene involved in cholesterol homeostasis. Inhibitors of the human convertase PCSK9 have emerged as a promising new class of cardioprotective drugs, as loss of PCSK9 is associated with a reduced risk of cardiovascular disease and lower levels of low-density lipoprotein (LDL) cholesterol.

Polyadenylated and capped CAS9 mRNA was obtained from ThermoFisher, and **gRNA** (5 µg) targeting PCSK9 Exon 1 was obtained from Eurogentec. CPP/CAS9mRNA/gRNA complexes were prepared by mixing CAS9 mRNA (5 µg) and gRNA (5 µg) with ADGN-100a, VEPEP-6, or VEPEP-9 peptides at a 20:1 ratio of CPP to nucleic acid (mRNA + gRNA) and incubating for 30 min at 37°C prior to administration. Swiss mice received a single intravenous injection (100 µl) of ADGN-100a/CAS9 mRNA/gRNA (4 animals/group), VEPEP-6/CAS9 mRNA/gRNA (4 animals per group), or VEPEP-9/CAS9 mRNA/gRNA (4 animals per group) complexes in physiological buffer. Free CAS9 mRNA/gRNA was injected as a control.

Blood samples were collected by saphenous vein bleeds at multiple time points (day 3, 6, 10, and 21), collecting no more than 100 µl or 10% of total blood volume per week. Serum levels of PCSK9 were determined by ELISA using the Mouse Proprotein Convertase 9/PCSK9 Quantikine ELISA Kit (MPC-900, R&DSystems). Total cholesterol levels in the serum were measured using the Infinity Cholesterol Reagent (Thermo Fisher).

As shown in FIG. 36, serum levels of PSCK9 decreased by 51%, 62%, 69%, 65%, 65%, and 60% using ADGN-100a for delivery at day 1, day 3, day 6, day 10, day 14, and day 20, respectively. The serum levels of PSCK9 decreased by 64%, 73%, 75%, 73% , 71%, and 70% using VEPEP-6 for delivery at day 1, day 3, day 6, day 10, day 14, and day 20, respectively. Finally, serum levels of PSCK9 decreased by only 13%, 29%, 31%, 27%, 25%, and 24% using VEPEP-9 for delivery at day 1, day 3, day 6, day 10, day 14, and day 20, respectively. As shown in FIG. 37, for all conditions, the decrease in PCSK9 levels was associated with a reduction in serum cholesterol levels. Cholesterol levels decreased by about 35% to 42% at days 3, 6 10, 14, and 20 using ADGN-100a and VEPEP-6 peptides for delivery.

These results demonstrate that both ADGN-100a and VEPEP-6 peptides constitute potent carriers for *in vivo* functional delivery of CRISPR components, including CAS9 mRNA/gRNA, suggesting their suitability for use in therapeutic applications.

### Example 1.6: VEPEP-3-mediated in vivo delivery of CAS9 protein does not induce cytokine activation in mice

VEPEP-3a was evaluated for *in vivo* delivery of CAS9 protein in Swiss mice. S. pyogenes HIS-NLS-CAS9 was prepared as described above. CPP/CAS9 protein complexes were prepared by mixing CAS9 protein (5 µg) with VEPEP-3a at a 20:1 ratio of CPP to CAS9 protein. Mice received a single intravenous injection (100 µl) of CAS9/VEPEP-3a (6 animals/group) complexes in physiological buffer. Free CAS9 protein in physiological buffer was injected as a negative control, and administration of LPS was used as a positive control for cytokine activation. To evaluate VEPEP-3a/CAS9 delivery-associated *in vivo* cytokine induction, blood samples were collected by saphenous vein bleeds at multiple time points (12 hr, 24 hr, 48 hr, and 72 hr), collecting no more than 100 µl or 10% of total blood volume per week. Serum cytokine levels were measured using The Cytokine Mouse Magnetic 20-Plex Panel from Invritrogen (LMC 0006M). As shown in FIG. 38, little to no impact of VEPEP-3a on non-specific cytokine induction was observed at the different time points, in contrast to administration of LPS.

### Example 1.7: CPP-mediated functional delivery of CAS9-expressing mRNA and plasmid in primary human T cells

T cells were isolated from PBMCs using anti-CD3/anti-CD28 antibodies bound to paramagnetic beads (Dynabeads ClinExVivo CD3/CD28, Invitrogen,) at a ratio of 3:1 (beads:cells). The cells and beads were co-incubated for 2 hours at room temperature and CD3⁺ cell enrichment was performed using Dynal ClinExVIVO MPC magnet (Thermo Fisher). The anti-CD3/anti-CD28 paramagnetic beads were removed, washed and concentrated. Cells in the CD3⁺ fraction were resuspended in activation media at a concentration of 1×10⁶ cells/ml with AIM-V medium (Thermo Fishers) supplemented with 10% fetal calf serum, 300 IU/ml IL2 and 50 ng/ml OKT3 (MuromonAB-CD3) for two days.

Cell-penetrating peptides (VEPEP-6, VEPEP-9, VEPEP-3a, and ADGN-100a) were evaluated for cellular delivery of either CAS9 mRNA or CAS9-expressing plasmid in T cells (5 x 10⁶) isolated from a single donor. CAS9 mRNA was prepared as described above. CPP/CAS9 complexes were formed by mixing the CAS9 mRNA (0.5 µg) with CPP at a 20:1 molar ratio of CPP:CAS9 mRNA and incubating for 30 min at 37°C prior to cell transfection. CPP/CAS9-expressing plasmid complexes were formed by mixing the CAS9-expressing plasmid (Addgene MLM3639-Plasmid #42252) (0.5 µg) with CPP at a 20:1 molar ratio of CPP:plasmid and incubating for 30 min at 37°C prior to cell transfection.

Cells were transfected according to the protocol for suspension cells described in Example 13a. RNAiMAX and lipofectamine 2000 lipid-based delivery methods (Invitrogen Thermo Fisher France) were used as controls, and transfections were performed following the manufacturer's instructions. CAS9 protein expression level was analyzed at multiple time points over a period of 10 days as determine by ELISA on protein extracts using antibodies against CRISPR/Cas9 obtained from Diagenode (Diagenode cat. No. C15200223) and ABCAM (clone 7A9-ab191468).

Results for CAS9 protein expression are shown in FIGS. 39A and 39B (averages from 3 separate experiments). VEPEP-6 and ADGN-lOOa CPPs promoted efficient delivery of the CAS9 mRNA, as indicated by the robust CAS9 protein expression in the primary human T cells (FIG. 39A). ELISA signal for CAS9 protein expression was increased at day 3 to about 15 and 17 times the background using ADGN-lOOa and VEPEP-6 for CAS9 mRNA delivery, respectively, and to about 4 times the background using VEPEP-9. In all conditions, a high level of CAS9 protein expression was maintained at day 6, which was reduced by about 50% at day 10. In contrast, little to no CAS9 expression was observed with VEPEP-3a peptide or RNAiMAX. These results demonstrate that VEPEP-6 and ADGN-100a peptides are potent vehicles for the delivery of mRNA in hard to transfect cell types.

As shown in FIG. 39B, ADGN-100a and VEPEP-9 CPPs mediated efficient delivery of the CAS9-expressing plasmid, as indicated by the robust CAS9 protein expression in the primary human T cells. ELISA signal for CAS9 protein expression at day 3 was increased to about 4 times the background using ADGN-100a and VEPEP-9. In all conditions, a high level of CAS9 protein expression was maintained at day 6, which was reduced by about 50% at day 10. In contrast, little to no CAS9 expression was observed with VEPEP-3a or VEPEP-6 peptides, or with lipofectamine 2000. These results demonstrate that ADGN-100a and VEPEP-9 peptides are potent vehicles for the delivery of CAS9-expressing plasmid in hard to transfect cell types.

### Example 18: CPP-mediated functional delivery of EMX1-targeting RGEN/gRNA in primary human T cells using CAS9 mRNA, CAS9 protein, or CAS9-expressing plasmid

Cell-penetrating peptides (VEPEP-6, VEPEP-9, ADGN-100a, and VEPEP-3a) were evaluated for functional intracellular delivery of gRNA targeting the endogenous *EMX1* gene with CAS9 mRNA, CAS9 protein, or CAS9-expressing plasmid in primary human T cells.

*EMX1-*targeting gRNAs and CAS9 mRNA were prepared as described above. CPP/CAS9 mRNA/gRNA complexes were formed by mixing the CAS9 mRNA (0.5 µg) and gRNA (5µg) with CPP at a 20:1 molar ratio of CPP to nucleic acid (mRNA + gRNA) and incubating for 30 min at 37°C prior to cell transfection. CPP/plasmid/gRNA complexes were formed by mixing CAS9 expressing plasmid (Addgene MLM3639-Plasmid #42252) (0.5 µg) and gRNA (5µg) with CPP at a 20:1 molar ratio of CPP to nucleic acid (plasmid + gRNA) and incubating for 30 min at 37°C prior to cell transfection. CPP/CAS9/gRNA complexes were formed by mixing preloaded CAS9/gRNA complexes (2.5 µg/5 µg CAS9/gRNA) with CPP at a 20:1 molar ratio of CPP to complex and incubating for 30 min at 37 °C prior to cell transfection.

T cells were transfected according to the protocol for suspension cells described in Example 13a. Lipofectamine 2000- and RNAiMAX-mediated delivery methods (Invitrogen Thermo Fisher France) were included as controls. *EMX1* gene editing was analyzed at day 3, day 6, and day 10 by T7E1 assay and quantified using a Fragment Analyzer System

The indel frequencies in the *EMX1* gene are shown in FIGS. 40A-40C. VEPEP-6 and ADGN-100a mediated CAS9 mRNA/gRNA delivery, inducing a robust editing of the *EMX-1* gene in primary human T cells (FIG. 40A). For both peptides, efficient functional intracellular delivery of CAS9 mRNA/gRNA was observed after 72 hours. Mean indel frequencies of about 60-65% were obtained. In comparison, mean indel frequencies of about 10-15% were obtained using VEPEP-9 or RNAiMAX. VEPEP-3a- and ADGN-100a-mediated CAS9 protein/gRNA delivery induced a robust editing of the *EMX1* gene in primary T cells (FIG. 40B). Mean indel frequencies of about 60-65% were obtained with both peptides. Mean indel frequencies of about 20% were obtained using VEPEP-9. In contrast, little to no *EMX1* gene editing was observed with VEPEP-6 peptide or RNAiMAX. ADGN-100a and VEPEP-9 promoted delivery of CAS9-expressing plasmid and gRNA, inducing a robust editing of the *EMX1* gene in primary T cells (FIG. 40C). For both peptides, efficient functional intracellular delivery of CAS9-expressing plasmid and gRNA was observed at day 3, with mean indel frequencies of about 30%. In comparison, little to no *EMX1* gene editing was observed with VEPEP-6 peptide or RNAiMAX.

The robust *EMX1* gene disruption observed in primary human T cells was not associated with cellular toxicity. As shown in FIG. 40D, less than 10% toxicity was observed using ADGN-100a, VEPEP-6, VEPEP-9, and VEPEP-3a cargo complexes. By contrast, with RNAiMAX and Lipofectamine 2000 between 40-50% cell death was observed. These results demonstrate that CPPs are potent, non-toxic carriers useful for the delivery of varying types of cargo, including CAS9 mRNA/gRNA, CAS9 protein/gRNA, and CAS9 plasmid/gRNA, in primary human T cells.

### Example 19: Particle size measurement and Zeta potential of peptide-based particles with different cargoes

The CPP/CAS9-protein, CPP/CAS9-protein/sgRNA, CPP/CAS9-mRNA, CPP/CAS-mRNA/sgRNA particles were characterized; the mean size, polydispersity, and zeta potential were measured with a Zetasizer NANO ZS P apparatus (Malvern Ltd). CAS9-protein (2.5 µg) and preloaded CAS9-protein (2.5 ng)/gRNA (5 µg) were mixed with VEPEP-3a, VEPEP-9, ADGN-100a and VEPEP-6 peptides at a 20:1 molar ratio of CPP to cargo in physiological conditions (0.9% NaCl) at 20 °C and incubated for 30 min at 37 °C prior to analysis.

CAS9 mRNA (0.5 µg) and CAS9 mRNA (0.5 ng)/gRNA (5 µg) were mixed with VEPEP-6, VEPEP-9 and ADGN-100a at a 20:1 molar ratio of CPP to cargo in physiological conditions (0.9% NaCl) at 20 °C and incubated for 30 min at 37 °C prior to analysis.

The mean size and the polydispersity of the CCP/cargo complexes were determined at 25 °C for 5 min per measurement and zeta potential was measured with a Zetasizer NANO ZS P apparatus (Malvem Ltd). Data are shown in Table 14 for a mean of 3 separate experiments.

At a molar ratio 20:1, VEPEP-6, VEPEP-9 and ADGN-100a peptides were able to form stable nanoparticles with CAS9-mRNA and CAS9-mRNA/gRNA complex. Mean diameters of about 75 nm, 100 nm, and 120 nm, and polydispersity index (PI) of 0.25, 0.2, and 0.15 were obtained for ADGN-100, VEPEP-6 and VEPEP-9, respectively.

ADGN-100a/mRNA and ADGN-100a/CAS9-mRNA/gRNA particle charges obtained by zeta potential were -4.5 ± 0.5 mV and -5.8 ± 0.6 mV, respectively.

VEPEP-6/mRNA and VEPEP-6/CAS9-mRNA/gRNA particle charges obtained by zeta potential were -11.0 ± 2 mV and -12.7 ± 2.0 mV, respectively.

VEPEP-9/mRNA and VEPEP-9/CAS9-mRNA/gRNA particle charges obtained by zeta potential were 15.4 ± 4.0 mV and 11.1 ± 2.0 mV, respectively.

At a molar ratio of 20:1, VEPEP-3a and ADGN-100a peptides were able to form stable nanoparticles with CAS9-protein and CAS9-protein/gRNA complex. Mean diameters of about 60 nm and about 100 nm, and polydispersity index (PI) of about 0.25 and 0.3, were obtained for VEPEP-3a and ADGN-100a, respectively.

ADGN-100a/CAS9-protein and ADGN-100a/CAS9-protein/gRNA particle charges obtained by zeta potential were 3.6 ± 0.5 mV and 8.3 ± 1.5 mV, respectively.

VEPEP-3a/CAS9-protein and VEPEP-3a/CAS9-protein/gRNA particle charges obtained by zeta potential were 16.6 ± 5 mV and 18.0 ± 5 mV, respectively.

In contrast, VEPEP-9 formed larger particles with preloaded CAS9-protein/gRNA complex. At a molar ratio 20:1, mean particle size of 210 ± 20 and polydispersity index of 0.51 were obtained.

**Table 14: CPP/RGEN and CPP/RGEN:gRNA nanoparticle characterizations**

| **PARTICLES** | **SIZE (nm)** | **zeta potential (mV)** | **PI** |
|---|---|---|---|
| ADGN 100/CAS9-mRNA | 75 ± 5 | -4.5 ± 0.5 | 0.27 ± 0.1 |
| ADGN 100/CAS9-mRNA/gRNA | 71 ± 5 | -5.8 ± 0.6 | 0.308 ± 0.1 |
| ADGN 100/CAS9-protein | 96 ± 7 | 3.6 ± 0.5 | 0.28 ± 0.1 |
| ADGN 100/CAS9-protein /gRNA | 98 ± 6 | 8.3 ± 1.5 | 0.21 ± 0.1 |
| VEPEP 6/CAS9-mRNA | 102 ± 6 | -11± 2 | 0.18 ± 0.1 |
| VEPEP 6/CAS9-mRNA/gRNA | 97 ± 5 | -12.7 ± 2 | 0.21 ± 0.1 |
| VEPEP 9/CAS9-mRNA | 121 ± 7 | 15.4 ± 4 | 0.12 ± 0.1 |
| VEPEP 9/CAS9-mRNA/gRNA | 113 ± 6 | 11.1 ± 2 | 0.15 ± 0.1 |
| VEPEP 9/CAS9-protein | 210 ± 20 | 25.3 ± 5 | 0.46 ± 0.1 |
| VEPEP 9/CAS9-protein/gRNA | 189 ± 12 | 22.4 ± 5 | 0.51 ± 0.1 |
| VEPEP 3/CAS9-protein | 68 ± 5 | 16.6 ± 5 | 0.29 ± 0.1 |
| VEPEP 3/CAS9-protein/gRNA | 54 ± 5 | 18.0 ± 5 | 0.33 ± 0.1 |

### Example 20: CPP-mediated functional delivery of CAS9 mRNA and gRNA targeting Olig2, HEF1-alpha, BCL2L2, or mutant PTEN or mutant KRAS in glioblastoma or pancreatic cancer cell lines

Cell-penetrating peptides (VEPEP-6, VEPEP-9, and ADGN-100a) are evaluated for functional intracellular delivery of nucleoprotein complexes containing CAS9 mRNA and gRNA targeting Olig2, HIF1-alpha, BCL2L2, mutant PTEN, or mutant KRAS in glioblastoma or pancreatic cancer cell lines. CPP/CAS9 mRNA/gRNA complexes are formed by mixing the CAS9 mRNA (0.5 µg) and gRNA (5µg) with CPP at a 20:1 molar ratio of CPP:cargo and incubating for 30 min at 37°C prior to cell transfection. Cells are transfected according to the protocols described in Example 13a. RNAiMAX-mediated delivery (Invitrogen Thermo Fisher France) is used as a control. Gene editing is analyzed at day 3 and day 6 by T7E1 assay and quantified using a Fragment Analyzer System. Cell proliferation and cell death are measured using standard techniques.

### Example 21: CPP-mediated functional delivery of CAS9 mRNA and gRNA targeting mutant PTEN or mutant KRAS and gene editing via homology directed repair (HDR) in glioblastoma or pancreatic cancer cell lines

Cell-penetrating peptides (VEPEP-6, VEPEP-9, and ADGN-100a) are evaluated for functional intracellular delivery of RGEN/gRNA/ssDNA for genome engineering using precise Homology Direct Repair (HDR) in glioblastoma or pancreatic cancer cell lines. Delivery of one or more complexes containing CAS9 mRNA, gRNA targeting mutant PTEN or mutant KRAS, and an ssDNA donor template allows correction of the mutant gene by HDR.

Genome editing experiments are optimized for a high level of double strand break formation, using CPP/CAS9 mRNA/gRNA complexes formed by mixing the CAS9 mRNA (0.5 µg) and gRNA (5µg) with CPP at a 20:1 molar ratio of CPP:cargo. 50 nM of both REGN/gRNA and ssDNA-donor are associated to VEPEP-6 or ADGN-100a peptides at a 20:1 molar ratio either in a same particle (mRNA/gRNA/ssDNA/CPP ternary complex) or in two different particles (mRNA/gRNA/CPP and ssDNA/CPP).

Cells are transfected according to the protocols described in Example 13a. RNAiMAX-mediated delivery (Invitrogen Thermo Fisher France) is used as a control. Gene editing is analyzed at day 3 and day 6 by T7E1 assay and quantified using a Fragment Analyzer System. Cell proliferation and cell death are measured using standard techniques. Efficiency of HDR correction of targeted gene is analyzed using Restriction Fragment Length Polymorphisms (RFLP) assay and genome sequencing.

### Example 22: CPP-mediated in vivo delivery of CAS9-expressing mRNA and gRNA targeting Olig2, HIF1-alpha, BCL2L2, or mutant PTEN in a glioblastoma model

Nude mice bearing an orthotopic glioblastoma xenograft tumor (e.g. U87) are evaluated for antitumor activity of the peptide-based gene editing complexes. CPPs including ADGN-lOOa, VEPEP-6 and VEPEP-9 are evaluated for *in vivo* delivery of CAS9-expressing mRNA and gRNA targeting Olig2, HIF1-alpha, BCL2L2, or mutant PTEN.

Polyadenylated and capped CAS9 mRNA is obtained from ThermoFisher, and gRNA targeting the above targets are obtained commercially. CPP/CAS9 mRNA/gRNA complexes are prepared by mixing CAS9 mRNA (5 µg) and gRNA (5 µg) with ADGN-100a, VEPEP-6, or VEPEP-9 peptides at a 20:1 ratio of CPP to nucleic acid (mRNA + gRNA) and incubating for 30 min at 37°C prior to administration. Nude mice receive a single intravenous or intracranial/intratumoral injection (100 µl) of ADGN-100a/CAS9 mRNA/gRNA (4 animals/group), VEPEP-6/CAS9 mRNA/gRNA (4 animals per group), or VEPEP-9/CAS9 mRNA/gRNA (4 animals per group) complexes in physiological buffer. Free CAS9 mRNA/gRNA is injected as a control. Animals are treated 1x or 2x weekly and tumor sizes are measured over 6 weeks.

### Example 23: CPP-mediated in vivo delivery of CAS9-expressing mRNA and gRNA targeting mutant KRAS in a pancreatic cancer model

Nude mice bearing a pancreatic xenograft tumor are evaluated for antitumor activity of the peptide-based gene editing complexes. CPPs including ADGN-100a, VEPEP-6 and VEPEP-9 are evaluated for *in vivo* delivery of CAS9-expressing mRNA and gRNA targeting Olig2, HIF1-alpha, BCL2L2, or mutant KRAS.

Polyadenylated and capped CAS9 mRNA is obtained from ThermoFisher, and gRNA targeting the above targets is obtained commercially. CPP/CAS9mRNA/gRNA complexes are prepared by mixing CAS9 mRNA (5 µg) and gRNA (5 µg) with ADGN-100a, VEPEP-6, or VEPEP-9 peptides at a 20:1 ratio of CPP to nucleic acid (mRNA + gRNA) and incubating for 30 min at 37°C prior to administration. Nude mice receive a single intravenous injection (100 µl) of ADGN-100a/CAS9 mRNA/gRNA (4 animals/group), VEPEP-6/CAS9 mRNA/gRNA (4 animals per group), or VEPEP-9/CAS9 mRNA/gRNA (4 animals per group) complexes in physiological buffer. Free CAS9 mRNA/gRNA is injected as a control. Animals are treated 1x or 2x weekly and tumor sizes are measured over 6 weeks.

### Example 24: CPP-mediated in vivo delivery of CAS9-expressing mRNA and gRNA targeting mutant PTEN and gene editing via homology directed repair (HDR) in a glioblastoma model

Nude mice bearing an orthotopic glioblastoma xenograft tumor (e.g. U87) are evaluated for antitumor activity of the peptide-based gene editing complexes. CPPs including ADGN-lOOa, VEPEP-6 and VEPEP-9 are evaluated for *in vivo* delivery of CAS9-expressing mRNA, gRNA targeting mutant PTEN, and ssDNA donor template allowing correction of the mutant PTEN gene by HDR.

Polyadenylated and capped CAS9 mRNA is obtained from ThermoFisher, and gRNA targeting the above targets is obtained commercially. CPP/CAS9mRNA/gRNA/ssDNA complexes are prepared by mixing CAS9 mRNA (5 µg), gRNA (5 µg), and ssDNA (10 µg) with ADGN-100a, VEPEP-6, or VEPEP-9 peptides at a 20:1 ratio of CPP to nucleic acid (mRNA + gRNA) either in the same particle (mRNA/gRNA/ssDNA/CPP ternary complex) or in two different particles (mRNA/gRNA/CPP and ssDNA/CPP) and incubating for 30 min at 37°C prior to administration. Nude mice receive a single intravenous injection (100 µl) of ADGN-100a/CAS9 mRNA/gRNA/ssDNA (4 animals/group), VEPEP-6/CAS9 mRNA/gRNA/ssDNA (4 animals per group), or VEPEP-9/CAS9 mRNA/gRNA/ssDNA (4 animals per group) complexes in physiological buffer. Free CAS9 mRNA/gRNA/ssDNA is injected as a control. Animals are treated 1x or 2x weekly and tumor sizes are measured over 6 weeks. Efficiency of HDR correction of interest gene PTEN is analyzed by genome sequencing at 6 weeks.

### Example 25: CPP-mediated in vivo delivery of CAS9-expressing mRNA and gRNA targeting mutant KRAS and gene editing via homology directed repair (HDR) in a pancreatic cancer model

Nude mice bearing a pancreatic xenograft tumor are evaluated for antitumor activity of the peptide-based gene editing complexes. CPPs including ADGN-100a, VEPEP-6 and VEPEP-9 are evaluated for *in vivo* delivery of CAS9-expressing mRNA, gRNA targeting mutant KRAS, and ssDNA donor template allowing the correction of the mutant KRAS gene by HDR.

Polyadenylated and capped CAS9 mRNA is obtained from ThermoFisher, and gRNA targeting the above targets is obtained commercially. CPP/CAS9mRNA/gRNA/ssDNA complexes are prepared by mixing CAS9 mRNA (5 µg), gRNA (5 µg), and ssDNA (10 µg) with ADGN-100a, VEPEP-6, or VEPEP-9 peptides at a 20:1 ratio of CPP to nucleic acid (mRNA + gRNA) either in the same particle (mRNA/gRNA/ssDNA/CPP ternary complex) or in two different particles (mRNA/gRNA/CPP and ssDNA/CPP) and incubating for 30 min at 37°C prior to administration. Nude mice receive a single intravenous injection (100 µl) of ADGN-100a/CAS9 mRNA/gRNA/ssDNA (4 animals/group), VEPEP-6/CAS9 mRNA/gRNA/ssDNA (4 animals per group), or VEPEP-9/CAS9 mRNA/gRNA/ssDNA (4 animals per group) complexes in physiological buffer. Free CAS9 mRNA/gRNA/ssDNA is injected as a control. Animals are treated 1x or 2x weekly and tumor sizes are measured over 6 weeks. Efficiency of HDR correction of interest gene is analyzed by genome sequencing at 6 weeks.

**SEQUENCE LISTING**

| SEQID | Sequence | Annotations |
|---|---|---|
| 1 | X₁X₂X₃X₄X₅X₂X₃X₄X₆X₇X₃X₈X₉X₁ₒX₁₁X₁₂X₁₃ | VEPEP-3 |
| | | |
| | X₁ is beta-A or S, X₂ is K, R or L, X₃ is F or W, X₄ is F, W or Y, X₅ is E, R or S, X₆ is R, T or S, X₇ is E, R, or S, X₈ is none, F or W, X₉ is P or R, X₁₀ is R or L, X₁₁ is K, W or R, X₁₂ is R or F, and X₁₃ is R or K | |
| 2 | X₁X₂WX₄EX₂WX₄X₆X₇X₃PRX₁₁RX₁₃ | VEPEP-3 1 |
| | | |
| | X₁ is beta-A or S, X₂ is R or K, X₃ is W or F, X₄ is F, W, or Y, X₆ is T or R, X₇ is E or R, X₁₁ is R or K, and X₁₃ is R or K | |
| 3 | X₁KVVFERVVFREVVPRKRR | VEPEP-3 1a |
| | | |
| | X₁ is beta-A or S | |
| 4 | X₁KWWERWWREWPRKRR | VEPEP-3 1b |
| | | |
| | X₁ is beta-A or S | |
| 5 | X₁KWWERWWREWPRKRK | VEPEP-3 1c |
| | | |
| | X₁ is beta-A or S | |
| 6 | X₁RVVVVEKVVVVTRVVPRKRK | VEPEP-3 1d |
| | | |
| | X₁ is beta-A or S | |
| 7 | X₁RVVYEKVVYTEFPRRRR | VEPEP-3 1e |
| | X₁ is beta-A or S | |
| 8 | X₁KX₁₄WWERWWRX₁₄WPRKRK | VEPEP-3 1S |
| | | |
| | X₁ is beta-A or S and X₁₄ is a non-natural amino acid, and wherein there is a hydrocarbon linkage between the two non-natural amino acids | |
| 9 | X₁X₂X₃WX₅X₁₀X₃WX₆X₇WX₈X₉X₁₀WX₁₂R | VEPEP-3 2 |
| | | |
| | X₁ is beta-A or S, X₂ is K, R or L, X₃ is F or W, X₅ is R or S, X₆ is R or S, X₇ is R or S, X₈ is F or W, X₉ is R or P, X₁₀ is L or R, and X₁₂ is R or F | |
| 10 | X₁RWWRLWWRSWFRLWRR | VEPEP-3 2a |
| | | |
| | X₁ is beta-A or S | |
| 11 | X₁LWWRRWWSRWWPRWRR | VEPEP-3 2b |
| | | |
| | X₁ is beta-A or S | |
| 12 | X₁LWWSRWWRSWFRLWFR | VEPEP-3 2c |
| | | |
| | X₁ is beta-A or S | |
| 13 | X₁KFWSRFWRSWFRLWRR | VEPEP-3 2d |
| | | |
| | X₁ is beta-A or S | |
| 14 | X₁RWWX₁₄LWWRSWX₁₄RLWRR | VEPEP-3 2S |
| | | |
| | X₁ is a beta-alanine or a serine and X₁₄ is a non-natural amino acid, and wherein there is a hydrocarbon linkage between the two non-natural amino acids | |
| 15 | X₁LX₂RALWX₉LX₃X₉X₄LWX₉LX₅X₆X₇X₈ | VEPEP-6 1 |
| | | |
| | X₁ is beta-A or S, X₂ is F or W, X₃ is L, W, C or I, X₄ is S, A, N or T, X₅ is L or W, X₆ is W or R, X₇ is K or R, X₈ is A or none, and X₉ is R or S | |
| 16 | X₁LX₂LARWX₉LX₃X₉X₄LWX₉LX₅X₆X₇X₈ | VEPEP-6 2 |
| | | |
| | X₁ is beta-A or S, X₂ is F or W, X₃ is L, W, C or I, X₄ is S, A, N or T, X₅ is L or W, X₆ is W or R, X₇ is K or R, X₈ is A or none, and X₉ is R or S | |
| 17 | X₁LX₂ARLWX₉LX₃X₉X₄LWX₉LX₅X₆X₇X₈ | VEPEP-6 3 |
| | | |
| | X₁ is beta-A or S, X₂ is F or W, X₃ is L, W, C or I, X₄ is S, A, N or T, X₅ is L or W, X₆ is W or R, X₇ is K or R, X₈ is A or none, and X₉ is R or S | |
| 18 | X₁LX₂RALWRLX₃RX₄LWRLX₅X₆X₇X₈ | VEPEP-6 4 |
| | | |
| | X₁ is beta-A or S, X₂ is F or W, X₃ is L, W, C or I, X₄ is S, A, N or T, X₅ is L or W, X₆ is W or R, X₇ is K or R, and X₈ is A or none | |
| 19 | X₁LX₂RALWRLX₃RX₄LWRLX₅X₆KX₇ | VEPEP-6 5 |
| | | |
| | X₁ is beta-A or S, X₂ is F or W, X₃ is L or W, X₄ is S, A or N, X₅ is L or W, X₆ is W or R, X₇ is A or none | |
| 20 | X₁LFRALWRLLRX₂LWRLLWX₃ | VEPEP-6 6 |
| | | |
| | X₁ is beta-A or S, X₂ is S or T,and X₃ is K or R | |
| 21 | X₁LWRALWRLWRX₂LWRLLWX₃A | VEPEP-6 7 |
| | | |
| | X₁ is beta-A or S, X₂ is S or T, and X₃ is K or R | |
| 22 | X₁LWRALWRLX₄RX₂LWRLWRX₃A | VEPEP-6 8 |
| | | |
| | X₁ is beta-A or S, X₂ is S or T, X₃ is K or R, and X₄ is L, C or I | |
| 23 | X₁LWRALWRLWRX₂LWRLWRX₃A | VEPEP-6 9 |
| | | |
| | X₁ is beta-A or S, X₂ is S or T, and X₃ is K or R | |
| 24 | X₁LWRALWRLX₅RALWRLLWX₃A | VEPEP-6 10 |
| | | |
| | X₁ is beta-A or S, X₃ is K or R, and X₅ is L or I | |
| 25 | X₁LWRALWRLX₄RNLWRLLWX₃A | VEPEP-6 11 |
| | | |
| | X₁ is beta-A or S, X₃ is K or R, and X₄ is L, C or I | |
| 26 | Ac-X₁LFRALWRLLRSLWRLLWK-cysteamide | VEPEP-6a |
| | | |
| | X₁ is beta-A or S | |
| 27 | Ac-X₁LWRALWRLWRSLWRLLWKA-cysteamide | VEPEP-6b |
| | | |
| | X₁ is beta-A or S | |
| 28 | Ac-X₁LWRALWRLLRSLWRLWRKA-cysteamide | VEPEP-6c |
| | | |
| | X₁ is beta-A or S | |
| 29 | Ac-X₁LWRALWRLWRSLWRLWRKA-cysteamide | VEPEP-6d |
| | | |
| | X₁ is beta-A or S | |
| 30 | Ac-X₁LWRALWRLLRALWRLLWKA-cysteamide | VEPEP-6e |
| | | |
| | X₁ is beta-A or S | |
| 31 | Ac-X₁LWRALWRLLRNLWRLLWKA-cysteamide | VEPEP-6f |
| | | |
| | X₁ is beta-A or S | |
| 32 | Ac-X₁LFRALWRₛLLRSₛLWRLLWK-cysteamide | ST-VEPEP-6a |
| | | |
| | X₁ is beta-A or S and the residues followed by an inferior "s" are linked by a hydrocarbon linkage | |
| 33 | Ac-X₁LFLARWRₛLLRSₛLWRLLWK-cysteamide | ST-VEPEP-6aa |
| | | |
| | X₁ is beta-A or S and the residues followed by an inferior "s" are linked by a hydrocarbon linkage | |
| 34 | Ac-X₁LFRALWSₛLLRSₛLWRLLWK-cysteamide | ST-VEPEP-6ab |
| | | |
| | X₁ is beta-A or S and the residues followed by an inferior "s" are linked by a hydrocarbon linkage | |
| 35 | Ac-X₁LFLARWSₛLLRSₛLWRLLWK-cysteamide | ST-VEPEP-6ad |
| | | |
| | X₁ is beta-A or S and the residues followed by an inferior "s" are linked by a hydrocarbon linkage | |
| 36 | Ac-X₁LFRALWRLLRₛSLWSₛLLWK-cysteamide | ST-VEPEP-6b |
| | | |
| | X₁ is beta-A or S and the residues followed by an inferior "s" are linked by a hydrocarbon linkage | |
| 37 | Ac-X₁LFLARWRLLRₛSLWSₛLLWK-cysteamide | ST-VEPEP-6ba |
| | | |
| | X₁ is beta-A or S and the residues followed by an inferior "s" are linked by a hydrocarbon linkage | |
| 38 | Ac-X₁LFRALWRLLSₛSLWSₛLLWK-cysteamide | ST-VEPEP-6bb |
| | | |
| | X₁ is beta-A or S and the residues followed by an inferior "s" are linked by a hydrocarbon linkage | |
| 39 | Ac-X₁LFLARWRLLSₛSLWSₛLLWK-cysteamide | ST-VEPEP-6bd |
| | | |
| | X₁ is beta-A or S and the residues followed by an inferior "s" are linked by a hydrocarbon linkage | |
| 40 | Ac-X₁LFARₛLWRLLRSₛLWRLLWK-cysteamide | ST-VEPEP-6c |
| | | |
| | X₁ is beta-A or S and the residues followed by an inferior "s" are linked by a hydrocarbon linkage | |
| 41 | X₁X₂X₃WWX₄X₅WAX₆X₃X₇X₈X₉X₁₀X₁₁X₁₂WX ₁₃R | VEPEP-91 |
| | | |
| | X₁ is beta-A or S, X₂ is L or none, X₃ is R or none, X₄ is L, R or G, X₅ is R, W or S, X₆ is S, P or T, X₇ is W or P, X₈ is F, A or R, X₉ is S, L, P or R, X₁₀ is R or S, X₁₁ is W or none, X₁₂ is A, R or none and X₁₃ is W or F, and wherein if X₃ is none, then X₂, X₁₁ and X₁₂ are none as well | |
| 42 | X₁X₂RWWLRWAX₆RWX₈X₉X₁₀WX₁₂WX₁₃R | VEPEP-9 2 |
| | | |
| | X₁ is beta-A or S, X₂ is L or none, X₆ is S or P, X₉ is F or A, X₉ is S, L or P, X₁₀ is R or S, X₁₂ is A or R, and X₁₃ is W or F | |
| 43 | X₁LRWWLRWASRWFSRWAWWR | VEPEP9a1 |
| | | |
| | X₁ is beta-A or S | |
| 44 | X₁LRWWLRWASRWASRWAWFR | VEPEP9a2 |
| | | |
| | X₁ is beta-A or S | |
| 45 | X₁RWWLRWASRWALSWRWWR | VEPEP9bl |
| | | |
| | X₁ is beta-A or S | |
| 46 | X₁RWWLRWASRWFLSWRWWR | VEPEP9b2 |
| | | |
| | X₁ is beta-A or S | |
| 47 | X₁RWWLRWAPRWFPSWRWWR | VEPEP9c1 |
| | | |
| | X₁ is beta-A or S | |
| 48 | X₁RWWLRWASRWAPSWRWWR | VEPEP9c2 |
| | | |
| | X₁ is beta-A or S | |
| 49 | X₁WWX₄X₅WAX₆X₇X₈RX₁₀WWR | VEPEP-9 3 |
| | | |
| | X₁ is beta-A or S | |
| 50 | X₁WWRWWASWARSWWR | VEPEP9d |
| | | |
| | X₁ is beta-A or S | |
| 51 | X₁WWGSWATPRRRWWR | VEPEP9e |
| | | |
| | X₁ is beta-A or S | |
| 52 | X₁WWRWWAPWARSWWR | VEPEP9f |
| | | |
| | X₁ is beta-A or S | |
| 53 | X₁KWRSX₂X₃X₄RWRLWRX₅X₆X₇X₈SR | ADGN-100 |
| | | |
| | X₁ is any amino acid or none, and X₂-X₈ are any amino acid | |
| 54 | X₁KWRSX₂X₃X₄RWRLWRX₅X₆X₇X₈SR | ADGN-100 1 |
| | | |
| | X₁ is βA, S, or none, X₂ is A or V, X₃ is G or L, X₄ is W or Y, X₅ is V or S, X₆ is R, V, or A, X₇ is S or L, and X₈ is W or Y | |
| 55 | KWRSAGWRWRLWRVRSWSR | ADGN-100a |
| 56 | KWRSALYRWRLWRVRSWSR | ADGN-100b |
| 57 | KWRSALYRWRLWRSRSWSR | ADGN-100c |
| 58 | KWRSALYRWRLWRSALYSR | ADGN-100d |
| 59 | KWRS_{S}AGWR_{S}WRLWRVRSWSR | ADGN-100 aa |
| | | |
| | the residues marked with a subscript "S" are linked by a hydrocarbon linkage | |
| 60 | KWR_{S}SAGWRWR_{S}LWRVRSWSR | ADGN-100 ab |
| | | |
| | the residues marked with a subscript "S" are linked by a hydrocarbon linkage | |
| 61 | KWRSAGWR_{S}WRLWRVR_{S}SWSR | ADGN-100 ac |
| | | |
| | the residues marked with a subscript "S" are linked by a hydrocarbon linkage | |
| 62 | KWRS_{S}ALYR_{S}WRLWRSRSWSR | ADGN-100 ba |
| | | |
| | the residues marked with a subscript "S" are linked by a hydrocarbon linkage | |
| 63 | KWR_{S}SALYRWR_{S}LWRSRSWSR | ADGN-100 bb |
| | | |
| | the residues marked with a subscript "S" are linked by a hydrocarbon linkage | |
| 64 | KWRSALYR_{S}WRLWRSR_{S}SWSR | ADGN-100 bc |
| | | |
| | the residues marked with a subscript "S" are linked by a hydrocarbon linkage | |
| 65 | KWRSALYRWR_{S}LWRS_{S}RSWSR | ADGN-100 bd |
| | | |
| | the residues marked with a subscript "S" are linked by a hydrocarbon linkage | |
| 66 | KWRSALYRWRLWRS_{S}RSWS_{S}R | ADGN-100 be |
| | | |
| | the residues marked with a subscript "S" are linked by a hydrocarbon linkage | |
| 67 | KWR_{S}SALYRWR_{S}LWRSALYSR | ADGN-100 ca |
| | | |
| | the residues marked with a subscript "S" are linked by a hydrocarbon linkage | |
| 68 | KWRS_{S}ALYR_{S}WRLWRSALYSR | ADGN-100 cb |
| | | |
| | the residues marked with a subscript "S" are linked by a hydrocarbon linkage | |
| 69 | KWRSALYRWR_{S}LWRS_{S}ALYSR | ADGN-100 cc |
| | | |
| | the residues marked with a subscript "S" are linked by a hydrocarbon linkage | |
| 70 | KWRSALYRWRLWRS_{S}ALYS_{S}R | ADGN-100 cd |
| | | |
| | the residues marked with a subscript "S" are linked by a hydrocarbon linkage | |
| 71 | GGGCACGGGCAGCTTGCCGG | EGFP target site |
| 72 | GAGTCCGAGCAGAAGAAGAA | EMX1 target site |
| 73 | GACTGTAAGTGAATTACTT | HPRT target site |
| 74 | GGGGCCACTAGGGACAGGAT | AAVS target site |
| 75 | beta-AKWFERWFREWPRKRR | VEPEP-3a |
| 76 | beta-AKWWERWWREWPRKRR | VEPEP-3b |
| 77 | beta-ALWRALWRLWRSLWRLLWKA | VEPEP-6 |
| 78 | beta-ALRWWLRWASRWFSRWAWWR | VEPEP-9 |
| 79 | beta-AKWRSAGWRWRLWRVRSWSR | ADGN-100a |
| 80 | beta-AKWRSALYRWRLWRVRSWSR | ADGN-100b |
| 81 | GLWRALWRLLRSLWRLLWKV | CADY |
| 82 | GGGGAGGACATCGATGTCACCTCCAATGA C AAGCTTGCTAGC GGTGGGCAACCACAAACCCACGAGGGCA GA | ssDonor template |
| 83 | PKKKRKV | SV40 NLS |
| 84 | KRPAATKKAGQAKKKK | nucleoplasmin NLS |
| 85 | PAAKRVKLD | c-myc NLS 1 |
| 86 | RQRRNELKRSP | c-myc NLS 2 |
| 87 | NQSSNFGPMKGGNFGGRSSGPYGGGGQY FAKPRNQGGY | hRNPA1 M9 NLS |
| 88 | RMRIZFKNKGKDTAELRRRRVEVSVELRKAK KDEQILKRRNV | IBB domain from importin-alpha |
| 89 | VSRKRPRP | myoma T protein NLS 1 |
| 90 | PPKKARED | myoma T protein NLS 2 |
| 91 | PQPKKKPL | p53 NLS |
| 92 | SALIKKKKKMAP | mouse cab1 IV NLS |
| 93 | DRLRR | influenza virus NS1 NLS 1 |
| 94 | PKQKKRK | influenza virus NS1 NLS 2 |
| 95 | RKLKKKIKKL | Hepatitis virus delta antigen NLS |
| 96 | REKKKFLKRR | mouse Mx1 NLS |
| 97 | KRKGDEVDGVDEVAKKKSKK | human poly(ADP-ribose) polymerase NLS |
| 98 | RKCLQAGMNLEARKTKK | steroid hormone receptors (human) glucocorticoid NLS |
| 99 | ACCGCGTGACTTCCACATGAGCG | PD1sgA1 target site |
| 100 | AAACCGCTCATGTGGAAGTCACG | hPD1sgB1 target site |
| 101 | ACCGCAGTTGTGTGACACGGAAG | hPD1sgC1f target site |
| 102 | AAACCTTCCGTGTCACACAACTG | hPD1sgD2 target site |
| 103 | ACAACTTTACCGACCGCGCC | Luciferase target site |
| 104 | GGCACTCGTAGCCGATCTTA | LDLR target site 1 |
| 105 | GTGGCCCAGCGAAGATGCGA | LDLR target site 2 |
| 106 | TCCAGATATCGATGAGTGTC | LDLR target site 3 |
| 107 | AGACTCACCGCACTCTTTGA | LDLR target site 4 |
| 108 | CCAGCTGGACCCCCACACGA | LDLR target site 5 |
| 109 | CCATGTTGCAGACTTTGTCC | LDLR target site 6 |
| 110 | AAACATCGAGAAATTTCAGG | LDLR SAM target site 1 |
| 111 | ACAAATCAAGTCGCCTGCCC | LDLR SAM target site 2 |
| 112 | AGGGGGCGTCAGCTCTTCAC | LDLR SAM target site 3 |
| 113 | CGACCCGATTCAAGCACCTC | ApoB target site 1 |
| 114 | CTGATTCAAGAAGTGCCACC | ApoB target site 2 |
| 115 | AGCTGGCGATGGACCCGCCG | ApoB target site 3 |
| 116 | ATGCCTTACTTGGACAGACC | ApoB target site 4 |
| 117 | CGCCAGCAGCGCCAGCAGCG | ApoB target site 5 |
| 118 | TCTTACAGAAGAGGAAATGC | ApoB target site 6 |
| 119 | CTTTGAGCCTTGAAGAGCCT | ApoB SAM target site 1 |
| 120 | GCAAACAGGTCAGGCCCGGG | ApoB SAM target site 2 |
| 121 | GCTGCGGCTGCATCCCAGGT | ApoB SAM target site 3 |
| 122 | CGCCGACTTGAGCGCGTCCA | LDLRAP1 target site 1 |
| 123 | TGTAGCCACGATCCTCTTGA | LDLRAP1 target site 2 |
| 124 | GCTCCACTAGCGTCATGCCC | LDLRAP1 target site 3 |
| 125 | TCTGTCAGGATAATTCCCCG | LDLRAP1 target site 4 |
| 126 | GGCCATGGACGCGCTCAAGT | LDLRAP1 target site 5 |
| 127 | TGGACACGTTCTCAATGAGC | LDLRAP1 target site 6 |
| 128 | AAACTCTTGGTGCCTGGACC | LDLRAP1 SAM target site 1 |
| 129 | CTGGCGCCTTCGGGCGGCCT | LDLRAP1 SAM target site 2 |
| 130 | GAGGAAAGCGGGGGCGTCTA | LDLRAP1 SAM target site 3 |
| 131 | GGTGCTAGCCTTGCGTTCCG | PCSK9 target site 1 |
| 132 | CTGGCCGAAGCACCCGAGCA | PCSK9 target site 2 |
| 133 | ACTGTATGGTCAGCACACTC | PCSK9 target site 3 |
| 134 | GTCGTGCTGGTCACCGCTGC | PCSK9 target site 4 |
| 135 | CGCCCGCTGCGCCCCAGATG | PCSK9 target site 5 |
| 136 | AGTAGAGGCAGGCATCGTCC | PCSK9 target site 6 |

### ASPECTS OF THE INVENTION

1. A genome-editing complex for modifying a target polynucleotide comprising a cell-penetrating peptide and one or more molecules of a genome-editing system, wherein the cell-penetrating peptide is selected from the group consisting of VEPEP-3 peptides, VEPEP-6 peptides, VEPEP-9 peptides, and ADGN-100 peptides, and wherein the one or more genome-editing system molecules are selected from the group consisting of:
   a) one or both of an RNA-guided endonuclease (RGEN) and a guide RNA (gRNA), wherein the gRNA comprises a guide sequence complementary to a target sequence in the target polynucleotide;
   b) one or both of a DNA-guided endonuclease (DGEN) and a guide DNA (gDNA), wherein the gDNA comprises a guide sequence complementary to a target sequence in the target polynucleotide;
   c) a zinc finger protein (ZFP), wherein the ZFP recognizes a target sequence in the target polynucleotide;
   d) a transcription activator-like effector nuclease (TALEN), wherein the TALEN recognizes a target sequence in the target polynucleotide;
   e) a homing endonuclease, wherein the homing endonuclease recognizes a target sequence in the target polynucleotide; and
   f) an integrase, wherein the integrase recognizes a recombination site in the target polynucleotide.
2. The genome-editing complex of aspect 1, wherein the cell-penetrating peptide is a VEPEP-3 peptide.
3. The genome-editing complex of aspect 2, wherein the cell-penetrating peptide comprises an amino acid sequence selected from the group consisting of SEQ ID NOs: 1-14.
4. The genome-editing complex of aspect 2, wherein the cell-penetrating peptide comprises the amino acid sequence of SEQ ID NO: 75 or 76.
5. The genome-editing complex of aspect 1, wherein the cell-penetrating peptide is a VEPEP-6 peptide.
6. The genome-editing complex of aspect 5, wherein the cell-penetrating peptide comprises an amino acid sequence selected from the group consisting of SEQ ID NOs: 15-40.
7. The genome-editing complex of aspect 5, wherein the cell-penetrating peptide comprises the amino acid sequence of SEQ ID NO: 77.
8. The genome-editing complex of aspect 1, wherein the cell-penetrating peptide is a VEPEP-9 peptide.
9. The genome-editing complex of aspect 8, wherein the cell-penetrating peptide comprises an amino acid sequence selected from the group consisting of SEQ ID NOs: 41-52.
10. The genome-editing complex of aspect 8, wherein the cell-penetrating peptide comprises the amino acid sequence of SEQ ID NO: 78.
11. The genome-editing complex of aspect 1, wherein the cell-penetrating peptide is an ADGN-100 peptide.
12. The genome-editing complex of aspect 11, wherein the cell-penetrating peptide comprises an amino acid sequence selected from the group consisting of SEQ ID NOs: 53-70.
13. The genome-editing complex of aspect 11, wherein the cell-penetrating peptide comprises the amino acid sequence of SEQ ID NO: 79 or 80.
14. The genome-editing complex of any one of aspects 1-13, wherein the cell-penetrating peptide further comprises one or more moieties covalently linked to the N-terminus of the cell-penetrating peptide, and wherein the one or more moieties are selected from the group consisting of an acetyl, a fatty acid, a cholesterol, a poly-ethylene glycol, a nuclear localization signal, nuclear export signal, an antibody, a polysaccharide and a targeting molecule.
15. The genome-editing complex of aspect 14, wherein the cell-penetrating peptide comprises an acetyl group covalently linked to its N-terminus.
16. The genome-editing complex of any one of aspects 1-15, wherein the cell-penetrating peptide further comprises one or more moieties covalently linked to the C-terminus of the cell-penetrating peptide, and wherein the one or more moieties are selected from the group consisting of a cysteamide, a cysteine, a thiol, an amide, a nitrilotriacetic acid optionally substituted, a carboxyl, a linear or ramified C₁-C₆ alkyl optionally substituted, a primary or secondary amine, an osidic derivative, a lipid, a phospholipid, a fatty acid, a cholesterol, a poly-ethylene glycol, a nuclear localization signal, nuclear export signal, an antibody, a polysaccharide and a targeting molecule.
17. The genome-editing complex of aspect 16, wherein the cell-penetrating peptide comprises a cysteamide group covalently linked to its C-terminus.
18. The genome-editing complex of any one of aspects 1-17, wherein at least some of the cell-penetrating peptides in the genome-editing complex are linked to a targeting moiety by a linkage.
19. The genome-editing complex of aspect 18, wherein the linkage is covalent.
20. The genome-editing complex of any one of aspects 1-19, wherein the genome-editing complex comprises one or both of an RGEN and a gRNA.
21. The genome-editing complex of aspect 20, wherein the genome-editing complex comprises an RGEN and a gRNA.
22. The genome-editing complex of aspect 20 or 21, wherein the gRNA is a single guide RNA (sgRNA).
23. The genome-editing complex of any one of aspects 20-22, wherein the RGEN is Cas9.
24. The genome-editing complex of any one of aspects 20-23, wherein the molar ratio of RGEN to gRNA is between about 1:10 and about 10:1.
25. The genome-editing complex of any one of aspects 20-24, wherein the molar ratio of the cell-penetrating peptide to the RGEN is between about 1:1 and about 80:1.
26. The genome-editing complex of aspect 25, wherein the molar ratio of the cell-penetrating peptide to the RGEN is between about 5:1 and about 20:1.
27. The genome-editing complex of any one of aspects 20-26, further comprising one or more additional gRNAs comprising different guide sequences.
28. The genome-editing complex of aspect 27, wherein the target sequence is present in a target gene, and the one or more additional gRNAs individually comprise a guide sequence complementary to a sequence in the target gene.
29. The genome-editing complex of aspect 27, wherein the target sequence is present in a first target gene, and the one or more additional gRNAs individually comprise a guide sequence complementary to a sequence present in one or more additional target genes.
30. The genome-editing complex of any one of aspects 1-19, wherein the genome-editing complex comprises one or both of a DGEN and a gDNA.
31. The genome-editing complex of any one of aspect 20, wherein the genome-editing complex comprises a DGEN and a gDNA.
32. The genome-editing complex of any one of aspects 1-19, wherein the genome-editing complex comprises a ZFP.
33. The genome-editing complex of any one of aspects 1-19, wherein the genome-editing complex comprises a TALEN.
34. The genome-editing complex of any one of aspects 1-19, wherein the genome-editing complex comprises a homing nuclease.
35. The genome-editing complex of any one of aspects 20-34, further comprising a donor nucleic acid for introducing a modification to the target polynucleotide, wherein the donor nucleic acid comprises a sequence corresponding to a portion of the target polynucleotide modified to comprise the modification.
36. The genome-editing complex of aspect 35, wherein the modification is addition, deletion, or substitution of one or more nucleotides in the target polynucleotide.
37. The genome-editing complex of aspect 35 or 36, wherein the donor nucleic acid is a single-stranded DNA oligonucleotide.
38. The genome-editing complex of aspect 35, wherein the modification is insertion of a heterologous nucleic acid in the target polynucleotide.
39. The genome-editing complex of aspect 35 or 38, wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified.
40. The genome-editing complex of any one of aspects 35-39, further comprising one or more additional donor nucleic acids for introducing modifications to one or more additional target polynucleotides.
41. The genome-editing complex of any one of aspects 1-19, wherein the genome-editing complex comprises an integrase.
42. The genome-editing complex of aspect 41, further comprising a donor nucleic acid for insertion into the target polynucleotide, wherein the integrase recognizes a recombination site in the donor nucleic acid and is capable of mediating recombination between the recombination site in the target polynucleotide and the recombination site in the donor nucleic acid to insert the donor nucleic acid into the target polynucleotide.
43. The genome-editing complex of any one of aspects 1-42, wherein the average diameter of the genome-editing complex is between about 10 nm and about 300 nm.
44. A nanoparticle comprising a core comprising the genome-editing complex of any one of aspects 1-43.
45. The nanoparticle of aspect 44, wherein the core further comprises one or more additional genome-editing complexes of any one of aspects 1-43.
46. The nanoparticle of aspect 44 or 45, wherein the core further comprises a donor nucleic acid for introducing a modification to the target polynucleotide comprising a sequence corresponding to a portion of the target polynucleotide modified to comprise the modification, wherein the donor nucleic acid is complexed with a second cell-penetrating peptide.
47. The nanoparticle of aspect 46, wherein the second cell-penetrating peptide is selected from the group consisting of VEPEP-3 peptides, VEPEP-6 peptides, VEPEP-9 peptides, and ADGN-100 peptides.
48. The nanoparticle of aspect 47, wherein the second cell-penetrating peptide comprises an amino acid sequence selected from the group consisting of SEQ ID NOs: 1-70 and 75-80.
49. The nanoparticle of any one of aspects 46-48, wherein the modification is addition, deletion, or substitution of one or more nucleotides in the target polynucleotide.
50. The nanoparticle of aspect 49, wherein the donor nucleic acid is a single-stranded DNA oligonucleotide.
51. The nanoparticle of any one of aspects 46-48, wherein the modification is insertion of a heterologous nucleic acid in the target polynucleotide.
52. The nanoparticle of aspect 51, wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified.
53. The nanoparticle of any one of aspects 46-52, wherein the core further comprises one or more additional donor nucleic acids for introducing modifications to one or more additional target polynucleotides.
54. The nanoparticle of any one of aspects 44-53, wherein at least some of the cell-penetrating peptides in the nanoparticle are linked to a targeting moiety by a linkage.
55. The nanoparticle of any one of aspects 44-53, wherein the core is coated by a shell comprising a peripheral cell-penetrating peptide.
56. The nanoparticle of aspect 55, wherein the peripheral cell-penetrating peptide is selected from the group consisting of VEPEP-3 peptides, VEPEP-6 peptides, VEPEP-9 peptides, and ADGN-100 peptides.
57. The nanoparticle of aspect 56, wherein the peripheral cell-penetrating peptide comprises an amino acid sequence selected from the group consisting of SEQ ID NOs: 1-70 and 75-80.
58. The nanoparticle of any one of aspects 55-57, wherein at least some of the peripheral cell-penetrating peptides in the shell are linked to a targeting moiety by a linkage.
59. The nanoparticle of aspect 54 or 58, wherein the linkage is covalent.
60. The nanoparticle of any one of aspects 44-59, wherein the average diameter of the nanoparticle is between about 10 nm and about 400 nm.
61. A pharmaceutical composition comprising the genome-editing complex of any one of aspects 1-43 or the nanoparticle of any one of aspects 44-60, and a pharmaceutically acceptable carrier.
62. The pharmaceutical composition of aspect 61, further comprising an expression complex comprising a third cell-penetrating peptide and a nucleic acid molecule encoding an exogenous protein.
63. The pharmaceutical composition of aspect 62, wherein the exogenous protein is a recombinant receptor capable of being expressed on the surface of a cell.
64. The pharmaceutical composition of aspect 63, wherein the recombinant receptor is a chimeric antigen receptor (CAR).
65. The pharmaceutical composition of any one of aspects 62-64, wherein the third cell-penetrating peptide is selected from the group consisting of VEPEP-3 peptides, VEPEP-6 peptides, VEPEP-9 peptides, and ADGN-100 peptides.
66. The pharmaceutical composition of aspect 65, wherein the third cell-penetrating peptide comprises an amino acid sequence selected from the group consisting of SEQ ID NOs: 1-70 and 75-80.
67. A method of preparing the genome-editing complex of any one of aspects 1-19, comprising combining the cell-penetrating peptide with the one or more genome-editing system molecules, thereby forming the genome-editing complex.
68. The method of aspect 67, wherein the one or more genome-editing system molecules include an RGEN and a gRNA, and wherein the cell-penetrating peptide and the RGEN are combined at a ratio from about 1:1 to about 80:1, respectively, and the RGEN and the gRNA are combined at a ratio from about 10:1 to about 1:10, respectively.
69. The method of aspect 68, wherein the cell-penetrating peptide and the RGEN are combined at a ratio from about 5:1 to about 20:1, respectively.
70. The method of aspect 68 or 69, wherein the RGEN and the gRNA are combined at a ratio of about 1:1.
71. A method of delivering one or more molecules of a genome-editing system into a cell, comprising contacting the cell with the genome-editing complex of any one of aspects 1-43 or the nanoparticle of any one of aspects 44-60, wherein the genome-editing complex or the nanoparticle comprises the one or more genome-editing system molecules.
72. The method of aspect 71, wherein the contacting of the cell with the genome-editing complex or nanoparticle is carried out *in vivo.*
73. The method of aspect 71, wherein the contacting of the cell with the genome-editing complex or nanoparticle is carried out *ex vivo.*
74. The method of aspect 71, wherein the contacting of the cell with the genome-editing complex or nanoparticle is carried out *in vitro.*
75. The method of any one of aspects 71-74, wherein the cell is a granulocyte, a mast cell, a monocyte, a dendritic cell, a B cell, a T cell, a natural killer cell, a fibroblast, a hepatocyte, a lung progenitor cell, or a neuronal cell.
76. The method of aspect 75, wherein the cell is a T cell.
77. The method of aspect 75 or 76, wherein the genome-editing system targets a sequence in a gene selected from the group consisting of PD-1, PD-L1, PD-L2, TIM-3, BTLA, VISTA, LAG-3, CTLA-4, TIGIT, 4-1BB, OX40, CD27, TIM-1, CD28, HVEM, GITR, and ICOS.
78. The method of any one of aspects 71-77, further comprising contacting the cell with an expression complex comprising a fourth cell-penetrating peptide and a nucleic acid molecule encoding an exogenous protein.
79. The method of aspect 78, wherein the exogenous protein is a recombinant receptor capable of being expressed on the surface of a cell.
80. The method of aspect 79, wherein the recombinant receptor is a chimeric antigen receptor (CAR).
81. The method of any one of aspects 78-80, wherein the fourth cell-penetrating peptide is selected from the group consisting of VEPEP-3 peptides, VEPEP-6 peptides, VEPEP-9 peptides, and ADGN-100 peptides.
82. The method of aspect 81, wherein the fourth cell-penetrating peptide comprises an amino acid sequence selected from the group consisting of SEQ ID NOs: 1-70 and 75-80.
83. A method of modifying a target polynucleotide in a cell, comprising contacting the cell with the genome-editing complex of any one of aspects 1-43 or the nanoparticle of any one of aspects 44-60, wherein the genome-editing complex or the nanoparticle comprises one or more molecules of a genome-editing system that targets a sequence in the target polynucleotide.
84. A method of treating a disease in an individual comprising administering to the individual an effective amount of the pharmaceutical composition of any one of aspects 61-66.
85. The method of aspect 84, wherein the disease is selected from the group consisting of cancer, diabetes, inflammatory diseases, fibrosis, viral infectious diseases, hereditary diseases, ocular diseases, liver diseases, lung diseases, kidney diseases, aging and degenerative diseases, and diseases characterized by cholesterol level abnormality.
86. The method of aspect 85, wherein the disease is cancer.
87. The method of aspect 86, wherein the cancer is a solid tumor, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis.
88. The method of aspect 87, wherein the cancer is cancer of the liver, lung, or kidney.
89. The method of aspect 86, wherein the cancer is a hematological malignancy, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis.
90. The method of aspect 85, wherein the disease is a viral infection disease, and wherein the pharmaceutical composition comprises a genome-editing complex or nanoparticle comprising one or more molecules of a genome-editing system that modulates the expression of one or more proteins involved in the viral infectious disease development and/or progression.
91. The method of aspect 85, wherein the disease is a hereditary disease, and wherein the pharmaceutical composition comprises a genome-editing complex or nanoparticle comprising one or more molecules of a genome-editing system that modulates the expression of one or more proteins involved in the hereditary disease development and/or progression.
92. The method of aspect 85, wherein the disease is an aging or degenerative disease, and wherein the pharmaceutical composition comprises a genome-editing complex or nanoparticle comprising one or more molecules of a genome-editing system that modulates the expression of one or more proteins involved in the aging or degenerative disease development and/or progression.
93. The method of aspect 85, wherein the disease is a fibrotic or inflammatory disease, and wherein the pharmaceutical composition comprises a genome-editing complex or nanoparticle comprising one or more molecules of a genome-editing system that modulates the expression of two or more proteins involved in the fibrotic or inflammatory disease development and/or progression.
94. The method of any one of aspects 84-93, wherein the individual is human.
95. A kit comprising a composition comprising the genome-editing complex of any one of aspects 1-43 and/or the nanoparticle of any one of aspects 44-60.

## Claims

1. A genome-editing complex for modifying a target polynucleotide comprising a cell-penetrating peptide and one or more molecules of a genome-editing system, wherein the cell-penetrating peptide is an VEPEP-6 peptide, and wherein the one or more genome-editing system molecules are selected from the group consisting of:
a) one or both of an RNA-guided endonuclease (RGEN) and a guide RNA (gRNA), wherein the gRNA comprises a guide sequence complementary to a target sequence in the target polynucleotide;
b) one or both of a DNA-guided endonuclease (DGEN) and a guide DNA (gDNA), wherein the gDNA comprises a guide sequence complementary to a target sequence in the target polynucleotide;
c) a zinc finger protein (ZFP), wherein the ZFP recognizes a target sequence in the target polynucleotide;
d) a transcription activator-like effector nuclease (TALEN), wherein the TALEN recognizes a target sequence in the target polynucleotide;
e) a homing endonuclease, wherein the homing endonuclease recognizes a target sequence in the target polynucleotide; and
f) an integrase, wherein the integrase recognizes a recombination site in the target polynucleotide.

2. The genome-editing complex of claim 1, wherein:
a) the cell-penetrating peptide comprises:
i) an amino acid sequence selected from the group consisting of SEQ ID NOs: 15-40; or
ii) the amino acid sequence of SEQ ID NO: 77; and/or
b) the cell-penetrating peptide further comprises one or more moieties covalently linked to the N-terminus of the cell-penetrating peptide, and wherein the one or more moieties are selected from the group consisting of an acetyl, a fatty acid, a cholesterol, a poly-ethylene glycol, a nuclear localization signal, nuclear export signal, an antibody, a polysaccharide and a targeting molecule, optionally wherein the cell-penetrating peptide comprises an acetyl group covalently linked to its N-terminus; and/or
c) the cell-penetrating peptide further comprises one or more moieties covalently linked to the C-terminus of the cell-penetrating peptide, and wherein the one or more moieties are selected from the group consisting of a cysteamide, a cysteine, a thiol, an amide, a nitrilotriacetic acid optionally substituted, a carboxyl, a linear or ramified C₁-C₆ alkyl optionally substituted, a primary or secondary amine, an osidic derivative, a lipid, a phospholipid, a fatty acid, a cholesterol, a poly-ethylene glycol, a nuclear localization signal, nuclear export signal, an antibody, a polysaccharide and a targeting molecule, optionally wherein the cell-penetrating peptide comprises a cysteamide group covalently linked to its C-terminus; and/or
d) at least some of the cell-penetrating peptides in the genome-editing complex are linked to a targeting moiety by a linkage, optionally wherein the linkage is covalent; and/or
e) the genome-editing complex comprises:
i) one or both of a DGEN and a gDNA; or
ii) a ZFP; or
iii) a TALEN; or
iv) a homing nuclease.

3. The genome-editing complex of any one of claims 1-2d, wherein the genome-editing complex comprises one or both of an RGEN and a gRNA, optionally:
a) wherein the gRNA is a single guide RNA (sgRNA); and/or
b) wherein the RGEN is Cas9; and/or
c) wherein the molar ratio of RGEN to gRNA is between about 1:10 and about 10:1; and/or
d) wherein the molar ratio of the cell-penetrating peptide to the RGEN is between about 1:1 and about 80:1, optionally wherein the molar ratio of the cell-penetrating peptide to the RGEN is between about 5:1 and about 20:1; and/or
e) further comprising one or more additional gRNAs comprising different guide sequences, optionally wherein the target sequence is present:
i) in a target gene, and the one or more additional gRNAs individually comprise a guide sequence complementary to a sequence in the target gene; or
ii) in a first target gene, and the one or more additional gRNAs individually comprise a guide sequence complementary to a sequence present in one or more additional target genes.

4. The genome-editing complex of any one of claims 2e-3, further comprising a donor nucleic acid for introducing a modification to the target polynucleotide, wherein the donor nucleic acid comprises a sequence corresponding to a portion of the target polynucleotide modified to comprise the modification, optionally:
a) wherein the modification is addition, deletion, or substitution of one or more nucleotides in the target polynucleotide; and/or
b) wherein the donor nucleic acid is a single-stranded DNA oligonucleotide; or
c) wherein the modification is insertion of a heterologous nucleic acid in the target polynucleotide; and/or
d) wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified; and/or
e) further comprising one or more additional donor nucleic acids for introducing modifications to one or more additional target polynucleotides.

5. The genome-editing complex of any one of claims 1-2d, wherein the genome-editing complex comprises an integrase, optionally further comprising a donor nucleic acid for insertion into the target polynucleotide, wherein the integrase recognizes a recombination site in the donor nucleic acid and is capable of mediating recombination between the recombination site in the target polynucleotide and the recombination site in the donor nucleic acid to insert the donor nucleic acid into the target polynucleotide.

6. The genome-editing complex of any one of claims 1-5, wherein the average diameter of the genome-editing complex is between about 10 nm and about 300 nm.

7. A nanoparticle comprising a core comprising the genome-editing complex of any one of claims 1-6.

8. The nanoparticle of claim 7:
a) wherein the core further comprises one or more additional genome-editing complexes of any one of claims 1-6; and/or
b) wherein the core further comprises a donor nucleic acid for introducing a modification to the target polynucleotide comprising a sequence corresponding to a portion of the target polynucleotide modified to comprise the modification, wherein the donor nucleic acid is complexed with a second cell-penetrating peptide, optionally wherein:
i) the second cell-penetrating peptide is selected from the group consisting of VEPEP-3 peptides, VEPEP-6 peptides, VEPEP-9 peptides, and ADGN-100 peptides, optionally wherein the second cell-penetrating peptide comprises an amino acid sequence selected from the group consisting of SEQ ID NOs: 1-70 and 75-80; and/or
ii) wherein the modification is addition, deletion, or substitution of one or more nucleotides in the target polynucleotide, optionally wherein the donor nucleic acid is a single-stranded DNA oligonucleotide; or
iii) wherein the modification is insertion of a heterologous nucleic acid in the target polynucleotide, optionally wherein the donor nucleic acid is a double-stranded DNA comprising the heterologous nucleic acid flanked by a 5' homology arm and a 3' homology arm, and wherein the 5' homology arm and the 3' homology arm are homologous to sequences flanking the region of the target polynucleotide to be modified; and/or
iv) wherein the core further comprises one or more additional donor nucleic acids for introducing modifications to one or more additional target polynucleotides; and/or
c) wherein:
i) at least some of the cell-penetrating peptides in the nanoparticle are linked to a targeting moiety by a linkage, optionally wherein the linkage is covalent; or
ii) the core is coated by a shell comprising a peripheral cell-penetrating peptide, optionally wherein:
1) the peripheral cell-penetrating peptide is selected from the group consisting of VEPEP-3 peptides, VEPEP-6 peptides, VEPEP-9 peptides, and ADGN-100 peptides, optionally wherein the peripheral cell-penetrating peptide comprises an amino acid sequence selected from the group consisting of SEQ ID NOs: 1-70 and 75-80; and/or
2) at least some of the peripheral cell-penetrating peptides in the shell are linked to a targeting moiety by a linkage, optionally wherein the linkage is covalent; and/or
d) wherein the average diameter of the nanoparticle is between about 10 nm and about 400 nm.

9. A pharmaceutical composition comprising the genome-editing complex of any one of claims 1-6, or the nanoparticle of claim 7 or 8, and a pharmaceutically acceptable carrier, optionally further comprising an expression complex comprising a third cell-penetrating peptide and a nucleic acid molecule encoding an exogenous protein, optionally wherein:
a) the exogenous protein is a recombinant receptor capable of being expressed on the surface of a cell, optionally wherein the recombinant receptor is a chimeric antigen receptor (CAR); and/or
b) wherein the third cell-penetrating peptide is selected from the group consisting of VEPEP-3 peptides, VEPEP-6 peptides, VEPEP-9 peptides, and ADGN-100 peptides, optionally wherein the third cell-penetrating peptide comprises an amino acid sequence selected from the group consisting of SEQ ID NOs: 1-70 and 75-80.

10. A method of preparing the genome-editing complex of any one of claims 1-2d, comprising combining the cell-penetrating peptide with the one or more genome-editing system molecules, thereby forming the genome-editing complex, optionally wherein:
a) the one or more genome-editing system molecules include an RGEN and a gRNA, and wherein the cell-penetrating peptide and the RGEN are combined at a ratio from about 1:1 to about 80:1, respectively, and the RGEN and the gRNA are combined at a ratio from about 10:1 to about 1:10, respectively; optionally wherein the cell-penetrating peptide and the RGEN are combined at a ratio from about 5:1 to about 20:1, respectively; and/or
b) the RGEN and the gRNA are combined at a ratio of about 1:1.

11. An *ex vivo* or *in vitro* method of delivering one or more molecules of a genome-editing system into a cell, comprising contacting the cell with the genome-editing complex of any one of claims 1-6 or the nanoparticle of claim 7 or 8, wherein the genome-editing complex or the nanoparticle comprises the one or more genome-editing system molecules, optionally:
a) wherein the cell is a granulocyte, a mast cell, a monocyte, a dendritic cell, a B cell, a T cell, a natural killer cell, a fibroblast, a hepatocyte, a lung progenitor cell, or a neuronal cell, optionally wherein the cell is a T cell; and/or
b) wherein the genome-editing system targets a sequence in a gene selected from the group consisting of PD-1, PD-L1, PD-L2, TIM-3, BTLA, VISTA, LAG-3, CTLA-4, TIGIT, 4-1BB, OX40, CD27, TIM-1, CD28, HVEM, GITR, and ICOS; and/or
c) further comprising contacting the cell with an expression complex comprising a fourth cell-penetrating peptide and a nucleic acid molecule encoding an exogenous protein, optionally wherein:
i) the exogenous protein is a recombinant receptor capable of being expressed on the surface of a cell, optionally wherein the recombinant receptor is a chimeric antigen receptor (CAR); and/or
ii) the fourth cell-penetrating peptide is selected from the group consisting of VEPEP-3 peptides, VEPEP-6 peptides, VEPEP-9 peptides, and ADGN-100 peptides, optionally wherein the fourth cell-penetrating peptide comprises an amino acid sequence selected from the group consisting of SEQ ID NOs: 1-70 and 75-80.

12. An *ex vivo* or *in vitro* method of modifying a target polynucleotide in a cell, comprising contacting the cell with the genome-editing complex of any one of claims 1-6 or the nanoparticle of claim 7 or 8, wherein the genome-editing complex or the nanoparticle comprises one or more molecules of a genome-editing system that targets a sequence in the target polynucleotide.

13. The pharmaceutical composition of claim 9, for use in a method of treating a disease in an individual comprising administering an effective amount of said composition to the individual.

14. The composition for use according to claim 13, wherein the disease is selected from the group consisting of cancer, diabetes, inflammatory diseases, fibrosis, viral infectious diseases, hereditary diseases, ocular diseases, liver diseases, lung diseases, kidney diseases, aging and degenerative diseases, and diseases **characterized by** cholesterol level abnormality; optionally wherein:
a) the disease is cancer, optionally wherein:
i) the cancer is a solid tumor, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis, optionally wherein the cancer is cancer of the liver, lung, or kidney; or
ii) the cancer is a hematological malignancy, and wherein the pharmaceutical composition comprises a genome-editing complex or nanoparticle comprising one or more molecules of a genome-editing system that modulates the expression of one or more proteins selected from the group consisting of growth factors and cytokines, cell surface receptors, signaling molecules and kinases, transcription factors and other modulators of transcription, regulators of protein expression and modification, tumor suppressors, and regulators of apoptosis and metastasis; or
b) the disease is a viral infection disease, and wherein the pharmaceutical composition comprises a genome-editing complex or nanoparticle comprising one or more molecules of a genome-editing system that modulates the expression of one or more proteins involved in the viral infectious disease development and/or progression; or
c) the disease is a hereditary disease, and wherein the pharmaceutical composition comprises a genome-editing complex or nanoparticle comprising one or more molecules of a genome-editing system that modulates the expression of one or more proteins involved in the hereditary disease development and/or progression; or
d) the disease is an aging or degenerative disease, and wherein the pharmaceutical composition comprises a genome-editing complex or nanoparticle comprising one or more molecules of a genome-editing system that modulates the expression of one or more proteins involved in the aging or degenerative disease development and/or progression; or
e) the disease is a fibrotic or inflammatory disease, and wherein the pharmaceutical composition comprises a genome-editing complex or nanoparticle comprising one or more molecules of a genome-editing system that modulates the expression of two or more proteins involved in the fibrotic or inflammatory disease development and/or progression; and/or
f) the individual is human.

15. A kit comprising a composition comprising the genome-editing complex of any one of claims 1-6 and/or the nanoparticle of claim 7 or 8.


SEQUENCE LISTING

<110> AADIGEN, LLC .
      DIVITA, Grilles
      DESAI, Niel

<120> PEPTIDES AND NANOPARTICLES FOR INTRACELLULAR
DELIVERY OF GENOME-EDITING MOLECULES

<130> 737372000840

<140> Not Yet Assigned
<141> Concurrently Herewith

<150> 62/477,357
<151> 2017-03-27

<150> 62/394,140
<151> 2016-09-13

<150> 62/394,140
<151> 2016-05-27

<160> 136

<170> FastSEQ for Windows Version 4.0

<210> 1
<211> 17
<212> PRT
<213> Artificial Sequence

<220>
<223> Synthetic construct VEPEP-3

<220>
<221> VARIANT
<222> 1
<223> Xaa = bAla or Ser

<220>
<221> VARIANT
<222> 2,6
<223> Xaa = Lys, Arg or Leu

<220>
<221> VARIANT
<222> 3,7,11
<223> Xaa = Phe or Trp

<220>
<221> VARIANT
<222> 4,8
<223> Xaa =  Phe, Trp or Tyr

<220>
<221> VARIANT
<222> 5
<223> Xaa = Glu, Arg or Ser

<220>

<221> VARIANT
<222> 9
<223> Xaa = Arg, Thr or Ser

<220>
<221> VARIANT
<222> 10
<223> Xaa = Glu, Arg, or Ser

<220>
<221> VARIANT
<222> 12
<223> Xaa = Phe or Trp or absent

<220>
<221> VARIANT
<222> 13
<223> Xaa = Pro or Arg

<220>
<221> VARIANT
<222> 14
<223> Xaa = Arg or Leu

<220>
<221> VARIANT
<222> 15
<223> Xaa = Lys, Trp or Arg

<220>
<221> VARIANT
<222> 16
<223> Xaa = Arg or Phe

<220>
<221> VARIANT
<222> 17
<223> Xaa = Arg or Lys

<400> 1
Xaa Xaa Xaa Xaa Xaa Xaa Xaa Xaa Xaa Xaa Xaa Xaa Xaa Xaa Xaa Xaa
1               5                   10                  15
Xaa


<210> 2
<211> 16
<212> PRT
<213> Artificial Sequence

<220>
<223> Synthetic construct VEPEP-3 1

<220>
<221> VARIANT
<222> 1
<223> Xaa = bAla or Ser

<220>
<221> VARIANT
<222> 2,6
<223> Xaa = Arg or Lys

<220>
<221> VARIANT
<222> 4,8
<223> Xaa = Phe, Trp, or Tyr

<220>
<221> VARIANT
<222> 11
<223> Xaa = Trp or Phe

<220>
<221> VARIANT
<222> 9
<223> Xaa = Thr or Arg

<220>
<221> VARIANT
<222> 10
<223> Xaa = Glu or Arg

<220>
<221> VARIANT
<222> 14
<223> Xaa = Arg or Lys

<220>
<221> VARIANT
<222> 16
<223> Xaa = Arg or Lys

<400> 2
Xaa Xaa Trp Xaa Glu Xaa Trp Xaa Xaa Xaa Xaa Pro Arg Xaa Arg Xaa
1               5                   10                  15


<210> 3
<211> 16
<212> PRT
<213> Artificial Sequence

<220>
<223> Synthetic construct VEPEP-3 1a


<220>
<221> VARIANT
<222> 1
<223> Xaa = bAla or Ser

<400> 3
Xaa Lys Trp Phe Glu Arg Trp Phe Arg Glu Trp Pro Arg Lys Arg Arg
1               5                   10                  15


<210> 4
<211> 16
<212> PRT
<213> Artificial Sequence

<220>
<223> Synthetic construct VEPEP-3 1b

<220>

<221> VARIANT
<222> 1
<223> Xaa = bAla or Ser

<400> 4
Xaa Lys Trp Trp Glu Arg Trp Trp Arg Glu Trp Pro Arg Lys Arg Arg
1               5                   10                  15


<210> 5
<211> 16
<212> PRT
<213> Artificial Sequence

<220>
<223> Synthetic construct VEPEP-3 1c

<220>
<221> VARIANT
<222> 1
<223> Xaa = bAla or Ser

<400> 5
Xaa Lys Trp Trp Glu Arg Trp Trp Arg Glu Trp Pro Arg Lys Arg Lys
1               5                   10                  15


<210> 6
<211> 16
<212> PRT
<213> Artificial Sequence

<220>
<223> Synthetic construct VEPEP-3 1d

<220>
<221> VARIANT
<222> 1
<223> Xaa = bAla or Ser

<400> 6
Xaa Arg Trp Trp Glu Lys Trp Trp Thr Arg Trp Pro Arg Lys Arg Lys
1               5                   10                  15


<210> 7
<211> 16
<212> PRT
<213> Artificial Sequence

<220>
<223> Synthetic construct VEPEP-3 1e

<220>
<221> VARIANT
<222> 1
<223> Xaa = bAla or Ser

<400> 7
Xaa Arg Trp Tyr Glu Lys Trp Tyr Thr Glu Phe Pro Arg Arg Arg Arg
1               5                   10                  15

<210> 8
<211> 17
<212> PRT
<213> Artificial Sequence

<220>
<223> Synthetic construct VEPEP-3 1S

<220>
<221> VARIANT
<222> 1
<223> Xaa = bAla or Ser

<220>
<221> VARIANT
<222> 3, 11
<223> Xaa = any non-natural amino acid

<220>
<221> Misc_feature
<222> 3,11
<223> Hydrocarbon linkage

<400> 8
Xaa Lys Xaa Trp Trp Glu Arg Trp Trp Arg Xaa Trp Pro Arg Lys Arg
 1               5                   10                  15
Lys

<210> 9
<211> 17
<212> PRT
<213> Artificial Sequence

<220>
<223> Synthetic construct VEPEP-3 2

<220>
<221> VARIANT
<222> 1
<223> Xaa = bAla or Ser

<220>
<221> VARIANT
<222> 2
<223> Xaa = Lys, Arg or Leu

<220>
<221> VARIANT
<222> 3,7
<223> Xaa = Phe or Trp

<220>
<221> VARIANT
<222> 5
<223> Xaa = Arg or Ser

<220>
<221> VARIANT
<222> 6, 14
<223> Xaa = Leu or Arg

<220>
<221> VARIANT
<222> 9
<223> Xaa = Arg or Ser

<220>
<221> VARIANT
<222> 10
<223> Xaa = Arg or Ser

<220>
<221> VARIANT
<222> 12
<223> Xaa = Phe or Trp

<220>
<221> VARIANT
<222> 13
<223> Xaa = Arg or Pro

<220>
<221> VARIANT
<222> 16
<223> Xaa = Arg or Phe


<400> 9
Xaa Xaa Xaa Trp Xaa Xaa Xaa Trp Xaa Xaa Trp Xaa Xaa Xaa Trp Xaa
 1               5                   10                  15
Arg




<210> 10
<211> 17
<212> PRT
<213> Artificial Sequence

<220>
<223> Synthetic construct VEPEP-3 2a

<220>
<221> VARIANT
<222> 1
<223> Xaa = bAla or Ser

<400> 10
Xaa Arg Trp Trp Arg Leu Trp Trp Arg Ser Trp Phe Arg Leu Trp Arg
 1               5                   10                  15
Arg




<210> 11
<211> 17
<212> PRT
<213> Artificial Sequence

<220>
<223> Synthetic construct VEPEP-3 2b

<220>
<221> VARIANT

<222> 1
<223> Xaa = bAla or Ser

<400> 11
Xaa Leu Trp Trp Arg Arg Trp Trp Ser Arg Trp Trp Pro Arg Trp Arg
1               5                   10                  15
Arg

<210> 12
<211> 17
<212> PRT
<213> Artificial Sequence

<220>
<223> Synthetic construct VEPEP-3 2c

<220>
<221> VARIANT
<222> 1
<223> Xaa = bAla or Ser

<400> 12
Xaa Leu Trp Trp Ser Arg Trp Trp Arg Ser Trp Phe Arg Leu Trp Phe
1               5                   10                  15
Arg

<210> 13
<211> 17
<212> PRT
<213> Artificial Sequence

<220>
<223> Synthetic construct VEPEP-3 2d

<220>
<221> VARIANT
<222> 1
<223> Xaa = bAla or Ser

<400> 13
Xaa Lys Phe Trp Ser Arg Phe Trp Arg Ser Trp Phe Arg Leu Trp Arg
1               5                   10                  15
Arg

<210> 14
<211> 17
<212> PRT
<213> Artificial Sequence

<220>
<223> Synthetic construct VEPEP-3 2S

<220>
<221> VARIANT
<222> 1
<223> Xaa = bAla or Ser

<220>
<221> VARIANT
<222> 5, 12
<223> Xaa = Any non-natural amino acid

<220>
<221> Misc_feature
<222> 5, 12
<223> Hydrocarbon linkage

<400> 14
Xaa Arg Trp Trp Xaa Leu Trp Trp Arg Ser Trp Xaa Arg Leu Trp Arg
 1               5                   10                  15
Arg




<210> 15
<211> 20
<212> PRT
<213> Artificial Sequence

<220>
<223> Synthetic construct VEPEP-6 1

<220>
<221> VARIANT
<222> 1
<223> Xaa = bAla or Ser

<220>
<221> VARIANT
<222> 3
<223> Xaa = Phe or Trp

<220>
<221> VARIANT
<222> 8, 11, 15
<223> Xaa = Arg or Ser

<220>
<221> VARIANT
<222> 10
<223> Xaa = Leu, Trp , Cys or Ile

<220>
<221> VARIANT
<222> 12
<223> Xaa = Ser, Ala, Asn or Thr

<220>
<221> VARIANT
<222> 17
<223> Xaa = Leu or Trp

<220>
<221> VARIANT
<222> 18
<223> Xaa = Trp or Arg

<220>
<221> VARIANT
<222> 19

<223> Xaa = Lys or Arg

<220>
<221> VARIANT
<222> 20
<223> Xaa = Ala or absent

<400> 15
Xaa Leu Xaa Arg Ala Leu Trp Xaa Leu Xaa Xaa Xaa Leu Trp Xaa Leu
1               5                   10                  15
Xaa Xaa Xaa Xaa
            20


<210> 16
<211> 20
<212> PRT
<213> Artificial Sequence

<220>
<223> Synthetic construct VEPEP-6 2

<220>
<221> VARIANT
<222> 1
<223> Xaa = bAla or Ser

<220>
<221> VARIANT
<222> 3
<223> Xaa = Phe or Trp

<220>
<221> VARIANT
<222> 8, 11, 15
<223> Xaa = Arg or Ser

<220>
<221> VARIANT
<222> 10
<223> Xaa = Leu, Trp , Cys or Ile

<220>
<221> VARIANT
<222> 12
<223> Xaa = Ser, Ala, Asn or Thr

<220>
<221> VARIANT
<222> 17
<223> Xaa = Leu or Trp

<220>
<221> VARIANT
<222> 18
<223> Xaa = Trp or Arg

<220>
<221> VARIANT
<222> 19
<223> Xaa = Lys or Arg

<220>

<221> VARIANT
<222> 20
<223> Xaa = Ala or absent

<400> 16
Xaa Leu Xaa Leu Ala Arg Trp Xaa Leu Xaa Xaa Xaa Leu Trp Xaa Leu
 1               5                   10                  15
Xaa Xaa Xaa Xaa
            20


<210> 17
<211> 20
<212> PRT
<213> Artificial Sequence

<220>
<223> Synthetic construct VEPEP-6 3


<220>
<221> VARIANT
<222> 1
<223> Xaa = bAla or Ser

<220>
<221> VARIANT
<222> 3
<223> Xaa = Phe or Trp

<220>
<221> VARIANT
<222> 8, 11, 15
<223> Xaa = Arg or Ser

<220>
<221> VARIANT
<222> 10
<223> Xaa = Leu, Trp , Cys or Ile

<220>
<221> VARIANT
<222> 12
<223> Xaa = Ser, Ala, Asn or Thr

<220>
<221> VARIANT
<222> 17
<223> Xaa = Leu or Trp

<220>
<221> VARIANT
<222> 18
<223> Xaa = Trp or Arg

<220>
<221> VARIANT
<222> 19
<223> Xaa = Lys or Arg

<220>
<221> VARIANT
<222> 20

<223> Xaa = Ala or absent

<400> 17
Xaa Leu Xaa Ala Arg Leu Trp Xaa Leu Xaa Xaa Xaa Leu Trp Xaa Leu
1               5                   10                  15
Xaa Xaa Xaa Xaa
            20

<210> 18
<211> 20
<212> PRT
<213> Artificial Sequence

<220>
<223> Synthetic construct VEPEP-6 4

<220>
<221> VARIANT
<222> 1
<223> Xaa = bAla or Ser

<220>
<221> VARIANT
<222> 3
<223> Xaa = Phe or Trp

<220>
<221> VARIANT
<222> 10
<223> Xaa = Leu, Trp , Cys or Ile

<220>
<221> VARIANT
<222> 12
<223> Xaa = Ser, Ala, Asn or Thr

<220>
<221> VARIANT
<222> 17
<223> Xaa = Leu or Trp

<220>
<221> VARIANT
<222> 18
<223> Xaa = Trp or Arg

<220>
<221> VARIANT
<222> 19
<223> Xaa = Lys or Arg

<220>
<221> VARIANT
<222> 20
<223> Xaa = Ala or absent

<400> 18
Xaa Leu Xaa Arg Ala Leu Trp Arg Leu Xaa Arg Xaa Leu Trp Arg Leu
1               5                   10                  15
Xaa Xaa Xaa Xaa
            20

<210> 19
<211> 20
<212> PRT
<213> Artificial Sequence

<220>
<223> Synthetic construct VEPEP-6 5

<220>
<221> VARIANT
<222> 1
<223> Xaa = bAla or Ser

<220>
<221> VARIANT
<222> 3
<223> Xaa = Phe or Trp

<220>
<221> VARIANT
<222> 10
<223> Xaa = Leu or Trp

<220>
<221> VARIANT
<222> 12
<223> Xaa = Ser, Ala or Asn

<220>
<221> VARIANT
<222> 17
<223> Xaa = Leu or Trp

<220>
<221> VARIANT
<222> 18
<223> Xaa = Trp or Arg

<220>
<221> VARIANT
<222> 20
<223> Xaa = Ala or absent


<400> 19
Xaa Leu Xaa Arg Ala Leu Trp Arg Leu Xaa Arg Xaa Leu Trp Arg Leu
 1               5                   10                  15
Xaa Xaa Lys Xaa
            20


<210> 20
<211> 19
<212> PRT
<213> Artificial Sequence

<220>
<223> Synthetic construct VEPEP-6 6

<220>
<221> VARIANT

<222> 1
<223> Xaa = bAla or Ser

<220>
<221> VARIANT
<222> 12
<223> Xaa = Ser or Thr

<220>
<221> VARIANT
<222> 19
<223> Xaa = Lys or Arg

<400> 20
Xaa Leu Phe Arg Ala Leu Trp Arg Leu Leu Arg Xaa Leu Trp Arg Leu
 1               5                   10                  15
Leu Trp Xaa

<210> 21
<211> 20
<212> PRT
<213> Artificial Sequence

<220>
<223> Synthetic construct VEPEP-6 7

<220>
<221> VARIANT
<222> 1
<223> Xaa = bAla or Ser

<220>
<221> VARIANT
<222> 12
<223> Xaa = Ser or Thr

<220>
<221> VARIANT
<222> 19
<223> Xaa = Lys or Arg

<400> 21
Xaa Leu Trp Arg Ala Leu Trp Arg Leu Trp Arg Xaa Leu Trp Arg Leu
 1               5                   10                  15
Leu Trp Xaa Ala
            20

<210> 22
<211> 20
<212> PRT
<213> Artificial Sequence

<220>
<223> Synthetic construct VEPEP-6 8

<220>
<221> VARIANT
<222> 1
<223> Xaa = bAla or Ser

<220>
<221> VARIANT
<222> 10
<223> Xaa = Leu, Cys or Ile

<220>
<221> VARIANT
<222> 12
<223> Xaa = Ser or Thr

<220>
<221> VARIANT
<222> 19
<223> Xaa = Lys or Arg

<400> 22
Xaa Leu Trp Arg Ala Leu Trp Arg Leu Xaa Arg Xaa Leu Trp Arg Leu
1               5                   10                  15
Trp Arg Xaa Ala
            20


<210> 23
<211> 20
<212> PRT
<213> Artificial Sequence

<220>
<223> Synthetic construct VEPEP-6 9

<220>
<221> VARIANT
<222> 1
<223> Xaa = bAla or Ser

<220>
<221> VARIANT
<222> 12
<223> Xaa = Ser or Thr

<220>
<221> VARIANT
<222> 19
<223> Xaa = Lys or Arg

<400> 23
Xaa Leu Trp Arg Ala Leu Trp Arg Leu Trp Arg Xaa Leu Trp Arg Leu
1               5                   10                  15
Trp Arg Xaa Ala
            20


<210> 24
<211> 20
<212> PRT
<213> Artificial Sequence

<220>
<223> Synthetic construct VEPEP-6 10

<220>
<221> VARIANT
<222> 1

<223> Xaa = bAla or Ser

<220>
<221> VARIANT
<222> 10
<223> Xaa = Lys or Arg

<220>
<221> VARIANT
<222> 19
<223> Xaa = Leu or Ile

<400> 24
Xaa Leu Trp Arg Ala Leu Trp Arg Leu Xaa Arg Ala Leu Trp Arg Leu
 1               5                   10                  15
Leu Trp Xaa Ala
            20


<210> 25
<211> 20
<212> PRT
<213> Artificial Sequence

<220>
<223> Synthetic construct VEPEP-6 11

<220>
<221> VARIANT
<222> 1
<223> Xaa = bAla or Ser

<220>
<221> VARIANT
<222> 10
<223> Xaa = Lys or Arg

<220>
<221> VARIANT
<222> 19
<223> Xaa = Leu, Cys or Ile

<400> 25
Xaa Leu Trp Arg Ala Leu Trp Arg Leu Xaa Arg Asn Leu Trp Arg Leu
 1               5                   10                  15
Leu Trp Xaa Ala
            20


<210> 26
<211> 19
<212> PRT
<213> Artificial Sequence

<220>
<223> Synthetic construct VEPEP-6a

<220>
<221> ACETYLATION
<222> 1

<220>
<221> VARIANT

<222> 1
<223> Xaa = bAla or Ser

<220>
<221> VARIANT
<222> 19
<223> Xaa = Lys attached to Cysteamide

<400> 26
Xaa Leu Phe Arg Ala Leu Trp Arg Leu Leu Arg Ser Leu Trp Arg Leu
 1               5                   10                  15
Leu Trp Xaa



<210> 27
<211> 20
<212> PRT
<213> Artificial Sequence

<220>
<223> Synthetic construct VEPEP-6b

<220>
<221> ACETYLATION
<222> 1

<220>
<221> VARIANT
<222> 1
<223> Xaa = bAla or Ser

<220>
<221> VARIANT
<222> 20
<223> Xaa = Ala attached to Cysteamide

<400> 27
Xaa Leu Trp Arg Ala Leu Trp Arg Leu Trp Arg Ser Leu Trp Arg Leu
 1               5                   10                  15
Leu Trp Lys Xaa
            20


<210> 28
<211> 20
<212> PRT
<213> Artificial Sequence

<220>
<223> Synthetic construct VEPEP-6c

<220>
<221> ACETYLATION
<222> 1

<220>
<221> VARIANT
<222> 1
<223> Xaa = bAla or Ser

<220>
<221> VARIANT

<222> 20
<223> Xaa = Ala attached to Cysteamide

<400> 28
Xaa Leu Trp Arg Ala Leu Trp Arg Leu Leu Arg Ser Leu Trp Arg Leu
1               5                   10                  15
Trp Arg Lys Xaa
            20


<210> 29
<211> 20
<212> PRT
<213> Artificial Sequence

<220>
<223> Synthetic construct VEPEP-6d

<220>
<221> ACETYLATION
<222> 1

<220>
<221> VARIANT
<222> 1
<223> Xaa = bAla or Ser

<220>
<221> VARIANT
<222> 20
<223> Xaa = Ala attached to Cysteamide

<400> 29
Xaa Leu Trp Arg Ala Leu Trp Arg Leu Trp Arg Ser Leu Trp Arg Leu
1               5                   10                  15
Trp Arg Lys Xaa
            20


<210> 30
<211> 20
<212> PRT
<213> Artificial Sequence

<220>
<223> Synthetic construct VEPEP-6e

<220>
<221> ACETYLATION
<222> 1

<220>
<221> VARIANT
<222> 1
<223> Xaa = bAla or Ser

<220>
<221> VARIANT
<222> 20
<223> Xaa = Ala attached to Cysteamide

<400> 30
Xaa Leu Trp Arg Ala Leu Trp Arg Leu Leu Arg Ala Leu Trp Arg Leu

1                   5                   10                  15
Leu Trp Lys Xaa
            20



<210> 31
<211> 20
<212> PRT
<213> Artificial Sequence

<220>
<223> Synthetic construct VEPEP-6f

<220>
<221> ACETYLATION
<222> 1

<220>
<221> VARIANT
<222> 1
<223> Xaa = bAla or Ser

<220>
<221> VARIANT
<222> 20
<223> Xaa = Ala attached to Cysteamide

<400> 31
Xaa Leu Trp Arg Ala Leu Trp Arg Leu Leu Arg Asn Leu Trp Arg Leu
1                   5                   10                  15
Leu Trp Lys Xaa
            20



<210> 32
<211> 19
<212> PRT
<213> Artificial Sequence

<220>
<223> Synthetic construct ST-VEPEP-6a

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 8,12
<223> Hydrocarbon linkage

<400> 32
Xaa Leu Phe Arg Ala Leu Trp Arg Leu Leu Arg Ser Leu Trp Arg Leu

 1                 5                   10                  15
Leu Trp Xaa

<210> 33
<211> 19
<212> PRT
<213> Artificial Sequence

<220>
<223> Synthetic construct ST-VEPEP-6aa

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 8,12
<223> Hydrocarbon linkage

<400> 33
Xaa Leu Phe Leu Ala Arg Trp Arg Leu Leu Arg Ser Leu Trp Arg Leu
 1                 5                   10                  15
Leu Trp Xaa

<210> 34
<211> 19
<212> PRT
<213> Artificial Sequence

<220>
<223> Synthetic construct ST-VEPEP-6ab

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature

<222> 8,12
<223> Hydrocarbon linkage

<400> 34
Xaa Leu Phe Arg Ala Leu Trp Ser Leu Leu Arg Ser Leu Trp Arg Leu
1               5                   10                  15
Leu Trp Xaa

<210> 35
<211> 19
<212> PRT
<213> Artificial Sequence

<220>
<223> Synthetic construct ST-VEPEP-6ad

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 8,12
<223> Hydrocarbon linkage

<400> 35
Xaa Leu Phe Leu Ala Arg Trp Ser Leu Leu Arg Ser Leu Trp Arg Leu
1               5                   10                  15
Leu Trp Xaa

<210> 36
<211> 19
<212> PRT
<213> Artificial Sequence

<220>
<223> Synthetic construct ST-VEPEP-6b

<220>
<221> ACETYLATION
<222> 1

<220>
<221> VARIANT
<222> 1
<223> Xaa = bAla or Ser

<220>
<221> VARIANT

<222> 19
<223> Xaa = Lys attached to Cysteamide

<220>
<221> Misc_feature
<222> 11,15
<223> Hydrocarbon linkage

<400> 36
Xaa Leu Phe Arg Ala Leu Trp Arg Leu Leu Arg Ser Leu Trp Ser Leu
 1               5                   10                  15
Leu Trp Xaa




<210> 37
<211> 19
<212> PRT
<213> Artificial Sequence

<220>
<223> Synthetic construct ST-VEPEP-6ba

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 11,15
<223> Hydrocarbon linkage

<400> 37
Xaa Leu Phe Leu Ala Arg Trp Arg Leu Leu Arg Ser Leu Trp Ser Leu
 1               5                   10                  15
Leu Trp Xaa




<210> 38
<211> 19
<212> PRT
<213> Artificial Sequence

<220>
<223> Synthetic construct ST-VEPEP-6bb

<220>
<221> ACETYLATION
<222> 1

<220>
<221> VARIANT

<222> 1
<223> Xaa = bAla or Ser

<220>
<221> VARIANT
<222> 19
<223> Xaa = Lys attached to Cysteamide

<220>
<221> Misc_feature
<222> 11,15
<223> Hydrocarbon linkage

<400> 38
Xaa Leu Phe Arg Ala Leu Trp Arg Leu Leu Ser Ser Leu Trp Ser Leu
 1               5                   10                  15
Leu Trp Xaa

<210> 39
<211> 19
<212> PRT
<213> Artificial Sequence

<220>
<223> Synthetic construct ST-VEPEP-6bd

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 11,15
<223> Hydrocarbon linkage

<400> 39
Xaa Leu Phe Leu Ala Arg Trp Arg Leu Leu Ser Ser Leu Trp Ser Leu
 1               5                   10                  15
Leu Trp Xaa

<210> 40
<211> 19
<212> PRT
<213> Artificial Sequence

<220>
<223> Synthetic construct ST-VEPEP-6c

<220>
<221> ACETYLATION
<222> 1

<220>
<221> VARIANT
<222> 1
<223> Xaa = bAla or Ser

<220>
<221> VARIANT
<222> 19
<223> Xaa = Lys attached to Cysteamide

<220>
<221> Misc_feature
<222> 5,12
<223> Hydrocarbon linkage

<400> 40
Xaa Leu Phe Ala Arg Leu Trp Arg Leu Leu Arg Ser Leu Trp Arg Leu
 1               5                   10                  15
Leu Trp Xaa



<210> 41
<211> 20
<212> PRT
<213> Artificial Sequence

<220>
<223> Synthetic construct VEPEP-9 1

<220>
<221> VARIANT
<222> 1
<223> Xaa = bAla or Ser

<220>
<221> VARIANT
<222> 2
<223> Xaa = Leu or absent

<220>
<221> VARIANT
<222> 3, 11
<223> Xaa = Arg or absent

<220>
<221> VARIANT
<222> 6
<223> Xaa = Leu, Arg or Gly

<220>
<221> VARIANT
<222> 7
<223> Xaa = Arg, Trp or Ser

<220>
<221> VARIANT
<222> 10
<223> Xaa = Ser, Pro or Thr

<220>
<221> VARIANT
<222> 12
<223> Xaa = Trp or Pro

<220>
<221> VARIANT
<222> 13
<223> Xaa = Phe, Ala or Arg

<220>
<221> VARIANT
<222> 14
<223> Xaa =  Ser, Leu, Pro or Arg

<220>
<221> VARIANT
<222> 15
<223> Xaa =  Arg or Ser

<220>
<221> VARIANT
<222> 16
<223> Xaa =  Trp or absent

<220>
<221> VARIANT
<222> 17
<223> Xaa = Ala, Arg or absent

<220>
<221> VARIANT
<222> 19
<223> Xaa = Trp or Phe


<400> 41
Xaa Xaa Xaa Trp Trp Xaa Xaa Trp Ala Xaa Xaa Xaa Xaa Xaa Xaa Xaa
 1               5                   10                  15
Xaa Trp Xaa Arg
            20



<210> 42
<211> 20
<212> PRT
<213> Artificial Sequence

<220>
<223> Synthetic construct VEPEP-9 2

<220>
<221> VARIANT
<222> 1
<223> Xaa = bAla or Ser

<220>
<221> VARIANT
<222> 2
<223> Xaa = Leu or absent

<220>

<221> VARIANT
<222> 10
<223> Xaa = Ser or Pro

<220>
<221> VARIANT
<222> 13
<223> Xaa = Phe or Ala

<220>
<221> VARIANT
<222> 14
<223> Xaa = Ser, Leu or Pro

<220>
<221> VARIANT
<222> 15
<223> Xaa = Arg or Ser

<220>
<221> VARIANT
<222> 17
<223> Xaa = Ala or Arg

<220>
<221> VARIANT
<222> 19
<223> Xaa = Trp or Phe


<400> 42
Xaa Xaa Arg Trp Trp Leu Arg Trp Ala Xaa Arg Trp Xaa Xaa Xaa Trp
 1               5                   10                  15
Xaa Trp Xaa Arg
            20



<210> 43
<211> 20
<212> PRT
<213> Artificial Sequence

<220>
<223> Synthetic construct VEPEP9a1

<220>
<221> VARIANT
<222> 1
<223> Xaa = bAla or Ser

<400> 43
Xaa Leu Arg Trp Trp Leu Arg Trp Ala Ser Arg Trp Phe Ser Arg Trp
 1               5                   10                  15
Ala Trp Trp Arg
            20



<210> 44
<211> 20
<212> PRT
<213> Artificial Sequence

<220>

<223> Synthetic construct VEPEP9a2

<220>
<221> VARIANT
<222> 1
<223> Xaa = bAla or Ser

<400> 44
Xaa Leu Arg Trp Trp Leu Arg Trp Ala Ser Arg Trp Ala Ser Arg Trp
 1               5                   10                  15
Ala Trp Phe Arg
            20



<210> 45
<211> 19
<212> PRT
<213> Artificial Sequence

<220>
<223> Synthetic construct VEPEP9b1

<220>
<221> VARIANT
<222> 1
<223> Xaa = bAla or Ser

<400> 45
Xaa Arg Trp Trp Leu Arg Trp Ala Ser Arg Trp Ala Leu Ser Trp Arg
 1               5                   10                  15
Trp Trp Arg



<210> 46
<211> 19
<212> PRT
<213> Artificial Sequence

<220>
<223> Synthetic construct VEPEP9b2



<220>
<221> VARIANT
<222> 1
<223> Xaa = bAla or Ser

<400> 46
Xaa Arg Trp Trp Leu Arg Trp Ala Ser Arg Trp Phe Leu Ser Trp Arg
 1               5                   10                  15
Trp Trp Arg



<210> 47
<211> 19
<212> PRT
<213> Artificial Sequence

<220>
<223> Synthetic construct VEPEP9c1

<220>
<221> VARIANT
<222> 1
<223> Xaa = bAla or Ser

<400> 47
Xaa Arg Trp Trp Leu Arg Trp Ala Pro Arg Trp Phe Pro Ser Trp Arg
 1               5                   10                  15
Trp Trp Arg

<210> 48
<211> 19
<212> PRT
<213> Artificial Sequence

<220>
<223> Synthetic construct VEPEP9c2

<220>
<221> VARIANT
<222> 1
<223> Xaa = bAla or Ser

<400> 48
Xaa Arg Trp Trp Leu Arg Trp Ala Ser Arg Trp Ala Pro Ser Trp Arg
 1               5                   10                  15
Trp Trp Arg

<210> 49
<211> 15
<212> PRT
<213> Artificial Sequence

<220>
<223> Synthetic construct VEPEP-9 3

<220>
<221> VARIANT
<222> 1
<223> Xaa = bAla or Ser

<220>
<221> VARIANT
<222> 4
<223> Xaa = Arg or Gly

<220>
<221> VARIANT
<222> 5
<223> Xaa = Trp or Ser

<220>
<221> VARIANT
<222> 8
<223> Xaa = Ser, Thr or Pro

<220>
<221> VARIANT
<222> 9

<223> Xaa = Trp or Pro

<220>
<221> VARIANT
<222> 10
<223> Xaa =  Ala or Arg

<220>
<221> VARIANT
<222> 12
<223> Xaa = Ser or Arg


<400> 49
Xaa Trp Trp Xaa Xaa Trp Ala Xaa Xaa Xaa Arg Xaa Trp Trp Arg
 1               5                   10                  15


<210> 50
<211> 15
<212> PRT
<213> Artificial Sequence

<220>
<223> Synthetic construct VEPEP9d

<220>
<221> VARIANT
<222> 1
<223> Xaa = bAla or Ser

<400> 50
Xaa Trp Trp Arg Trp Trp Ala Ser Trp Ala Arg Ser Trp Trp Arg
 1               5                   10                  15


<210> 51
<211> 15
<212> PRT
<213> Artificial Sequence

<220>
<223> Synthetic construct VEPEP9e

<220>
<221> VARIANT
<222> 1
<223> Xaa = bAla or Ser

<400> 51
Xaa Trp Trp Gly Ser Trp Ala Thr Pro Arg Arg Arg Trp Trp Arg
 1               5                   10                  15


<210> 52
<211> 15
<212> PRT
<213> Artificial Sequence

<220>
<223> Synthetic construct VEPEP9f

<220>

<221> VARIANT
<222> 1
<223> Xaa = bAla or Ser

<400> 52
Xaa Trp Trp Arg Trp Trp Ala Pro Trp Ala Arg Ser Trp Trp Arg
1               5                   10                  15


<210> 53
<211> 20
<212> PRT
<213> Artificial Sequence

<220>
<223> Synthetic construct ADGN-100

<220>
<221> VARIANT
<222> 1
<223> Xaa = Any Amino Acid or absent

<220>
<221> VARIANT
<222> 6, 7, 8, 15, 16, 17, 18
<223> Xaa = Any Amino Acid

<400> 53
Xaa Lys Trp Arg Ser Xaa Xaa Xaa Arg Trp Arg Leu Trp Arg Xaa Xaa
1               5                   10                  15
Xaa Xaa Ser Arg
            20


<210> 54
<211> 20
<212> PRT
<213> Artificial Sequence

<220>
<223> Synthetic construct ADGN-100 1

<220>
<221> VARIANT
<222> 1
<223> Xaa = bAla, Ser or absent

<220>
<221> VARIANT
<222> 6
<223> Xaa = Ala or Val

<220>
<221> VARIANT
<222> 7
<223> Xaa = Gly or Leu

<220>
<221> VARIANT
<222> 8
<223> Xaa = Trp or Tyr

<220>

<221> VARIANT
<222> 15
<223> Xaa = Val or Ser

<220>
<221> VARIANT
<222> 16
<223> Xaa = Arg, Val, or Ala

<220>
<221> VARIANT
<222> 17
<223> Xaa = Ser or Leu

<220>
<221> VARIANT
<222> 18
<223> Xaa = Trp or Tyr


<400> 54
Xaa Lys Trp Arg Ser Xaa Xaa Xaa Arg Trp Arg Leu Trp Arg Xaa Xaa
 1               5                   10                  15
Xaa Xaa Ser Arg
            20



<210> 55
<211> 19
<212> PRT
<213> Artificial Sequence

<220>
<223> Synthetic construct ADGN-100a

<400> 55
Lys Trp Arg Ser Ala Gly Trp Arg Trp Arg Leu Trp Arg Val Arg Ser
 1               5                   10                  15
Trp Ser Arg




<210> 56
<211> 19
<212> PRT
<213> Artificial Sequence

<220>
<223> Synthetic construct ADGN-100b

<400> 56
Lys Trp Arg Ser Ala Leu Tyr Arg Trp Arg Leu Trp Arg Val Arg Ser
 1               5                   10                  15
Trp Ser Arg




<210> 57
<211> 19
<212> PRT
<213> Artificial Sequence

<220>

<223> Synthetic construct ADGN-100c

<400> 57
Lys Trp Arg Ser Ala Leu Tyr Arg Trp Arg Leu Trp Arg Ser Arg Ser
1               5                   10                  15
Trp Ser Arg

<210> 58
<211> 19
<212> PRT
<213> Artificial Sequence

<220>
<223> Synthetic construct ADGN-100d

<400> 58
Lys Trp Arg Ser Ala Leu Tyr Arg Trp Arg Leu Trp Arg Ser Ala Leu
1               5                   10                  15
Tyr Ser Arg

<210> 59
<211> 19
<212> PRT
<213> Artificial Sequence

<220>
<223> Synthetic construct ADGN-100 aa

<220>
<221> Misc_feature
<222> 4,8
<223> Hydrocarbon linkage

<400> 59
Lys Trp Arg Ser Ala Gly Trp Arg Trp Arg Leu Trp Arg Val Arg Ser
1               5                   10                  15
Trp Ser Arg

<210> 60
<211> 19
<212> PRT
<213> Artificial Sequence

<220>
<223> Synthetic construct ADGN-100 ab

<220>
<221> Misc_feature
<222> 3,10
<223> Hydrocarbon linkage

<400> 60
Lys Trp Arg Ser Ala Gly Trp Arg Trp Arg Leu Trp Arg Val Arg Ser
1               5                   10                  15
Trp Ser Arg

<210> 61
<211> 19
<212> PRT
<213> Artificial Sequence

<220>
<223> Synthetic construct ADGN-100 ac

<220>
<221> Misc_feature
<222> 8,15
<223> Hydrocarbon linkage

<400> 61
Lys Trp Arg Ser Ala Gly Trp Arg Trp Arg Leu Trp Arg Val Arg Ser
1               5                   10                  15
Trp Ser Arg

<210> 62
<211> 19
<212> PRT
<213> Artificial Sequence

<220>
<223> Synthetic construct ADGN-100 ba

<220>
<221> Misc_feature
<222> 4,8
<223> Hydrocarbon linkage

<400> 62
Lys Trp Arg Ser Ala Leu Tyr Arg Trp Arg Leu Trp Arg Ser Arg Ser
1               5                   10                  15
Trp Ser Arg

<210> 63
<211> 19
<212> PRT
<213> Artificial Sequence

<220>
<223> Synthetic construct ADGN-100 bb

<220>
<221> Misc_feature
<222> 3,10
<223> Hydrocarbon linkage

<400> 63
Lys Trp Arg Ser Ala Leu Tyr Arg Trp Arg Leu Trp Arg Ser Arg Ser
1               5                   10                  15
Trp Ser Arg

<210> 64
<211> 19

<212> PRT
<213> Artificial Sequence

<220>
<223> Synthetic construct ADGN-100 bc

<220>
<221> Misc_feature
<222> 8,15
<223> Hydrocarbon linkage

<400> 64
Lys Trp Arg Ser Ala Leu Tyr Arg Trp Arg Leu Trp Arg Ser Arg Ser
1               5                   10                  15
Trp Ser Arg

<210> 65
<211> 19
<212> PRT
<213> Artificial Sequence

<220>
<223> Synthetic construct ADGN-100 bd

<220>
<221> Misc_feature
<222> 10,14
<223> Hydrocarbon linkage

<400> 65
Lys Trp Arg Ser Ala Leu Tyr Arg Trp Arg Leu Trp Arg Ser Arg Ser
1               5                   10                  15
Trp Ser Arg

<210> 66
<211> 19
<212> PRT
<213> Artificial Sequence

<220>
<223> Synthetic construct ADGN-100 be

<220>
<221> Misc_feature
<222> 14,18
<223> Hydrocarbon linkage

<400> 66
Lys Trp Arg Ser Ala Leu Tyr Arg Trp Arg Leu Trp Arg Ser Arg Ser
1               5                   10                  15
Trp Ser Arg

<210> 67
<211> 19
<212> PRT
<213> Artificial Sequence

<220>
<223> Synthetic construct ADGN-100 ca

<220>
<221> Misc_feature
<222> 3,9
<223> Hydrocarbon linkage

<400> 67
Lys Trp Arg Ser Ala Leu Tyr Arg Trp Arg Leu Trp Arg Ser Ala Leu
1               5                   10                  15
Tyr Ser Arg

<210> 68
<211> 19
<212> PRT
<213> Artificial Sequence

<220>
<223> Synthetic construct ADGN-100 cb

<220>
<221> Misc_feature
<222> 4,8
<223> Hydrocarbon linkage

<400> 68
Lys Trp Arg Ser Ala Leu Tyr Arg Trp Arg Leu Trp Arg Ser Ala Leu
1               5                   10                  15
Tyr Ser Arg

<210> 69
<211> 19
<212> PRT
<213> Artificial Sequence

<220>
<223> Synthetic construct ADGN-100 cc

<220>
<221> Misc_feature
<222> 10,14
<223> Hydrocarbon linkage

<400> 69
Lys Trp Arg Ser Ala Leu Tyr Arg Trp Arg Leu Trp Arg Ser Ala Leu
1               5                   10                  15
Tyr Ser Arg

<210> 70
<211> 19
<212> PRT
<213> Artificial Sequence

<220>
<223> Synthetic construct ADGN-100 cd

<220>
<221> Misc_feature
<222> 14,18
<223> Hydrocarbon linkage

<400> 70
Lys Trp Arg Ser Ala Leu Tyr Arg Trp Arg Leu Trp Arg Ser Ala Leu
1               5                   10                  15
Tyr Ser Arg


<210> 71
<211> 20
<212> DNA
<213> Artificial Sequence

<220>
<223> EGFP target site

<400> 71
gggcacgggc agcttgccgg                                                   20

<210> 72
<211> 20
<212> DNA
<213> Homo sapiens

<220>
<223> EMX1 target site

<400> 72
gagtccgagc agaagaagaa                                                   20

<210> 73
<211> 19
<212> DNA
<213> Homo sapiens

<220>
<223> HPRT target site

<400> 73
gactgtaagt gaattactt                                                    19

<210> 74
<211> 20
<212> DNA
<213> Homo sapiens

<220>
<223> AAVS target site

<400> 74
ggggccacta gggacaggat                                                   20


<210> 75
<211> 16
<212> PRT
<213> Artificial Sequence

<220>
<223> Synthetic construct VEPEP-3a

<220>
<221> VARIANT
<222> 1
<223> Xaa = bAla

<400> 75
Xaa Lys Trp Phe Glu Arg Trp Phe Arg Glu Trp Pro Arg Lys Arg Arg
1               5                   10                  15


<210> 76
<211> 16
<212> PRT
<213> Artificial Sequence

<220>
<223> Synthetic construct VEPEP-3b

<220>
<221> VARIANT
<222> 1
<223> Xaa = bAla

<400> 76
Xaa Lys Trp Trp Glu Arg Trp Trp Arg Glu Trp Pro Arg Lys Arg Arg
1               5                   10                  15


<210> 77
<211> 20
<212> PRT
<213> Artificial Sequence

<220>
<223> Synthetic construct VEPEP-6

<220>
<221> VARIANT
<222> 1
<223> Xaa = bAla

<400> 77
Xaa Leu Trp Arg Ala Leu Trp Arg Leu Trp Arg Ser Leu Trp Arg Leu
1               5                   10                  15
Leu Trp Lys Ala
            20


<210> 78
<211> 20
<212> PRT
<213> Artificial Sequence

<220>
<223> Synthetic construct VEPEP-9

<220>
<221> VARIANT
<222> 1
<223> Xaa = bAla

<400> 78

Xaa Leu Arg Trp Trp Leu Arg Trp Ala Ser Arg Trp Phe Ser Arg Trp
1               5                   10                  15
Ala Trp Trp Arg
            20

<210> 79
<211> 20
<212> PRT
<213> Artificial Sequence

<220>
<223> Synthetic construct ADGN-100a

<220>
<221> VARIANT
<222> 1
<223> Xaa = bAla

<400> 79
Xaa Lys Trp Arg Ser Ala Gly Trp Arg Trp Arg Leu Trp Arg Val Arg
1               5                   10                  15
Ser Trp Ser Arg
            20

<210> 80
<211> 20
<212> PRT
<213> Artificial Sequence

<220>
<223> Synthetic construct ADGN-100b

<220>
<221> VARIANT
<222> 1
<223> Xaa = bAla

<400> 80
Xaa Lys Trp Arg Ser Ala Leu Tyr Arg Trp Arg Leu Trp Arg Val Arg
1               5                   10                  15
Ser Trp Ser Arg
            20

<210> 81
<211> 20
<212> PRT
<213> Artificial Sequence

<220>
<223> Synthetic construct CADY

<400> 81
Gly Leu Trp Arg Ala Leu Trp Arg Leu Leu Arg Ser Leu Trp Arg Leu
1               5                   10                  15
Leu Trp Lys Val
            20

<210> 82
<211> 72
<212> DNA

<213> Artificial Sequence

<220>
<223> Synthetic construct ssDonor template

<400> 82
ggggaggaca tcgatgtcac ctccaatgac aagcttgcta gcggtgggca accacaaacc 60
cacgagggca ga 72

<210> 83
<211> 7
<212> PRT
<213> Unknown

<220>
<223> SV40 Virus NLS

<400> 83
Pro Lys Lys Lys Arg Lys Val
1               5

<210> 84
<211> 16
<212> PRT
<213> Unknown

<220>
<223> nucleoplasmin NLS

<400> 84
Lys Arg Pro Ala Ala Thr Lys Lys Ala Gly Gln Ala Lys Lys Lys Lys
1               5                   10                  15

<210> 85
<211> 9
<212> PRT
<213> Homo sapiens

<220>
<223> c-myc NLS 1

<400> 85
Pro Ala Ala Lys Arg Val Lys Leu Asp
1               5

<210> 86
<211> 11
<212> PRT
<213> Homo sapiens

<220>
<223> c-myc NLS 2

<400> 86
Arg Gln Arg Arg Asn Glu Leu Lys Arg Ser Pro
1               5                   10

<210> 87
<211> 38
<212> PRT
<213> Homo sapiens

<220>
<223> hRNPA1 M9 NLS

<400> 87
Asn Gln Ser Ser Asn Phe Gly Pro Met Lys Gly Gly Asn Phe Gly Gly
1               5                   10                  15
Arg Ser Ser Gly Pro Tyr Gly Gly Gly Gly Gln Tyr Phe Ala Lys Pro
            20                  25                  30
Arg Asn Gln Gly Gly Tyr
        35


<210> 88
<211> 42
<212> PRT
<213> Homo sapiens

<220>
<223> IBB domain from importin-alpha

<400> 88
Arg Met Arg Ile Glx Phe Lys Asn Lys Gly Lys Asp Thr Ala Glu Leu
1               5                   10                  15
Arg Arg Arg Arg Val Glu Val Ser Val Glu Leu Arg Lys Ala Lys Lys
            20                  25                  30
Asp Glu Gln Ile Leu Lys Arg Arg Asn Val
        35                  40


<210> 89
<211> 8
<212> PRT
<213> Unknown

<220>
<223> Virus myoma T protein NLS 1

<400> 89
Val Ser Arg Lys Arg Pro Arg Pro
1               5


<210> 90
<211> 8
<212> PRT
<213> Unknown

<220>
<223> Virus myoma T protein NLS 2

<400> 90
Pro Pro Lys Lys Ala Arg Glu Asp
1               5


<210> 91
<211> 8

<212> PRT
<213> Homo sapiens

<220>
<223> p53 NLS

<400> 91
Pro Gln Pro Lys Lys Lys Pro Leu
1               5

<210> 92
<211> 12
<212> PRT
<213> Mus musculus

<220>
<223> mouse c-ab1 IV NLS

<400> 92
Ser Ala Leu Ile Lys Lys Lys Lys Lys Met Ala Pro
1               5                   10

<210> 93
<211> 5
<212> PRT
<213> Unknown

<220>
<223> influenza virus NS1 NLS 1

<400> 93
Asp Arg Leu Arg Arg
1               5

<210> 94
<211> 7
<212> PRT
<213> Unknown

<220>
<223> influenza virus NS1 NLS 2

<400> 94
Pro Lys Gln Lys Lys Arg Lys
1               5

<210> 95
<211> 10
<212> PRT
<213> Unknown

<220>
<223> Hepatitis virus delta antigen NLS

<400> 95
Arg Lys Leu Lys Lys Lys Ile Lys Lys Leu
1               5                   10

<210> 96
<211> 10
<212> PRT
<213> Mus musculus

<220>
<223> mouse Mx1 NLS

<400> 96
Arg Glu Lys Lys Lys Phe Leu Lys Arg Arg
1               5                   10


<210> 97
<211> 20
<212> PRT
<213> Homo sapiens

<220>
<223> human poly(ADP-ribose) polymerase NLS

<400> 97
Lys Arg Lys Gly Asp Glu Val Asp Gly Val Asp Glu Val Ala Lys Lys
1               5                   10                  15
Lys Ser Lys Lys
            20


<210> 98
<211> 17
<212> PRT
<213> Homo sapiens

<220>
<223> steroid hormone receptors (human) glucocorticoid NLS

<400> 98
Arg Lys Cys Leu Gln Ala Gly Met Asn Leu Glu Ala Arg Lys Thr Lys
1               5                   10                  15
Lys


<210> 99
<211> 23
<212> DNA
<213> Homo sapiens

<220>
<223> hPD1sgA1 target site

<400> 99
accgcgtgac ttccacatga gcg                                              23

<210> 100
<211> 23
<212> DNA
<213> Homo sapiens

<220>
<223> hPD1sgB1 target site

<400> 100

aaaccgctca tgtggaagtc acg                                                23

<210> 101
<211> 23
<212> DNA
<213> Homo sapiens

<220>
<223> hPD1sgC1f target site

<400> 101
accgcagttg tgtgacacgg aag                                                23

<210> 102
<211> 23
<212> DNA
<213> Homo sapiens

<220>
<223> hPD1sgD2 target site

<400> 102
aaaccttccg tgtcacacaa ctg                                                23

<210> 103
<211> 20
<212> DNA
<213> Homo sapiens

<220>
<223> Luciferase target site

<400> 103
acaactttac cgaccgcgcc                                                    20

<210> 104
<211> 20
<212> DNA
<213> Homo sapiens

<220>
<223> LDLR target site 1

<400> 104
ggcactcgta gccgatctta                                                    20

<210> 105
<211> 20
<212> DNA
<213> Homo sapiens

<220>
<223> LDLR target site 2

<400> 105
gtggcccagc gaagatgcga                                                    20

<210> 106
<211> 20
<212> DNA
<213> Homo sapiens

<220>

<223> LDLR target site 3

<400> 106
tccagatatc gatgagtgtc                                                    20

<210> 107
<211> 20
<212> DNA
<213> Homo sapiens

<220>
<223> LDLR target site 4

<400> 107
agactcaccg cactctttga                                                    20

<210> 108
<211> 20
<212> DNA
<213> Homo sapiens

<220>
<223> LDLR target site 5

<400> 108
ccagctggac ccccacacga                                                    20

<210> 109
<211> 20
<212> DNA
<213> Homo sapiens

<220>
<223> LDLR target site 6

<400> 109
ccatgttgca gactttgtcc                                                    20

<210> 110
<211> 20
<212> DNA
<213> Homo sapiens

<220>
<223> LDLR SAM target site 1

<400> 110
aaacatcgag aaatttcagg                                                    20

<210> 111
<211> 20
<212> DNA
<213> Homo sapiens

<220>
<223> LDLR SAM target site 2

<400> 111
acaaatcaag tcgcctgccc                                                    20

<210> 112
<211> 20
<212> DNA

<213> Homo sapiens

<220>
<223> LDLR SAM target site 3

<400> 112
   agggggcgtc agctcttcac                                                    20

<210> 113
<211> 20
<212> DNA
<213> Homo sapiens

<220>
<223> ApoB target site 1

<400> 113
cgacccgatt caagcacctc                                                   20

<210> 114
<211> 20
<212> DNA
<213> Homo sapiens

<220>
<223> ApoB target site 2

<400> 114
ctgattcaag aagtgccacc                                                   20

<210> 115
<211> 20
<212> DNA
<213> Homo sapiens

<220>
<223> ApoB target site 3

<400> 115
agctggcgat ggacccgccg                                                   20

<210> 116
<211> 20
<212> DNA
<213> Homo sapiens

<220>
<223> ApoB target site 4

<400> 116
atgccttact tggacagacc                                                   20

<210> 117
<211> 20
<212> DNA
<213> Homo sapiens

<220>
<223> ApoB target site 5

<400> 117
cgccagcagc gccagcagcg                                                   20

<210> 118
<211> 20
<212> DNA
<213> Homo sapiens

<220>
<223> ApoB target site 6

<400> 118
tcttacagaa gaggaaatgc                                                   20

<210> 119
<211> 20
<212> DNA
<213> Homo sapiens

<220>
<223> ApoB SAM target site 1

<400> 119
ctttgagcct tgaagagcct                                                   20

<210> 120
<211> 20
<212> DNA
<213> Homo sapiens

<220>
<223> ApoB SAM target site 2

<400> 120
gcaaacaggt caggcccggg                                                   20

<210> 121
<211> 20
<212> DNA
<213> Homo sapiens

<220>
<223> ApoB SAM target site 3

<400> 121
gctgcggctg catcccaggt                                                   20

<210> 122
<211> 20
<212> DNA
<213> Homo sapiens

<220>
<223> LDLRAP1 target site 1

<400> 122
cgccgacttg agcgcgtcca                                                   20

<210> 123
<211> 20
<212> DNA
<213> Homo sapiens

<220>
<223> LDLRAP1 target site 2

<400> 123
tgtagccacg atcctcttga                                                    20

<210> 124
<211> 20
<212> DNA
<213> Homo sapiens

<220>
<223> LDLRAP1 target site 3

<400> 124
gctccactag cgtcatgccc                                                    20

<210> 125
<211> 20
<212> DNA
<213> Homo sapiens

<220>
<223> LDLRAP1 target site 4

<400> 125
tctgtcagga taattccccg                                                    20

<210> 126
<211> 20
<212> DNA
<213> Homo sapiens

<220>
<223> LDLRAP1 target site 5

<400> 126
ggccatggac gcgctcaagt                                                    20

<210> 127
<211> 20
<212> DNA
<213> Homo sapiens

<220>
<223> LDLRAP1 target site 6

<400> 127
tggacacgtt ctcaatgagc                                                    20

<210> 128
<211> 20
<212> DNA
<213> Homo sapiens

<220>
<223> LDLRAP1 SAM target site 1

<400> 128
aaactcttgg tgcctggacc                                                    20

<210> 129
<211> 20
<212> DNA
<213> Homo sapiens

<220>
<223> LDLRAP1 SAM target site 2

<400> 129
ctggcgcctt cgggcggcct                                                    20

<210> 130
<211> 20
<212> DNA
<213> Homo sapiens

<220>
<223> LDLRAP1 SAM target site 3

<400> 130
gaggaaagcg gggcgtcta                                                     20

<210> 131
<211> 20
<212> DNA
<213> Homo sapiens

<220>
<223> PCSK9 target site 1

<400> 131
ggtgctagcc ttgcgttccg                                                    20

<210> 132
<211> 20
<212> DNA
<213> Homo sapiens

<220>
<223> PCSK9 target site 2

<400> 132
ctggccgaag cacccgagca                                                    20

<210> 133
<211> 20
<212> DNA
<213> Homo sapiens

<220>
<223> PCSK9 target site 3

<400> 133
actgtatggt cagcacactc                                                    20

<210> 134
<211> 20
<212> DNA
<213> Homo sapiens

<220>
<223> PCSK9 target site 4

<400> 134
gtcgtgctgg tcaccgctgc                                                    20

<210> 135
<211> 20

<212> DNA
<213> Homo sapiens

<220>
<223> PCSK9 target site 5

<400> 135
cgcccgctgc gccccagatg                                                    20

<210> 136
<211> 20
<212> DNA
<213> Homo sapiens

<220>
<223> PCSK9 target site 6

<400> 136
agtagaggca ggcatcgtcc                                                    20